# FASCICULE DE BREVET EUROPEEN

(11) **EP 1 187 847 B1**
(45) Date de publication et mention de la délivrance du brevet: **03.12.2003**
(21) Numéro de dépôt: 00949570.6
(22) Date de dépôt: 29.06.2000
(51) Int. Cl.: C07K 5/078, A61K 38/05, A61P 31/04

(54) **DERIVES DE STREPTOGRAMINES, LEUR PREPARATION ET LES COMPOSITIONS QUI LES CONTIENNENT**
STREPTOGRAMINDERIVATE, IHRE HERSTELLUNG UND SIE ENTHALTENDE ZUBEREITUNGEN
STREPTOGRAMIN DERIVATIVES, PREPARATION AND COMPOSITIONS CONTAINING THEM

(30) Priorité: 30.06.1999 FR 9908375
(43) Date de publication de la demande: 20.03.2002
(73) Titulaire: Aventis Pharma S.A., 92160 Antony (FR)
(72) Inventeur: ACHARD, Daniel, F-94320 Thiais (FR); BACQUE, Eric, 91190 GIF SUR YVETTE (FR); BARRIERE, Jean-Claude, F-91400 Bures Sur Yvette (FR); BOUQUEREL, Jean, F-93700 Drancy (FR); DESMAZEAU, Pascal, F-91250 Tigery (FR); GRISONI, Serge, F-94600 Choisy le Roi (FR); LECONTE, Jean-Pierre, F-91800 Brunoy (FR); RIBEILL, Yves, Raleigh, NC 27615 (US); RONAN, Baptiste, F-92140 Clamart (FR)
(86) Numéro de dépôt international: FR0001818
(87) Numéro de publication internationale: WO01002427

(56) Documents cités:
- WO-A-89/03843
- WO-A-99/05165

## Description

La présente invention concerne des dérivés du groupe A des streptogramines de formule générale : 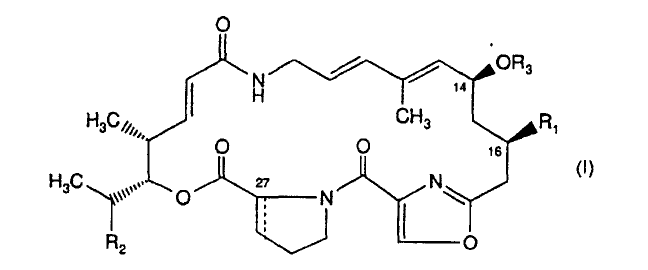 ainsi que leurs sels, qui présentent une activité antibactérienne particulièrement intéressante.

Parmi les streptogramines connues, la pristinamycine (RP 7293), antibactérien d'origine naturelle produit par *Streptomyces pristinaespiralis* a été isolée pour la première fois en 1955. La pristinamycine commercialisée sous le nom de Pyostacine® est constituée principalement de pristinamycine IIA associée à la pristinamycine IA.

Un autre antibactérien de la classe des streptogramines : la virginiamycine, a été isolé à partir de *Streptomyces virginiae*, ATCC 13161 [Antibiotics and Chemotherapy, 5, 632 (1955)]. La virginiamycine (Staphylomycine®) est constituée principalement de facteur M₁ (VM1) associé au facteur S (VS).

Des dérivés hémisynthétiques de streptogramines de structure : 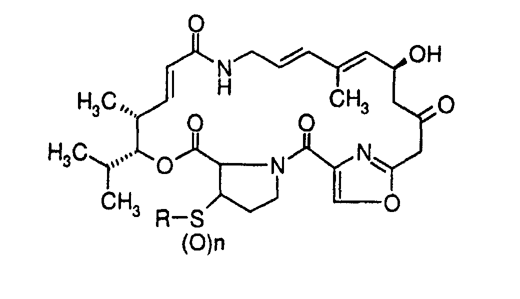 pour lesquels n est 0 à 2 ont été décrits dans les brevets EP 135410 et EP 191662. Associés à une composante hemisynthétique du groupe B des streptogramines, ils manifestent une synergie d'action et sont utilisables par voie injectable.

Dans la demande de brevet international WO 99/05165 ont été décrits des dérivés du groupe A des streptogramines de formule générale : 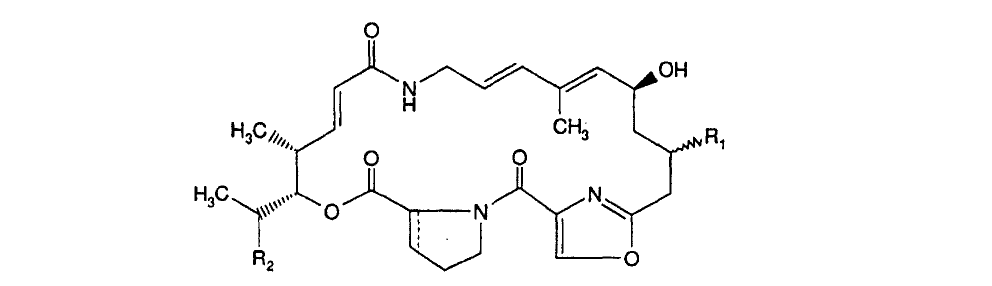 dans laquelle R₁ est un radical -NR'R" ou -NR'OR"', R₂ est hydrogène, méthyle ou éthyle, et la liaison --- est une liaison simple ou une liaison double, qui sont des agents antimicrobiens.
Cependant ces dérivés n'atteignent pas des niveaux d'activité spécialement élevés et par ailleurs n'ont pas toujours un spectre aussi large que souhaité.

Il a été maintenant trouvé, que les dérivés de streptogramine du groupe A de formule générale (I) dans laquelle :
- R₁ représente un atome d'halogène ou un radical azido ou thiocyanato,
- R₂ représente un atome d'hydrogène ou un radical méthyle ou éthyle,
- R₃ représente un atome d'hydrogène, ou le reste d'un ester aliphatique, cycloaliphatique, aromatique, araliphatique, hétérocyclique ou hétérocyclylaliphatique pouvant être substitué, et
- la liaison --- représente une liaison simple (stéréochimie 27R) ou une liaison double,
et leurs sels lorsqu'ils existent, présentent une activité antibactérienne particulièrement puissante, seuls ou associés à un dérivé de streptogramine du groupe B, et/ou manifestent également un spectre élargi par comparaison au spectre habituel des streptogramines.

Selon l'invention, lorsque R₁ représente un atome d'halogène, il peut être choisi parmi le fluor, le chlore, le brome ou l'iode ;
lorsque le radical R₃ représente le reste d'un ester aliphatique, cycloaliphatique, aromatique, araliphatique, hétérocyclique ou hétérocyclylaliphatique pouvant être substitué, ce dernier peut être choisi à titre d'exemple parmi des radicaux R'₃-CO-pour lesquels R'₃ est phényle ou phénylalcoyle non substitués ou substitués sur le radical phényle [par un ou plusieurs radicaux choisis parmi alcoyle, portant éventuellement un radical NR'R" dont les radicaux R' et R" identiques ou différents peuvent être des atomes d'hydrogène ou des radicaux alcoyle pouvant former ensemble avec l'atome d'azote auquel ils sont attachés un radical hétérocyclyle saturé ou insaturé de 3 à 8 chaînons, comprenant éventuellement un autre hétéroatome choisi parmi l'oxygène, le soufre ou l'azote, ledit hétérocycle pouvant être lui même substitué par un ou plusieurs radicaux (alcoyle, hydroxyalcoyle, alcoyloxyalcoyle, alcoyloxycarbonylalcoyle, aryle, hétérocyclyle, hétérocyclylalcoyle saturés ou insaturés de 3 à 8 chaînons ou -CH₂-CO-NR'R"), ou bien R' et/ou R" peuvent être un radical hydroxyalcoyle, phényle, hétérocyclylalcoyle saturé ou insaturé de 3 à 8 chaînons, -CO-NR'R" pour lequel NR'R" est défini comme précédemment, ou alcoyle ou acyle substitués par NR'R" défini tel que ci-dessus], ou bien R'₃ peut être choisi parmi des radicaux phényle ou phénylalcoyle substitués sur le radical phényle par un ou plusieurs radicaux [choisis parmi alcoyle, pouvant être substitués par un radical alcoyloxy ou alcoylthio éventuellement portant eux-même un radical carboxy ou un radical NR'R" tel que défini ci-dessus, ou choisis parmi acyloxy pouvant être substitué par NR'R" défini tel que précédemment], ou bien R'₃ peut être choisi parmi des radicaux alcoyle ou cycloalcoyle éventuellement substitués [par un radical carboxy, carboxyalcoyldisulfanyle ou par un radical NR'R", -CH₂-NR'R", -CO-NR'R", ou par un radical alcoyloxycarbonyle, alcoyloxy, ou alcoyldisulfanyle éventuellement substitués par NR'R" ou -CO-NR'R" pour lesquels NR'R" est défini tel que précédemment], ou bien R'₃ peut être choisi parmi des radicaux hétérocyclyle saturés ou insaturés de 3 à 8 chaînons éventuellement substitués [par alcoyle ou acyle eux-même éventuellement substitués par NR'R"].

Dans la formule générale (I), sauf mention spéciale, les radicaux ou portions alcoyle ou acyle sont droits ou ramifiés et contiennent 1 à 12 atomes de carbone, les radicaux hétérocyclyle peuvent être choisis notamment parmi pyrrolidinyle, pyrrolyle, furyle, thiényle, imidazolyle, pyridyle, pipéridinyle, pipérazinyle, morpholinyle, thiomorpholinyle, pyrizanyle, pyrimidinyle, pyridazinyle, imidazolidinyle, les radicaux aryle peuvent être notamment choisis parmi phényle éventuellement substitué et plus particulièrement phényle substitué par alcoyle, alcoyloxy, halogène, ou par un radical -CH₂OH, -(CH₂)n-NH₂, -(CH₂)ₙ-NHalcoyle ou -(CH₂)ₙ-N(alcoyle)₂.

Les dérivés de streptogramine de formule générale (I) peuvent être préparés par halogénation, par transformation en azide ou par transformation en thiocyanate, d'un dérivé de streptogramine de formule générale : 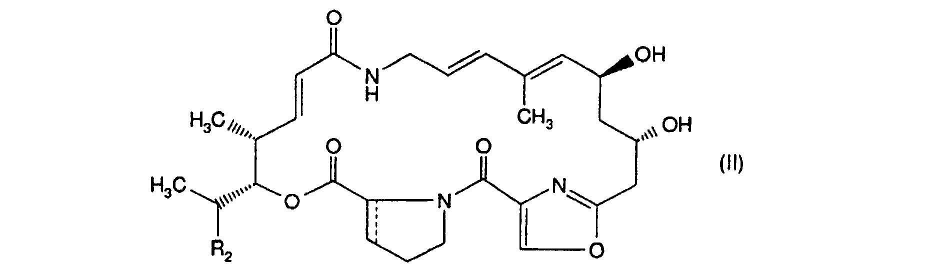 dans laquelle R₂ est défini comme précédemment, la liaison --- représente une liaison simple (stéréochimie 27R) ou une liaison double, et dont la fonction hydroxy en position 14 a été préalablement protégée, suivie de l'élimination du radical protecteur et le cas échéant, pour obtenir un dérivé de formule générale (I) pour lequel R₃ est autre que l'atome d'hydrogène, introduction du reste d'ester aliphatique, cycloaliphatique, aromatique, araliphatique, hétérocyclique ou hétérocyclylaliphatique pouvant être substitué (R₃) selon les méthodes habituelles qui n'altèrent pas le reste de la molécule.

Les réactions d'halogénation, de transformation en azide ou de transformation en thiocyanate peuvent être mises en oeuvre en présence d'un trifluorure d'aminosoufre (trifluorure de diéthylamino soufre, trifluorure de bis(2-méthoxyéthyl)amino soufre (Deoxofluor®), trifluorure de morpholino soufre par exemple) ou alternativement en présence de tétrafluorure de soufre, au moyen d'un réactif comme un halogénure, un azoture ou un thiocyanate de tétra alkylammonium, de tri alkyl benzylammonium ou de tri alkyl phénylammonium ou au moyen d'un halogénure, d'un azoture ou d'un thiocyanate de métal alcalin additionné éventuellement d'un éther couronne. Les réactions de fluoration peuvent être également mises en oeuvre par action d'un agent de fluoration comme un fluorure de soufre [par exemple trifluorure de morpholino soufre, tétrafluorure de soufre (J. Org. Chem., 40, 3808 (1975)), trifluorure de diéthylamino soufre (Tetrahedron, 44, 2875 (1988)), trifluorure de bis(2-méthoxyéthyl)amino soufre (Deoxofluor®). Alternativement les réactions de fluoration peuvent aussi s'effectuer au moyen d'un agent de fluoration comme l'hexafluoropropyl diéthylamine (JP 2 039 546) ou la N-(chloro-2 trifluoro-1,1,2 éthyl) diéthylamine.

Lorsque l'on met en oeuvre un halogénure, un azoture ou un thiocyanate de tétra alkylammonium, ce dernier peut être choisi, à titre d'exemple, parmi les halogénures, azotures ou thiocyanates de tétra méthylammonium, de tétra éthylammonium, de tétra propylammonium, de tétra butylammonium (tétra n-butylammonium par exemple), de tétra pentylammonium, de tétra cyclohexylammonium, de tri éthyl méthylammonium de tri butyl méthylammonium, ou de tri méthyl propylammonium.

On opère dans un solvant organique tel qu'un solvant chloré (par exemple dichlorométhane, dichloréthane, chloroforme) ou dans un éther (tétrahydrofurane par exemple) à une température comprise entre -78 et 40°C (de préférence entre 0 et 30°C). Il est avantageux d'opérer sous argon ou sous azote. Il est entendu que la mise en oeuvre du dérivé hydroxy de configuration (16S) conduit au dérivé de configuration (16R).

La protection et la déprotection du radical hydroxy en position 14 s'effectue selon les méthodes habituelles qui n'affectent pas le reste de la molécule, notamment par application des méthodes décrites par T.W. Greene et P.G.M. Wuts, Protective Groups in Organic Synthesis (2^{ème} éd.), A. Wiley - Interscience Publication (1991), ou par Mc Omie, Protective Groups in Organic Chemistry, Plenum Press ( 1973). Par exemple on opère par protection par un radical trialcoylsilyle, alcoyldiphénylsilyle (t.butyldiphénylsilyle et t.butyldiméthylsilyle par exemple) ou allyle qui sont mis en place et éliminés comme décrit ci-après dans les exemples.

Le cas échéant, lorsque l'on souhaite préparer un produit de formule générale (I) pour lequel R₃ est un ester aliphatique, cycloaliphatique, aromatique, araliphatique, hétérocyclique ou hétérocyclylaliphatique pouvant être substitué, l'opération subséquente d'estérification est effectuée selon les méthodes habituelles qui n'altèrent pas le reste de la molécule. Plus particulièrement l'estérification est mise en oeuvre par réaction de l'acide ou d'un dérivé réactif de l'acide (chlorure d'acide, ester réactif, anhydride par exemple), en présence ou non d'un agent de couplage comme un carbodiimide (dicyclohexylcarbodiimide par exemple) et d'une amine tertiaire (trialcoylamine comme la triéthylamine ou la diisopropyléthylamine, ou la pyridine ou un dérivé) et éventuellement un catalyseur comme la 4-N-diméthylaminopyridine, à une température comprise entre -40 et + 80°C dans un solvant organique tel qu'un amide (diméthylformamide ou N-méthyl 2-pyrrolidinone par exemple), la pyridine, un solvant halogéné (dichlorométhane, dichloroéthane ou chloroforme par exemple) ou un éther (tétrahydrofurane, dioxane, diméthoxyéthane). Il est entendu que les fonctions pouvant interférer avec la réaction sont préalablement protégées, puis libérées après la réaction.

L'acide ou le dérivé réactif d'acide mis en oeuvre est préparé comme décrit ci-après dans les exemples ou par analogie avec les méthodes décrites.

Le dérivé dihydroxylé de streptogramine du groupe A de formule générale (II) peut être obtenu par réduction sélective de la composante de la pristinamycine naturelle de formule générale : 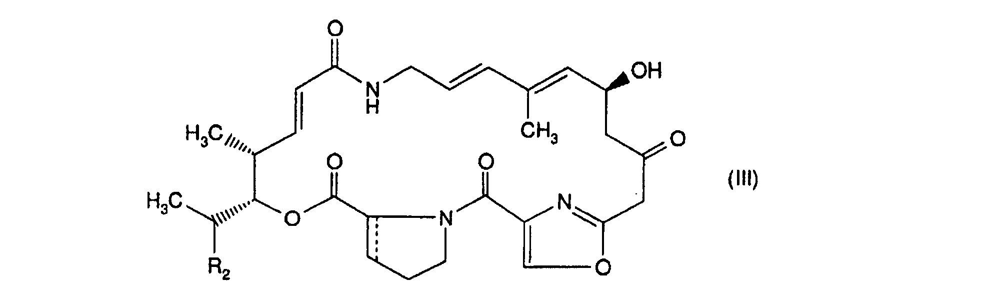 dans laquelle R₂ est défini comme ci-dessus et la liaison --- représente une liaison simple (stéréochimie 27R) ou une liaison double, suivie de la séparation de la forme épimère 16S.

La réduction s'effectue avantageusement en présence d'un agent réducteur tel qu'un borohydrure alcalin par exemple le borohydrure de sodium ou le triacétoxyborohydrure de sodium, dans un solvant organique choisi parmi les solvants chlorés (par exemple dichlorométhane, dichloréthane, chloroforme) le tétrahydrofurane, l'acide acétique et des alcools comme le méthanol, l'éthanol, ou le 2-propanol, à une température comprise entre -78 et 40°C

La séparation de la forme épimère 16R et de la forme épimère 16S s'effectue selon les méthodes habituelles ; par exemple la séparation des formes épimères peut s'effectuer par chromatographie, chromatographie flash, chromatographie liquide haute performance (CLHP), sur phase chirale ou non, ou chromatographie par partage centrifuge (CPC), à partir du mélange des épimères 16R et 16S. Ou par cristallisation.

Notamment la (16*S*)-16-hydroxy pristinamycine II_{A} peut être préparée selon F. Le Goffic et coll. ; Eur. J. Med. - Chimica Therapeutica ; January - February, - 16(1), 69-72(1981).

Les dérivés de pristinamycine de formule générale (III) correspondent respectivement à la pristinamycine IIA (PIIA), à la pristinamycine IIB (PIIB), à la pristinamycine IIC (PIIC), à la pristinamycine IID (PIID), à la pristinamycine IIF (PIIF) et à la pristinamycine IIG (PIIG) qui sont des composantes connues de la pristinamycine naturelle. Les composantes PIIF et PIIG ont été décrites dans le brevet européen EP 614910.

La pristinamycine IIC (PIIC), et la pristinamycine IID (PIID) peuvent être obtenues comme décrit par J.C. Barrière et coll., Expert. Opin. Invest. Drugs, 3(2), 115-31 (1994).

La préparation et la séparation des composantes des streptogramines naturelles du groupe A [streptogramines de formule générale (III)] est effectuée par fermentation et isolement des constituants à partir du môut de fermentation selon ou par analogie avec la méthode décrite par J. Preud'homme et coll., Bull. Soc. Chim. Fr., vol. 2, 585 (1968) ou dans le brevet européen EP 614910. Alternativement la préparation des composantes naturelles du groupe A peut être effectuée par fermentation spécifique, comme décrit dans la demande de brevet FR 2 689 518.

Les dérivés de streptogramine de formule générale (I) peuvent être purifiés le cas échéant par des méthodes physiques telles que la cristallisation, la chromatographie ou la CPC.

Certains des dérivés de streptogramine de formule générale (I) peuvent être transformés à l'état de sels d'addition avec les acides, par les méthodes connues. Il est entendu que ces sels, lorsqu'ils existent, entrent aussi dans le cadre de la présente invention.

Comme exemples de sels d'addition avec des acides pharmaceutiquement acceptables, peuvent être cités les sels formés avec les acides minéraux (chlorhydrates, bromhydrates, sulfates, nitrates, phosphates) ou avec les acides organiques (succinates, fumarates, tartrates, acétates, propionates, maléates, citrates, méthanesulfonates, éthanesulfonates, phénylsulfonates, p.toluènesulfonates, iséthionates, naphtylsulfonates ou camphorsulfonates, ou avec des dérivés de substitution de ces composés).

Les dérivés de streptogramine selon la présente invention présentent des propriétés antibactériennes et des propriétés synergisantes de l'activité antibactérienne des dérivés de streptogramine du groupe B. Ils sont particulièrement intéressants du fait de leur activité puissante seuls ou associés.

Lorsqu'ils sont associés avec une composante ou un dérivé du groupe B des streptogramines, ces derniers peuvent être choisis selon que l'on désire obtenir une forme administrable par voie orale ou parentérale, parmi les composantes naturelles : pristinamycine IA, pristinamycine IB, pristinamycine IC, pristinamycine ID, pristinamycine IE, pristinamycine IF, pristinamycine IG, virginiamycine S1, S3 ou S4, vernamycine B ou C, étamycine ou parmi des dérivés d'hémisynthèse tels que décrits dans les brevets ou demandes de brevet US 4618599, US 4798827, US 5326782, EP 772630 ou EP 770132, notamment des dérivés de streptogramines de formule générale : 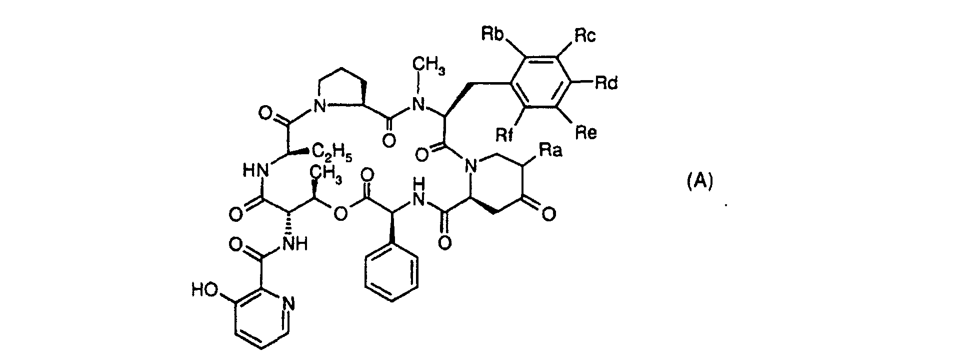 dans laquelle,
1. Rb, Rc, Re et Rf sont des atomes d'hydrogène, Rd est un atome d'hydrogène ou un radical diméthylamino, et Ra est un radical de structure -CH₂R'a pour lequel R'a est pyrrolidinyl-3thio, pipéridyl-3(ou -4)thio pouvant être substitués par alcoyle, alcoylthio substitué par 1 ou 2 hydroxysulfonyle, alcoylamino, dialcoylamino (lui même éventuellement substitué par mercapto ou dialcoylamino), ou substitué par 1 ou 2 cycles pipérazine éventuellement substitué, morpholino, thiomorpholino, pipéridino, pyrrolidinyle-1, pipéridyle-2,-3 ou -4, ou pyrrolidinyle-2 ou -3 (pouvant être substitués par alcoyle), ou bien Ra est un radical de structure =CHR'a pour lequel R'a est pyrrolidinyl-3amino, pipéridyl-3(ou -4)amino, pyrrolidinyl-3oxy, pipéridyl-3(ou -4)oxy, pyrrolidinyl-3thio, pipéridyl-3(ou -4)thio pouvant être substitués par alcoyle, ou R'a est alcoylamino, alcoyloxy ou alcoylthio substitués par 1 ou 2 hydroxysulfonyle, alcoylamino, dialcoylamino (lui même éventuellement substitué par dialcoylamino), ou par trialcoylammonio, imidazolyl-4 ou -5, ou par 1 ou 2 cycles pipérazine éventuellement substitué, morpholino, thiomorpholino, pipéridino, pyrrolidinyle-1, pipéridyle-2,-3 ou -4, ou pyrrolidinyle-2 ou -3 (pouvant être substitués par alcoyle), ou
   Ra est un radical quinuclidinyl-3(ou -4)thiométhyle ou bien
2. Ra est un atome d'hydrogène et
   a) soit Rb, Re et Rf sont des atomes d'hydrogène, Rd est un radical -NHCH₃ ou - N(CH₃)₂ et Rc est un atome de chlore ou de brome, ou représente un radical alcényle contenant 3 à 5 atomes de carbone [si Rd est -N(CH₃)₂],
   b) soit Rb, Rd, Re et Rf représentent un atome d'hydrogène et Rc est un halogène, ou un radical aminomonoalkyle, aminodialkyle, alcoyloxy, trifluorométhyloxy, thioalcoyle,alcoyle en C₁ à C₃ ou trihalogénométhyle
   c) soit Rb, Rc, Re et Rf représentent un atome d'hydrogène et Rd est un halogène, ou un radical éthylamino, diéthylamino ou méthyléthylamino, alcoyloxy ou trifluorométhyloxy, thioalkyle, alcoyle en C₁ à C₆, aryle ou trihalogénométhyle
   d) soit Rb, Re et Rf représentent un atome d'hydrogène et Rc est halogène ou un radical aminomonoalkyle ou aminodialkyle, alcoyloxy ou trifluorométhyloxy, thioalkyle, alcoyle en C₁ à C₃, et Rd est halogène ou un radical amino, aminomonoalkyle ou aminodialkyle, alcoyloxy ou trifluorométhyloxy, thioalkyle, alcoyle en C₁ à C₆ ou trihalogénométhyle,
   e) soit Rc, Re et Rf représentent un atome d'hydrogène et Rb et Rd représentent un radical méthyle ;
ou encore parmi les dérivés d'hémisynthèse du groupe B des streptogramines de formule générale : 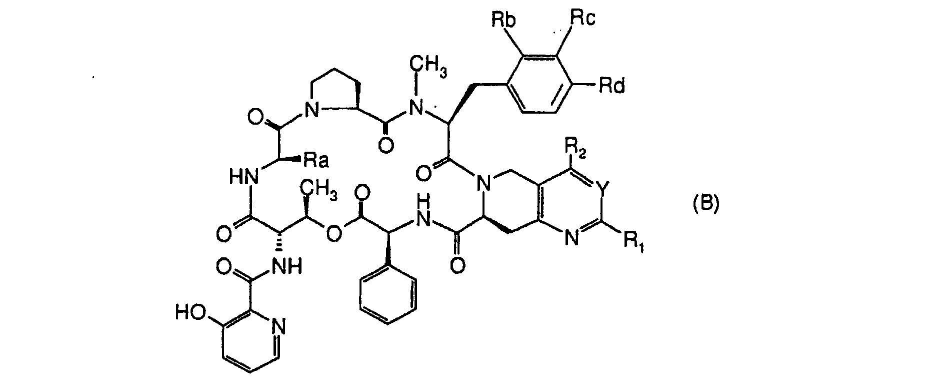 dans laquelle
Y est un atome d'azote ou un radical =CR₃-,
R₁ est un atome d'hydrogène, un radical alcoyle (1 à 8 carbones), alcényle (2 à 8 carbones), cycloalcoyle (3 à 8 carbones), hétérocyclyle saturé ou insaturé (3 à 8 chaînons), phényle, phényle substitué [par un ou plusieurs atomes d'halogène ou radicaux hydroxy, alcoyle, alcoyloxy, alcoylthio, alcoylsulfinyle, alcoylsulfonyle, amino, alcoylamino ou dialcoylamino] ou un radical NR'R", R' et R" identiques ou différents pouvant être des atomes d'hydrogène ou des radicaux alcoyle (1 à 3 carbones), ou pouvant former ensemble avec l'atome d'azote auquel ils sont attachés, un hétérocycle de 3 à 8 chaînons contenant éventuellement un autre hétéroatome choisi parmi l'oxygène le soufre ou l'azote éventuellement substitué [par un radical alcoyle, alcényle (2 à 8 carbones), cycloalcoyle (3 à 6 carbones), hétérocyclyle saturé ou insaturé (4 à 6 chaînons), benzyle, phényle ou phényle substitué tel que défini ci-dessus pour la définition de R₁]
   ou bien lorsque Y est un radical =CR₃-, R₁ peut être aussi halogénométhyle, hydroxyméthyle, alcoyloxyméthyle, alcoylthiométhyle dont la partie alcoyle est éventuellement substituée par NR'R", alcoylsulfinylméthyle, alcoylsulfonylméthyle, acyloxyméthyle, benzoyloxyméthyle, cyclopropylaminométhyle ou -(CH₂)ₙNR'R" (n étant un entier de 1 à 4 et R' et R" étant définis comme ci-dessus), ou bien si R₃ est un atome d'hydrogène, R₁ peut être aussi formyle, carboxy, alcoyloxycarbonyle, ou -CONR'R" pour lequel R' et R" sont définis comme ci-dessus,
   ou bien lorsque Y est un atome d'azote, R₁ peut être aussi un radical -XR° pour lequel X est un atome d'oxygène ou de soufre, un radical sulfinyle ou sulfonyle, ou un radical NH et R° est un radical alcoyle (1 à 8 carbones), cycloalcoyle (3 à 6 carbones), hétérocyclyle saturé ou insaturé (3 à 8 chaînons), hétérocyclylméthyle (3 à 8 chaînons) dont la partie hétérocyclyle est attachée au radical méthyle par un atome de carbone, phényle, phényle substitué [par un ou plusieurs atomes d'halogène ou radicaux hydroxy, alcoyle, alcoyloxy, alcoylthio, alcoylsulfinyle, alcoylsulfonyle, amino, alcoylamino ou dialcoylamino] ou un radical -(CH₂)ₙNR'R" pour lequel R' et R" sont définis comme ci-dessus et n est un entier de 2 à 4, ou bien, si X représente NH, R° peut aussi représenter l'atome d'hydrogène,
R₂ est un atome d'hydrogène ou un radical alcoyle (1 à 3 carbones),
R₃ est un atome d'hydrogène ou un radical alcoyle, carboxy, alcoyloxycarbonyle ou carbamoyle de structure -CO-NR'R" dans laquelle R' et R" sont définis comme précédemment,
Ra est un radical méthyle ou éthyle, et
Rb, Rc et Rd ont les définitions ci-après :
   1) Rb et Rc sont des atomes d'hydrogène et Rd est un atome d'hydrogène ou un radical méthylamino ou diméthylamino,
   2) Rb est un atome d'hydrogène, Rc est un atome d'hydrogène, de chlore ou de brome, ou représente un radical alcényle (3 à 5C), et Rd est un radical -NMe-R''' pour lequel R''' représente un radical alcoyle, hydroxyalcoyle (2 à 4C), ou alcényle (2 à 8C) éventuellement substitué par phényle, cycloalcoyl(3 à 6C)méthyle, benzyle, benzyle substitué [par un ou plusieurs atomes d'halogène ou radicaux hydroxy, . alcoyle, alcoyloxy, alcoylthio, alcoylsulfinyle, alcoylsulfonyle, amino, alcoylamino ou dialcoylamino], hétérocyclylméthyle ou hétérocyclyléthyle dont la partie hétérocyclyle est saturée ou insaturée et contient 5 à 6 chaînons et 1 ou 2 hétéroatomes choisis parmi le soufre, l'oxygène ou l'azote éventuellement substitué [par un radical alcoyle, alcényle (2 à 8 carbones), cycloalcoyle (3 à 6 carbones), hétérocyclyle saturé ou insaturé (4 à 6 chaînons), phényle, phényle substitué tel que défini ci-avant pour la définition de R₁ ou benzyle], ou bien R''' représente un radical cyanométhyle, ou -CH₂CORe pour lequel soit Re est -OR'e, R'e étant hydrogène, alcoyle (1 à 6 carbones), alcényle (2 à 6 carbones), benzyle ou hétérocyclylméthyle dont la partie hétérocyclyle contient 5 à 6 chaînons et 1 ou 2 hétéroatomes choisis parmi le soufre, l'oxygène ou l'azote soit Re est un radical alcoylamino, alcoyl méthyl amino, hétérocyclylamino ou hétérocyclyl méthyl amino dont la partie hétérocyclyle est saturée et contient 5 à 6 chaînons et 1 ou 2 hétéroatomes choisis parmi le soufre, l'oxygène ou l'azote éventuellement substitué par un radical alcoyle, benzyle ou alcoyloxycarbonyle,
   3) Rb est un atome d'hydrogène, Rd est un radical -NHCH₃ ou -N(CH₃)₂ et Rc est un atome de chlore ou de brome, ou représente un radical alcényle (3 à 5C), [si Rd est -N(CH₃)₂],
   4) Rb et Rd sont des atomes d'hydrogène et Rc est un atome d'halogène, ou un radical alcoylamino ou dialcoylamino, alcoyloxy, trifluorométhoxy, thioalcoyle, alcoyle (1 à 6C) ou trihalogénométhyle,
   5) Rb et Rc sont des atomes d'hydrogène et Rd est un atome d'halogène, ou un radical éthylamino, diéthylamino ou méthyl éthyl amino, alcoyloxy ou trifluorométhoxy, alcoylthio, alcoylsulfinyle, alcoylsulfonyle, alcoyle (1 à 6C), phényle ou trihalogénométhyle,
   6) Rb est un atome d'hydrogène et Rc est un atome d'halogène ou un radical alcoylamino ou dialcoylamino, alcoyloxy ou trifluorométhoxy, thioalcoyle, alcoyle (1 à 3C), et Rd est un atome d'halogène ou un radical amino, alcoylamino ou dialcoylamino, alcoyloxy ou trifluorométhoxy, thioalcoyle, alcoyle (1 à 6C) ou trihalogénométhyle,
   7) Rc est un atome d'hydrogène et Rb et Rd représentent un radical méthyle, ainsi que leurs sels.

Il est entendu que les associations des dérivés selon l'invention et des streptogramines du groupe B entrent également dans le cadre de la présente invention.

Les dérivés du groupe B des streptogramines de structure (B) peuvent être préparés selon les méthodes décrites dans la demande internationale WO 9943699.

*In vitro* sur *Staphylococcus aureus 209P*, les dérivés de streptogramine selon l'invention se sont montrés actifs à des concentrations comprises entre 0,015 et 32 µg/ml seuls ou associés à un dérivé du groupe B comme la pristinamycine IB et à des concentrations comprises entre 0,015 et 32 µg/ml sur *Staphylococcus aureus Schiclia* (résistant à la méticilline) seuls ou associés à la pristinamycine IB ; in vivo, ils synergisent l'activité antimicrobienne de la pristinamycine I_{B} sur les infections expérimentales de la souris à *Staphylococcus aureus IP8203* à des doses comprises entre 5 et 150 mg/kg par voie sous cutanée (DC₅₀) et la majorité d'entre eux également par voie orale, à des doses comprises entre 30 et 150 mg/kg (DC₅₀).

Enfin, les produits selon l'invention sont particulièrement intéressants du fait de leur faible toxicité. Aucun des produits n'a manifesté de toxicité à des doses de 150 mg/kg sur Staphylococcus *aureus IP8203*, 2 fois par jour, par voie sous-cutanée ou par voie orale chez la souris.

D'un intérêt particulier sont les produits de formule générale (I) pour lesquels :
- R₁ représente un atome de fluor, de chlore, de brome ou d'iode ou un radical azido ou thiocyanato,

- R₂ représente un radical méthyle,
- R₃ représente un atome d'hydrogène, ou un radical R'₃-CO- pour lequel R'₃ est phényle ou phénylalcoyle non substitués ou substitués sur le radical phényle [par un ou plusieurs radicaux choisis parmi alcoyle, portant éventuellement un radical NR'R" dont les radicaux R' et R" identiques ou différents peuvent être des atomes d'hydrogène ou des radicaux alcoyle pouvant former ensemble avec l'atome d'azote auquel ils sont attachés un radical hétérocyclyle saturé ou insaturé de 3 à 8 chaînons, comprenant éventuellement un autre hétéroatome choisi parmi l'oxygène, le soufre ou l'azote, ledit hétérocycle pouvant être lui même substitué par un ou plusieurs radicaux (alcoyle, hydroxyalcoyle, alcoyloxyalcoyle, alcoyloxycarbonylalcoyle, aryle, hétérocyclyle, hétérocyclylalcoyle saturés ou insaturés de 3 à 8 chaînons ou -CH₂-CO-NR'R"), ou bien R' et/ou R" peuvent être un radical hydroxyalcoyle, phényle, hétérocyclylalcoyle saturé ou insaturé de 3 à 8 chaînons, -CO-NR'R" pour lequel NR'R" est défini comme précédemment, ou alcoyle ou acyle substitués par NR'R" défini tel que ci-dessus], ou bien R'₃ peut être un radical phényle substitué par un ou plusieurs radicaux [choisis parmi alcoyle, pouvant être substitués par un radical alcoyloxy ou alcoylthio éventuellement portant eux-même un radical carboxy ou un radical NR'R" tel que défini ci-dessus, ou choisis parmi acyloxy pouvant être substitué par NR'R" défini tel que précédemment], ou bien R'₃ peut être choisi parmi des radicaux alcoyle ou cycloalcoyle éventuellement substitués [par un radical carboxy, carboxyalcoyldisulfanyle ou par un radical NR'R", -CH₂-NR'R", -CO-NR'R", ou par un radical alcoyloxycarbonyle, alcoyloxy, ou alcoyldisulfanyle éventuellement substitués par NR'R" ou -CO-NR'R" pour lesquels NR'R" est défini tel que précédemment], ou bien R'₃ peut être choisi parmi des radicaux hétérocyclyle saturés ou insaturés de 3 à 8 chaînons éventuellement substitués [par alcoyle ou acyle eux-même éventuellement substitués par NR'R"], étant entendu que les hétérocycles sont choisis parmi pyrrolidinyle, imidazolyle, pyridyle, pipéridinyle, pipérazinyle, ou morpholynyle, et
- la liaison --- représente une liaison simple (stéréochimie 27R) ou une liaison double,
   ainsi que leurs sels lorsqu'ils existent ;
   et parmi ces produits, plus spécialement les :
   ― (16*R*)-16-désoxo-16-fluoro pristinamycine II_{B ;}
   ― ( 16*R*)-16-désoxo-16-thiocyanato pristinamycine II_{B ;}
   ― (16*R*)-16-désoxo-16-chloro pristinamycine II_{B ;}
   ― ( 16*R*)-16-azido-16-désoxo pristinamycine II_{B ;}
   ― (16*R*)-16-désoxo-16-fluoro pristinamycine II_{A.}

Les exemples suivants, donnés à titre non limitatif, illustrent la présente invention.

Dans les exemples qui suivent, la nomenclature 16-désoxo pristinamycine IIA (ou IIB) signifie le remplacement de la fonction cétone en position 16 par 2 atomes d'hydrogène. Au fur et à mesure de la chromatographie, toutes les fractions sont analysées par chromatographie en couche mince (CCM), sur plaques de silice Merck 60F254. Les fractions correspondants à un même tâche en CCM sont regroupées puis concentrées à sec, sous pression réduite (30°C; 2,7 kPa). Les résidus ainsi obtenus sont analysés par les techniques spectroscopiques habituelles (RMN; IR; MS) ce qui permet d'identifier le produit attendu.

### Exemple 1

### (16R)-16-Désoxo-16-fluoro pristinamycine II_{B}

A 1,12 g de (16*R*)-16-désoxo-16-fluoro-14-O-(tert-butyldiphénylsilyl) pristinamycine II_{B} en solution dans 10 cm³ de tétrahydrofurane, on ajoute à 20°C, sous atmosphère d'argon, 0,2 cm³ d'acide acétique et 0,6 g de fluorure de tétra n-butylammonium trihydraté. Après 168 heures d'agitation, le mélange réactionnel est concentré à sec sous pression réduite (2,7 kPa) pour donner 1 g d'une huile marron qui est purifiée par chromatographie-flash [éluant : dichlorométhane / méthanol / acétonitrile (90 / 5 / 5 en volumes)]. On obtient 0,3 g de (16*R*)-16-désoxo-16-fluoro pristinamycine II_{B}, sous forme d'un solide beige clair fondant vers 125°C (déc.).

Spectre de R.M.N. ¹H (400 MHz, CDCl₃, δ en ppm) : 0,96 (d, J = 6,5 Hz : 3H) ; 1,00 (d, J = 6,5 Hz : 3H) ; 1,10 (d, J = 6,5 Hz : 3H) ; de 1,55 à 2,05 (mt : 5H) ; 1,83 (s : 3H) ; de 2,10 à 2,30 (mt : 2H) ; 2,76 (mt : 1H) ; 2,98 (mt : 1H) ; 3,21 (mt : 1H) ; 3,48 (mt : 1H) ; 3,87 (mt : 1H) ; 4,07 (mt : 1H) ; 4,55 (mt : 1H) ; de 4,75 à 4,90 (mt : 3H) ; 5,14 (doublet démultiplié, J _{HF} = 48 Hz : 1H) ; 5,39 (d, J = 9 Hz : 1H) ; 5,71 (mt : 1H) ; 5,82 (dd, J = 17 et 2 Hz : 1H) ; 6,00 (mt : 1H) ; 6,21 (d, J = 16 Hz : 1H) ; 6,52 (dd, J = 17 et 5 Hz : 1H) ; 8,12 (s : 1H).

La (16*R*)-16-désoxo-16-fluoro-14-O-(tert-butyldiphénylsilyl) pristinamycine II_{B} peut être préparée de la manière suivante :

A 2 g de (16*S*)-16-hydroxy-14-O-(tert-butyldiphénylsilyl) pristinamycine II_{B} en solution dans 50 cm³ de dichlorométhane, on ajoute lentement à 20°C, sous atmosphère d'argon, 0,464 cm³ de trifluorure de diéthylaminosulfure. Après 2 heures d'agitation, le mélange réactionnel est versé sur 100 cm³ d'une solution aqueuse saturée de bicarbonate de sodium. La phase organique est décantée, lavée par 2 fois 100 cm³ d'eau, séchée sur sulfate de magnésium, filtrée puis concentrée à sec sous pression réduite (2,7 kPa) pour donner 2,1 g d'un solide ocre qui est purifié par chromatographie-flash [éluant : gradient dichlorométhane / acétonitrile / méthanol (100 / 0/ 0; 99/ 0,5 / 0,5 puis 98 / 1 / 1 en volumes)]. On obtient 1,35 g de (16*R*)-16-désoxo-16-fluoro-14-O-(tert-butyldiphénylsilyl) pristinamycine II_{B}, sous forme d'un solide blanc fondant vers 116°C (déc.).

Spectre de R.M.N. ¹H (400 MHz, CDCl₃, δ en ppm) : 0,96 (d, J = 6,5 Hz : 3H) ; 0,99 (d, J = 6,5 Hz : 3H) ; de 1,00 à 1,15 (mt : 12H) ; 1,29 (s : 3H) ; de 1,55 à 1,95 (mt : 4H) ; 1,96 (mt : 1H) ; 2,13 (mt : I H) ; 2,24 (mt : 1H) ; 2,76 (mt : 1H) ; 2,85 (mt : 1H) ; 3,03 (mt : 1H) ; 3,39 (mt : 1H) ; 3,80 (mt : 1H) ; 4,01 (mt : 1H) ; 4,57 (mt : 1H) ; 4,72 (mt : 1H) ; de 4,75 à 4,85 (mt : 2H) ; 5,01 (doublet démultiplié, J _{HF} = 48 Hz : 1H) ; 5,38 (d, J = 9 Hz : 1H) ; 5,50 (mt : 1H) ; 5,81 (dd, J = 17 et 1,5 Hz : 1H) ; 5,97 (mt : 1H) ; 6,10 (d, J = 15,5 Hz : 1H) ; 6,49 (dd, J = 17 et 5 Hz : 1H) ; de 7,30 à 7,50 (mt : 6H) ; 7,63 (d large, J = 7 Hz : 2H) ; 7,68 (d large, J = 7 Hz : 2H) ; 8,08 (s : 1H).

La (16*S*)-16-hydroxy-14-O-(tert-butyldiphénylsilyl) pristinamycine II_{B} peut être préparée de la manière suivante :

A 22 g de (16*S*)-16-hydroxy pristinamycine II_{B} en solution dans 200 cm³ de dichlorométhane, on ajoute à 20°C, sous atmosphère d'argon, 29 cm³ de diisopropyléthylamine, 43,2 cm³ de tert-butyldiphénylchlorosilane goutte à goutte et 1,01 g de 4-diméthylaminopyridine. Après 22 heures d'agitation, le mélange réactionnel est versé sur 600 cm³ d'une solution aqueuse saturée de bicarbonate de sodium. La phase aqueuse est décantée puis extraite par 2 fois 100 cm³ de dichlorométhane. Les phases organiques sont rassemblées, lavées par 400 cm³ d'une solution aqueuse saturée de chlorure de sodium, séchées sur sulfate de sodium, filtrées puis concentrées à sec sous pression réduite (2,7 kPa) pour donner 70,6 g d'une huile visqueuse orange qui est agité dans 600 cm³ d'éther diisopropylique pendant 16 heures. Après filtration et séchage sous pression réduite (2,7 kPa) à 20°C, on obtient 28 g de (16*S*)-16-hydroxy-14-O-(tert-butyldiphénylsilyl) pristinamycine II_{B}, sous forme d'un solide rosé, fondant vers 133°C (déc.).

Spectre de R.M.N. ¹H (400 MHz, CDCl₃, δ en ppm) : 0,95 (d, J = 6,5 Hz : 3H) ; de 1,00 à 1,05 (mt : 9H) ; 1,08 (s : 9H) ; de 1,40 à 1,80 (mt : 3H) ; de 1,90 à 2,15 (mt : 3H) ; 2,23 (d large, J = 14 Hz : 1H) ; 2,75 (mt : 1H) ; 2,83 (dd, J = 17 et 11 Hz : 1H) ; 3,10 (dd, J = 17 et 2,5 Hz : 1H) ; 3,25 (mt : 1H) ; de 3,60 à 3,75 (mt : 2H) ; 4,49 (mt : 1H) ; 4,56 (mt : 1H) ; de 4,60 à 4,70 (mt : 2H) ; 4,87 (mt : 1H) ; 5,49 (mt : 1H) ; 5,74 (dd, J = 17 et 2 Hz : 1H) ; 5,78 (d, J = 9 Hz : 1H) ; 5,95 (mt : 1H) ; 6,04 (d, J = 16 Hz : 1H) ; 6,41 (dd, J = 17 et 4 Hz : 1H) ; de 7,30 à 7,50 (mt : 6H) ; 7,64 (dd, J = 7 et 1,5 Hz : 2H) ; 7,69 (dd, J = 7 et 1,5 Hz : 2H) ; 8,11 (s : 1H).

La (16*S*)-16-hydroxy pristinamycine II_{B} peut être préparée de la manière suivante :

Une suspension de 11,35 g de borohydrure de sodium dans 550 cm³ de dichlorométhane est chauffée au reflux pendant 20 minutes. On ajoute alors, goutte à goutte, en environ 30 minutes, 68,6 cm³ d'acide acétique puis une solution (préalablement séchée sur sulfate de sodium) de 52,75 g de pristinamycine II_{B} dans 230 cm³ de dichlorométhane, en environ 45 minutes. Le mélange réactionnel est agité 4,5 heures au reflux puis 16 heures à 20°C. On ajoute alors au mélange réactionnel 500 cm³ de dichlorométhane et 1500 cm³ d'eau. La phase organique est décantée et la phase aqueuse est extraite par 500 cm³ de dichlorométhane. Les phases organiques sont réunies et le pH est ajusté à 8 par une addition lente de 1000 cm³ d'une solution aqueuse saturée de bicarbonate de sodium. La phase organique résultante est lavée successivement par 1000 cm³ d'eau et 1000 cm³ d'une solution aqueuse saturée de chlorure de sodium puis traitée au noir végétal 3S, séchée sur sulfate de sodium, filtrée et concentrée à sec sous pression réduite (2,7 kPa) pour donner 50 g d'un solide jaune clair. A une solution du solide précédent dans 900 cm³ de dichlorométhane, on ajoute à 20°C, 378 cm³ d'une solution aqueuse d'hydroxyde d'ammonium 0,5 M. Après 16 heures d'agitation à 20°C, la phase organique est décantée, lavée par 1000 cm³ d'eau puis 1000 cm³ d'une solution aqueuse saturée de chlorure de sodium, séchée sur sulfate de sodium, filtrée et concentrée à sec sous pression réduite (2,7 kPa) pour donner 46 g d'un solide jaune pâle qui est purifié par chromatographie-flash [éluant : gradient dichlorométhane / méthanol (98 / 2 et 97 / 3 en volumes)]. On obtient 31,68 g de (16*S*)-16-hydroxy pristinamycine II_{B}, sous forme d'un solide blanc cassé fondant vers 131°C (déc.).

Spectre de R.M.N. ¹H (400 MHz, CDCl₃, δ en ppm) : 0,96 (d, J = 6,5 Hz : 3H) ; 1,02 (d, J = 6,5 Hz : 3H) ; 1,07 (d, J = 6,5 Hz : 3H) ; de 1,70 à 1,90 (mt : 3H) ; 1,76 (s : 3H) ; 1,97 (mt : 2H) ; 2,12 (mt : 1 H) ; 2,26 (d large : 14,5 Hz : 1H) ; 2,56 (d, J = 3 Hz : 1H) ; 2,76 (mt : 1H) ; 2,90 (dd, J = 16 et 10 Hz : 1H) ; 3,08 (dd, J = 16 et 3 Hz : 1H) ; 3,35 (mt : 1H) ; 3,82 (mt : 2H) ; 3,99 (d, J = 2,5 Hz : 1H) ; de 4,40 à 4,55 (mt : 2H) ; de 4,65 à 4,75 (mt : 2H) ; 5,03 (mt : 1H) ; de 5,65 à 5,85 (mt : 3H) ; 6,01 (mt : 1H) ; 6,21 (d, J = 16 Hz : 1H) ; 6,46 (dd, J = 17 et 5 Hz : 1H) ; 8,13 (s : 1H).

### Exemple 2

### (16R)-16-Désoxo-16-fluoro pristinamycine II_{B}

A 257 g de (16*R*)-16-désoxo-16-fluoro-14-O-(tert-butyldiméthylsilyl) pristinamycine II_{B} en solution dans 2500 cm³ de dichlorométhane, on ajoute lentement, en 40 minutes à 40°C, sous atmosphère d'argon, 970 cm³ de trifluorhydrate de triéthylamine. Après 2 heure d'agitation à 40°C, le mélange réactionnel est refroidi à 8°C puis hydrolysé par une addition lente, en 25 minutes, de 1940 cm³ d'eau. Après 10 minutes d'agitation, les phases organique et aqueuse sont décantées et l'interface laiteuse est reprise par 4000 cm³ d'eau et 1000 cm³ de dichlorométhane. Après agitation des deux phases, la phase organique est décantée et les phases aqueuses sont réunies puis extraites par 1000 cm³ de dichlorométhane. Les phases organiques sont réunies et additionnées à 1500 cm³ d'eau. Le pH de la phase aqueuse est ajusté à 7 par addition lente, en 15 minutes à 10°C, de 400 cm³ d'une solution aqueuse saturée de bicarbonate de sodium. La phase organique est décantée, diluée avec 1500 cm³ d'acétate d'éthyle puis lavée par 1000 cm³ d'eau. La phase organique est décantée et la phase aqueuse est extraite par 2 fois 1500 cm³ d'acétate d'éthyle puis filtrée sur Célite® sur verre fritté. La Célite® est rincée par 3 fois 400 cm³ d'acétate d'éthyle. Les phases organiques sont réunies, séchées sur sulfate de sodium puis filtrées sur Célite® sur verre fritté. La Célite® est rincée par 4 fois 800 cm³ d'acétate d'éthyle. Le filtrat est concentré à sec sous pression réduite (2,7 kPa) pour donner, après séchage à poids constant à 25°C, 218,5 g de (16*R*)-16-désoxo-16-fluoro pristinamycine II_{B}, sous forme d'une poudre cristalline blanche fondant vers 133°C (déc.).

Spectre de R.M.N. ¹H (400 MHz, CDCl₃, δ en ppm) : 0,96 (d, J = 6,5 Hz : 3H) ; 1,00 (d, J = 6,5 Hz : 3H) ; 1,10 (d, J = 6,5 Hz : 3H) ; de 1,55 à 2,05 (mt : 5H) ; 1,83 (s : 3H) ; de 2,10 à 2,30 (mt : 2H) ; 2,76 (mt : 1H) ; 2,98 (mt : 1H) ; 3,21 (mt : 1H) ; 3,48 (mt : 1H) ; 3,87 (mt : 1H) ; 4,07 (mt : 1H) ; 4,55 (mt : 1H) ; de 4,75 à 4,90 (mt : 3H) ; 5,14 (doublet démultiplié, J _{HF} = 48 Hz : 1H) ; 5,39 (d, J = 9 Hz : 1H) ; 5,71 (mt : 1H) ; 5,82 (dd, J = 17 et 2 Hz : 1H) ; 6,00 (mt : 1H) ; 6,21 (d, J = 16 Hz : 1H) ; 6,52 (dd, J = 17 et 5 Hz : 1H); 8,12 (s : 1H).

La (16*R*)-16-désoxo-16-fluoro-14-O-(tert-butyldiméthylsilyl) pristinamycine II_{B} peut être préparée de la manière suivante :

En opérant d'une manière analogue à celle décrite à l'exemple 1, mais à partir de 22 g de (16*S*)-16-hydroxy-14-O-(tert-butyldiméthylsilyl) pristinamycine II_{B} en solution dans 500 cm³ de dichlorométhane, on ajoute lentement à 20°C, sous atmosphère d'argon, 6,7 cm³ de trifluorure de diéthylaminosulfure. Après 4 heures d'agitation et traitement analogue à celui de l'exemple 1, on obtient 25 g d'une huile orange qui est purifiée par chromatographie-flash [éluant : gradient dichlorométhane / méthanol / acétonitrile (100 / 0 / 0; 99 / 0,5 / 0,5 à 96 / 2 / 2 en volumes)]. On obtient 6,6 g de (16*R*)-16-désoxo-16-fluoro-14-O-(tert-butyldiméthylsilyl) pristinamycine II_{B}, sous forme d'un solide jaune clair fondant vers 128°C (déc.).

Spectre de R.M.N. ¹H (400 MHz, CDCl₃, δ en ppm) : 0,04 (s : 3H) ; 0,07 (s : 3H) ; 0,89 (s : 9H) ; 0,96 (d, J = 6,5 Hz : 3H) ; 0,99 (d, J = 6,5 Hz : 3H) ; 1,09 (d, J = 6,5 Hz : 3H) ; de 1,50 à 2,05 (mt : 5H) ; 1,79 (s : 3H) ; de 2,10 à 2,25 (mt : 2H) ; 2,76 (mt : 1H) ; 2,90 (mt : 1H) ; 3,15 (mt : 1H) ; 3,43 (mt : 1H) ; 3,88 (mt : 1H) ; 4,09 (mt : 1H) ; 4,59 (mt : 1H) ; 4,75 (mt : 1H) ; 4,79 (dd, J = 10 et 1,5 Hz : 1H) ; 4,84 (dd, J = 9 et 3 Hz : 1H) ; 5,12 (doublet démultiplié, J _{HF} = 48 Hz : 1H) ; 5,34 (d, J = 9 Hz : 1H) ; 5,64 (mt : 1H) ; 5,82 (dd, J = 17 et 2 Hz : 1H) ; 5,99 (mt : 1H) ; 6,20 (d, J = 16 Hz : 1H) ; 6,50 (dd, J = 17 et 5 Hz : 1H) ; 8,09 (s : 1H).

La (16*S*)-16-hydroxy-14-O-(tert-butyldiméthylsilyl) pristinamycine II_{B} peut être préparée de la manière suivante :

A 27 g de (16*S*)-16-hydroxy pristinamycine II_{B} (préparée comme décrit dans l'exemple 1) en solution dans 270 cm³ de dichlorométhane, on ajoute à 20°C, sous atmosphère d'argon, 44,4 cm³ de diisopropyléthylamine et 39,6 g de tert-butyldiméthylchlorosilane en solution dans 140 cm³ de dichlorométhane. Après 17 heures d'agitation, le mélange réactionnel est lavé par 3 fois 300 cm³ d'eau. La phase organique est séchée sur sulfate de magnésium, filtrée puis concentrée à sec sous pression réduite (2,7 kPa) pour donner une huile orange qui est purifiée par chromatographie-flash [éluant : gradient dichlorométhane / méthanol / acétonitrile (100 / 0 / 0; 99 / 0,5 / 0,5 à 96 / 2 / 2 en volumes)]. On obtient 24 g de (16*S*)-16-hydroxy-14-O-(tert-butyldiméthylsilyl) pristinamycine II_{B}, sous forme d'un solide jaune clair fondant vers 139°C (déc.).

Spectre de R.M.N. ¹H (400 MHz, CDCl₃, δ en ppm) : 0,05 (s : 3H) ; 0,10 (s : 3H) ; 0,91 (s : 9H) ; 0,96 (d, J = 6,5 Hz : 3H) ; 1,02 (d, J = 6,5 Hz : 3H) ; 1,06 (d, J = 6,5 Hz : 3H) ; de 1,70 à 2,05 (mt : 5H) ; 1,71 (s : 3H) ; 2,12 (mt : 1H) ; 2,31 (d large, J = 14 Hz : 1H) ; de 2,70 à 2,80 (mt : 1H) ; 2,80 (dd, J = 17 et 11 Hz : 1H) ; 3,06 (dd, J = 17 et 2,5 Hz : 1H) ; 3,30 (mt : 1H) ; 3,79 (mt : 2H) ; 4,47 (t large, J = 10 Hz : 1H) ; 4,52 (s : 1H) ; 4,54 (mt : 1H) ; de 4,65 à 4,75 (mt : 2H) ; 4,99 (mt : 1H) ; 5,69 (mt : 1H) ; 5,76 (dd, J = 17 et 2 Hz : 1H) ; 5,78 (d, J = 9 Hz : 1H) ; 6,00 (mt : 1H) ; 6,20 (d, J = 16 Hz : 1H) ; 6,43 (dd, J = 17 et 4 Hz : 1H) ; 8,12 (s : 1H).

### Exemple 3

### (16R)-16-Désoxo-16-fluoro pristinamycine II_{B}

A une suspension de 0,09 g de *para*-toluènesulfinate de sodium dans 6 cm³ de dichlorométhane, on ajoute à 0°C sous atmosphère d'argon, 0,5 cm³ d'une solution aqueuse d'acide chlorhydrique 1 N. Après 15 minutes d'agitation à 20°C, la phase organique est décantée, séchée sur du sulfate de magnésium et filtrée. Au filtrat on ajoute, à 20°C sous atmosphère d'argon, 0,26 g de (16*R*)-14-O-allyl-16-désoxo-16-fluoro pristinamycine II_{B} et 0,04 g de tétrakis(triphénylphosphine)palladium. Après deux heures d'agitation, on ajoute 0,01g de triéthylamine. Après 30 minutes d'agitation supplémentaire, le mélange réactionnel est concentré à sec puis purifiée par chromatographie-flash [éluant : dichlorométhane / méthanol / acétonitrile (90 / 5 / 5 en volumes)] pour conduireà 0,2 g de (16*R*)-16-désoxo-16-fluoro pristinamycine II_{B}.

Spectre de R.M.N. ¹H (400 MHz, CDCl₃, δ en ppm) : 0,96 (d, J = 6,5 Hz : 3H) ; 1,00 (d, J = 6,5 Hz : 3H) ; 1,10 (d, J = 6,5 Hz : 3H) ; de 1,50 à 2,05 (mt : 5H) ; 1,62 (d, J = 4 Hz : 1H) ; 1,83 (s : 3H) ; de 2,10 à 2,30 (mt : 2H) ; 2,77 (mt : 1H) ; 2,98 (mt : 1H) ; 3,21 (mt : 1H) ; 3,48 (mt : 1H) ; 3,87 (mt : 1H) ; 4,06 (mt : 1H) ; 4,55 (mt : 1H) ; de 4,75 à 4,85 (mt : 3H) ; 5,14 (doublet démultiplié, J _{HF} = 48 Hz : 1H) ; 5,39 (d, J = 9 Hz : 1H) ; 5,72 (mt : 1H) ; 5,82 (dd, J = 16 et 2 Hz : 1H) ; 5,98 (mt : 1H) ; 6,21 (d, J = 16 Hz : 1H) ; 6,51 (dd, J = 16 et 5 Hz : 1H); 8,11 (s : 1H).

La (16*R*)-14-O-allyl-16-désoxo-16-fluoro pristinamycine II_{B} peut être préparée de la manière suivante :

A 0,8 g de (16*S*)-14-O-allyl-16-hydroxy pristinamycine II_{B} en solution dans 25 cm³ de dichlorométhane, on ajoute goutte à goutte, à 0°C, sous atmosphère d'argon, 0,28 cm³ de trifluorure de diéthylaminosulfure en solution dans 5 cm³ de dichlorométhane. Après 45 minutes d'agitation à 0°C puis retour à 20°C, le mélange réactionnel est versé sur 70 cm³ d'une solution aqueuse saturée d'hydrogénocarbonate de sodium à 0°C. Le mélange résultant est extrait par 100 cm³ de dichlorométhane. La phase organique est lavée successivement par 70 cm³ d'une solution aqueuse saturée d'hydrogénocarbonate de sodium et 70 cm³ d'une solution aqueuse saturée de chlorure de sodium puis séchée sur du sulfate de magnésium. Après filtration et concentration à sec sous pression réduite (2,7 kPa) à 20°C, on obtient 0,8 g de (16*R*)-14-O-allyl-16-désoxo-16-fluoro pristinamycine II_{B} sous la forme d'un solide de couleur crème.

Spectre de R.M.N. ¹H (400 MHz, CDCl₃, δ en ppm) : 0,96 (d, J = 6,5 Hz : 3H) ; 1,00 (d, J = 6,5 Hz : 3H) ; 1,09 (d, J = 6,5 Hz : 3H) ; de 1,50 à 2,05 (mt : 5H) ; 1,81 (s : 3H) ; 2,16 (mt : 1H) ; 2,29 (mt : 1H) ; 2,76 (mt : 1H) ; 2,93 (mt : 1H) ; 3,18 (dt, J = 17 et 7 Hz : 1H) ; 3,45 (mt : 1H) ; de 3,80 à 3,95 (mt : 2H) ; de 4,00 à 4,15 (mt : 2H) ; 4,47 (dt, J = 9,5 et 4 Hz : 1H) ; 4,59 (mt : 1H) ; 4,78 (dd, J = 10 et 1,5 Hz : 1H) ; 4,83 (dd, J = 9 et 3 Hz : 1H) ; de 5,05 à 5,35 (mt : 4H) ; 5,69 (mt : 1H) ; 5,82 (dd, J = 16 et 2 Hz : 1H) ; 5,90 (mt : 1H) ; 6,00 (mt : 1H) ; 6,25 (d, J = 16 Hz : 1H) ; 6,51 (dd, J = 16 et 4 Hz : 1H) ; 8,11 (s : 1H).

La (16*S*)-14-O-allyl-16-hydroxy pristinamycine II_{B} peut être préparée de la manière suivante :

A 10,6 g de (16*S*)-16-hydroxy pristinamycine II_{B} (préparée comme décrit dans l'exemple 1) en solution dans 500 cm³ de 2-butanone, on ajoute 19 g de carbonate de potassium et 31,5 cm³ de bromure d'allyle. Le mélange réactionnel est chauffé au reflux pendant 90 heures. Après refroidissement à 20°C et filtration, le mélange réactionnel est concentré sous pression réduite (2,7 kPa). Le résidu est repris par 100 cm³ d'eau distillée et par 300 cm³ de dichlorométhane. La phase organique est décantée puis lavée par 100 cm³ d'eau distillée, séchée sur du sulfate de magnésium, filtrée et concentrée à sec, sous pression réduite (2,7 kPa), pour donner 12,5 g d'un résidu qui est purifié par chromatographie-flash [éluant : dichlorométhane / méthanol (95 / 5 en volumes)]. On obtient un solide qui est agité dans l'éther diéthylique, filtré et séché (2,7 kPa) à 20°C, pour donner 1,4 g de (16*S*)-14-O-allyl-16-hydroxy pristinamycine II_{B} sous forme d'une poudre blanche.

Spectre de R.M.N. ¹H (400 MHz, CDCl₃, δ en ppm) : 0,97 (d, J = 6,5 Hz : 3H) ; 1,04 (d, J = 6,5 Hz : 3H) ; 1,07 (d, J = 6,5 Hz : 3H) ; de 1,70 à 2,05 (mt : 5H) ; 1,75 (s : 3H) ; 2,12 (mt : 1H) ; 2,34 (d large, J = 16 Hz : 1H) ; 2,77 (mt : 1H) ; 2,85 (dd, J = 16 et 10 Hz : 1H) ; 3,09 (dd, J = 16 et 3 Hz : 1H) ; 3,32 (mt : 1H) ; de 3,75 à 3,90 (mt : 3H) ; de 4,05 à 4,15 (mt : 2H) ; 4,42 (mt : 1H) ; 4,53 (mt : 1H) ; de 4,65 à 4,75 (mt : 3H) ; de 5,15 à 5,30 (mt : 2H) ; de 5,65 à 5,80 (mt : 1H) ; 5,70 (d large, J = 9 Hz : 1H) ; 5,80 (dd, J = 16 et 1,5 Hz : 1H) ; 5,79 (mt : 1H) ; 5,98 (mt : 1H) ; 6,25 (d, J = 16 Hz : 1H) ; 6,45 (dd, J = 16 et 4 Hz : 1H) ; 8,14 (s : 1H).

### Exemple 4

### (16R)-16-Désoxo-16-thiocyanato pristinamycine II_{B}

A 0,85 g de (16*R*)-16-désoxo-16-thiocyanato-14-O-(tert-butyldiphénylsilyl) pristinamycine II_{B} en solution dans 40 cm³ de dichlorométhane, on ajoute à 20°C, 5 cm³ de trifluorhydrate de triéthylamine. Après 20 heures d'agitation au reflux, on ajoute 3 cm³ de trifluorhydrate de triéthylamine et on maintient le reflux pendant encore 3 heures. Le mélange réactionnel est alors versé sur 80 cm³ d'eau puis neutralisé par addition lente de bicarbonate de sodium. La phase organique est décantée, séchée sur sulfate de magnésium, filtrée et concentrée à sec sous pression réduite (2,7 kPa) pour donner 0,61 g d'un solide qui est purifié par chromatographie-flash [éluant : dichlorométhane / méthanol (96 / 4 en volumes)]. On obtient 0,36 g de (16*R*)-16-désoxo-16-thiocyanato pristinamycine II_{B}, sous forme d'un solide blanc fondant vers 140°C (déc.).

Spectre de R.M.N. ¹H (400 MHz, CDCl₃, δ en ppm) : 0,96 (d, J = 6,5 Hz : 3H) ; 1,00 (d, J = 6,5 Hz : 3H) ; 1,09 (d, J = 6,5 Hz : 3H) ; de 1,75 à 2,05 (mt : 5H) ; 1,85 (s : 3H) ; de 2,10 à 2,30 (mt : 2H) ; 2,77 (mt : 1H) ; 3,15 (dd, J = 17 et 7 Hz : 1H) ; 3,40 (dd, J = 17 et 7 Hz : 1H) ; 3,52 (mt : 1H) ; 3,62 (mt : 1H) ; 3,83 (mt : 1H) ; 4,02 (mt : 1H) ; 4,52 (mt : 1H) ; de 4,75 à 4,85 (mt : 2H) ; 4,77 (dd, J = 10 et 2 Hz : 1H) ; 5,36 (d, J = 9 Hz : 1H) ; 5,77 (mt : 1H) ; 5,81 (dd, J = 16 et 2 Hz : 1H) ; 6,05 (mt : 1H) ; 6,21 (d, J = 16 Hz : 1H) ; 6,52 (dd, J = 16 et 5 Hz : 1H) ; 8,14 (s : 1H).

La (16*R*)-16-désoxo-16-thiocyanato-14-O-(tert-butyldiphénylsilyl) pristinamycine II_{B} peut être préparée de la manière suivante :

A 5,85 g de thiocyanate de tétra n-butylammonium en solution dans 70 cm³ de dichlorométhane, on ajoute à 20°C, sous atmosphère d'argon, 2,6 g de (16*S*)-16-hydroxy-14-O-(tert-butyldiphénylsilyl) pristinamycine II_{B} (préparée comme décrit dans l'exemple 1) puis, goutte à goutte, 1,56 cm³ de trifluorure de diéthylaminosulfure. Après 10 minutes d'agitation, le mélange réactionnel est dilué par 100 cm³ de dichlorométhane. La solution obtenue est lavée successivement par 2 fois 100 cm³ d'eau et 100 cm³ d'une solution aqueuse saturée de chlorure de sodium puis séchée sur sulfate de magnésium, filtrée et concentrée à sec sous pression réduite (2,7 kPa) pour donner 7,6 g d'une huile jaune foncé qui est purifiée par chromatographie-flash [éluant : cyclohexane / acétate d'éthyle (6 / 4 en volumes)]. On obtient 0,87 g de (16*R*)-16-désoxo-16-thiocyanato-14-O-(tert-butyldiphénylsilyl) pristinamycine II_{B}, sous forme d'un solide blanc.

Spectre de R.M.N. ¹H (400 MHz, CDCl₃, δ en ppm) : 0,96 (d, J = 6,5 Hz : 3H) ; 1,00 (d, J = 6,5 Hz : 3H) ; de 1,05 à 1,10 (mt : 3H) ; 1,06 (s : 9H) ; 1,28 (s : 3H) ; de 1,70 à 1,95 (mt : 4H) ; 1,96 (mt : 1H) ; de 2,10 à 2,25 (mt : 2H) ; 2,77 (mt : 1H) ; 3,04 (dd, J = 17 et 6 Hz : 1H) ; 3,27 (dd, J = 17 et 8 Hz : 1H) ; de 3,40 à 3,50 (mt : 2H) ; 3,75 (mt : 1H) ; 3,95 (mt : 1H) ; 4,53 (mt : 1H) ; 4,67 (mt : 1H) ; de 4,75 à 4,85 (mt : 2H) ; 5,35 (d, J = 9 Hz : 1H) ; 5,56 (mt : 1H) ; 5,81 (dd, J = 16 et 1,5 Hz : 1H) ; 6,04 (mt : 1H) ; 6,11 (d, J = 16 Hz : 1H) ; 6,49 (dd, J = 16 et 5 Hz : 1H) ; de 7,30 à 7,50 (mt : 6H) ; 7,63 (d large, J = 7 Hz : 2H) ; 7,69 (mt : 2H) ; 8,12 (s : 1H).

### Exemple 5

### (16R)-16-Désoxo-16-bromo pristinamycine II_{B}

En opérant d'une manière analogue à celle décrite à l'exemple 4, mais à partir de 1,2 g de (16*R*)-16-désoxo-16-bromo-14-O-(tert-butyldiphénylsilyl) pristinamycine II_{B} en solution dans 30 cm³ de dichlorométhane, on ajoute à 20°C, 7,5 cm³ de trifluorhydrate de triéthylamine. Après 25 heures d'agitation au reflux et traitement analogue à celui de l'exemple 4 on obtient un solide orangé qui est purifié par chromatographie-flash [éluant : dichlorométhane / méthanol (97 / 3 en volumes)]. On obtient 0,78 g d'un solide blanc qui est recristallisé dans 5 cm³ d'acétonitrile pour donner 0,48 g de (16*R*)-16-désoxo-16-bromo pristinamycine II_{B}, sous forme de cristaux blanc cassé fondant vers 146°C (déc.).

Spectre de R.M.N. ¹H (400 MHz, CDCl₃, δ en ppm) : 0,96 (d, J = 6,5 Hz : 3H) ; 1,01 (d, J = 6,5 Hz : 3H) ; 1,09 (d, J = 6,5 Hz : 3H) ; de 1,75 à 2,05 (mt : 5H) ; 1,90 (s : 3H) ; 2,15 (mt : 1H) ; 2,32 (mt : 1H) ; 2,77 (mt : 1H) ; 3,22 (dd, J = 17 et 7 Hz : 1H) ; 3,44 (dd, J = 17 et 7 Hz : 1H) ; 3,52 (mt : 1H) ; 3,83 (mt : 1H) ; 4,09 (mt : 1H) ; 4,37 (mt : 1H) ; 4,52 (mt : 1H) ; de 4,75 à 4,90 (mt : 2H) ; 4,79 (dd, J = 10 et 1,5 Hz : 1H) ; 5,34 (d, J = 9 Hz : 1H) ; 5,75 (mt : 1H) ; 5,82 (dd, J = 17 et 1,5 Hz : 1H) ; 6,03 (mt : 1H) ; 6,22 (d, J = 16 Hz : 1H) ; 6,52 (dd, J = 17 et 5 Hz : 1H) ; 8,11 (s : 1H).

La (16*R*)-16-désoxo-16-bromo-14-O-(tert-butyldiphénylsilyl) pristinamycine II_{B} peut être préparée de la manière suivante :

En opérant d'une manière analogue à celle décrite à l'exemple 4, mais à partir de 5,25 g de bromure de tétra n-butylammonium en solution dans 60 cm³ de dichlorométhane, on ajoute à 20°C, sous atmosphère d'argon, 2,5 g de (16*S*)-16-hydroxy-14-O-(tert-butyldiphénylsilyl) pristinamycine II_{B} (préparée comme décrit dans l'exemple 1) puis, lentement, 1,5 cm³ de trifluorure de diéthylaminosulfure. Après 10 minutes d'agitation et traitement analogue à celui de l'exemple 4, on obtient 4,6 g d'un solide jaune qui est purifié par chromatographie-flash [éluant : cyclohexane / acétate d'éthyle (6 / 4 en volumes)]. On obtient 1,38 g de (16*R*)-16-désoxo-16-bromo-14-O-(tert-butyldiphénylsilyl) pristinamycine II_{B}, sous forme d'un solide blanc.

Spectre de R.M.N. ¹H (400 MHz, CDCl₃, δ en ppm) : 0,96 (d, J = 6,5 Hz : 3H) ; 1,00 (d, J = 6,5 Hz : 3H) ; 1,07 (s : 9H) ; 1,10 (d, J = 6,5 Hz : 3H) ; 1,37 (s : 3H) ; de 1,70 à 2,05 (mt : 5H) ; 2,14 (mt : 1H) ; 2,31 (mt : 1H) ; 2,77 (mt : 1H) ; 3,12 (dd, J = 17 et 5,5 Hz : 1H) ; 3,30 (dd, J = 17 et 8 Hz : 1H) ; 3,45 (mt : 1H) ; 3,77 (mt : 1H) ; 4,03 (mt : 1H) ; 4,23 (mt : 1H) ; 4,55 (mt : 1H) ; de 4,75 à 4,85 (mt : 3H) ; 5,33 (d, J = 9 Hz : 1H) ; 5,54 (mt : 1H) ; 5,82 (d large, J = 16 Hz : 1H) ; 6,00 (mt : 1H) ; 6,12 (d, J = 16 Hz : 1H) ; 6,50 (dd, J = 16 et 5 Hz : 1H) ; de 7,30 à 7,45 (mt : 6H) ; 7,63 (d large, J = 7 Hz : 2H) ; 7,69 (d large, J = 7 Hz : 2H) ; 8,08 (s : 1H).

### Exemple 6

### (16R)-16-Désoxo-16-chloro pristinamycine II_{B}

En opérant d'une manière analogue à celle décrite à l'exemple 4, mais à partir de 1,4 g de (16*R*)-16-désoxo-16-chloro-14-O-(tert-butyldiphénylsilyl) pristinamycine II_{B} en solution dans 35 cm³ de dichlorométhane, on ajoute à 20°C, 11 cm³ de trifluorhydrate de triéthylamine. Après 20 heures d'agitation au reflux et traitement analogue à celui de l'exemple 4, on obtient un solide orangé qui est purifiée par chromatographie-flash [éluant : dichlorométhane / méthanol (97 / 3 en volumes)]. On obtient 1 g d'un solide blanc auquel on ajoute 0,5 g obtenu d'un essai identique. Après recristallisation dans 10 cm³ d'acétonitrile, on obtient 1,12 g de (16*R*)-16-désoxo-16-chloro pristinamycine II_{B}, sous forme de cristaux blanc fondant vers 142°C (déc.).

Spectre de R.M.N. ¹H (400 MHz, CDCl₃, δ en ppm) : 0,96 (d, J = 6,5 Hz : 3H) ; 1,00 (d, J = 6,5 Hz : 3H) ; 1,10 (d, J = 6,5 Hz : 3H) ; de 1,75 à 2,05 (mt : 5H) ; 1,88 (s : 3H) ; de 2,10 à 2,25 (mt : 2H) ; 2,75 (mt : 1H) ; 3,10 (dd, J = 17 et 7 Hz : 1H) ; 3,30 (dd, J = 17 et 6,5 Hz : 1H) ; 3,53 (mt : 1H) ; 3,84 (mt : 1H) ; 4,06 (mt : 1H) ; 4,37 (mt : 1H) ; 4,49 (mt : 1H) ; de 4,75 à 4,90 (mt : 3H) ; 5,35 (d, J = 9 Hz : 1H) ; 5,76 (mt : 1H) ; 5,83 (dd, J = 17 et 2 Hz : 1H) ; 5,99 (mt : 1H) ; 6,22 (d, J = 15,5 Hz : 1H) ; 6,51 (dd, J = 17 et 5 Hz : 1H); 8,09 (s : 1H).

La (16*R*)-16-désoxo-16-chloro-14-O-(tert-butyldiphénylsilyl) pristinamycine II_{B} peut être préparée de la manière suivante :

En opérant d'une manière analogue à celle décrite à l'exemple 4, mais à partir de 7,27 g de chlorure de tétra n-butylammonium en solution dans 50 cm³ de dichlorométhane, on ajoute à 20°C, sous atmosphère d'argon, 4 g de (16*S*)-16-hydroxy-14-O-(tert-butyldiphénylsilyl) pristinamycine II_{B} (préparée comme décrit dans l'exemple 1) puis, goutte à goutte, 2,44 cm³ de trifluorure de diéthylaminosulfure. Après 10 minutes d'agitation et traitement analogue à celui de l'exemple 4, on obtient 4,45 g d'un solide jaune qui est purifié par chromatographie-flash [éluant : cyclohexane / acétate d'éthyle (55 / 45 en volumes)]. On obtient 0,51 g de (16R)-16-désoxo-16-chloro-14-O-(tert-butyldiphénylsilyl) pristinamycine II_{B}, sous forme d'un solide blanc.

Spectre de R.M.N. ¹H (400 MHz, CDCl₃, δ en ppm) : 0,96 (d, J = 6,5 Hz : 3H) ; 1,00 (d, J = 6,5 Hz : 3H) ; 1,07 (s : 9H) ; 1,10 (d, J = 6,5 Hz : 3H) ; 1,33 (s : 3H) ; de 1,70 à 1,90 (mt : 4H) ; 1,96 (mt : 1H) ; de 2,05 à 2,25 (mt : 2H) ; 2,77 (mt : 1H) ; 2,98 (dd, J = 17 et 5,5 Hz : 1H) ; 3,16 (dd, J = 17 et 8 Hz : 1H) ; 3,45 (mt : 1H) ; 3,76 (mt : 1H) ; 4,03 (mt : 1H) ; 4,25 (mt : 1H) ; 4,55 (mt : 1H) ; de 4,75 à 4,85 (mt : 3H) ; 5,34 (d, J = 9 Hz : 1H) ; 5,54 (mt : 1H) ; 5,82 (dd, J = 16 et 2 Hz : 1H) ; 5,99 (mt : 1H) ; 6,11 (d, J = 16 Hz : 1H) ; 6,50 (dd, J = 16 et 5 Hz : 1H) ; de 7,30 à 7,45 (mt : 6H) ; 7,63 (dd, J = 7 et 1,5 Hz : 2H) ; 7,68 (dd, J = 7 et 1,5 Hz : 2H) ; 8,08 (s : 1H).

### Exemple 7

### (16R)-16-Désoxo-16-iodo pristinamycine II_{B}

En opérant d'une manière analogue à celle décrite à l'exemple 4, mais à partir de 0,83 g de (16*R*)-16-désoxo-16-iodo-14-O-(tert-butyldiphénylsilyl) pristinamycine II_{B} en solution dans 15 cm³ de dichlorométhane, on ajoute à 20°C, 6,2 cm³ de trifluorhydrate de triéthylamine. Après 23 heures d'agitation au reflux et traitement analogue à celui de l'exemple 4, on obtient 0,9 g d'un solide blanc cassé qui est purifié par chromatographie-flash [éluant : dichlorométhane / méthanol / acétonitrile (98 / 1 / 1 en volumes)]. On obtient 0,41 g d'un solide jaune qui est purifié par chromatographie liquide haute performance sur colonne Kromasil® de silice C18 5 µm (diamètre colonne = 2 cm, longueur colonne = 25 cm) [éluant : gradient eau / acétonitrile (80 / 20 à 60 / 40 en volumes)]. On obtient 0,18 g d'un solide blanc auquel on ajoute 0,12 g obtenus d'essais équivalents pour donner 0,3 g de (16*R*)-16-désoxo-16-iodo pristinamycine II_{B}, sous forme d'un solide beige fondant vers 140°C (déc.).

Spectre de R.M.N. ¹H (400 MHz, CDCl₃, δ en ppm) : 0,96 (d, J = 6,5 Hz : 3H) ; 1,01 (d, J = 6,5 Hz : 3H) ; 1,09 (d, J = 6,5 Hz : 3H) ; 1,67 (d, J = 3 Hz : 1H) ; 1,73 (mt : 1H) ; de 1,80 à 2,05 (mt : 4H) ; 1,94 (s : 3H) ; 2,17 (mt : 1H) ; 2,36 (mt : 1H) ; 2,77 (mt : 1H) ; 3,31 (dd, J = 17 et 7 Hz : 1H) ; de 3,45 à 3,60 (mt : 1H) ; 3,53 (dd, J = 17 et 7 Hz : 1H) ; 3,85 (mt : 1H) ; 4,10 (mt : 1H) ; 4,32 (mt : 1H) ; 4,53 (mt : 1H) ; de 4,75 à 4,85 (mt : 3H) ; 5,31 (d, J = 9 Hz : 1H) ; 5,76 (mt : 1H) ; 5,82 (dd, J = 17 et 1,5 Hz : 1H) ; 6,00 (mt : 1H) ; 6,21 (d, J = 16 Hz : 1H) ; 6,51 (dd, J = 17 et 5 Hz : 1H) ; 8,11 (s : 1H).

La (16*R*)-16-désoxo-16-iodo-14-O-(tert-butyldiphénylsilyl) pristinamycine II_{B} peut être préparée de la manière suivante :

En opérant d'une manière analogue à celle décrite à l'exemple 4, mais à partir de 7,2 g d'iodure de tétra n-butylammonium en solution dans 60 cm³ de dichlorométhane, on ajoute à 20°C, sous atmosphère d'argon, 3 g de (16*S*)-16-hydroxy-14-O-(tert-butyldiphénylsilyl) pristinamycine II_{B} (préparée comme décrit dans l'exemple 1) puis, goutte à goutte à 0°C, 1,8 cm³ de trifluorure de diéthylaminosulfure. Après 20 minutes d'agitation à 0°C et traitement analogue à celui de l'exemple 4, on obtient 10,5 g d'une huile orange qui est agitée pendant 15 minutes dans 30 cm³ d'un mélange cyclohexane / acétate d'éthyle (60 / 40). Après filtration et concentration du filtrat sous pression réduite (2,7 kPa), on obtient 5,5 g d'un solide jaune qui est purifié par chromatographie-flash [éluant : cyclohexane / acétate d'éthyle (60 / 40 en volumes)].

On obtient 1,81 g de (16*R*)-16-désoxo-16-iodo-14-O-(tert-butyldiphénylsilyl) pristinamycine II_{B}, sous forme d'un solide blanc fondant vers 105°C (déc.).

Spectre de R.M.N. ¹H (400 MHz, CDCl₃, δ en ppm) : 0,96 (d, J = 6,5 Hz : 3H) ; 1,00 (d, J = 6,5 Hz : 3H) ; 1,05 (s : 9H) ; 1,09 (d, J = 6,5 Hz : 3H) ; 1,41 (s : 3H) ; de 1,70 à 1,95 (mt : 4H) ; 1,96 (mt : 1H) ; 2,14 (mt : 1H) ; 2,33 (mt : 1H) ; 2,77 (mt : 1H) ; 3,20 (dd, J = 17 et 6 Hz : 1H) ; 3,38 (dd, J = 17 et 8 Hz : 1H) ; 3,45 (mt : 1H) ; 3,79 (mt : 1H) ; 4,03 (mt : 1H) ; 4,17 (mt : 1H) ; 4,54 (mt : 1H) ; 4,71 (dt, J = 9 et 3 Hz : 1H) ; 4,78 (dd, J = 10 et 2,5 Hz : 1H) ; de 4,75 à 4,85 (mt : 1H) ; 5,30 (d, J = 9 Hz : 1H) ; 5,55 (mt : 1H) ; 5,81 (dd, J = 17 et 2 Hz : 1H) ; 5,99 (mt : 1H) ; 6,11 (d, J = 16 Hz : 1H) ; 6,49 (dd, J = 17 et 5 Hz : 1H) ; de 7,30 à 7,45 (mt : 6H) ; 7,63 (dd, J = 7 et 1,5 Hz : 2H) ; 7,69 (dd, J = 7 et 1,5 Hz : 2H) ; 8,08 (s : 1H).

### Exemple 8

### (16R)-16-Désoxo-16-fluoro pristinamycine II_{A}

A 0,97 g de (16*R*)-16-désoxo-16-fluoro-14-O-(tert-butyldiphénylsilyl) pristinamycine II_{A} en solution dans 8 cm³ de tétrahydrofurane, on ajoute à 20°C, sous atmosphère d'argon, 0,2 cm³ d'acide acétique et 0,6 g de fluorure de tétra n-butylammonium trihydraté. Après 168 heures d'agitation, le mélange réactionnel est versé sur 50 cm³ d'une solution aqueuse saturée par du bicarbonate de sodium. La phase organique est décantée et la phase aqueuse est extraite par 30 cm³ de dichlorométhane. Les phases organiques sont réunies, séchées sur sulfate de magnésium, filtrées puis concentrées à sec sous pression réduite (2,7 kPa) pour donner un résidu qui est purifié par chromatographie-flash [éluant : dichlorométhane / méthanol / acétonitrile (95 / 2,5 / 2,5 en volumes)]. On obtient 0,38 g d'un solide jaune auquel on ajoute 0,21 g obtenu d'un essai identique. Après agitation dans l'éther, filtration et séchage sous pression réduite (2,7 kPa) à 20°C, on obtient 0,58 g de (16*R*)-16-désoxo-16-fluoro pristinamycine II_{A}, sous forme d'un solide jaune clair fondant vers 110°C (déc.).

Spectre de R.M.N. ¹H (400 MHz, CDCl₃, δ en ppm) : 0,99 (mt : 6H) ; 1,14 (d, J = 6,5 Hz : 3H) ; 1,77 (s : 3H) ; de 1,85 à 2,10 (mt : 2H) ; 2,28 (mt : 1H) ; de 2,65 à 2,90 (mt : 3H) ; 3,08 (mt : 1H) ; 3,25 (mt : 1H) ; 3,98 (d large, J = 17 Hz : 1H) ; de 4,10 à 4,25 (mt : 2H) ; 4,31 (mt : 1H) ; de 4,55 à 4,80 (mt : 2H) ; de 4,90 à 5,00 (mt : 2H) ; 5,69 (mt : 1H) ; 5,96 (d, J = 16 Hz : 1H) ; 5,99 (d, J = 16 Hz : 1H) ; 6,17 (t large, J = 3 Hz : 1H) ; 6,61 (dd, J = 16 et 7 Hz : 1H) ; 7,06 (mt : 1H) ; 7,93 (s : 1H).

La (16*R*)-16-désoxo-16-fluoro-14-O-(tert-butyldiphénylsilyl) pristinamycine II_{A} peut être préparée de la manière suivante :

A 65 g de (16*S*)-16-hydroxy-14-O-(tert-butyldiphénylsilyl) pristinamycine II_{A} en solution dans 700 cm³ de dichlorométhane, on ajoute lentement à 20°C, sous atmosphère d'argon, 20,5 cm³ de trifluorure de diéthylaminosulfure. Après 3 heures d'agitation, le mélange réactionnel est versé lentement sur 1000 cm³ d'une solution aqueuse saturée de bicarbonate de sodium. La phase organique est décantée et la phase aqueuse est extraite par 2 fois 500 cm³ de dichlorométhane. Les phases organiques sont réunies, séchées sur sulfate de magnésium, filtrées puis concentrées à sec sous pression réduite (2,7 kPa) pour donner 67,62 g d'un solide orange. Après agitation de ce solide dans du pentane, filtration et séchage sous pression réduite (2,7 kPa) à 20°C, on obtient 65,55 g de (16*R*)-16-désoxo-16-fluoro-14-O-(tert-butyldiphénylsilyl) pristinamycine II_{A}, sous forme d'un solide jaune orange.

Spectre de R.M.N. ¹H (400 MHz, CDCl₃, δ en ppm) : de 0,95 à 1,05 (mt : 6H) ; 1,05 (s : 9H) ; 1,13 (d, J = 6,5 Hz : 3H) ; 1,28 (s : 3H) ; de 1,80 à 2,10 (mt : 2H) ; de 2,10 à 2,30 (mt : 1H) ; de 2,65 à 2,85 (mt : 3H) ; de 2,90 à 3,15 (mt : 2H) ; de 3,95 à 4,10 (mt : 2H) ; 4,16 (mt : 1H) ; 4,28 (mt : 1H) ; de 4,40 à 4,60 (mt : 2H) ; 4,99 (dd, J = 10 et 1,5 Hz : 1H) ; 5,05 (d, J = 9 Hz : 1H) ; 5,50 (mt : 1H) ; 5,90 (d, J = 16 Hz : 1H) ; 6,00 (d large, J = 17 Hz : 1H) ; 6,16 (t, J = 3 Hz : 1H) ; 6,61 (dd, J = 17 et 7 Hz : 1H) ; 7,02 (t, J = 5,5 Hz : 1H) ; de 7,25 à 7,50 (mt : 6H) ; 7,58 (d large, J = 7 Hz : 2H) ; 7,67 (d large, J = 7 Hz : 2H) ; 7,89 (s : 1H).

La (16*S*)-16-hydroxy-14-O-(tert-butyldiphénylsilyl) pristinamycine II_{A} peut être préparée de la manière suivante :

En opérant d'une manière analogue à celle décrite à l'exemple 1, mais à partir de 10 g de (16*S*)-16-hydroxy pristinamycine II_{A} en solution dans 100 cm³ de dichlorométhane, on ajoute à 20°C, sous atmosphère d'argon, 13,2 cm' de diisopropyléthylamine, 19,7 cm³ de tert-butyldiphénylchlorosilane goutte à goutte et 0,46 g de 4-diméthylaminopyridine. Après 20 heures d'agitation et traitement analogue à celui de l'exemple 1, on obtient 35 g d'une huile marron qui est purifiée par chromatographie-flash [éluant : dichlorométhane / méthanol / acétonitrile (96 / 2 / 2 en volumes)]. On obtient 10,5 g de (16*S*)-16-hydroxy-14-O-(tert-butyldiphénylsilyl) pristinamycine II_{A}, sous forme d'un solide beige.

Spectre de R.M.N. ¹H (400 MHz, CDCl₃, δ en ppm) : de 0,90 à 1,05 (mt : 6H) ; de 1,05 à 1,15 (mt : 15H) ; de 1,90 à 2,05 (mt : 3H) ; 2,54 (mt : 1H) ; 2,66 (mt : 1H) ; 2,76 (mt : 1H) ; 2,82 (dd, J = 16 et 11 Hz : 1H) ; 3,11 (dd, J = 16 et 3 Hz : 1H) ; 3,28 (mt : 1H) ; 3,83 (s large : 1H) ; de 4,00 à 4,15 (mt : 2H) ; 4,50 (mt : 1H) ; 4,57 (mt : 1H) ; 4,81 (mt : I H) ; 4,94 (dd, J = 10 et 2 Hz : 1H) ; 5,38 (mt : 1H) ; 5,63 (d, J = 9 Hz : 1H) ; 5,88 (dd, J = 16 et 1,5 Hz : 1H) ; 5,94 (d, J = 16 Hz : 1H) ; 6,01 (t, J = 3 Hz : 1H) ; 6,34 (mt : 1H) ; 6,47 (dd, J = 16 et 5 Hz : 1H) ; de 7,25 à 7,50 (mt : 6H) ; 7,62 (dd, J = 7 et 1,5 Hz : 2H) ; 7,68 (dd, J = 7 et 1,5 Hz : 2H) ; 8,06 (s : 1H).

La (16*S*)-16-hydroxy pristinamycine II_{A} peut être préparée selon F. Le Goffic et coll.; Eur. J. Med. - Chimica Therapeutica ; January - February, - 16(1), 69-72 (1981).

### Exemple 9

Méthanesulfonate de la (16*R*)-16-Désoxo-16-fluoro-14-O-[4-(morpholin-4-yl méthyl) benzoyl] pristinamycine II_{B}

A 0,15 g de (16*R*)-16-désoxo-16-fluoro-14-O-[4-(morpholin-4-yl méthyl) benzoyl] pristinamycine II_{B} en solution dans 5 cm' de méthanol, on ajoute à 20°C, 2 cm³ d'une solution éthanolique d'acide méthanesulfonique 0,1 N. Après 10 minutes d'agitation, le mélange réactionnel est concentré à sec sous pression réduite (2,7 kPa) pour donner un résidu blanc qui est agité dans 10 cm³ d'éther. Après filtration, rinçage du solide par 10 cm³ d'éther diéthylique et séchage sous pression réduite (2,7 kPa) à 20°C, on obtient 0,15 g du méthanesulfonate de la (16*R*)-16-désoxo-16-fluoro-14-O-[4-(morpholin-4-yl méthyl) benzoyl] pristinamycine II_{B}, sous forme d'un solide blanc fondant vers 150°C (déc.).

Spectre de R.M.N. ¹H (400 MHz, (CD₃)₂SO d6, δ en ppm) : 0,85 (d, J = 6,5 Hz : 3H); 0,95 (d, J = 6,5 Hz : 3H) ; 1,05 (d, J = 6,5 Hz : 3H) ; 1,54 (mt : 1H) ; de 1,75 à 2,00 (mt : 3H) ; 1,86 (s : 3H) ; de 2,05 à 2,25 (mt : 2H) ; de 2,30 à 2,45 (mt : 1H) ; 2,32 (s : 3H) ; 2,78 (mt : 1H) ; de 3,05 à 3,45 (mt : 6H) ; de 3,55 à 3,70 (mt : 3H) ; 3,72 (mt : 1H) ; 3,84 (mt : 1H) ; de 3,90 à 4,05 (mt : 3H) ; 4,47 (mf : 2H) ; de 4,70 à 4,80 (mt : 2H) ; 5,18 (doublet démultiplié, J _{HF} = 48 Hz : 1H) ; 5,55 (d, J = 9,5 Hz : 1H) ; 5,66 (mt : 1H) ; 5,81 (d large, J = 16 Hz : 1H) ; 6,00 (mt : 1H) ; 6,21 (d, J = 16 Hz : 1H) ; 6,64 (dd, J = 16 et 5 Hz : 1H) ; 7,68 (d large, J = 7,5 Hz : 2H) ; 8,08 (d large, J = 7,5 Hz : 2H) ; 8,16 (mt : 1H) ; 8,55 (s : 1H) ; 9,95 (mf : 1H).

La (16*R*)-16-désoxo-16-fluoro-14-O-[4-(morpholin-4-yl méthyl) benzoyl] pristinamycine II_{B} peut être préparée de la manière suivante :

A 2 g de (16*R*)-16-désoxo-16-fluoro-14-O-[4-(chlorométhyl) benzoyl] pristinamycine II_{B} en solution dans 50 cm³ de tétrahydrofurane, on ajoute à 20°C, 0,44 g d'iodure de sodium et 0,5 cm³ de morpholine. Après 17 heures d'agitation à reflux, le mélange réactionnel est versé sur 50 cm³ d'eau puis extrait par 2 fois 50 cm³ de dichlorométhane. Les phases organiques sont réunies, lavées par 2 fois 100 cm³ d'eau, séchées sur sulfate de magnésium, filtrées puis concentrées à sec sous pression réduite (2,7 kPa) pour donner 2,5 g d'un résidu qui est purifié par chromatographie-flash [éluant : gradient dichlorométhane / méthanol (100 / 0, 99 / 1 et 98 / 2 en volumes)]. On obtient un solide qui est agité dans du pentane, filtré et séché (90 Pa) à 30°C, pour donner 1,69 g de (16*R*)-16-désoxo-16-fluoro-14-O-[4-(morpholin-4-yl méthyl) benzoyl] pristinamycine II_{B}, sous forme d'un solide cassé fondant vers 120°C (déc.).

Spectre de R.M.N. ¹H (400 MHz, CDCl₃, δ en ppm) : 0,96 (d, J = 6,5 Hz : 3H) ; 1,01 (d, J = 6,5 Hz : 3H) ; 1,10 (d, J = 6,5 Hz : 3H) ; de 1,75 à 2,05 (mt : 5H) ; 1,95 (s : 3H) ; 2,16 (mt : 1H) ; de 2,30 à 2,50 (mt : 1H) ; 2,44 (mt : 4H) ; 2,76 (mt : 1H) ; 3,05 (dt, J = 17 et 6 Hz : 1H) ; 3,29 (mt : 1H) ; 3,49 (mt : 1H) ; 3,55 (s : 2H) ; 3,71 (mt : 4H) ; 3,89 (mt : 1H) ; 4,09 (mt : 1H) ; 4,52 (mt : 1H) ; 4,79 (d large, J = 10 Hz : 1H) ; 4,83 (dd, J = 9 et 3 Hz : 1H) ; 5,18 (doublet démultiplié, J _{HF} = 48 Hz : 1H) ; 5,45 (d, J = 9,5 Hz : 1H) ; de 5,70 à 5,85 (mt : 1H) ; 5,82 (d large, J = 17 Hz : 1H) ; 5,94 (mt : 1H) ; 6,06 (dt, J = 9,5 et 5 Hz : 1H) ; 6,22 (d, J = 16 Hz : 1H) ; 6,52 (dd, J = 17 et 5 Hz : 1H) ; 7,41 (d, J = 8 Hz : 2H) ; 7,98 (d, J = 8 Hz : 2H) ; 8,14 (s : 1H).

La (16*R*)-16-désoxo-16-fluoro-14-O-[4-(chlorométhyl) benzoyl] pristinamycine II_{B} peut être préparée de la manière suivante :

A 5,32 g de (16*R*)-16-désoxo-16-fluoro pristinamycine II_{B} (préparée comme décrit dans l'exemple 1) en solution dans 150 cm' de dichlorométhane et 2,1 cm³ de triéthylamine, on ajoute à 10°C, 2,84 g de chlorure de 4-(chlorométhyl) benzoyle et 0,095 g de 4-diméthylaminopyridine. Après 20 heures d'agitation à 20°C, le mélange réactionnel est successivement lavé par 50 cm³ d'eau, 50 cm³ d'une solution aqueuse à 5% de bicarbonate de sodium, 30 cm³ d'eau et 30 cm³ d'une solution aqueuse saturée de chlorure de sodium. La phase organique est séchée sur sulfate de magnésium, filtrée puis concentrée à sec sous pression réduite (2,7 kPa) pour donner 7,2 g d'un résidu beige qui est purifié par chromatographie-flash [éluant : gradient dichlorométhane / méthanol / acétonitrile (99 / 0,5 / 0,5 et 98 / 1 / 1 en volumes)]. On obtient 4,5 g de (16*R*)-16-désoxo-16-fluoro-14-O-[4-(chlorométhyl) benzoyl] pristinamycine II_{B}, sous forme d'un solide jaune clair.

Spectre de R.M.N. ¹H (400 MHz, CDCl₃, δ en ppm) : 0,96 (d, J = 6,5 Hz : 3H) ; 1,01 (d, J = 6,5 Hz : 3H) ; 1,10 (d, J = 6,5 Hz : 3H) ; de 1,75 à 2,05 (mt : 5H) ; 1,95 (s : 3H) ; 2,16 (mt : 1H) ; 2,40 (mt : 1H) ; 2,76 (mt : 1H) ; 3,04 (dt, J = 17 et 6 Hz : 1H) ; 3,29 (mt : 1H) ; 3,49 (mt : 1H) ; 3,88 (mt : 1H) ; 4,09 (mt : 1H) ; 4,53 (mt : 1H) ; 4,62 (s : 2H) ; 4,78 (dd, J = 10 et 2 Hz : 1H) ; 4,83 (dd, J = 9 et 3 Hz : 1H) ; 5,18 (doublet démultiplié, J _{HF} = 48 Hz : 1H) ; 5,44 (d, J = 9,5 Hz : 1H) ; de 5,75 à 5,85 (mt : 1H) ; 5,82 (dd, J = 17 et 2 Hz : 1H) ; 5,94 (mt : 1H) ; 6,06 (dt, J = 9,5 et 5 Hz : 1H) ; 6,22 (d, J = 16 Hz : 1H) ; 6,52 (dd, J = 17 et 5 Hz : 1H) ; 7,47 (d, J = 8 Hz : 2H) ; 8,02 (d, J = 8 Hz : 2H) ; 8,14 (s : 1H).

### Exemple 10

### (16R)-16-désoxo-16-fluoro-14-O-[4-(imidazol-1-yl méthyl) benzoyl] pristinamycine II_{B}

En opérant d'une manière analogue à celle décrite à l'exemple 9, mais à partir de 0,499 g de (16*R*)-16-désoxo-16-fluoro-14-O-[4-(chlorométhyl) benzoyl] pristinamycine II_{B} (préparée comme décrit dans l'exemple 9) en solution dans 30 cm³ de tétrahydrofurane, on ajoute à 20°C, 0,11 g d'iodure de sodium et 0,099 g d'imidazole. Après 17 heures d'agitation à reflux et traitement analogue à celui de l'exemple 9, on obtient 0,5 g d'un solide jaune qui est purifié par chromatographie-flash [éluant : dichlorométhane / méthanol (97 / 3 en volumes)]. On obtient un solide qui est agité dans l'éther, filtré et séché (90 Pa) à 20°C, pour donner 0,289 g de (16*R*)-16-désoxo-16-fluoro-14-O-[4-(imidazol-1-yl méthyl) benzoyl] pristinamycine II_{B}, sous forme d'un solide jaune fondant vers 136°C (déc.).

Spectre de R.M.N. ¹H (400 MHz, CDCl₃, δ en ppm) : 0,96 (d, J = 6,5 Hz : 3H) ; 1,01 (d, J = 6,5 Hz : 3H) ; 1,10 (d, J = 6,5 Hz : 3H) ; de 1,75 à 2,05 (mt : 5H) ; 1,95 (s : 3H) ; 2,16 (mt : 1H) ; 2,39 (mt : 1H) ; 2,76 (mt : 1H) ; 3,04 (td, J = 17 et 6 Hz : 1H) ; 3,28 (mt : 1H) ; 3,49 (mt : 1H) ; 3,87 (mt : 1H) ; 4,08 (mt : 1H) ; 4,53 (mt : 1H) ; 4,78 (d large, J = 10 Hz : 1H) ; 4,83 (dd, J = 9 et 3 Hz : 1H) ; 5,17 (doublet démultiplié, J _{HF} = 48 Hz : 1H) ; 5,19 (s : 2H) ; 5,43 (d, J = 9 Hz : 1H) ; 5,78 (mt : 1H) ; 5,82 (dd, J = 17 et 1,5 Hz : 1H) ; 5,94 (mt : 1H) ; 6,05 (mt : 1H) ; 6,22 (d, J = 16 Hz : 1H) ; 6,52 (dd, J = 17 et 5 Hz : 1H) ; 6,90 (s : 1H) ; 7,13 (s : 1H) ; 7,20 (d, J = 8 Hz : 2H) ; 7,58 (s : 1H) ; 8,01 (d, J = 8 Hz : 2H) ; 8,14 (s : 1H).

### Exemple 11

### (16R)-16-Désoxo-16-fluoro-14-O-[4-(4-méthylpipérazin-1-yl méthyl) benzoyl] pristinamycine II_{B}

En opérant d'une manière analogue à celle décrite à l'exemple 9, mais à partir de 2 g de (16*R*)-16-désoxo-16-fluoro-14-O-[4-(chlorométhyl) benzoyl] pristinamycine II_{B} (préparée comme décrit dans l'exemple 9) en solution dans 90 cm³ de tétrahydrofurane, on ajoute à 20°C, 0,44 g d'iodure de sodium et 0,65 cm³ de méthylpipérazine en solution dans 10 cm³ de tétrahydrofurane. Après 20 heures d'agitation à reflux et traitement analogue à celui de l'exemple 9, on obtient 2,4 g d'un résidu qui est purifié par chromatographie-flash [éluant : dichlorométhane / méthanol (99 / 1 en volumes)]. On obtient un solide qui est agité dans 15 cm³ d'éther, filtré et séché (90 Pa) à 20°C, pour donner 1 g de (16*R*)-16-désoxo-16-fluoro-14-O-[4-(4-méthylpipérazin-1-yl méthyl) benzoyl] pristinamycine II_{B}, sous forme d'un solide jaune pâle fondant vers 160°C (déc.).

Spectre de R.M.N. ¹H (400 MHz, CDCl₃, δ en ppm) : 0,96 (d, J = 6,5 Hz : 3H) ; 1,01 (d, J = 6,5 Hz : 3H) ; 1,10 (d, J = 6,5 Hz : 3H) ; de 1,75 à 2,25 (mt : 6H) ; 1,95 (s : 3H) ; de 2,30 à 2,50 (mt : 1H) ; 2,40 (s : 3H) ; 2,62 (mf : 8H) ; 2,76 (mt : 1H) ; 3,04 (dt, J = 17 et 6 Hz : 1H) ; 3,29 (mt : 1H) ; 3,49 (mt : 1H) ; 3,59 (s : 2H) ; 3,88 (mt : 1H) ; 4,08 (mt : 1H) ; 4,52 (mt : 1H) ; 4,79 (d large, J = 10 Hz : 1H) ; 4,83 (dd, J = 9 et 3 Hz : 1H) ; 5,18 (doublet démultiplié, J _{HF} = 48 Hz : 1H) ; 5,44 (d, J = 9,5 Hz : 1H) ; de 5,70 à 5,85 (mt : 1H) ; 5,82 (dd, J = 17 et 1,5 Hz : 1H) ; 5,95 (mt : 1H) ; 6,05 (dt, J = 9,5 et 6 Hz : 1H) ; 6,22 (d, J = 16 Hz : 1H) ; 6,52 (dd, J = 17 et 5 Hz : 1H) ; 7,40 (d, J = 8,5 Hz : 2H) ; 7,97 (d, J = 8,5 Hz : 2H) ; 8,14 (s : 1H).

### Exemple 12

### (16R)-16-Désoxo-16-fluoro-14-O-[4-(diéthylaminométhyl) benzoyl] pristinamycine II_{B}

En opérant d'une manière analogue à celle décrite à l'exemple 9, mais à partir de 0,55 g de (16*R*)-16-désoxo-16-fluoro-14-O-[4-(chlorométhyl) benzoyl] pristinamycine II_{B} (préparée comme décrit dans l'exemple 9) en solution dans 30 cm³ de tétrahydrofurane, on ajoute à 20°C, 0,12 g d'iodure de sodium et 0,17 cm³ de diéthylamine. Après 15,5 heures d'agitation à reflux et traitement analogue à celui de l'exemple 9, on obtient un résidu qui est purifié par chromatographie-flash [éluant : dichlorométhane / méthanol (95 / 5 en volumes)]. On obtient un solide qui est agité dans 20 cm³de pentane, filtré et séché (90 Pa) à 20°C, pour donner 0,14 g de (16*R*)-16-désoxo-16-fluoro-14-O-[4-(diéthylaminométhyl) benzoyl] pristinamycine II_{B}, sous forme d'un solide jaune pâle fondant vers 150°C (déc.).

Spectre de R.M.N. ¹H (400 MHz, CDCl₃, δ en ppm) : 0,96 (d, J = 6,5 Hz : 3H) ; 1,03 (mt : 9H) ; 1,09 (d, J = 6,5 Hz : 3H) ; de 1,75 à 2,05 (mt : 5H) ; 1,95 (s : 3H) ; 2,16 (mt : 1H) ; 2,40 (mt : 1H) ; 2,52 (q, J = 7 Hz : 4H) ; 2,77 (mt : 1H) ; 3,05 (td, J = 17 et 6 Hz : 1H) ; 3,28 (mt : 1 H) ; 3,48 (mt : 1H) ; 3,61 (s : 2H) ; 3,89 (mt : 1H) ; 4,09 (mt : 1H) ; 4,53 (mt : 1H) ; 4,79 (d large, J = 10 Hz : 1H) ; 4,82 (dd, J = 9 et 3 Hz : 1H) ; 5,17 (doublet démultiplié, J _{HF} = 48 Hz : 1H) ; 5,44 (d, J = 9 Hz : 1H) ; 5,77 (mt: 1H) ; 5,82 (dd, J = 17 et 1,5 Hz : 1H) ; 5,92 (mt : 1H) ; 6,05 (mt : 1H) ; 6,21 (d, J = 6 Hz : 1H) ; 6,50 (dd, J = 16 et 5 Hz : 1H) ; 7,42 (d, J = 8 Hz : 2H) ; 7,96 (d, J = 8 Hz : 2H) ; 8,14 (s : 1H).

### Exemple 13

### (16R)-16-Désoxo-16-fluoro-14-O-{4-[((2S)-2-hydroxyméthyl)pyrrolidin-1-yl méthyl] benzoyl} pristinamycine II_{B}

En opérant d'une manière analogue à celle décrite à l'exemple 9, mais à partir de 1 g de (16*R*)-16-désoxo-16-fluoro-14-O-[4-(chlorométhyl) benzoyl] pristinamycine II_{B} (préparée comme décrit dans l'exemple 9) en solution dans 45 cm³ de tétrahydrofurane, on ajoute à 20°C, 0,22 g d'iodure de sodium et 0,29 cm³ de L-prolinol en solution dans 5 cm³ de tétrahydrofurane. Après 12 heures d'agitation à reflux et traitement analogue à celui de l'exemple 9, on obtient 1,25 g d'un résidu qui est purifié par chromatographie-flash [éluant : dichlorométhane / méthanol (98 / 2 en volumes)]. On obtient un solide qui est agité dans 10 cm³ d'éther, filtré et séché (90 Pa) à 20°C, pour donner 0,4 g de (16*R*)-16-désoxo-16-fluoro-14-O-{4-[((2S)-2-hydroxyméthyl) pyrrolidin-1-yl méthyl] benzoyl} pristinamycine II_{B}, sous forme d'un solide blanc fondant vers 158°C (déc.).

Spectre de R.M.N. ¹H (400 MHz, CDCl₃, δ en ppm) : 0,96 (d, J = 6,5 Hz : 3H) ; 1,01 (d, J = 6,5 Hz : 3H) ; 1,10 (d, J = 6,5 Hz : 3H) ; de 1,55 à 2,05 (mt : 8H) ; 1,96 (s : 3H) ; 2,17 (mt : 1H) ; 2,29 (mt : 1H) ; 2,41 (mt : 1H) ; 2,77 (mt : 2H) ; 2,96 (mt : 1H) ; 3,05 (td, J = 17 et 6 Hz : 1H) ; 3,29 (mt : 1H) ; de 3,40 à 3,55 (mt : 3H) ; 3,66 (dd, J = 11 et 4 Hz : 1H) ; 3,88 (mt : 1H) ; 4,02 (d, J = 13 Hz : 1H) ; 4,09 (mt : 1H) ; 4,53 (mt : 1H) ; 4,79 (dd, J = 10 et 2 Hz : 1H) ; 4,83 (dd, J = 9 et 3 Hz : 1H) ; 5,18 (doublet démultiplié, J _{HF} = 48 Hz : 1H) ; 5,45 (d, J = 9,5 Hz : 1H) ; 5,78 (mt : 1H) ; 5,82 (dd, J = 16 et 2 Hz : 1H) ; 5,95 (mt : 1 H) ; 6,05 (mt : 1H) ; 6,22 (d, J = 16 Hz : 1H) ; 6,52 (dd, J = 16 et 5 Hz : 1H) ; 7,38 (d, J = 8 Hz : 2H) ; 7,98 (d, J = 8 Hz : 2H) ; 8,14 (s : 1H).

### Exemple 14

### (16R)-16-Désoxo-16-fluoro-14-O-[4-(2-picolylaminométhyl) benzoyl] pristinamycine II_{B}

En opérant d'une manière analogue à celle décrite à l'exemple 9, mais à partir de 2 g de (16*R*)-16-désoxo-16-fluoro-14-O-[4-(chlorométhyl) benzoyl] pristinamycine II_{B} (préparée comme décrit dans l'exemple 9) en solution dans 20 cm³ de tétrahydrofurane, on ajoute à 20°C, 0,44 g d'iodure de sodium et 1,2 cm³ de 2-(aminométhyl) pyridine. Après 17 heures d'agitation à reflux et traitement analogue à celui de l'exemple 9, on obtient 2,1 g d'un solide jaune foncé qui est purifié par deux chromatographies-flash successives [éluant : respectivement dichlorométhane / méthanol / acétonitrile (92 / 4 / 4 et 95 / 2,5 / 2,5, en volumes)]. On obtient un solide qui est agité dans l'éther, filtré et séché (90 Pa) à 20°C, pour donner 1,59 g de (16R)-16-désoxo-16-fluoro-14-O-[4-(2-picolylaminométhyl) benzoyl] pristinamycine II_{B}, sous forme d'un solide blanc fondant vers 106°C (déc.).

Spectre de R.M.N. ¹H (400 MHz, CDCl₃, δ en ppm) : 0,96 (d, J = 6,5 Hz : 3H) ; 1,01 (d, J = 6,5 Hz : 3H) ; 1,10 (d, J = 6,5 Hz : 3H) ; de 1,75 à 2,05 (mt : 5H) ; 1,95 (s : 3H) ; 2,16 (mt : 1H) ; 2,40 (mt : 1H) ; 2,77 (mt : 1H) ; 3,05 (dt, J = 17 et 6 Hz : 1H) ; 3,29 (mt : 1H) ; 3,49 (mt : 1H) ; 3,90 (mt : 1H) ; 3,92 (s : 2H) ; 3,93 (s : 2H) ; 4,10 (mt : 1H) ; 4,53 (mt : 1H) ; 4,79 (dd, J = 10 et 2 Hz : 1H) ; 4,83 (dd, J = 9 et 3,5 Hz : 1H) ; 5,18 (doublet démultiplié, J _{HF} = 48 Hz : 1H) ; 5,45 (d, J = 9 Hz : 1H) ; 5,79 (mt : 1H) ; 5,82 (dd, J = 16 et 2 Hz : 1H) ; 5,92 (mt : 1H) ; 6,06 (mt : 1H) ; 6,22 (d, J = 16 Hz : 1H) ; 6,51 (dd, J = 16 et 5 Hz : 1H) ; 7,18 (dd, J = 8 et 5 Hz : 1H) ; 7,30 (d, J = 8 Hz : 1H) ; 7,45 (d, J = 9 Hz : 2H) ; 7,65 (dt, J = 8 et 2 Hz : 1H) ; 7,99 (d, J = 8 Hz : 2H) ; 8,14 (s : 1H) ; 8,58 (d large, J = 5 Hz : 1H).

### Exemple 15

### (16R)-16-Désoxo-16-fluoro-14-O-[4-(pyrrolidin-1-yl) benzoyl] pristinamycine II_{B}

En opérant d'une manière analogue à celle décrite à l'exemple 9, mais à partir de 1 g de (16*R*)-16-désoxo-16-fluoro-14-O-[4-(chlorométhyl) benzoyl] pristinamycine II_{B} (préparée comme décrit dans l'exemple 9) en solution dans 50 cm³ de tétrahydrofurane, on ajoute à 20°C, 0,22 g d'iodure de sodium et 0,24 cm³ de pyrrolidine. Après 7 heures d'agitation à reflux et traitement analogue à celui de l'exemple 9, on obtient un résidu qui est purifié par chromatographie-flash [éluant : dichlorométhane / méthanol (99 / 1 en volumes)]. On obtient ainsi, après agitation dans 10 cm³ de pentane, filtration et séchage (90 Pa) à 20°C, 0,4 g de (16*R*)-16-désoxo-16-fluoro-14-O-[4-(pyrrolidin-1-yl) benzoyl] pristinamycine II_{B}, sous forme d'un solide jaune fondant vers 170°C (déc.).

Spectre de R.M.N. ¹H (400 MHz, CDCl₃, δ en ppm) : 0,96 (d, J = 6,5 Hz : 3H) ; 1,01 (d, J = 6,5 Hz : 3H) ; 1,10 (d, J = 6,5 Hz : 3H) ; de 1,70 à 2,05 (mt : 5H) ; 1,81 (mt : 4H) ; 1,95 (s : 3H) ; 2,16 (mt : 1H) ; 2,40 (mt : 1H) ; 2,53 (mt : 4H) ; 2,76 (mt : 1H) ; 3,04 (td, J = 17 et 6 Hz : 1H) ; 3,28 (mt : 1H) ; 3,48 (mt : 1H) ; 3,68 (s : 2H) ; 3,88 (mt : 1H) ; 4,09 (mt : 1H) ; 4,53 (mt : 1H) ; 4,79 (dd, J = 10 et 1 Hz : 1H) ; 4,83 (dd, J = 9 et 3 Hz : 1H) ; 5,17 (doublet démultiplié, J _{HF} = 48 Hz : 1H) ; 5,45 (d, J = 9,5 Hz : 1H) ; 5,77 (mt : 1H) ; 5,81 (dd, J = 17 et 1,5 Hz : 1H) ; 5,95 (mt : 1H) ; 6,05 (mt: 1H) ; 6,22 (d, J = 16 Hz : 1H) ; 6,51 (dd, J = 17 et 5 Hz : 1H) ; 7,42 (d, J = 8 Hz : 2H) ; 7,97 (d, J = 8 Hz : 2H) ; 8,14 (s : 1H).

### Exemple 16

### (16R)-16-Désoxo-16-fluoro-14-O-[4-(pipéridin-1-yl) benzoyl] pristinamycine II_{B}

En opérant d'une manière analogue à celle décrite à l'exemple 9, mais à partir de 0,75 g de (16*R*)-16-désoxo-16-fluoro-14-O-[4-(chlorométhyl) benzoyl] pristinamycine II_{B} (préparée comme décrit dans l'exemple 9) en solution dans 20 cm³ de tétrahydrofurane, on ajoute à 20°C, 0,16 g d'iodure de sodium et 0,22 cm³ de pipéridine. Après 15 heures d'agitation à 66°C et traitement analogue à celui de l'exemple 9, on obtient 1 g d'un solide jaune qui est purifié par chromatographie-flash [éluant : dichlorométhane / méthanol (97 / 3 en volumes)]. On obtient ainsi, après agitation dans de l'éther diéthylique puis dans du pentane, filtration et séchage (90 Pa) à 20°C, 0,33 g de (16*R*)-16-désoxo-16-fluoro-14-O-[4-(pipéridin-1-yl) benzoyl] pristinamycine II_{B}, sous forme d'un solide blanc fondant vers 110°C (déc.).

Spectre de R.M.N. ¹H (400 MHz, CDCl₃, δ en ppm) : 0,96 (d, J = 6,5 Hz : 3H) ; 1,01 (d, J = 6,5 Hz : 3H) ; 1,10 (d, J = 6,5 Hz : 3H) ; de 1,35 à 1,70 (mt : 6H) ; de 1,75 à 2,05 (mt : 5H) ; 1,95 (s : 3H) ; 2,16 (mt : 1H) ; de 2,30 à 2,50 (mt : 5H) ; 2,76 (mt : 1H) ; 3,05 (td, J = 17 et 6 Hz : 1H) ; 3,29 (mt : 1H) ; 3,49 (mt : 1H) ; 3,52 (s : 2H) ; 3,89 (mt : 1H) ; 4,09 (mt : 1H) ; 4,53 (mt : 1H) ; 4,79 (dd, J = 10 et 1,5 Hz : 1H) ; 4,83 (dd, J = 9 et 3 Hz : 1H) ; 5,18 (doublet démultiplié, J _{HF} = 48 Hz : 1 H) ; 5,45 (d, J = 9,5 Hz : 1H) ; 5,77 (mt : 1H) ; 5,82 (dd, J = 16 et 2 Hz : 1H) ; 5,93 (mt : 1H) ; 6,05 (mt : 1H) ; 6,22 (d, J = 16 Hz : 1H) ; 6,51 (dd, J = 16 et 5 Hz : 1H) ; 7,40 (d, J = 8 Hz : 2H) ; 7,97 (d, J = 8 Hz : 2H) ; 8,14 (s : 1H).

### Exemple 17

### (16R)-16-Désoxo-16-fluoro-14-O-{4-[(2-hydroxyéthyl) aminométhyl] benzoyl} pristinamycine II_{B}

En opérant d'une manière analogue à celle décrite à l'exemple 9, mais à partir de 0,8 g de (16*R*)-16-désoxo-16-fluoro-14-O-[4-(chlorométhyl) benzoyl] pristinamycine II_{B} (préparée comme décrit dans l'exemple 9) en solution dans 20 cm³ de tétrahydrofurane, de 0,17 g d'iodure de sodium et de 0,14 cm³ d'éthanolamine et, après 16 heures d'agitation à reflux et traitement analogue à celui de l'exemple 9, on obtient 0,8 g d'un solide beige qui est purifié par chromatographie-flash [éluant : gradient dichlorométhane / méthanol (95 / 5 et 90 / 10 en volumes)]. Après agitation dans de l'éther, filtration et séchage (90 Pa), on obtient 0,36 g d'un solide blanc qui est purifié par chromatographie liquide haute performance sur colonne Hypersil® 5µm (diamètre colonne = 2 cm, longueur colonne = 25 cm) [éluant : gradient dichlorométhane / méthanol (97 / 3 en volumes)]. On obtient 0,129 g de (16*R*)-16-désoxo-16-fluoro-14-O-{4-[(2-hydroxyéthyl) aminométhyl] benzoyl} pristinamycine II_{B}, sous forme d'un solide blanc fondant vers 194°C (déc.).

Spectre de R.M.N. ¹H (400 MHz, CDCl₃, δ en ppm) : 0,96 (d, J = 6,5 Hz : 3H) ; 1,00 (d, J = 6,5 Hz : 3H) ; 1,09 (d, J = 6,5 Hz : 3H) ; de 1,70 à 2,05 (mt : 5H) ; 1,95 (s : 3H) ; 2,16 (mt : 1H) ; 2,39 (mt : 1H) ; 2,76 (mt : 1H) ; 2,81 (t, J = 5,5 Hz : 2H) ; 3,04 (td, J = 17 et 6 Hz : 1H) ; 3,28 (mt : 1H) ; 3,48 (mt : 1H) ; 3,67 (t, J = 5,5 Hz : 2H ) ; de 3,85 à 3,95 (mt : 1H) ; 3,88 (s : 2H) ; 4,09 (mt : 1H) ; 4,52 (mt : 1H) ; 4,78 (d large, J = 10 Hz : 1H) ; 4,82 (dd, J = 9 et 3 Hz : 1H) ; 5,17 (doublet démultiplié, J _{HF} = 48 Hz : 1H) ; 5,44 (d, J = 9,5 Hz : 1H) ; 5,78 (mt : 1H) ; 5,81 (d large, J = 16 Hz : 1H) ; 5,93 (mt : 1H) ; 6,05 (mt : 1H) ; 6,22 (d, J = 16 Hz : 1H) ; 6,51 (dd, J = 16 et 5 Hz : 1H) ; 7,40 (d, J = 8 Hz : 2H) ; 7,99 (d, J = 8 Hz : 2H) ; 8,13 (s : 1H).

### Exemple 18

### (16R)-16-Désoxo-16-fluoro-14-O-{4-[(2-diéthylamino) éthylthiométhyl] benzoyl} pristinamycine II_{B}

A 0,46 cm³ de 2-diéthylaminoéthanethiol en solution dans 10 cm³ de tétrahydrofurane, on ajoute à 0°C, 0,82 cm³ d'une solution de butyllithium (2,5 M dans l'hexane). Après 15 minutes d'agitation, on ajoute goutte à goutte en 15 minutes, 1,4 g de (16*R*)-16-désoxo-16-fluoro-14-O-[4-(chlorométhyl) benzoyl] pristinamycine II_{B} (préparée comme décrit dans l'exemple 9) en solution dans 15 cm³ de tétrahydrofurane. Après 6,5 heures d'agitation à 0°C et 15 heures à 20°C, on ajoute goutte à goutte une solution, préalablement préparée comme précédemment mais à partir de 0,153 cm³ de 2-diéthylaminoéthanethiol et de 0,205 cm³ d'une solution de butyllithium (2,5 M dans l'hexane) dans 0,5 cm³ de tétrahydrofurane. Après 3,5 heures d'agitation à 20°C, le mélange réactionnel est concentré à sec sous pression réduite (2,7 kPa) pour donner un résidu qui est repris dans 20 cm³ de dichlorométhane. Cette solution est lavée par 50 cm³ d'eau puis décantée. La phase aqueuse est extraite par 20 cm³ de dichlorométhane. Les phases organiques sont réunies, lavées par 2 fois 70 cm³ d'eau, séchées sur sulfate de magnésium, filtrées et concentrées à sec sous pression réduite (2,7 kPa) pour donner 1,6 g d'un solide jaune qui est purifié par chromatographie-flash [éluant : dichlorométhane / méthanol / acétonitrile (92 / 4 / 4 en volumes)]. On obtient ainsi, après agitation dans du pentane, filtration et séchage (2,7 kPa) à 20°C, 0,926 g de (16R)-16-désoxo-16-fluoro-14-0-{4-[(2-diéthylamino) éthylthiométhyl] benzoyl} pristinamycine II_{B}, sous forme d'un solide beige fondant vers 84°C (déc.).

Spectre de R.M.N. ¹H (400 MHz, CDCl₃, δ en ppm) : de 0,90 à 1,05 (mt : 12H) ; 1,10 (d, J = 6,5 Hz : 3H) ; de 1,75 à 2,05 (mt : 5H) ; 1,96 (s : 3H) ; 2,16 (mt : 1H) ; 2,40 (mt : 1H) ; 2,50 (mt : 6H) ; 2,62 (mt : 2H) ; 2,76 (mt : 1H) ; 3,05 (dt, J = 17 et 6 Hz : 1H) ; 3,29 (mt : 1H) ; 3,49 (mt : 1H) ; 3,77 (s : 2H) ; 3,89 (mt : 1H) ; 4,09 (mt : 1H) ; 4,53 (mt : 1H) ; 4,78 (dd, J = 10 et 1,5 Hz : 1H) ; 4,83 (dd, J = 9 et 3 Hz : 1H) ; 5,17 (doublet démultiplié, J _{HF} = 48 Hz : 1H) ; 5,45 (d, J = 9 Hz : 1H) ; 5,78 (mt : 1H) ; 5,82 (dd, J = 17 et 1,5 Hz : 1H) ; 5,92 (mt : 1H) ; 6,05 (mt : 1H) ; 6,22 (d, J = 16 Hz : 1H) ; 6,52 (dd, J = 17 et 5 Hz : 1H) ; 7,40 (d, J = 8,5 Hz : 2H) ; 7,97 (d, J = 8,5 Hz : 2H) ; 8,14 (s : 1H).

### Exemple 19

### (16R)-16-Désoxo-16-fluoro-14-O-[4-(carboxyméthylthiométhyl) benzoyl] pristinamycine II_{B}

A 0,4 cm³ d'acide mercaptoacétique en solution dans 10 cm³ de tétrahydrofurane, on ajoute à 0°C, 4,64 cm³ d'une solution de butyllithium (2,5 M dans l'hexane). Après 15 minutes d'agitation, on abaisse la température du mélange réactionnel à -50°C et on ajoute, en un goutte à goutte lent, 2 g de (16*R*)-16-désoxo-16-fluoro-14-O-[4-(chlorométhyl) benzoyl] pristinamycine II_{B} (préparée comme décrit dans l'exemple 9) en solution dans 10 cm³ de tétrahydrofurane. Après 3 heures d'agitation à -50°C et 15 heures à 20°C, on abaisse à nouveau la température du mélange réactionnel à -50°C et on ajoute, goutte à goutte, une solution, préalablement préparée comme précédemment à -50°C, mais à partir de 0,2 cm³ de d'acide mercaptoacétique et de 2,32 cm³ d'une solution de butyllithium (2,5 M dans l'hexane) dans 5 cm³ de tétrahydrofurane. Après 5 heures d'agitation à -50°C, le mélange réactionnel est versé sur 100 cm³ d'eau et le pH est ajusté à 3-4 par addition d'une solution aqueuse d'acide chlorhydrique 0,1 N. Après addition de 20 cm³ de dichlorométhane et 20 cm³ d'une solution aqueuse saturée de chlorure de sodium, la phase organique est décantée et la phase aqueuse extraite par 20 cm³ de dichlorométhane. Les phases organiques sont réunies, séchées sur sulfate de magnésium, filtrées et concentrées à sec sous pression réduite (2,7 kPa) pour donner 2,1 g d'un solide jaune qui est purifié par chromatographie-flash [éluant : dichlorométhane / méthanol / acétonitrile (90 / 5 / 5 en volumes)]. On obtient ainsi, après agitation dans de l'éther, filtration et séchage (90 Pa) à 20°C, 0,21 g de (16*R*)-16-désoxo-16-fluoro-14-O-[4-(carboxyméthylthiométhyl) benzoyl] pristinamycine II_{B}, sous forme d'un solide jaune fondant vers 142°C (déc.).

Spectre de R.M.N. ¹H (400 MHz, CDCl₃, δ en ppm) : 0,96 (d, J = 6,5 Hz : 3H) ; 1,00 (d, J = 6,5 Hz : 3H) ; 1,10 (d, J = 6,5 Hz : 3H) ; de 1,75 à 2,05 (mt : 5H) ; 1,95 (s : 3H) ; 2,17 (mt : 1H) ; 2,39 (mt : 1H) ; 2,76 (mt : 1H) ; 3,05 (dt, J = 17 et 6 Hz : 1H) ; 3,08 (s : 2H) ; 3,28 (mt : 1H) ; 3,50 (mt : 1H) ; 3,88 (mt : 1H) ; 3,88 (s : 2H) ; 4,10 (mt : 1H) ; 4,52 (mt : 1H) ; 4,78 (dd, J = 10 et 2 Hz : 1H) ; 4,83 (dd, J = 9 et 3,5 Hz : 1H) ; 5,19 (doublet démultiplié, J _{HF} = 48 Hz : 1H) ; 5,44 (d, J = 9 Hz : 1H) ; 5,78 (mt : 1H) ; 5,83 (dd, J = 16 et 2 Hz : 1H) ; 6,00 (mt : 1H) ; 6,05 (mt : 1H) ; 6,22 (d, J = 16 Hz : 1H) ; 6,52 (dd, J = 16 et 5 Hz : 1H) ; 7,41 (d, J = 8 Hz : 2H) ; 7,97 (d, J = 8 Hz : 2H) ; 8,16 (s : 1H).

### Exemple 20

### (16R)-16-Désoxo-16-fluoro-14-O-{4-[(2-diéthylamino) éthoxyméthyl] benzoyl} pristinamycine II_{B}

A 1 g de (16*R*)-16-désoxo-16-fluoro pristinamycine II_{B} (préparée comme décrit dans l'exemple 1) en solution dans 15 cm³ de dichlorométhane, on ajoute à 20°C, 0,6 g d'acide 4-((2-diéthylamino) éthoxyméthyl) benzoïque, 0,05 g de 4-diméthylaminopyridine, 0,43 g de N,N'-dicyclohexylcarbodiimide et 2 g de sulfate de magnésium. Après 40 heures d'agitation à 20°C, on ajoute 0,24 g d'acide 4-((2-diéthylamino) éthoxyméthyl) benzoïque, 0,025 g de 4-diméthylaminopyridine et 0,2 g de N,N'-dicyclohexylcarbodiimide. Après 3 heures d'agitation supplémentaires à 20°C, le mélange réactionnel est filtré et l'insoluble rincé par 20 cm³ de dichlorométhane. Le filtrat est lavé par 3 fois 100 cm³ d'eau, séché sur sulfate de magnésium, filtré puis concentré à sec sous pression réduite (2,7 kPa) pour donner 1,5 g d'un solide beige qui est purifié par chromatographie-flash [éluant : gradient dichlorométhane / méthanol / acétonitrile (90 / 5 / 5 et 84 / 8 / 8 en volumes)]. On obtient ainsi, après agitation dans 20 cm³ d'éther, filtration et séchage, 0,86 g d'un solide blanc qui est mis en solution dans 20 cm³ de dichlorométhane. La solution obtenue est lavée par 2 fois 50 cm³ d'une solution aqueuse saturée de bicarbonate de sodium, séchée sur sulfate de magnésium, filtrée puis concentrée sous pression réduite (2,7 kPa) à 20°C, pour donner 0,67 g de (16*R*)-16-désoxo-16-fluoro-14-O-{4-[(2-diéthylamino) éthoxyméthyl] benzoyl} pristinamycine II_{B}, sous forme d'un solide blanc fondant vers 76°C (déc.).

Spectre de R.M.N. ¹H (400 MHz, CDCl₃, δ en ppm) : 0,96 (d, J = 6,5 Hz : 3H) ; 1,01 (d, J = 6,5 Hz : 3H) ; 1,05 (t, J = 7 Hz : 6H) ; 1,10 (d, J = 6,5 Hz : 3H) ; de 1,75 à 2,05 (mt : 5H) ; 1,95 (s : 3H) ; 2,16 (mt : 1H) ; 2,40 (mt : 1H) ; 2,60 (q, J = 7 Hz : 4H) ; 2,72 (t, J = 6,5 Hz : 2H) ; 2,77 (mt : 1H) ; 3,04 (dt, J = 17,5 et 6 Hz : 1H) ; 3,29 (mt : 1H) ; 3,49 (mt : 1H) ; 3,59 (t, J = 6,5 Hz : 2H) ; 3,89 (mt : 1H) ; 4,09 (mt : 1H) ; 4,53 (mt : 1H) ; 4,59 (s : 2H) ; 4,79 (dd, J = 10 et 2 Hz : 1H) ; 4,83 (dd, J = 9 et 3,5 Hz : 1H) ; 5,18 (doublet démultiplié, J _{NF} = 48 Hz : 1H) ; 5,45 (d, J = 9 Hz : 1H) ; 5,78 (mt : 1H) ; 5,82 (dd, J = 17 et 2 Hz : 1H) ; 5,92 (mt : 1H) ; 6,05 (mt : 1H) ; 6,22 (d, J = 16 Hz : 1H) ; 6,51 (dd, J = 17 et 5 Hz : 1H) ; 7,41 (d, J = 8 Hz : 2H) ; 7,99 (d, J = 8 Hz : 2H) ; 8,14 (s : 1H).

L'acide 4-[(2-diéthylamino) éthoxyméthyl] benzoïque peut être préparé de la manière suivante :

A 3,67 g 4-[(2-diéthylamino) éthoxyméthyl] benzoate de méthyle en solution dans 50 cm³ de méthanol, on ajoute à 20°C, 28 cm³ d'une solution aqueuse d'hydroxyde de sodium 1 N. Après 2,5 heures d'agitation à 20°C, on ajuste le pH du mélange réactionnel à 5 par addition de 29 cm³ d'une solution aqueuse d'acide chlorhydrique 1 N. Le mélange réactionnel est concentré à sec sous pression réduite (2,7 kPa) pour donner un résidu qui est agité dans 50 cm³ de méthanol. Après filtration de l'insoluble, le filtrat est concentré à sec sous pression réduite (2,7 kPa) pour donner un résidu qui est agité dans 50 cm³ de dichlorométhane. Après filtration de l'insoluble, le filtrat est concentré à sec sous pression réduite (2,7 kPa), à 20°C, pour donner 3,5 g d'acide 4-[(2-diéthylamino) éthoxyméthyl] benzoïque, sous forme d'une huile jaune.

Spectre de R.M.N. ¹H (300 MHz, (CD₃)₂SO d6, δ en ppm) : 1,07 (t, J = 7,5 Hz : 6H) ; 2,79 (q, J = 7,5 Hz : 4H) ; 2,93 (t, J = 6,5 Hz : 2H) ; 3,66 (t, J = 6,5 Hz : 2H) ; 4,57 (s : 2H) ; 7,42 (d, J = 8 Hz : 2H) ; 7,92 (d, J = 8 Hz : 2H).

Le 4-[(2-diéthylamino) éthoxyméthyl] benzoate de méthyle peut être préparé de la manière suivante :

A 5,8 cm³ de N,N-diéthyléthanolamine en solution dans 15 cm³ de diméthylformamide, on ajoute à 0°C, 2,64 g d'hydrure de sodium (à 60 % en poids dans de l'huile de vaseline). Après 1 heure d'agitation à 0°C, on ajoute goutte à goutte en 20 minutes, 10 g de 4-(bromométhyl) benzoate de méthyle en solution dans 10 cm³ de diméthylformamide, puis 50 cm³ de diméthylformamide. Après 17 heures d'agitation à 20°C, on ajoute 100 cm³ de méthanol puis le mélange réactionnel est concentré à sec sous pression réduite (2,7 kPa) pour donner une huile jaune qui est diluée dans 200 cm³ d'acétate d'éthyle. La solution obtenue est lavée par 300 cm³ d'eau. La phase aqueuse est décantée puis extraite par 100 cm³ d'acétate d'éthyle. Les phases organiques sont réunies, lavées par 2 fois 500 cm³ d'eau, séchées sur sulfate de magnésium, filtrées et concentrées à sec sous pression réduite (2,7 kPa) pour donner 10 g d'une huile jaune qui est purifiée par chromatographie-flash [éluant : gradient dichlorométhane / méthanol (96 / 4 et 90 / 10 en volumes)]. On obtient ainsi 3,67 g de 4-[(2-diéthylamino) éthoxyméthyl] benzoate de méthyle, sous forme d'une huile jaune.

Spectre de R.M.N. ¹H (400 MHz, CDCl₃, δ en ppm) : 1,02 (t, J = 7,5 Hz : 6H) ; 2,58 (q, J = 7,5 Hz : 4H) ; 2,70 (t, J = 6 Hz : 2H) ; 3,57 (t, J = 6 Hz : 2H) ; 3,88 (s : 3H) ; 4,56 (s : 2H) ; 7,38 (d, J = 8 Hz : 2H) ; 7,99 (d, J = 8 Hz : 2H).

### Exemple 21

### (16R)-16-Désoxo-16-fluoro-14-O-[3-(morpholin-4-yl méthyl) benzoyl] pristinamycine II_{B}

En opérant d'une manière analogue à celle décrite à l'exemple 9, mais à partir de 1 g de (16*R*)-16-désoxo-16-fluoro-14-O-[3-(chlorométhyl) benzoyl] pristinamycine II_{B} en solution dans 25 cm³ de tétrahydrofurane, on ajoute à 20°C, 0,212 g d'iodure de sodium et 0,253 cm³ de morpholine Après 16 heures d'agitation à reflux et traitement analogue à celui de l'exemple 9, on obtient 1 g d'un solide jaune qui est purifié par chromatographie-flash [éluant : gradient dichlorométhane / méthanol / acétonitrile (100 / 0 / 0 puis 98 / 1 / 1 en volumes)]. On obtient ainsi 0,8 g d'un solide jaune qui donne, après agitation dans 10 cm³ d'éther, filtration et séchage (90 Pa) à 20°C, 0,68 g de (16*R*)-16-désoxo-16-fluoro-14-O-[3-(morpholin-4-yl méthyl) benzoyl] pristinamycine II_{B}, sous forme d'un solide blanc fondant vers 198°C (déc.).

Spectre de R.M.N. ¹H (400 MHz, CDCl₃, δ en ppm) : 0,96 (d, J = 6,5 Hz : 3H ) ; 1,01 (d, J = 6,5 Hz : 3H) ; 1,10 (d, J = 6,5 Hz : 3H) ; de 1,75 à 2,05 (mt : 5H) ; 1,96 (s : 3H) ; 2,17 (mt : 1H) ; de 2,35 à 2,50 (mt : 1H) ; 2,46 (mt : 4H) ; 2,76 (mt : 1H) ; 3,05 (td, J = 17 et 6 Hz : 1H) ; 3,29 (mt : 1H) ; de 3,45 à 3,60 (mt : 1H) ; 3,54 (s : 2H) ; 3,72 (mt : 4H) ; 3,88 (mt : 1H) ; 4,10 (mt : 1H) ; 4,53 (mt : 1H) ; 4,79 (dd, J = 10 et 1,5 Hz : 1H) ; 4,83 (dd, J = 9 et 3 Hz : 1H) ; 5,18 (doublet démultiplié, J _{HF} = 48 Hz : 1H) ; 5,45 (d, J = 9 Hz : 1H) ; 5,79 (mt : 1H) ; 5,82 (dd, J = 17 et 2 Hz : 1H) ; 5,93 (mt : 1H) ; 6,06 (mt : 1H) ; 6,22 (d, J = 16 Hz : 1H) ; 6,52 (dd, J = 17 et 5 Hz : 1H) ; 7,40 (t, J = 8 Hz : 1H) ; 7,56 (d large, J = 8 Hz : 1H) ; 7,92 (d large, J = 8 Hz : 1H) ; 7,96 (s large : 1H) ; 8,14 (s : 1H).

La (16*R*)-16-désoxo-16-fluoro-14-O-[3-(chlorométhyl) benzoyl] pristinamycine II_{B} peut être préparée de la manière suivante :

A 5 g de (16*R*)-16-désoxo-16-fluoro pristinamycine II_{B} (préparée comme décrit dans l'exemple 1) en solution dans 250 cm³ de dichlorométhane, on ajoute à 20°C, 2 cm³ de triéthylamine, 2 cm³ de chlorure de 3-(chlorométhyl) benzoyle et 0,23 g de 4-diméthylaminopyridine. Après 24 heures d'agitation à 20°C, le mélange réactionnel est lavé par 3 fois 120 cm³ d'eau, séché sur sulfate de magnésium, filtré puis concentré à sec sous pression réduite (2,7 kPa) pour donner un solide jaune qui est agité dans 50 cm³ d'éther. Après filtration, rinçage du solide par 2 fois 10 cm³ d'éther diéthylique et séchage sous pression réduite (2,7 kPa) à 20°C, on obtient 5,56 g de (16*R*)-16-désoxo-16-fluoro-14-O-[3-(chlorométhyl) benzoyl] pristinamycine II_{B}, sous forme d'un solide blanc fondant vers 190°C (déc.).

Spectre de R.M.N. ¹H (400 MHz, CDCl₃, δ en ppm) : 0,96 (d, J = 6,5 Hz : 3H) ; 1,01 (d, J = 6,5 Hz : 3H) ; 1,10 (d, J = 6,5 Hz : 3H) ; de 1,80 à 2,05 (mt : 5H) ; 1,96 (s : 3H) ; 2,17 (mt : 1H) ; 2,40 (mt : 1H) ; 2,76 (mt : 1H) ; 3,05 (dt, J = 17 et 6 Hz : 1H) ; 3,29 (mt : 1H) ; 3,50 (mt : 1H) ; 3,88 (mt : 1H) ; 4,10 (mt : 1H) ; 4,53 (mt : 1H) ; 4,63 (s : 2H) ; 4,79 (dd, J = 10 et 2 Hz : 1H) ; 4,84 (dd, J = 9 et 3 Hz : 1H) ; 5,18 (doublet démultiplié, J _{HF} = 48 Hz : 1H) ; 5,45 (d, J = 9,5 Hz : 1H) ; de 5,75 à 5,85 (mt : 1H) ; 5,82 (dd, J = 16 et 1,5 Hz : 1H) ; 5,92 (mt : 1H) ; 6,07 (dt, J = 9,5 et 5 Hz : 1H) ; 6,23 (d, J = 16 Hz : 1H) ; 6,52 (dd, J = 16 et 5 Hz : 1H) ; 7,46 (t, J = 8 Hz : 1H) ; 7,61 (d large, J = 8 Hz : 1H) ; 7,99 (d large, J = 8 Hz : 1H) ; 8,04 (s large : 1H) ; 8,14 (s : 1H).

### Exemple 22

### Méthanesulfonate de la (16R)-16-Désoxo-16-fluoro-14-O-[3-(imidazol-1-yl méthyl) benzoyl] pristinamycine II_{B}

A 0,450 g de (16*R*)-16-désoxo-16-fluoro-14-O-[3-(imidazol-1-yl méthyl) benzoyl] pristinamycine II_{B} en solution dans 10 cm³ d'éthanol, on ajoute à 20°C, 6,28 cm' d'une solution éthanolique d'acide méthanesulfonique 0,1 N. Après 10 minutes d'agitation, le mélange réactionnel est concentré à sec sous pression réduite (2,7 kPa) pour donner un résidu qui est agité dans 7 cm³ d'éther. Après filtration, lavage du solide par 2 fois 2 cm³ d'éther diéthylique et séchage sous pression réduite (2,7 kPa), on obtient 0,479 g du méthanesulfonate de la (16*R*)-16-désoxo-16-fluoro-14-O-[3-(imidazol-1-yl méthyl) benzoyl] pristinamycine II_{B}, sous forme d'un solide blanc fondant vers 160°C (déc.).

Spectre de R.M.N. ¹H (400 MHz, CDCl₃, δ en ppm) : 0,96 (d, J = 6,5 Hz : 3H) ; 1,00 (d, J = 6,5 Hz : 3H) ; 1,11 (d, J = 6,5 Hz : 3H) ; de 1,75 à 2,05 (mt : 5H) ; 1,96 (s : 3H) ; 2,17 (mt : 1H) ; 2,37 (mt : 1H) ; 2,77 (mt : 1H) ; 2,86 (s : 3H) ; 3,05 (mt : 1H) ; 3,29 (mt : 1H) ; 3,53 (mt : 1H) ; 3,85 (mt : 1H) ; 4,11 (mt : 1H) ; 4,53 (mt : 1H) ; 4,80 (d large, J = 10 Hz : 1H) ; 4,84 (dd, J = 9 et 3 Hz : 1H) ; 5,18 (doublet démultiplié, J _{HF} = 48 Hz : 1H) ; de 5,35 à 5,50 (mt : 3H) ; 5,82 (mt : 1H) ; 5,86 (d large, J = 17 Hz : 1H) ; 6,07 (mt : 1H) ; 6,11 (mt : 1H) ; 6,22 (d, J = 16 Hz : 1H) ; 6,55 (dd, J = 17 et 5 Hz : 1H) ; 7,10 (s large : 1H) ; 7,43 (s large : 1H) ; 7,54 (mt : 2H) ; 7,95 (s large : 1H) ; 8,09 (mt : 1H) ; 8,12 (s : 1H) ; 9,11 (s large : 1H).

La (16*R*)-16-désoxo-16-fluoro-14-O-[3-(imidazol-1-yl méthyl) benzoyl] pristinamycine II_{B} peut être préparée de la manière suivante :

En opérant d'une manière analogue à celle décrite à l'exemple 9, mais à partir de 1,2 g de (16*R*)-16-désoxo-16-fluoro-14-O-[3-(chlorométhyl) benzoyl] pristinamycine II_{B} (préparée comme décrit dans l'exemple 21) en solution dans 30 cm³ de tétrahydrofurane, on ajoute à 20°C, 0,263 g d'iodure de sodium et 0,238 g d'imidazole. Après 16 heures d'agitation à reflux et traitement analogue à celui de l'exemple 9, on obtient 0,9 g d'un solide jaune qui est purifié par chromatographie-flash [éluant : gradient dichlorométhane / méthanol / acétonitrile (98 / 1 / 1 puis 96 / 2 / 2 puis 90 / 5 / 5 en volumes)]. On obtient ainsi 0,53 g de (16*R*)-16-désoxo-16-fluoro-14-O-[3-(imidazol-1-yl méthyl) benzoyl] pristinamycine II_{B}, sous forme d'un solide jaune fondant vers 125°C (déc.).

Spectre de R.M.N. ¹H (400 MHz, CDCl₃, δ en ppm) : 0,96 (d, J = 6,5 Hz : 3H) ; 1,00 (d, J = 6,5 Hz : 3H) ; 1,10 (d, J = 6,5 Hz : 3H) ; de 1,75 à 2,05 (mt : 5H) ; 1,95 (s : 3H) ; 2,16 (mt : 1H) ; 2,38 (mt : 1H) ; 2,76 (mt : 1H) ; 3,04 (dt, J = 17 et 6 Hz : 1H) ; 3,28 (mt : 1H) ; 3,50 (mt : 1H) ; 3,86 (mt : 1H) ; 4,10 (mt : 1H) ; 4,53 (mt : 1H) ; 4,79 (dd, J = 10 et 2 Hz : 1H) ; 4,83 (dd, J = 9 et 4 Hz : 1H) ; 5,17 (s : 2H) ; 5,17 (doublet démultiplié, J _{HF} = 48 Hz : 1H) ; 5,43 (d, J = 9 Hz : 1H) ; 5,79 (mt : 1H) ; 5,82 (dd, J = 16 et 2 Hz : 1H) ; 5,97 (mt : 1H) ; 6,06 (mt : 1H) ; 6,22 (d, J = 16 Hz : 1H) ; 6,52 (dd, J = 16 et 5 Hz : 1H) ; 6,91 (mf: 1H) ; 7,11 (s large : 1H) ; 7,32 (d large, J = 8 Hz : 1H) ; 7,44 (t, J = 8 Hz : 1H) ; 7,57 (s large : 1H) ; 7,88 (s large : 1H) ; 7,99 (d large, J = 8 Hz : 1H) ; 8,14 (s : 1H).

### Exemple 23

### (16R)-16-Désoxo-16-fluoro-14-O-[3-(diéthylaminométhyl) benzoyl] pristinamycine II_{B}

En opérant d'une manière analogue à celle décrite à l'exemple 9, mais à partir de 1 g de (16*R*)-16-désoxo-16-fluoro-14-O-[3-(chlorométhyl) benzoyl] pristinamycine II_{B} (préparée comme décrit dans l'exemple 21) en solution dans 25 cm³ de tétrahydrofurane, on ajoute à 20°C, 0,217 g d'iodure de sodium et 0,3 cm³ de diéthylamine. Après 16 heures d'agitation à reflux et traitement analogue à celui de l'exemple 9, on obtient 1 g d'un solide jaune qui est purifié par chromatographie-flash [éluant : gradient dichlorométhane / méthanol / acétonitrile (100 / 0 / 0 puis 98 / 1 / 1 en volumes)]. On obtient un solide qui est agité dans du pentane, filtré et séché (2,7 kPa), à 20°C, pour donner 0,313 g de (16*R*)-16-désoxo-16-fluoro-14-O-[3-(diéthylaminométhyl) benzoyl] pristinamycine II_{B}, sous forme d'un solide jaune fondant vers 115°C (déc.).

Spectre de R.M.N. ¹H (400 MHz, CDCl₃, δ en ppm) : 0,96 (d, J = 6,5 Hz : 3H) ; 1,01 (d, J = 6,5 Hz : 3H) ; de 1,05 à 1,15 (mt : 9H) ; de 1,75 à 2,05 (mt : 5H) ; 1,97 (s : 3H) ; 2,16 (mt : 1H) ; 2,41 (mt : 1H) ; 2,56 (mt : 4H) ; 2,77 (mt : 1H) ; 3,05 (td, J = 17 et 6 Hz : 1H) ; 3,29 (mt : 1H) ; 3,49 (mt : 1H) ; 3,64 (s large : 2H) ; 3,89 (mt : 1H); 4,10 (mt : 1H) ; 4,53 (mt : 1H) ; 4,79 (dd, J = 10 et 1,5 Hz : 1H) ; 4,83 (dd, J = 9 et 3 Hz : 1H) ; 5,19 (doublet démultiplié, J _{HF} = 48 Hz : 1H) ; 5,46 (d, J = 9,5 Hz : 1H) ; 5,78 (mt : 1H) ; 5,82 (dd, J = 16 et 2 Hz : 1H) ; 5,94 (mt : 1H) ; 6,06 (mt : 1H) ; 6,23 (d, J = 16 Hz : 1H) ; 6,52 (dd, J = 16 et 5 Hz : 1H) ; 7,40 (t, J = 8 Hz : 1H) ; 7,62 (mt: 1H) ; 7,91 (d, J = 8 Hz : 1H) ; 7,97 (s large : 1H) ; 8,14 (s : 1H).

### Exemple 24

### (16R)-16-Désoxo-16-fluoro-14-O-[4-(morpholin-4-yl méthyl) phénylacétyl] pristinamycine II_{B}

A 0,87 g de (16*R*)-16-désoxo-16-fluoro-14-O-[4-(halogénométhyl) phénylacétyl] pristinamycine II_{B} (mélange des dérivés chloré et bromé) en solution dans 5 cm³ de diméthylformamide, on ajoute à 20°C, 0,25 cm³ de morpholine et quelques cristaux d'iodure de sodium. Après 15 minutes d'agitation à 85°C, le mélange réactionnel est versé sur 90 cm³ d'eau. La phase aqueuse est décantée puis extraite par 2 fois 50 cm³ d'acétate d'éthyle. Les phases organiques sont réunies, lavées successivement par 30 cm³ d'eau et 30 cm³ d'une solution aqueuse saturée de chlorure de sodium, puis séchées sur sulfate de magnésium, filtrées et concentrées à sec sous pression réduite (2,7 kPa). On obtient ainsi 0,91 g d'un solide brun-orangé qui est purifié par chromatographie-flash [éluant : dichlorométhane / méthanol / acétonitrile (92 / 4 / 4 en volumes)] pour donner 0,46 g d'un solide jaune qui est repris dans 5 cm³ de dichlorométhane. Après filtration sur Célite®, la Célite® est rincée avec du dichlorométhane. Le filtrat est concentré à sec sous pression réduite (2,7 kPa) pour donner 0,42 g d'un solide jaune qui est agité 15 minutes dans 5 cm³ d'eau. Après filtration et séchage sous pression réduite (2,7 kPa) à 20°C, on obtient 0,38 g de (16*R*)-16-désoxo-16-fluoro-14-O-[4-(morpholin-4-yl méthyl) phénylacétyl] pristinamycine II_{B,} sous forme d'un solide jaune fondant vers 100°C (déc.).

Spectre de R.M.N. ¹H (400 MHz, CDCl₃, δ en ppm) : 0,96 (d, J = 6,5 Hz : 3H) ; 1,01 (d, J = 6,5 Hz : 3H) ; 1,09 (d, J = 6,5 Hz : 3H) ; de 1,60 à 2,05 (mt : 5H) ; 1,85 (s : 3H) ; de 2,10 à 2,30 (mt : 2H) ; 2,44 (mt : 4H) ; 2,76 (mt : 1H) ; 2,97 (dt, J = 17 et 6 Hz : 1H) ; 3,22 (mt : 1H) ; de 3,40 à 3,55 (mt : 1H) ; 3,48 (s : 2H) ; 3,59 (s : 2H) ; 3,71 (mt : 4H) ; 3,85 (mt : 1H) ; 4,04 (mt : 1H) ; 4,52 (mt : 1H) ; 4,78 (d large, J = 10 Hz : 1H) ; 4,82 (dd, J = 9 et 3 Hz : 1H) ; 5,06 (doublet démultiplié, J _{HF} = 48 Hz : 1H) ; 5,3 (d, J = 9 Hz : 1H) ; de 5,75 à 5,85 (mt : 1H) ; 5,75 (mt : 1H) ; 5,81 (d large, J = 17 Hz : 1H) ; 5,93 (mt : 1H) ; 6,18 (d, J = 16 Hz : 1H) ; 6,51 (dd, J = 17 et 4 Hz : 1H) ; 7,22 (d, J = 8 Hz : 2H) ; 7,29 (d, J = 8 Hz : 2H) ; 8,12 (s : 1H).

La (16*R*)-16-désoxo-16-fluoro-14-O-[4-(halogénométhyl) phénylacétyl] pristinamycine II_{B} (mélange des dérivés chloré et bromé) peut être préparée de la manière suivante :

A 2 g de (16*R*)-16-désoxo-16-fluoro pristinamycine II_{B} (préparée comme décrit dans l'exemple 1) en solution dans 60 cm³ de dichlorométhane, on ajoute à 20°C, sous atmosphère d'argon, 1,3 cm³ de diisopropyléthylamine et 1,75 g de chlorure de 4-(bromométhyl)phénylacétyle en 5 minutes. Après 16 heures d'agitation à 20°C, le mélange réactionnel est lavé successivement par 2 fois 25 cm³ d'eau et 25 cm³ d'une solution aqueuse saturée de chlorure de sodium, puis séché sur sulfate de magnésium, filtré et concentré à sec sous pression réduite (2,7 kPa). On obtient ainsi 2,79 g d'un solide orangé qui est purifié par chromatographie-flash [éluant : dichlorométhane / méthanol (97 / 3 en volumes)] pour donner 1,3 g d'un solide jaune qui est repris dans 50 cm³ d'acétate d'éthyle. La solution obtenue est lavée par 2 fois 25 cm³ d'une solution aqueuse saturée de bicarbonate de sodium puis séchée sur sulfate de magnésium, filtrée et concentrée à sec sous pression réduite (2,7 kPa), à 20°C, pour donner 1,04 g de (16*R*)-16-désoxo-16-fluoro-14-O-[4-(halogénométhyl) phénylacéthyl] pristinamycine II_{B} (mélange des dérivés chloré et bromé), sous forme d'un solide jaune.

Le chlorure de 4-(bromométhyl)phénylacétyle peut être préparé selon la demande de brevet EP 274 999.

### Exemple 25

### Méthanesulfonate de la (16R)-16-Désoxo-16-fluoro-14-O-[(2-imidazol-1-yl éthoxy)acétyl] pristinamycine II_{B}

A 0,19 g de (16*R*)-16-désoxo-16-fluoro-14-O-[(2-imidazol-1-yl éthoxy)acétyl] pristinamycine II_{B} en solution dans 8 cm³ d'éthanol, on ajoute à 20°C, 0,26 cm³ d'une solution éthanolique d'acide méthanesulfonique 1,09 N. Après 10 minutes d'agitation, le mélange réactionnel est concentré à sec sous pression réduite (2,7 kPa) pour donner un résidu huileux qui est agité dans 5 cm³ d'éther diéthylique. Le solvant est ensuite éliminé sous pression réduite (2,7 kPa). Le solide ainsi obtenu est agité dans 5 cm³ d'éther diéthylique puis filtré, rincé avec de l'éther diéthylique et séché sous pression réduite (2,7 kPa) pour donner 0,189 g du méthanesulfonate de la (16*R*)-16-désoxo-16-fluoro-14-O-[(2-imidazol-1-yl éthoxy)acétyl] pristinamycine II_{B}, sous forme d'une poudre beige fondant vers 115°C (déc.).

Spectre de R.M.N. ¹H (400 MHz, CDCl₃, δ en ppm) : 0,95 (mt : 6H) ; 1,12 (d, J = 6,5 Hz : 3H) ; de 1,50 à 2,25 (mt : 7H) ; 1,86 (s : 3H) ; 2,79 (mt : 1H) ; 2,83 (s : 3H) ; 3,02 (mt : 1H) ; 3,26 (mt : 1H) ; 3,58 (mt : 1H) ; 3,72 (mt : 1H) ; de 3,80 à 3,90 (mt : 2H) ; de 4,00 à 4,15 (mt : 3H) ; 4,34 (mt : 1H) ; de 4,50 à 4,60 (mt : 2H) ; de 4,80 à 4,90 (mt : 2H) ; de 4,90 à 5,00 (mt : 1H) ; 4,94 (d large, J = 10 Hz : 1H) ; de 5,75 à 5,95 (mt : 2H) ; 6,06 (d large, J = 16 Hz : 1H) ; 6,12 (d, J = 16 Hz : 1H) ; 6,65 (dd, J = 16 et 5 Hz : 1H) ; 6,95 (mt : 1H) ; 7,30 (s large : 1H) ; 7,36 (s large : 1H) ; 8,12 (s : 1H) ; 9,18 (s large : 1H) ; 14,73 (mf large : 1H).

La (16*R*)-16-désoxo-16-fluoro-14-O-[(2-imidazol-1-yl éthoxy)acétyl] pristinamycine II_{B} peut être préparée de la manière suivante :

A 1,3 g de (16*R*)-16-désoxo-16-fluoro-14-O-[2-chloroéthoxyacétyl] pristinamycine II_{B} en solution dans 6,5 cm³ de diméthylsulfoxyde, on ajoute à 20°C, 0,41 g d'imidazole et quelques cristaux d'iodure de sodium. Après 4 heures d'agitation à 60°C, on ajoute 0,3 g d'iodure de sodium. Après encore 4 heures d'agitation à 60°C, 72 heures à 85°C puis 60 heures à 20°C, le mélange réactionnel est versé sur un mélange de 35 cm³ d'eau et de glace puis additionné de 0,17 g de bicarbonate de sodium. Après filtration, lavage du solide par de l'eau puis séchage à l'air, on obtient un résidu huileux qui est dilué dans 30 cm³ de dichlorométhane. Après concentration sous pression réduite (2,7 kPa), on obtient 1,23 g d'un solide jaune qui est purifié par chromatographie-flash sur silice Amicon® _{―}20-40 Å [éluant : dichlorométhane / méthanol / acétonitrile (88 / 6 / 6 en volumes)]. On obtient ainsi 0,64 g de (16*R*)-16-désoxo-16-fluoro-14-O-[(2-imidazol-1-yl éthoxy)acétyl] pristinamycine II_{B}, sous forme d'un solide jaune pâle.

Spectre de R.M.N. ¹H (400 MHz, CDCl₃, δ en ppm) : 0,96 (d, J = 6,5 Hz : 3H) ; 1,00 (d, J = 6,5 Hz : 3H) ; 1,10 (d, J = 6,5 Hz : 3H) ; de 1,65 à 2,05 (mt : 5H) ; 1,89 (s : 3H) ; de 2,10 à 2,30 (mt : 2H) ; 2,76 (mt : 1H) ; 2,99 (dt, J = 17 et 6,5 Hz : 1H) ; 3,24 (mt : 1H) ; 3,51 (mt : 1H) ; de 3,75 à 3,90 (mt : 3H) ; 4,03 (AB limite : 2H) ; 4,07 (mt : 1H) ; 4,17 (t, J = 5 Hz : 2H) ; 4,55 (mt : 1H) ; de 4,75 à 4,85 (mt : 2H) ; 5,10 (doublet démultiplié, J _{HF} = 48 Hz : 1H) ; 5,24 (d, J = 9 Hz : 1H) ; 5,78 (mt : 1H) ; de 5,80 à 5,95 (mt : 1H) ; 5,87 (dd, J = 16 et 2 Hz : 1H) ; 6,18 (d, J = 16 Hz : 1H) ; 6,31 (mt : 1H) ; 6,56 (dd, J = 16 et 5 Hz : 1H) ; 7,01 (s : 1H) ; 7,08 (s : 1H) ; 7,59 (s : 1H); 8,12 (s : 1H).

La (16*R*)-16-désoxo-16-fluoro-14-O-[2-chloroéthoxyacétyl] pristinamycine II_{B} peut être préparée de la manière suivante :

A 5,32 g de (16R)-16-désoxo-16-fluoro pristinamycine II_{B} (préparée comme décrit dans l'exemple 1) en solution dans 130 cm³ de dichlorométhane, on ajoute à 20°C, 3,1 g de N,N'-dicyclohexylcarbodiimide, 0,61 g de 4-diméthylaminopyridine et 2,08 g d'acide 2-chloroéthoxyacétique en solution dans 20 cm³ de dichlorométhane. Après 1 heure d'agitation à 20°C, le mélange réactionnel est filtré et le résidu est rincé par du dichlorométhane. Le filtrat est concentré à sec sous pression réduite (2,7 kPa) pour donner 7,68 g d'un solide orange qui est purifié par chromatographie-flash sur silice Amicon® _{―}20-40 Å [éluant : dichlorométhane / acétate d'éthyle (50 / 50 en volumes)]. On obtient ainsi 4,68 g de (16*R*)-16-désoxo-16-fluoro-14-O-[2-chloroéthoxyacétyl] pristinamycine II_{B} sous la forme d'une poudre blanche.

Spectre de R.M.N. ¹H (400 MHz, CDCl₃, δ en ppm) : 0,96 (d, J = 6,5 Hz : 3H) ; 1,00 (d, J = 6,5 Hz : 3H) ; 1,09 (d, J = 6,5 Hz : 3H) ; de 1,70 à 2,05 (mt : 5H) ; 1,90 (s : 3H) ; 2,15 (mt : 1H) ; 2,26 (mt : 1H) ; 2,76 (mt : 1H) ; 2,99 (dt, J = 17 et 6 Hz : 1H) ; 3,24 (mt : 1H) ; 3,50 (mt : 1H) ; 3,67 (t, J = 6 Hz : 2H) ; de 3,80 à 3,90 (mt : 1H) ; 3,83 (t, J = 6 Hz : 2H) ; 4,05 (mt : 1H) ; 4,14 (AB limite, J = 13 Hz : 2H) ; 4,52 (mt : 1H) ; 4,78 (dd, J = 10 et 1,5 Hz : 1H) ; 4,82 (dd, J = 9 et 3 Hz : 1H) ; 5,12 (doublet démultiplié, J _{HF} = 48 Hz : 1H) ; 5,31 (d large, J = 9 Hz : 1H) ; 5,78 (mt : 1H) ; 5,82 (dd, J = 16 et 2 Hz : 1H) ; de 5,85 à 5,95 (mt : 2H) ; 6,19 (d, J = 16 Hz : 1H) ; 6,52 (dd, J = 16 et 5 Hz : 1H) ; 8,12 (s : 1H).

L'acide 2-chloroéthoxyacétique peut être préparée selon E. J. Corey and Christopher J. Helal, Tetrahedron Letters, Vol. 37, No. 28, pp. 4837-4840, 1996.

### Exemple 26

### (16R)-14-O-{3,3-Diméthyl-3-[4,6-diméthyl-2-(4-morpholin-4-yl butyryloxy)phényl] propionyl}-16-désoxo-16-fluoro pristinamycine II_{B}

A 1,6 g de (16*R*)-14-O-{3-[2-(4-bromobutyryloxy)-4,6-diméthylphényl]-3,3-diméthylpropionyl}-16-désoxo-16-fluoro pristinamycine II_{B} en solution dans 8 cm³ de diméthylsulfoxyde, on ajoute à 20°C et sous atmosphère d'argon, 0,48 cm³ de morpholine. Après 30 minutes d'agitation à 60°C, le mélange réactionnel est versé sur 300 cm³ d'un mélange eau-glace. Le précipité est isolé par filtration, rincé par deux fois 20 cm³ d'eau distillée, puis dissous dans 150 cm³ de dichlorométhane. La solution obtenue est lavée par trois fois 20 cm³ d'eau distillée, séchée sur du sulfate de magnésium, filtrée, puis concentrée à sec sous pression réduite (2,7 kPa) pour donner 1,7 g d'un résidu qui est purifié par chromatographie-flash [éluant : dichlorométhane / méthanol (94 / 6 en volumes)]. On obtient un solide, qui, après agitation dans de l'éther diisopropylique, filtration et séchage (2,7 kPa) à 20°C, conduit à 0,52 g de (16*R*)-14-O-{3,3-diméthyl-3-[4,6-diméthyl-2-(4-morpholin-4-yl butyryloxy)phényl]propionyl}-16-désoxo-16-fluoro pristinamycine II_{B}, sous forme d'un solide blanc fondant vers 88°C (déc.).

Spectre de R.M.N. ¹H (400 MHz, CDCl₃, δ en ppm) : 0,96 (d, J = 6,5 Hz : 3H) ; 1,01 (d, J = 6,5 Hz : 3H) ; 1,09 (d, J = 6,5 Hz : 3H) ; de 1,40 à 1,60 (mt : 1H) ; 1,56 (s : 3H) ; 1,58 (s : 3H) ; de 1,75 à 2,05 (mt : 7H) ; 1,80 (s : 3H) ; 2,14 (mt : 1H) ; 2,25 (s : 3H) ; de 2,40 à 2,50 (mt : 6H) ; 2,54 (s : 3H) ; 2,63 (t, J = 7,5 Hz : 2H) ; de 2,70 à 2,80 (mt : 1H) ; 2,75 (d, J = 15 Hz : 1H) ; 2,85 (d, J = 15 Hz : 1H) ; 2,91 (mt : 1H) ; 3,15 (mt : 1H) ; 3,45 (mt : 1H) ; 3,73 (t, J = 5 Hz : 4H) ; 3,86 (mt : 1H) ; 4,01 (mt : 1H) ; 4,53 (mt : 1H); de 4,70 à 4,95 (mt : 1H) ; 4,77 (dd, J = 10 et 1,5 Hz : 1H) ; 4,80 (dd, J = 9 et 3 Hz : 1H) ; 5,11 (d, J = 9 Hz : 1H) ; 5,66 (mt : 1H) ; 5,71 (mt : 1H) ; 5,81 (dd, J = 17 et 2 Hz : 1H) ; 5,93 (mt : 1H) ; 6,12 (d, J = 16 Hz : 1H) ; 6,50 (dd, J = 17 et 5 Hz : 1H) ; 6,61 (d, J = 1,5 Hz : 1H) ; 6,81 (s large : 1H) ; 8,12 (s : 1H).

La (16*R*)-14-O-{3-[2-(4-bromobutyryloxy)-4,6-diméthylphényl]-3,3-diméthylpropionyl}-16-désoxo-16-fluoro pristinamycine II_{B} peut être préparée de la manière suivante :

A 2,7 g d'acide 3-[2-(4-bromobutyryloxy)-4,6-diméthylphényl]-3,3-diméthylpropionique en solution dans 150 cm³ de dichlorométhane, on ajoute à 20°C sous atmosphère d'argon, 1,2 g de N,N'-dicyclohexylcarbodiimide, 2,06 g de (16*R*)-16-désoxo-16-fluoro pristinamycine II_{B} (préparée comme décrit dans l'exemple 1) et 0,07 g de 4-diméthylaminopyridine. Après 18 heures d'agitation, le mélange réactionnel est filtré pour éliminer l'insoluble. Le filtrat est lavé par quatre fois 25 cm³ d'eau distillée, séché sur du sulfate de magnésium, filtré puis concentré à sec sous pression réduite (2,7 kPa) pour donner 4,6 g d'un résidu qui est purifié par deux chromatographies-flash successives (éluant : dichlorométhane / méthanol, 96 / 4 puis 97 / 3 en volumes). On obtient 1,6 g de (16*R*)-14-O-{3-[2-(4-bromobutyryloxy)-4,6-diméthylphényl]-3,3-diméthylpropionyl)-16-désoxo-16-fluoro pristinamycine II_{B} sous forme d'un solide crème.

Spectre de R.M.N. ¹H (400 MHz, CDCl₃, δ en ppm) : 0,96 (d, J = 6,5 Hz : 3H) ; 1,00 (d, J = 6,5 Hz : 3H) ; 1,09 (d, J = 6,5 Hz : 3H) ; de 1,45 à 2,05 (mt : 12H) ; 1,79 (s : 3H) ; 2,13 (mt : 1H) ; 2,24 (s : 3H) ; 2,29 (mt : 2H) ; 2,54 (s : 3H) ; de 2,70 à 2,90 (mt : 5H) ; 2,91 (mt : 1H) ; 3,15 (mt : 1H) ; 3,45 (mt : 1H) ; 3,56 (t, J = 6,5 Hz : 2H) ; 3,86 (mt : 1H) ; 4,01 (mt : 1H) ; 4,53 (mt : 1H) ; de 4,75 à 4,95 (mt : 1H) ; 4,77 (dd, J = 10 et 2 Hz : 1H) ; 4,80 (dd, J = 9 et 3 Hz : 1H) ; 5,12 (d, J = 9 Hz : 1H) ; de 5,60 à 5,75 (mt : 2H) ; 5,81 (dd, J = 16 et 2 Hz : 1H) ; 5,92 (mt : 1H) ; 6,13 (d, J = 16 Hz : 1H) ; 6,50 (dd, J = 16 et 5 Hz : 1H) ; 6,59 (d, J = 1,5 Hz : 1H) ; 6,82 (d, J = 1,5 Hz : 1H) ; 8,12 (s : 1H).

L'acide 3-[2-(4-bromobutyryloxy)-4,6-diméthylphényl]-3,3-diméthylpropionique peut être préparé de la manière suivante :

A 2,3 g de 3-[2-(4-bromobutyryloxy)-4,6-diméthylphényl]-3,3-diméthylpropanal en solution dans 200 cm³ d'acétone, on ajoute goutte à goutte à 20°C, une solution de 1 g de permanganate de potassium dans un mélange de 46 cm³ d'eau distillée et de 30 cm³ d'acétone. Après 24 heures d'agitation, le mélange réactionnel est additionné de 100 cm' d'eau distillée, acidifié jusqu'à pH 1-2 par ajout d'une solution aqueuse d'acide chlorhydrique 1 N, puis extrait par cinq fois 100 cm³ de dichlorométhane. Les phases organiques sont réunies, séchées sur du sulfate de magnésium, filtrées puis concentrées à sec sous pression réduite (2,7 kPa). On obtient 2,7 g d'acide 3-[2-(4-bromobutyryloxy)-4,6-diméthylphényl]-3,3-diméthylpropionique sous la forme d'une huile incolore.

Spectre de R.M.N. ¹H (400 MHz, CDCl₃, δ en ppm); (2 conformères dans les proportions 80-20) : 1,58 et 1,59 (2 s : 6H en totalité) ; 2,23 et 2,24 (2 s : 3H en totalité) ; 2,29 (mt : 2H) ; 2,54 et 2,55 (2 s : 3H en totalité) ; 2,78 (t, J = 7 Hz : 2H) ; 2,84 et 2,87 (2 s : 2H en totalité) ; 3,55 (t, J = 6,5 Hz : 2H) ; 6,57 et 6,59 (2 s larges : 1H en totalité) ; 6,81 et 6,83 (2 s larges : 1H en totalité).

Le 3-[2-(4-bromobutyryloxy)-4,6-diméthylphényl]-3,3-diméthylpropanal peut être préparé de la manière suivante :

A 3,1 g de chlorochromate de pyridinium en suspension dans 900 cm³ de dichlorométhane, on ajoute goutte à goutte, à 24°C, 2,5 g de 3-[2-(4-bromobutyryloxy)-4,6-diméthylphényl]-3,3-diméthylpropan-1-ol en solution dans 160 cm³ de dichlorométhane. Après 2 heures d'agitation à 24°C, le mélange réactionnel est filtré sur 630 g de silice (granulométrie 0,063-0,2 mm) en éluant successivement avec du dichlorométhane pur, puis un mélange de dichlorométhane et d'acétate d'éthyle (80 / 20 en volumes). On obtient ainsi 2,4 g de 3-[2-(4-bromobutyryloxy)-4,6-diméthylphényl]-3,3-diméthylpropanal sous forme d'une huile visqueuse incolore.

Spectre de R.M.N. ¹H (400 MHz, CDCl₃, δ en ppm); (2 conformères dans les proportions 80-20) : 1,57 (s : 6H) ; 2,25 (s : 3H) ; 2,29 (mt : 2H) ; 2,55 (s : 3H) ; 2,78 (t, J = 7 Hz : 2H) ; de 2,80 à 2,90 (mt : 2H) ; 3,56 (t, J = 6 Hz : 2H) ; 6,59 et 6,61 (2 s larges : 1H en totalité) ; 6,85 et 6,87 (2 s larges : 1H en totalité) ; 9,55 (s large : 1H).

Le 3-[2-(4-bromobutyryloxy)-4,6-diméthylphényl]-3,3-diméthylpropan-1-ol peut être préparé de la manière suivante :

A 5,4 g de 4-bromobutyrate de 2-{3-(tert-butyldiméthylsilyloxy])1,1-diméthylpropyl}-3,5-diméthylphénol en solution dans 60 cm³ de dichlorométhane, on ajoute goutte à goutte, à 0°C, sous atmosphère d'argon, 2,24 cm' de trifluorhydrate de triéthylamine. Après 42 heures d'agitation à 20°C, le mélange réactionnel est filtré sur 750 g de silice (granulométrie 0,063-0,2 mm) en éluant successivement avec du dichlorométhane, puis avec un mélange dichlorométhane / acétate d'éthyle (90 / 10 en volumes). On obtient ainsi 2,6 g de 3-[2-(4-bromobutyryloxy)-4,6-diméthylphényl]-3,3-diméthylpropan-1-ol sous forme d'une huile visqueuse incolore.

Spectre de R.M.N. ¹H (400 MHz, CDCl₃, δ en ppm); (2 conformères dans les proportions 80-20) : 1,50 et 1,52 (2 s : 6H en totalité) ; de 2,00 à 2,10 (mt : 2H) ; 2,23 et 2,24 (2 s : 3H en totalité) ; 2,30 (mt : 2H) ; 2,54 (s : 3H) ; 2,78 (t, J = 7 Hz : 2H) ; de 3,50 à 3,60 (mt : 4H) ; 6,55 et 6,56 (2 s larges : 1H en totalité) ; 6,82 et 6,84 (2 s larges : 1H en totalité).

Le 4-bromobutyrate de 2-{3-(tert-butyldiméthylsilyloxy)-1,1-diméthylpropyl}-3,5-diméthylphénol peut être préparé de la manière suivante :

A 3,22 g de 2-{3-(tert-butyldiméthylsilyloxy)-1,1-diméthylpropyl}-3,5-diméthylphénol en solution dans 60 cm³ de tétrahydrofurane, on ajoute à 20°C sous atmosphère d'argon et par petites portions, 0,32 g d'hydrure de sodium (à 75 % dans de l'huile minérale), puis 15 minutes plus tard 1,16 cm³ de chlorure de 4-bromobutyryle. Après 40 minutes d'agitation, on ajoute 100 cm³ d'éther, 10 cm³ d'eau distillée et 10 cm³ d'une solution aqueuse saturée de chlorure de sodium. Après agitation, la phase organique est décantée, séchée sur sulfate de magnésium, filtrée puis concentrée à sec sous pression réduite (2,7 kPa) pour donner un résidu qui est filtré sur 75 g de silice (granulométrie 0,063-0,2 mm) en éluant successivement avec du cyclohexane, puis avec un mélange cyclohexane / acétate d'éthyle (90 / 10 en volumes). On obtient ainsi 2 g de 4-bromobutyrate de 2-{3-(tert-butyldiméthylsilyloxy)-1,1-diméthylpropyl}-3,5-diméthylphénol sous forme d'une huile incolore.

Spectre de R.M.N. ¹H (300 MHz, CDCl₃, δ en ppm); (2 conformères dans les proportions 80-20) : 0,00 et 0,03 (2 s : 6H en totalité) ; 0,87 et 0,89 (2 s : 9H en totalité) ; de 1,45 à 1,55 (mt : 6H) ; 2,05 (t, J = 7,5 Hz : 2H) ; 2,24 et 2,25 (2 s : 3H en totalité) ; 2,31 (mt : 2H) ; 2,54 (s : 3H) ; 2,77 (t, J = 7,5 Hz : 2H) ; de 3,40 à 3,65 (mt : 4H) ; 6,56 et 6,58 (2 s larges : 1H en totalité) ; 6,81 et 6,83 (2 s larges : 1H en totalité).

Le 2-{3-(tert-butyldiméthylsilyloxy)-1,1-diméthylpropyl}-3,5-diméthyl-phénol peut être préparé selon Amsberry K. L., Gerstenberger A. E., Borchardt R. T., Pharm. Res. 1991, 8(4), 455-61.

### Exemple 27

### Méthanesulfonate de la (16R)-16-Désoxo-16-fluoro-14-O-[4-(morpholin-4-yl) butyryl] pristinamycine II_{B}

A 0,453 g de (16*R*)-16-désoxo-16-fluoro-14-O-[4-(morpholin-4-yl) butyryl] pristinamycine II_{B} en solution dans 10 cm³ d'éthanol, on ajoute à 20°C, 6,58 cm³ d'une solution éthanolique d'acide méthanesulfonique 0,1 N. Après 10 minutes d'agitation, le mélange réactionnel est concentré à sec sous pression réduite (2,7 kPa) pour donner un résidu qui est agité dans 10 cm³ d'éther éthylique. Après filtration, rinçage du solide par 4 cm³ d'éther diéthylique et séchage sous pression réduite (2,7 kPa) à 20°C, on obtient 0,506 g du méthanesulfonate de la (16*R*)-16-désoxo-16-fluoro-14-O-[4-(morpholin-4-yl) butyryl] pristinamycine II_{B}, sous forme d'un solide blanc fondant vers 122°C (déc.).

Spectre de R.M.N. ¹H (400 MHz, CDCl₃, δ en ppm) : 0,96 (d, J = 6,5 Hz : 3H) ; 0,99 (d, J = 6,5 Hz : 3H) ; 1,10 (d, J = 6,5 Hz : 3H) ; de 1,60 à 2,00 (mt : 5H) ; 1,89 (s : 3H) ; de 2,10 à 2,30 (mt : 4H) ; 2,48 (t, J = 7 Hz : 2H) ; 2,77 (mt : 1H) ; 2,82 (s : 3H) ; 2,88 (mt : 2H) ; 2,99 (dt, J = 17 et 6 Hz : 1H) ; 3,13 (mt : 2H) ; 3,23 (dt, J = 17 et 6 Hz : 1H) ; de 3,45 à 3,60 (mt : 3H) ; 3,82 (mt : 1H) ; de 3,95 à 4,20 (mt : 5H) ; 4,53 (mt : 1H) ; 4,79 (dd, J = 10 et 2 Hz : 1H) ; 4,83 (dd, J = 9 et 3,5 Hz : 1H) ; 5,11 (doublet démultiplié, J _{HF} = 48 Hz : 1H) ; 5,28 (d, J = 10 Hz : 1H) ; de 5,75 à 5,85 (mt : 2H) ; 5,84 (dd, J = 17 et 2 Hz : 1H) ; 6,09 (mt : 1H) ; 6,19 (d, J = 16 Hz : 1H) ; 6,53 (dd, J = 17 et 5 Hz : 1H) ; 8,11 (s : 1H).

La (16*R*)-16-désoxo-16-fluoro-14-O-[4-(morpholin-4-yl) butyryl] pristinamycine II_{B} peut être préparée de la manière suivante :

A 1,3 g de (16*R*)-16-désoxo-16-fluoro pristinamycine II_{B} (préparée comme décrit dans l'exemple 1) en solution dans 35 cm³ de dichlorométhane, on ajoute à 20°C, 0,512 g de chlorhydrate d'acide 4-(morpholin-4-yl) butyrique et 0,343 cm³ de triéthylamine en solution dans 35 cm³ dichlorométhane, 0503 g de N,N'-dicyclohexylcarbodiimide et 0,03 g de 4-diméthylaminopyridine. Après 16 heures d'agitation à 20°C, le mélange réactionnel est concentré à sec sous pression réduite (2,7 kPa). Le résidu est repris dans 10 cm³ de dichlorométhane puis filtré. Le filtrat est dilué avec 40 cm³ de dichlorométhane, lavé par 50 cm³ d'eau puis séché sur sulfate de magnésium, filtré et concentré à sec sous pression réduite (2,7 kPa) pour donner 1 g d'un solide jaune qui est purifié par chromatographie-flash [éluant : gradient dichlorométhane / méthanol / acétonitrile (98 / 1 / 1 puis 96 / 2 / 2 puis 94 / 3 / 3 en volumes)]. On obtient ainsi, 0,584 g d'un solide blanc qui est repris dans 20 cm³ de dichlorométhane. La solution obtenue est lavée par 20 cm³ d'eau, séchée sur sulfate de magnésium, filtrée puis concentrée à sec sous pression réduite (2,7 kPa) pour donner, après agitation dans du pentane, filtration et séchage (2,7 kPa) à 20°C, 0,524 g de (16*R*)-16-désoxo-16-fluoro-14-O-[4-(morpholin-4-yl)butyryl] pristinamycine II_{B}, sous forme d'un solide blanc fondant vers 100°C (déc.).

Spectre de R.M.N. ¹H (400 MHz, CDCl₃, δ en ppm) : 0,96 (d, J = 6,5 Hz : 3H) ; 1,00 (d, J = 6,5 Hz : 3H) ; 1,10 (d, J = 6,5 Hz : 3H) ; de 1,60 à 2,05 (mt : 7H) ; 1,89 (s : 3H) ; 2,16 (mt : 1H) ; 2,23 (mt : 1H) ; 2,35 (mt : 4H) ; 2,43 (mt : 4H) ; 2,76 (mt : 1H) ; 2,99 (dt, J = 17 et 6 Hz : 1H) ; 3,24 (mt : 1H) ; 3,49 (mt : 1H) ; 3,71 (mt : 4H) ; 3,85 (mt : 1H) ; 4,07 (mt : 1H) ; 4,54 (mt : 1H) ; 4,78 (dd, J = 10 et 1,5 Hz : 1H) ; 4,82 (dd, J = 9 et 3 Hz : 1H) ; 5,11 (doublet démultiplié, J _{HF} = 48 Hz : 1H) ; 5,30 (d, J = 9 Hz : 1H) ; de 5,75 à 5,85 (mt : 1H) ; 5,76 (mt : 1H) ; 5,81 (dd, J = 17 et 1,5 Hz : 1H) ; 5,95 (mt : 1H) ; 6,19 (d, J = 16 Hz : 1H) ; 6,51 (dd, J = 17 et 5 Hz : 1H) ; 8,12 (s : 1H).

Le chlorhydrate de l'acide 4-(morpholin-4-yl)butyrique peut être préparé selon Raj K. Razdan, Barbara Zitko Terris, Harry G. Pars, J. Med. Chem. 1976, 19 (4), 454 - 461.

### Exemple 28

### (16R)-16-Désoxo-16-fluoro-14-O (4-imidazol-1-yl butyryl) pristinamycine II_{B}

A 600 mg de (16*R*)-14-O-(4-bromobutyryl)-16-désoxo-16-fluoro pristinamycine II_{B} en solution dans 2,5 cm³ de diméthylformamide, on ajoute 150 mg d'imidazole. Après 4 heures d'agitation à 60°C, on ajoute 0,5 g d'imidazole supplémentaire et l'agitation est poursuivie 2 heures à 65°C. Le mélange est concentré sous pression réduite (2,7 kPa) pour donner un résidu qui est repris par 20 cm³ d'eau distillée et 25 cm³ de dichlorométhane. La phase organique est décantée puis lavée par 2 fois 20 cm³ d'eau distillée. La phases organique est séchée sur sulfate de magnésium, filtrée, puis concentrée à sec sous pression réduite (2,7 kPa) pour donner 1,13 g d'un solide qui est purifié par chromatographie-flash [éluant : dichlorométhane-méthanol (98-2 en volumes puis 95-5 en volumes)]. On obtient ainsi 325 mg d'un produit qui est agité dans l'éther diéthylique, filtré puis séché à 20°C (90 Pa) pour donner 248 mg de (16*R*)-16-désoxo-16-fluoro-14-O-(4-imidazol-1-yl butyryl) pristinamycine II_{B}, sous forme d'un solide beige fondant à 125°C.

Spectre de R.M.N. ¹H (400 MHz, CDCl₃, δ en ppm) : 0,96 (d, J = 6,5 Hz : 3H) ; 1,00 (d, J = 6,5 Hz : 3H) ; 1,10 (d, J = 6,5 Hz : 3H) ; de 1,60 à 2,05 (mt : 5H) ; 1,90 (s : 3H) ; de 2,00 à 2,35 (mt : 2H) ; 2,08 (mt : 2H) ; 2,26 (mt : 2H) ; 2,76 (mt : 1H) ; 2,99 (dt, J = 17 et 6,5 Hz : 1H) ; 3,24 (mt : 1H) ; 3,50 (mt : 1H) ; 3,94 (mt : 1H) ; 4,01 (t, J = 7 Hz : 2H) ; 4,06 (mt : 1H) ; 4,54 (mt : 1H) ; 4,79 (dd, J = 10 et 2 Hz : 1 H) ; 4,82 (dd, J = 9 et 3 Hz : 1H) ; 5,11 (doublet démultiplié, J _{HF} = 48 Hz : 1H) ; 5,28 (d, J = 9 Hz : 1H) ; de 5,75 à 5,90 (mt : 1H) ; 5,78 (mt : 1H) ; 5,84 (dd, J = 16 et 1,5 Hz : 1H) ; 6,09 (mt : 1H) ; 6,20 (d, J = 16 Hz : 1H) ; 6,53 (dd, J = 16 et 5 Hz : 1H) ; 6,91 (s : 1H) ; 7,07 (s : 1H) ; 7,44 (s : 1H) ; 8,12 (s : 1H).

La (16*R*)-14-O-(4-Bromobutyryl)-16-désoxo-16-fluoro pristinamycine II_{B} peut être préparée de la façon suivante :

A 5 g de (16*R*)-16-désoxo-16-fluoro pristinamycine II_{B} (préparée comme décrit dans l'exemple 1) en solution dans 150 cm³ de dichlorométhane, on ajoute 2,64 cm³ de triéthylamine puis 2,3 cm³ de chlorure de l'acide 4-bromobutyrique. Après 18 heures d'agitation à 25°C, on ajoute 1,32 cm³ de triéthylamine et 1,15 cm³ de chlorure de l'acide 4-bromobutyrique supplémentaire. Le mélange réactionnel est agité pendant 2 heures à 25°C puis lavé par 2 fois 100 cm³ d'eau distillée. La phase organique est séchée sur sulfate de magnésium, filtrée, puis concentrée à sec sous pression réduite (2,7 kPa) pour donner 8,19 g d'une huile brune qui est purifiée par chromatographie-flash [éluant : dichlorométhane-méthanol (97-3 en volumes)]. On obtient ainsi 3,4 g d'un produit qui est agité dans de l'éther diisopropylique, filtré, séché puis repurifié par chromatographie-flash [éluant : dichlorométhane-méthanol (98-2 en volumes)]. On obtient ainsi, après agitation dans de l'éther diisopropylique, filtration et séchage à 20°C (90 Pa), 1,32 g de (16*R*)-14-O-(4-bromobutyryl)-16-désoxo-16-fluoro pristinamycine II_{B}, sous forme d'un solide blanc cassé utilisé tel quel.

Spectre de R.M.N. ¹H (400 MHz, CDCl₃, δ en ppm) : 0,96 (d, J = 6,5 Hz : 3H) ; 1,00 (d, J = 6,5 Hz : 3H) ; 1,10 (d, J = 6,5 Hz : 3H) ; de 1,65 à 2,05 (mt : 5H) ; 1,89 (s : 3H) ; de 2,10 à 2,35 (mt : 2H) ; 2,17 (mt : 2H) ; 2,49 (mt : 2H) ; 2,77 (mt : 1H) ; 2,99 (mt : 1H) ; 3,24 (mt : 1H) ; de 3,45 à 3,55 (mt : 1H) ; 3,47 (t, J = 6,5 Hz : 2H) ; 3,86 (mt : 1H) ; 4,06 (mt : 1H) ; 4,54 (mt : 1H) ; 4,78 (dd, J = 10 et 1,5 Hz : 1H) ; 4,82 (dd, J = 9 et 3 Hz : 1H) ; 5,13 (doublet démultiplié, J _{HF} = 48 Hz : 1H) ; 5,31 (d, J = 9 Hz : 1H) ; de 5,75 à 5,90 (mt : 1H) ; 5,77 (mt : 1H) ; 5,82 (dd, J = 16 et 2 Hz : 1H) ; 5,93 (mt : 1H) ; 6,20 (d, J = 15 Hz : 1H) ; 6,52 (dd, J = 16 et 5 Hz : 1H) ; 8,12 (s : 1H).

### Exemple 29

### (16R)-16-Désoxo-16-fluoro-14-O-{4-[4-(pyrrolidin-1-yl carbonylméthyl) pipérazin-1-yl) butyryl}pristinamycine II_{B}

A 680 mg de (16*R*)-14-O-(4-bromobutyryl)-16-désoxo-16-fluoro pristinamycine II_{B} (préparée comme décrit dans l'exemple 28) en solution dans 3 cm³ de diméthylformamide, on ajoute 590 mg de 1-(pyrrolidinocarbonylméthyl) pipérazine. Après 4 heures d'agitation à 60°C, le mélange est concentré sous pression réduite (2,7 kPa) pour donner un résidu qui est repris par 40 cm³ d'eau distillée et 20 cm³ de dichlorométhane. Après ajout de chlorure de sodium, la phase aqueuse est décantée puis extraite par 2 fois 20 cm³ de dichlorométhane. Les phases organiques sont réunies, séchées sur sulfate de magnésium, filtrées, puis concentrées à sec sous pression réduite (2,7 kPa) pour donner 1 g d'un solide marron qui est purifié par chromatographie-flash [éluant : dichlorométhane-méthanol (95-5 en volumes)]. On obtient ainsi 650 mg d'un produit qui est agité dans l'éther diéthylique pendant 1 heure, filtré puis séché à 20°C (90 Pa), pour donner 413 mg de (16*R*)-16-désoxo-16-fluoro-14-O-{4-[4-(pyrrolidin-1-yl carbonylméthyl) pipérazin-1-yl) butyryl} pristinamycine II_{B}, sous forme d'un solide blanc cassé fondant à 128°C.

Spectre de R.M.N. ¹H (400 MHz, CDCl₃, δ en ppm) : 0,96 (d, J = 6,5 Hz : 3H) ; 1,00 (d, J = 6,5 Hz : 3H) ; 1,10 (d, J = 6,5 Hz : 3H) ; de 1,60 à 2,05 (mt : 11H) ; 1,88 (s : 3H) ; de 2,10 à 2,35 (mt : 2H) ; 2,32 (t, J = 7,5 Hz : 2H) ; 2,40 (mf : 2H) ; de 2,50 à 2,70 (mf : 8H) ; 2,75 (mt : 1H) ; 2,99 (dt, J = 17 et 6 Hz : 1H) ; 3,12 (s : 2H) ; 3,24 (mt : 1H) ; de 3,40 à 3,55 (mt : 5H) ; 3,86 (mt : 1H) ; 4,06 (mt : 1H) ; 4,53 (mt : 1H) ; 4,78 (dd, J = 10 et 1,5 Hz : 1H) ; 4,82 (dd, J = 9 et 3 Hz : 1H) ; 5,09 (doublet démultiplié, J _{HF} = 48 Hz : 1H) ; 5,29 (d, J = 9 Hz : 1H) ; de 5,75 à 5,85 (mt : 1H) ; 5,75 (mt : 1H) ; 5,83 (dd, J = 17 et 1,5 Hz : 1H) ; 6,05 (mt : 1H) ; 6,18 (d, J = 16 Hz : 1H) ; 6,52 (dd, J = 17 et 5 Hz : 1H) ; 8,12 (s : 1H).

### Exemple 30

### Méthanesulfonate de la (16R)-16-Désoxo-16-fluoro-14-O-[5-(morpholin-4-yl) pentanoyl] pristinamycine II_{B}

A 0,79 g de (16*R*)-16-désoxo-16-fluoro-14-O-[5-(morpholin-4-yl) pentanoyl] pristinamycine II_{B} en solution dans 15 cm³ d'éthanol, on ajoute à 20°C, 11 cm³ d'une solution éthanolique d'acide méthanesulfonique 0,1 N. Après 20 minutes d'agitation, le mélange réactionnel est concentré à sec sous pression réduite (2,7 kPa) pour donner un résidu qui est agité dans 10 cm³ d'éther. Après filtration, rinçage du solide par 5 cm³ d'éther diéthylique et séchage sous pression réduite (2,7 kPa) à 20°C, on obtient 0,89 g du méthanesulfonate de la (16*R*)-16-désoxo-16-fluoro-14-O-[5-(morpholin-4-yl) pentanoyl] pristinamycine II_{B}, sous forme d'un solide blanc cassé fondant vers 120°C (déc.).

Spectre de R.M.N. ¹H (400 MHz, (CD₃)₂SO d6, δ en ppm) : 0,85 (d, J = 6,5 Hz : 3H) ; 0,94 (d, J = 6,5 Hz : 3H) ; 1,03 (d, J = 6,5 Hz : 3H) ; de 1,45 à 1,70 (mt : 5H) ; de 1,70 à 2,25 (mt : 6H) ; 1,77 (s : 3H) ; 2,32 (s : 3H) ; 2,39 (t, J = 7 Hz : 2H) ; 2,77 (mt : 1H) ; de 2,95 à 3,50 (mt : 8H) ; de 3,50 à 3,70 (mt : 4H) ; 3,80 (mt : 1H) ; de 3,90 à 4,05 (mt : 3H) ; de 4,70 à 4,80 (mt : 2H) ; 5,09 (doublet démultiplié, J _{HF} = 48 Hz : 1H) ; 5,42 (d, J = 9,5 Hz : 1H) ; 5,62 (mt : 1H) ; 5,75 (mt : 1H) ; 5,80 (d large, J = 16 Hz : 1H) ; 6,18 (d, J = 16 Hz : 1H) ; 6,62 (dd, J = 16 et 4 Hz : 1H) ; 8,15 (mt : 1H) ; 8,52 (s : 1H) ; 9,46 (mf large : 1H).

La (16*R*)-16-désoxo-16-fluoro-14-O-[5-(morpholin-4-yl) pentanoyl] pristinamycine II_{B} peut être préparée de la manière suivante :

A 2 g de (16*R*)-16-désoxo-16-fluoro-14-O-(5-chloropentanoyl) pristinamycine II_{B} en solution dans 30 cm³ de tétrahydrofurane, on ajoute à 20°C, 0,46 g d'iodure de sodium et 0,54 cm³ de morpholine. Après 27 heures d'agitation à reflux, on ajoute 0,54 cm³ de morpholine supplémentaire. Après 16 heures d'agitation supplémentaires à reflux, on ajoute 1 cm³ de diméthylformamide et 0,54 cm³ de morpholine. Après encore 24 heures d'agitation à reflux, le mélange réactionnel est dilué par 20 cm³ de dichlorométhane et lavé par 50 cm³ d'eau. La phase organique est décantée et la phase aqueuse est extraite par 50 cm³ de dichlorométhane. Les phases organiques sont réunies, lavées par 3 fois 150 cm³ d'eau, séchées sur sulfate de magnésium, filtrées puis concentrées à sec sous pression réduite (2,7 kPa) pour donner 2 g d'un résidu qui est purifié par chromatographie-flash [éluant : dichlorométhane / méthanol / acétonitrile (92 / 4 / 4 en volumes)]. On obtient ainsi, après agitation dans du pentane, filtation et séchage (2,7 kPa) à 20°C, 0,81 g de (16*R*)-16-désoxo-16-fluoro-14-O-[5-(morpholin-4-yl) pentanoyl] pristinamycine II_{B}, sous forme d'un solide beige.

Spectre de R.M.N. ¹H (400 MHz, CDCl₃, δ en ppm) : 0,96 (d, J = 6,5 Hz : 3H) ; 1,00 (d, J = 6,5 Hz : 3H) ; 1,10 (d, J = 6,5 Hz : 3H) ; 1,52 (mt : 2H) ; de 1,55 à 2,05 (mt : 7H) ; 1,89 (s : 3H) ; de 2,10 à 2,35 (mt : 2H) ; 2,32 (mt : 4H) ; 2,42 (mf : 4H) ; 2,76 (mt : 1H) ; 2,99 (dt, J = 17 et 6 Hz : 1H) ; 3,24 (mt : 1H) ; 3,48 (mt : 1H) ; 3,72 (t, J = 5 Hz : 4H) ; 3,86 (mt : 1H) ; 4,07 (mt : 1H) ; 4,53 (mt : 1H) ; 4,78 (dd, J = 10 et 2 Hz : 1H) ; 4,82 (dd, J = 9 et 3 Hz : 1H) ; 5,11 (doublet démultiplié, J _{HF} = 48 Hz : 1H) ; 5,30 (d, J = 9 Hz : 1H) ; de 5,70 à 5,85 (mt : 2H) ; 5,82 (dd, J = 16,5 et 2 Hz : 1H) ; 5,96 (mt : 1H) ; 6,19 (d, J = 16 Hz : 1H) ; 6,51 (dd, J = 16,5 et 5 Hz : 1H) ; 8,12 (s : 1H).

La (16*R*)-16-désoxo-16-fluoro-14-O-(5-chloropentanoyl) pristinamycine II_{B} peut être préparée de la manière suivante :

A 4 g de (16*R*)-16-désoxo-16-fluoro pristinamycine II_{B} (préparée comme décrit dans l'exemple 1) en solution dans 100 cm³ de dichlorométhane, on ajoute à 28°C, 2,1 cm³ de triéthylamine, 2 cm³ de chlorure de 5-chloropentanoyle et 0,18 g de 4-diméthylaminopyridine. Après 2 heures d'agitation à 28°C, le mélange réactionnel est versé sur 100 cm³ d'eau. La phase organique est décantée et la phase aqueuse est extraite par 100 cm³ de dichlorométhane. Les phases organiques sont réunies, lavées par 2 fois 200 cm³ d'eau, séchées sur sulfate de magnésium, filtrées puis concentrées à sec sous pression réduite (2,7 kPa) pour donner 6 g d'un solide marron qui est purifié par chromatographie-flash [éluant : dichlorométhane / méthanol (98 / 2 en volumes)]. On obtient ainsi, après agitation dans du pentane, filtation et séchage (2,7 kPa) à 20°C, 3,77 g de (16*R*)-16-désoxo-16-fluoro-14-O-(5-chloropentanoyl) pristinamycine II_{B}, sous forme d'un solide blanc.

Spectre de R.M.N. ¹H (400 MHz, CDCl₃, δ en ppm) : 0,96 (d, J = 6,5 Hz : 3H) ; 1,00 (d, J = 6,5 Hz : 3H) ; 1,10 (d, J = 6,5 Hz : 3H) ; de 1,65 à 2,05 (mt : 9H) ; 1,89 (s : 3H) ; 2,15 (mt : 1H) ; 2,24 (mt : 1H) ; 2,33 (t, J = 7 Hz : 2H) ; 2,76 (mt : 1H) ; 2,99 (mt : 1H) ; 3,24 (mt : 1H) ; 3,49 (mt : 1H) ; 3,55 (t, J = 7 Hz : 2H) ; 3,86 (mt : 1H) ; 4,06 (mt : 1H) ; 4,52 (mt : 1H) ; 4,78 (dd, J = 10 et 2 Hz : 1H) ; 4,82 (dd, J = 9 et 3 Hz : 1H) ; 5,11 (doublet démultiplié, J _{HF} = 48 Hz : 1H) ; 5,30 (d, J = 9 Hz : 1H) ; de 5,75 à 5,85 (mt : 1H) ; 5,76 (mt : 1H) ; 5,82 (dd, J = 17 et 2 Hz : 1H) ; 5,96 (mt : 1H) ; 6,19 (d, J = 16 Hz : 1H) ; 6,51 (dd, J = 17 et 5 Hz : 1H) ; 8,13 (s : 1H).

### Exemple 31

### Méthanesulfonate de la (16R)-16-Désoxo-16-fluoro-14-O-[5-(imidazol-1-yl) pentanoyl] pristinamycine II_{B}

A 0,66 g de (16*R*)-16-désoxo-16-fluoro-14-O-[5-(imidazol-1-yl) pentanoyl] pristinamycine II_{B} en solution dans 10 cm³ d'éthanol, on ajoute à 20°C, 10,2 cm³ d'une solution éthanolique d'acide méthanesulfonique 0,095 N. Après 10 minutes d'agitation, le mélange réactionnel est concentré à sec sous pression réduite (2,7 kPa) pour donner un résidu qui est agité dans 20 cm³ d'éther. Après filtration, lavage du solide par 5 cm³ d'éther diéthylique et séchage sous pression réduite (2,7 kPa), on obtient 0,712 g du méthanesulfonate de la (16*R*)-16-désoxo-16-fluoro-14-O-[5-(imidazol-1-yl) pentanoyl] pristinamycine II_{B}, sous forme d'un solide jaune fondant vers 126°C (déc.).

Spectre de R.M.N. ¹H (400 MHz, (CD₃)₂SO d6, δ en ppm) : 0,85 (d, J = 6,5 Hz : 3H) ; 0,94 (d, J = 6,5 Hz : 3H) ; 1,03 (d, J = 6,5 Hz : 3H) ; de 1,40 à 1,60 (mt : 3H) ; de 1,70 à 2,30 (mt : 8H) ; 1,76 (s : 3H) ; 2,32 (s : 3H) ; 2,37 (t, J = 7 Hz : 2H) ; 2,77 (mt : 1H) ; de 3,10 à 3,40 (mt : 2H) ; 3,58 (mt : 1H) ; 3,67 (mt : 1H) ; 3,80 (mt : 1H) ; 3,97 (mt : 1H) ; 4,21 (t, J = 7 Hz : 2H) ; de 4,65 à 4,80 (mt : 2H) ; 5,08 (doublet démultiplié, J _{HF} = 48 Hz : 1H) ; 5,40 (d, J = 9 Hz : 1H) ; 5,61 (mt : 1H) ; 5,73 (mt : 1H) ; 5,80 (d large, J = 16 Hz : 1H) ; 6,18 (d, J = 16 Hz : 1H) ; 6,63 (dd, J = 16 et 4 Hz : 1H) ; 7,72 (s large : 1H) ; 7,80 (s large : 1H) ; 8,15 (t, J = 6 Hz : 1H) ; 8,52 (s : 1H) ; 9,14 (s large : 1H).

La (16*R*)-16-désoxo-16-fluoro-14-O-[5-(imidazol-1-yl) pentanoyl] pristinamycine II_{B} peut être préparée de la manière suivante :

A 1,4 g de (16*R*)-16-désoxo-16-fluoro-14-O-(5-chloropentanoyl) pristinamycine II_{B} (préparée comme décrit dans l'exemple 30) en solution dans 20 cm³ de tétrahydrofurane, on ajoute à 20°C, 0,29 g d'imidazole et 0,32 g d'iodure de sodium. Après 20 heures d'agitation à reflux, on ajoute 1 cm³ de diméthylformamide et 0,29 g d'imidazole. Après 8,5 heures d'agitation supplémentaires à reflux, on ajoute 0,29 g d'imidazole. Après encore 15,5 heures d'agitation à reflux, le mélange réactionnel est dilué par 50 cm³ de dichlorométhane puis lavé par 100 cm³ d'eau. La phase organique est décantée et la phase aqueuse est extraite par 50 cm³ de dichlorométhane. Les phases organiques sont réunies, séchées sur sulfate de magnésium, filtrées puis concentrées à sec sous pression réduite (2,7 kPa) pour donner 1,6 g d'un solide orange qui est purifié par deux chromatographies-flash successives [éluant : dichlorométhane / méthanol (97 / 3 en volumes)]. On obtient ainsi, après agitation dans de l'éther éthylique, filtration et séchage (2,7 kPa) à 20°C, 0,66 g de (16*R*)-16-désoxo-16-fluoro-14-O-[5-(imidazol-1-yl) pentanoyl] pristinamycine II_{B}, sous forme d'un solide jaune.

Spectre de R.M.N. ¹H (400 MHz, CDCl₃, δ en ppm) : 0,96 (d, J = 6,5 Hz : 3H) ; 1,00 (d, J = 6,5 Hz : 3H) ; 1,11 (d, J = 6,5 Hz : 3H) ; de 1,60 à 2,05 (mt : 9H) ; 1,90 (s : 3H) ; de 2,10 à 2,25 (mt : 2H) ; 2,31 (t, J = 7 Hz : 2H) ; 2,76 (mt : 1H) ; 3,00 (mt : 1H) ; 3,25 (dt, J = 17 et 5,5 Hz : 1H) ; 3,51 (mt : 1H) ; 3,84 (mt : 1H) ; 3,94 (t, J = 7 Hz : 2H) ; 4,06 (mt : 1 H) ; 4,56 (mt : 1H) ; de 4,75 à 4,90 (mt : 2H) ; 5,06 (doublet démultiplié, J _{HF} = 48 Hz : 1H) ; 5,24 (d, J = 9 Hz : 1H) ; de 5,75 à 5,90 (mt : 2H) ; 5,90 (dd, J = 16 et 1,5 Hz : 1H) ; 6,17 (d, J = 15 Hz : 1H) ; 6,52 (mt : 1H) ; 6,57 (dd, J = 16 et 5 Hz : 1H) ; 6,89 (s : 1H) ; 7,06 (s : 1H) ; 7,45 (s : 1H) ; 8,10 (s : 1H).

### Exemple 32

### (16R)-16-Désoxo-16-fluoro-14-O-{5-[4-(pyrrolidin-1-yl carbonylméthyl) pipérazin-1-yl] pentanoyl} pristinamycine II_{B}

A 1,8 g de (16*R*)-16-désoxo-16-fluoro-14-O-(5-chloropentanoyl) pristinamycine II_{B} (préparée comme décrit dans l'exemple 30) en solution dans 20 cm³ de diméthylformamide, on ajoute à 20°C, 1,1 g de (pyrrolidin-1-yl carbonylméthyl) pipérazine et 0,42 g d'iodure de sodium. Après 27,5 heures d'agitation à 50°C, on ajoute 0,55 g de (pyrrolidin-1-yl carbonylméthyl) pipérazine. Après 27,5 heures d'agitation supplémentaires à 54°C, le mélange réactionnel est concentré à sec sous pression réduite (2,7 kPa) pour donner un résidu qui est dilué dans 100 cm³ de dichlorométhane. La solution obtenue est lavée par 4 fois 300 cm³ d'eau. La phase organique est séchée sur sulfate de magnésium, filtrée et concentrée à sec sous pression réduite (2,7 kPa) pour donner 1,61 g d'un solide jaune qui est purifié par chromatographie-flash [éluant : dichlorométhane / méthanol (95 / 5 en volumes)]. On obtient ainsi, après agitation dans du pentane, filtation et séchage (2,7 kPa), 0,726 g de (16*R*)-16-désoxo-16-fluoro-14-O-{5-[4-(pyrrolidin-1-yl carbonylméthyl) pipérazin-1-yl] pentanoyl} pristinamycine II_{B}, sous forme d'un solide beige fondant vers 100°C (déc.).

Spectre de R.M.N. ¹H (400 MHz, CDCl₃, δ en ppm) : 0,96 (d, J = 6,5 Hz : 3H) ; 1,00 (d, J = 6,5 Hz : 3H) ; 1,09 (d, J = 6,5 Hz : 3H) ; 1,51 (mt : 2H) ; de 1,55 à 1,80 (mt : 3H) ; de 1,75 à 2,05 (mt : 8H) ; 1,88 (s : 3H) ; 2,15 (mt : 1H) ; 2,23 (mt : 1H) ; de 2,25 à 2,40 (mt : 4H) ; 2,50 (mf : 4H) ; 2,58 (mf : 4H) ; 2,76 (mt : 1H) ; 2,99 (dt, J = 17 et 6 Hz : 1H) ; 3,11 (s : 2H) ; 3,24 (mt : 1H) ; de 3,45 à 3,55 (mt : 5H) ; 3,86 (mt : 1H) ; 4,06 (mt : 1H) ; 4,53 (mt : 1H) ; 4,78 (dd, J = 10 et 2 Hz : 1H) ; 4,82 (dd, J = 9 et 3 Hz : 1H) ; 5,09 (doublet démultiplié, J _{HF} = 48 Hz : 1H) ; 5,30 (d, J = 9 Hz : 1H) ; de 5,75 à 5,90 (mt : 1H) ; 5,75 (mt : 1H) ; 5,83 (dd, J = 17 et 1,5 Hz : 1H) ; 6,04 (mt : 1H) ; 6,19 (d, J = 16 Hz : 1H) ; 6,53 (dd, J = 17 et 5 Hz : 1H) ; 8,12 (s : 1H).

### Exemple 33

### (16R)-16-Désoxo-16-fluoro-14-O-[(RR,SS)-trans-2-(morpholinométhyl)-1-cyclopropane carbonyl] pristinamycine II_{B}, mélange de deux diastéréoisomères dans les proportions 50/50.

A 2,1 g de (16*R*)-16-désoxo-16-fluoro pristinamycine II_{B} (préparée comme décrit dans l'exemple 1) en solution dans 80 cm³ de dichlorométhane, on ajoute à 20°C sous atmosphère d'argon, 1,1 g d'acide (*RR,SS*)-*trans*-2-(morpholinométhyl)-1-cyclopropane carboxylique, 1,24 g de N,N'-dicyclohexylcarbodiimide et 0,73 g de 4-diméthylaminopyridine. Après 16 heures d'agitation, le mélange réactionnel est filtré pour éliminer l'insoluble, puis concentré à sec sous pression réduite (2,7 kPa) pour donner 4,2 g d'un résidu qui est purifié par chromatographie-flash [éluant : gradient dichlorométhane / méthanol (98 / 2 à 95 / 5 en volumes)]. On obtient un solide qui est agité dans l'éther diisopropylique, filtré et séché (2,7 kPa) à 20°C, pour donner 1,5 g de (16*R*)-16-désoxo-16-fluoro-14-O-[(*RR*,*SS*)-*trans*-2-(morpholinométhyl)-1-cyclopropane carbonyl] pristinamycine II_{B,} mélange de deux diastéréoisomères dans les proportions 50/50, sous forme d'une poudre blanchâtre.

Spectre de R.M.N. ¹H (400 MHz, CDCl₃, δ en ppm). Nous observons la présence de deux diastéréoisomères dans les proportions 50-50 : 0,77 (mt : 1H) ; 0,96 (d, J = 6,5 Hz : 3H) ; 1,00 (d, J = 6,5 Hz : 3H) ; 1,09 (d, J = 6,5 Hz : 3H) ; 1,24 (mt : 1H) ; 1,41 (mt : 1H) ; 1,52 (mt : 1H) ; de 1,60 à 2,05 (mt : 5H) ; 1,87 (s : 3H) ; de 2,05 à 2,45 (mt : 4H) ; 2,50 (mf : 4H) ; 2,76 (mt : 1H) ; 2,99 (mt : 1H) ; 3,24 (mt : 1H) ; 3,49 (mt: 1H) ; 3,73 (t, J = 5 Hz : 4H) ; 3,84 (mt : 1H) ; 4,06 (mt : 1H) ; 4,53 (mt : 1H) ; de 4,75 à 4,85 (mt : 2H) ; 5,09 (doublet démultiplié, J _{HF} = 48 Hz : 1H) ; 5,30 (mt : 1H) ; de 5,70 à 5,85 (mt : 3H) ; 5,96 et 6,03 (2 mts : 1H en totalité) ; 6,19 (d, J = 16 Hz : 1H) ; 6,51 (mt : 1H) ; 8,12 (s : 1H).

L'acide (*RR*,*SS*)-*trans*-2-(morpholinométhyl)-1-cyclopropane carboxylique peut être préparé de la manière suivante :

A 1,0 g de (*RR*,*SS*)-*trans*-2-(morpholinoinéthyl)-1-cyclopropane carboxylate d'éthyle en solution dans 50 cm³ d'éthanol, on ajoute à 20°C, 10 cm³ de solution aqueuse d'hydroxyde de sodium 1 N. Après 2 heures d'agitation à 80°C, l'éthanol est concentré sous pression réduite (2,7 kPa) et la phase aqueuse résiduelle est lavée par 50 cm³ d'acétate d'éthyle. La phase aqueuse est ajusté à pH 6 par addition d'une solution aqueuse d'acide chlorhydrique 1 N, puis concentrée à sec sous pression réduite (2,7 kPa). Par deux fois le résidu est recouvert par 50 cm³ de toluène et concentré à sec sous pression réduite (2,7 kPa). Le résidu est repris par 50 cm³ d'éthanol à 50°C, l'insoluble est éliminé par filtration et le filtrat est concentré sous pression réduite (2,7 kPa). On obtient ainsi 0,9 g d'acide (*RR,SS*)-*trans*-2-(morpholino méthyl)-1-cyclopropane carboxylique sous forme d'une huile jaune.

Spectre de R.M.N. ¹H (400 MHz, CDCl₃, δ en ppm) : 0,68 (mt : 1H) ; 1,20 (mt : 1H) ; 1,46 (mt : 1H) ; 1,56 (mt : 1H) ; 2,28 (dd, J = 13 et 8 Hz : 1H) ; 2,60 (dd, J = 13 et 5 Hz : 1H) ; 2,66 (mf : 2H) ; 2,77 (mf : 2H) ; 3,78 (t, J = 5 Hz : 4H).

Le (*RR,SS*)-*trans*-2-(morpholinométhyl)-1-cyclopropane carboxylate d'éthyle peut être préparé de la manière suivante :

A 2,82 g de (*RR,SS*)-*trans*-2-formyl-1-cyclopropane carboxylate d'éthyle en solution dans 100 cm³ de dichlorométhane, on ajoute à 20°C sous atmosphère d'argon, 1,74 cm³ de morpholine et 6,36 g de tris(acétato-O)hydridoborate de sodium. Après 4 heures d'agitation, on ajoute 100 cm³ d'eau distillée. Après agitation la phase organique est décantée, puis lavée par 100 cm³ d'eau distillée. La phase organique est séchée sur du sulfate de magnésium, filtrée puis concentrée à sec sous pression réduite (2,7 kPa). On obtient ainsi 1,1 g de (*RR,SS*)-*trans*-2-(morpholinométhyl)-1-cyclopropane carboxylate d'éthyle, sous forme d'une huile jaune.

Spectre de R.M.N. ¹H (400 MHz, CDCl₃, δ en ppm) : 0,76 (mt : 1 H) ; de 1,20 à 1,35 (mt : 4H) ; 1,44 (mt : 1H) ; 1,54 (mt : 1H) ; 2,31 (dd, J = 13 et 7 Hz : 1H) ; 2,36 (dd, J = 13 et 7 Hz : 1H) ; 2,50 (t large, J = 5 Hz : 4H) ; 3,73 (t, J = 5 Hz : 4H) ; 4,13 (mt : 2H).

### Exemple 34

### (16R)-16-Désoxo-16-fluoro-14-O-[6-(imidazol-1-yl) hexanoyl] pristinamycine II_{B}

A 1,42 g de (16*R*)-16-désoxo-16-fluoro-14-O-(6-bromohexanoyl) pristinamycine II_{B} en solution dans 7 cm³ de diméthylsulfoxyde, on ajoute à 20°C, 0,34 g d'imidazole et quelques cristaux d'iodure de sodium. Après 2 heures d'agitation à 53°C puis 16 heures à 20°C, le mélange réactionnel est versé sur 35 cm³ d'eau et filtré sur verre fritté. L'insoluble est lavé par de l'eau et dissous dans 70 cm³ d'acétate d'éthyle. La solution résultante est extraite par 25 cm' d'une solution aqueuse d'acide chlorhydrique 0,1 N. La phase aqueuse est décantée, extraite par 10 cm³ d'acétate d'éthyle puis neutralisée par 0,5 cm³ d'une solution aqueuse d'hydroxyde de sodium 5 N. La phase aqueuse est alors extraite par 2 fois 25 cm³ d'acétate d'éthyle. Les phases organiques sont réunies, séchées sur sulfate de magnésium, filtrées puis concentrées à sec sous pression réduite (2,7 kPa) pour donner 1 g d'un solide jaune pâle qui est purifié par chromatographie-flash [éluant : dichlorométhane / méthanol / acétonitrile (88 / 6 /6 en volumes)]. On obtient 0,59 g d'un solide blanc qui est repris dans 5 cm³ de dichlorométhane. La phase organique est filtrée sur Célite®. La Célite® est rincée par du dichlorométhane. Le filtrat est concentré à sec sous pression réduite (2,7 kPa) à 20°C, pour donner 0,58 g de (16*R*)-16-désoxo-16-fluoro-14-O-[6-(imidazol-1-yl) hexanoyl] pristinamycine II_{B}, sous forme d'un solide blanc fondant vers 90°C (déc.).

Spectre de R.M.N. ¹H (400 MHz, CDCl₃, δ en ppm) : 0,95 (d, J = 6,5 Hz : 3H) ; 0,99 (d, J = 6,5 Hz : 3H) ; 1,10 (d, J = 6,5 Hz : 3H) ; 1,27 (mt : 2H) ; de 1,60 à 2,10 (mt : 5H) ; 1,63 (mt : 2H) ; 1,78 (mt : 2H) ; 1,88 (s : 3H) ; de 2,10 à 2,25 (mt : 2H) ; 2,27 (t, J = 7 Hz : 2H) ; 2,75 (mt : 1H) ; 3,00 (mt : I H) ; 3,25 (dt, J = 17 et 5 Hz : 1H) ; 3,51 (mt : 1H) ; 3,82 (mt : 1H) ; 3,94 (t, J = 7 Hz : 2H) ; 4,08 (mt : 1H) ; 4,58 (mt : 1H) ; de 4,75 à 4,85 (mt : 2H) ; 5,07 (doublet démultiplié, J _{HF} = 48 Hz : 1H) ; 5,20 (d, J = 9 Hz : 1H) ; de 5,75 à 5,85 (mt : 2H) ; 5,90 (dd, J = 16 et 1,5 Hz : 1H) ; 6,18 (d, J = 16 Hz : 1H) ; 6,56 (dd, J = 16 et 5 Hz : 1H) ; 6,77 (mt : 1H) ; 6,90 (s : 1H) ; 7,10 (s : 1H) ; 7,45 (s : 1H) ; 8,12 (s : 1H).

La (16*R*)-16-désoxo-16-fluoro-14-O-(6-bromohexanoyl) pristinamycine II_{B} peut être préparée de la manière suivante :

A 5,32 g de (16*R*)-16-désoxo-16-fluoro pristinamycine II_{B} (préparée comme décrit dans l'exemple 1) en solution dans 150 cm³ de dichlorométhane, on ajoute à 28°C, 1,7 cm³ de triéthylamine et 1,8 cm³ de chlorure de 6-bromohexanoyle. Après 21,5 heures d'agitation à 23°C, on ajoute 0,43 cm³ de triéthylamine et 0,45 cm³ de chlorure de 6-bromohexanoyle. Après 21 heures d'agitation supplémentaires à 28°C, le mélange réactionnel est versé sur 50 cm³ d'eau. La phase organique est décantée, séchée sur sulfate de magnésium, filtrée puis concentrée à sec sous pression réduite (2,7 kPa) pour donner 8,05 g d'un solide brun qui est purifié par chromatographie-flash [éluant : dichlorométhane / méthanol / acétonitrile (96 / 2 / 2 en volumes)]. On obtient ainsi 5,6 g de (16*R*)-16-désoxo-16-fluoro-14-O-(6-bromohexanoyl) pristinamycine II_{B}, sous forme d'un solide jaune pâle.

Spectre de R.M.N. ¹H (400 MHz, CDCl₃, δ en ppm) : 0,96 (d, J = 6,5 Hz : 3H) ; 1,00 (d, J = 6,5 Hz : 3H) ; 1,09 (d, J = 6,5 Hz : 3H) ; 1,48 (mt : 2H) ; de 1,60 à 2,05 (mt : 9H) ; 1,88 (s : 3H) ; 2,15 (mt : 1H) ; 2,24 (mt : 1H) ; 2,31 (t, J = 7,5 Hz : 2H) ; 2,76 (mt : 1H) ; 2,99 (mt : 1H) ; 3,24 (mt : 1H) ; 3,41 (t, J = 7 Hz : 2H) ; 3,49 (mt : 1H) ; 3,86 (mt : 1H) ; 4,06 (mt : 1H) ; 4,53 (mt : 1H) ; 4,78 (dd, J = 10 et 2 Hz : 1H) ; 4,82 (dd, J = 9 et 3 Hz : 1H) ; 5,11 (doublet démultiplié, J _{HF} = 48 Hz : 1H) ; 5,31 (d, J = 9 Hz : 1H) ; de 5,75 à 5,85 (mt : 1H) ; 5,75 (mt : 1H) ; 5,82 (dd, J = 17 et 2 Hz : 1H) ; 5,95 (mt : 1H) ; 6,19 (d, J = 16 Hz : 1H) ; 6,51 (dd, J = 17 et 4 Hz : 1H) ; 8,13 (s : 1H).

### Exemple 35

### (16R)-16-Désoxo-16-fluoro-14-O-[6-(4-méthylpipérazin-1-yl) hexanoyl] pristinamycine II_{B}

A 1,42 g de (16*R*)-16-désoxo-16-fluoro-14-O-(6-bromohexanoyl) pristinamycine II_{B} (préparée comme décrit dans l'exemple 34) en solution dans 7 cm³ de diméthylsulfoxyde, on ajoute à 20°C, 0,67 cm³ de N-méthylpipérazine. Après 0,5 heure d'agitation à 60°C, le mélange réactionnel est versé sur 35 cm³ d'eau glacée et extrait par 50 cm³ de dichlorométhane. La phase organique est lavée par 25 cm³ d'une solution aqueuse saturée de chlorure de sodium, séchée sur sulfate de magnésium, filtrée puis concentrée à sec sous pression réduite (2,7 kPa) pour donner 1,2 g d'un solide orange qui est dissous dans 30 cm³ d'acétate d'éthyle. La solution résultante est extraite successivement par 20 cm³ d'une solution aqueuse d'acide chlorhydrique 0,1 N et 10 cm³ d'eau. Les phases aqueuses sont réunies et neutralisées par 0,4 cm³ d'une solution aqueuse d'hydroxyde de sodium 5 N. La phase aqueuse est extraite par 2 fois 25 cm³ de dichlorométhane. Les phases organiques sont réunies, séchées sur sulfate de magnésium, filtrées puis concentrées à sec sous pression réduite (2,7 kPa) pour donner 0,94 g d'un solide jaune qui est purifié par chromatographie-flash [éluant : dichlorométhane / méthanol (90 / 10 en volumes)]. On obtient ainsi 0,27 g d'un solide blanc qui est repris dans 5 cm³ de dichlorométhane. La phase organique est filtrée sur Célite®. La Célite® est rincée par du dichlorométhane. Le filtrat est concentré à sec sous pression réduite (2,7 kPa) à 20°C, pour donner 0,26 g de (16*R*)-16-désoxo-16-fluoro-14-O-[6-(4-méthylpipérazin-1-yl) hexanoyl] pristinamycine II_{B}, sous forme d'un solide blanc fondant vers 95°C (déc.).

Spectre de R.M.N. ¹H (400 MHz, CDCl₃, δ en ppm) : 0,96 (d, J = 6,5 Hz : 3H) ; 1,00 (d, J = 6,5 Hz : 3H) ; 1,09 (d, J = 6,5 Hz : 3H) ; 1,33 (mt : 2H) ; 1,51 (mt : 2H) ; de 1,55 à 2,05 (mt : 7H) ; 1,88 (s : 3H) ; de 2,10 à 2,65 (mt : 14H) ; 2,29 (s : 3H) ; 2,76 (mt : 1H) ; 2,99 (mt : 1H) ; 3,24 (mt : 1H) ; 3,48 (mt : 1H) ; 3,86 (mt : 1H) ; 4,06 (mt: 1H) ; 4,53 (mt : 1H) ; 4,78 (dd, J = 10 et 2 Hz : 1H) ; 4,82 (dd, J = 9 et 3 Hz : 1H) ; 5,10 (doublet démultiplié, J _{HF} = 48 Hz : 1H) ; 5,30 (d, J = 9 Hz : 1H) ; de 5,75 à 5,85 (mt : 1H) ; 5,75 (mt : 1H) ; 5,82 (dd, J = 17 et 2 Hz : 1H) ; 5,98 (mt : 1H) ; 6,19 (d, J = 16 Hz : 1H) ; 6,52 (dd, J = 17 et 5 Hz : 1H) ; 8,13 (s : 1H).

### Exemple 36

### (16R)-16-Désoxo-16-fluoro-14-O-{6-[4-(pyrrolidin-1-yl carbonylméthyl) pipérazin-1-yl] hexanoyl} pristinamycine II_{B}

A 1,35 g de (16*R*)-16-désoxo-16-fluoro-14-O-(6-bromohexanoyl) pristinamycine II_{B} (préparée comme décrit dans l'exemple 34) en solution dans 7 cm³ de diméthylsulfoxyde, on ajoute à 20°C, 1,18 g de (pyrrolidin-1-yl carbonylméthyl) pipérazine. Après 3 heures d'agitation à 60°C, le mélange réactionnel est versé sur 35 cm³ d'eau glacée puis extrait par 2 fois 25 cm³ d'acétate d'éthyle. Les phases organiques sont réunies, séchées sur sulfate de magnésium, filtrées puis concentrées à sec sous pression réduite (2,7 kPa) pour donner 1,53 g d'un résidu gommeux qui est purifié par chromatographie-flash [éluant : dichlorométhane / méthanol (90 / 10 en volumes)]. On obtient 0,96 g d'une gomme collante qui est reprise dans 10 cm³ de dichlorométhane. Cette solution est filtrée sur Célite®. La Célite® est rincée par du dichlorométhane. Le filtrat est concentré à sec sous pression réduite (2,7 kPa) pour donner une gomme qui est agitée successivement dans de l'éther diéthylique et du pentane. Après filtration puis séchage sous pression réduite (2,7 kPa), on obtient 0,78 g de (16*R*)-16-désoxo-16-fluoro-14-O-{6-[4-(pyrrolidin-1-yl carbonylméthyl) pipérazin-1-yl] hexanoyl} pristinamycine II_{B}, sous forme d'un solide jaune fondant vers 80°C (déc.).

Spectre de R.M.N. ¹H (400 MHz, CDCl₃, δ en ppm) : 0,96 (d, J = 6,5 Hz : 3H) ; 1,00 (d, J = 6,5 Hz : 3H) ; 1,09 (d, J = 6,5 Hz : 3H) ; 1,32 (mt : 2H) ; 1,50 (mt : 2H) ; de 1,60 à 2,05 (mt : 11H) ; 1,88 (s : 3H) ; de 2,10 à 2,40 (mt : 6H) ; 2,50 (mf : 4H) ; 2,59 (mf : 4H) ; 2,76 (mt : 1H) ; 2,99 (mt : 1H) ; 3,12 (s : 2H) ; 3,24 (mt : 1H) ; de 3,40 à 3,55 (mt : 5H) ; 3,86 (mt : 1H) ; 4,05 (mt : 1H) ; 4,52 (mt : 1H) ; 4,78 (dd, J = 10 et 2 Hz : 1H) ; 4,82 (dd, J = 9 et 3 Hz : 1H) ; 5,10 (doublet démultiplié, J _{HF} = 48 Hz : 1H) ; 5,30 (d, J = 9 Hz : 1H) ; de 5,75 à 5,90 (mt : 1H) ; 5,75 (mt : 1H) ; 5,82 (dd, J = 17 et 2 Hz : 1H) ; 6,02 (mt : 1H) ; 6,19 (d, J = 16 Hz : 1H) ; 6,52 (dd, J = 17 et 5 Hz : 1H) ; 8,13 (s : 1H).

### Exemple 37

### Méthanesulfonate de la (16R)-16-Désoxo-16-fluoro-14-O-[7-(imidazol-1-yl) heptanoyl] pristinamycine II_{B}

A 1,96 g de (16*R*)-16-désoxo-16-fluoro-14-O-[7-(imidazol-1-yl) heptanoyl] pristinamycine II_{B} en solution dans 10 cm³ d'éthanol, on ajoute à 20°C, 2,7 cm³ d'une solution éthanolique d'acide méthanesulfonique 1 N. Après 10 minutes d'agitation, le mélange réactionnel est concentré à sec sous pression réduite (2,7 kPa) pour donner un résidu qui est agité dans 10 cm³ d'éther. Après filtration, lavage du solide par 5 cm³ d'éther diéthylique et séchage sous pression réduite (2,7 kPa), on obtient 2,13 g du méthanesulfonate de la (16*R*)-16-désoxo-16-fluoro-14-O-[7-(imidazol-1-yl) heptanoyl] pristinamycine II_{B}, sous forme d'un solide crème fondant vers 110°C (déc.).

Spectre de R.M.N. ¹H (400 MHz, CDCl₃, δ en ppm) : 0,95 (d, J = 6,5 Hz : 3H) ; 0,98 (d, J = 6,5 Hz : 3H) ; 1,09 (d, J = 6,5 Hz : 3H) ; de 1,25 à 1,45 (mt : 4H) ; de 1,55 à 2,05 (mt : 9H) ; 1,88 (s : 3H) ; de 2,10 à 2,30 (mt : 2H) ; 2,28 (t, J = 7,5 Hz : 2H) ; 2,76 (mt : 1H) ; 2,85 (s : 3H) ; 3,00 (dt, J = 17 et 6 Hz : 1H) ; 3,24 (mt : 1H) ; 3,51 (mt : 1H) ; 3,84 (mt : 1H) ; 4,05 (mt : 1H) ; 4,18 (t, J = 7,5 Hz : 2H) ; 4,49 (mt : 1H) ; 4,78 (dd, J = 10 et 2 Hz : 1H) ; 4,82 (dd, J = 9 et 3 Hz : 1H) ; 5,09 (doublet démultiplié, J _{HF} = 48 Hz : 1H) ; 5,28 (d, J = 9,5 Hz : 1H) ; de 5,70 à 5,85 (mt : 2H) ; 5,84 (dd, J = 16 et 2 Hz : 1H) ; 6,13 (mt : 1H) ; 6,18 (d, J = 16 Hz : 1 H) ; 6,53 (dd, J = 16 et 5 Hz : 1H) ; 7,13 (s : 1H) ; 7,41 (s : 1H) ; 8,12 (s : 1H) ; 8,92 (s : 1H).

La (16*R*)-16-désoxo-16-fluoro-14-O-[7-(imidazol-1-yl) heptanoyl] pristinamycine II_{B} peut être préparée de la manière suivante : )

A 3,2 g de (16*R*)-16-désoxo-16-fluoro pristinamycine II_{B} (préparée comme décrit dans l'exemple 1) en solution dans 100 cm³ de dichlorométhane, on ajoute à 23°C, 1,49 g de N,N'-dicyclohexylcarbodiimide, 0,075 g de 4-diméthylaminopyridine, 1,68 g de chlorhydrate d'acide 7-(imidazol-1-yl) heptanoïque et 1 cm³ de triéthylamine,. Après 84 heures d'agitation à 23°C, le mélange réactionnel est filtré et l'insoluble est rincé avec du dichlorométhane. Le filtrat est concentré à sec sous pression réduite (2,7 kPa) pour donner un résidu huileux qui est dilué dans 100 cm³ d'acétate d'éthyle. La solution résultante est lavée successivement par 2 fois 40 cm³ d'eau et 40 cm³ d'une solution aqueuse saturée de chlorure de sodium puis extraite par 60 cm³ d'une solution aqueuse d'acide chlorhydrique 0,1 N. La phase aqueuse acide est alors extraite par 20 cm³ d'acétate d'éthyle, ajustée à pH 8 par addition de 6 cm³ d'une solution aqueuse d'hydroxyde de sodium 1 N puis extraite par 2 fois 40 cm³ d'acétate d'éthyle. Les phases organiques sont réunies, lavées par une solution aqueuse saturée de chlorure de sodium, séchées sur sulfate de magnésium, filtrées puis concentrées à sec sous pression réduite (2,7 kPa) pour donner 3,05 g d'un solide jaune pâle qui est purifié par chromatographie-flash [éluant : dichlorométhane / méthanol / acétonitrile (86 / 7 / 7 en volumes)]. On obtient ainsi, 2,58 g d'un solide qui est repris dans 10 cm³ de dichlorométhane. Cette solution est filtrée sur Célite®. La Célite® est rincée par du dichlorométhane. Le filtrat est concentré à sec sous pression réduite (2,7 kPa) à 20°C, pour donner, 2,58 g de (16*R*)-16-désoxo-16-fluoro-14-O-[7-(imidazol-1-yl) heptanoyl] pristinamycine II_{B}, sous forme d'un solide blanc fondant vers 80°C (déc.).

Spectre de R.M.N. ¹H (400 MHz, CDCl₃, δ en ppm) : 0,95 (d, J = 6,5 Hz : 3H) ; 0,99 (d, J = 6,5 Hz : 3H) ; 1,09 (d, J = 6,5 Hz : 3H) ; de 1,25 à 1,40 (mt : 4H) ; de 1,55 à 2,05 (mt : 9H) ; 1,88 (s : 3H) ; de 2,10 à 2,30 (mt : 2H) ; 2,27 (t, J = 7,5 Hz : 2H) ; 2,76 (mt : 1H) ; 2,99 (dt, J = 17 et 6 Hz : 1H) ; 3,24 (mt : 1H) ; 3,48 (mt : 1H) ; 3,85 (mt : 1H) ; 3,93 (t, J = 7,5 Hz : 2H) ; 4,06 (mt : 1H) ; 4,53 (mt : 1H) ; 4,78 (dd, J = 10 et 2 Hz : 1H) ; 4,82 (dd, J = 9 et 3 Hz : 1H) ; 5,09 (doublet démultiplié, J _{HF} = 48 Hz : 1H) ; 5,29 (d, J = 9 Hz : 1H) ; de 5,70 à 5,85 (mt : 2H) ; 5,83 (dd, J = 16,5 et 2 Hz : 1H) ; 6,17 (mt : 1H) ; 6,19 (d, J = 15 Hz : 1H) ; 6,52 (dd, J = 16,5 et 5 Hz : 1H) ; 6,90 (s : 1H) ; 7,06 (s : 1H) ; 7,45 (s : 1H) ; 8,12 (s : 1H).

Le chlorhydrate de l'acide 7-(imidazol-1-yl) heptanoique peut être préparé selon Kinji Iizuka et *al*, J. Med. Chem., Vol. 24, N° 10, pages 1139 à 1148 (1981).

### Exemple 38

### (16R)-16-Désoxo-16-fluoro-14-O-(4-méthylpipérazin-1-yl)carbonyl acétyl pristinamycine II_{B}

A 2,5 g de (16*R*)-16-désoxo-16-fluoro pristinamycine II_{B} (préparée comme décrit dans l'exemple 1) en solution dans 100 cm³ de dichlorométhane, on ajoute, à 20°C sous atmosphère d'argon, 1,74 g d'acide 4-méthylpipérazin-1-yl carbonyl acétique, 1,44 g de N,N'-dicyclohexylcarbodiimide et 0,09 g de 4-diméthylaminopyridine. Après 18 heures d'agitation, le mélange réactionnel est additionné de 50 cm³ de dichlorométhane, filtré pour éliminer l'insoluble, puis lavé par quatre fois 50 cm³ d'eau distillée. La phase organique est décantée, séchée sur du sulfate de magnésium puis concentrée à sec sous pression réduite (2,7 kPa). Le résidu (3,4 g) est purifié par chromatographie-flash [éluant : gradient dichlorométhane / méthanol (97 / 3 et 92 / 8 en volumes)]. On obtient un solide blanc qui donne, après agitation dans de l'éther diéthylique, filtration et séchage (2,7 kPa), 1,3 g de (16*R*)-16-désoxo-16-fluoro-14-O-(4-méthylpipérazin-1-yl) carbonyl acétyl pristinamycine II_{B} sous forme d'un solide blanc.

Spectre de R.M.N. ¹H (500 MHz, CDCl₃, δ en ppm) : 0,96 (d, J = 6,5 Hz : 3H) ; 1,01 (d, J = 6,5 Hz : 3H) ; 1,10 (d, J = 6,5 Hz : 3H) ; de 1,70 à 2,00 (mt : 5H) ; 1,89 (s : 3H) ; 2,15 (mt : 1H) ; de 2,25 à 2,35 (mt : 1H) ; 2,31 (s : 3H) ; 2,40 (mt : 4H) ; 2,76 (mt : 1H) ; 3,01 (dt, J = 16 et 6 Hz : 1H) ; 3,23 (mt : 1H) ; de 3,40 à 3,55 (mt : 5H) ; 3,66 (mt : 2H) ; 3,87 (mt : 1H) ; 4,05 (mt : 1H) ; 4,53 (mt : 1H) ; 4,78 (d large, J = 10 Hz : 1H) ; 4,82 (dd, J = 9 et 3 Hz : 1H) ; 5,14 (doublet démultiplié, J _{HF} = 48 Hz : 1H). ; 5,34 (d, J = 9,5 Hz : 1H) ; 5,77 (mt : 1H) ; 5,82 (dd, J = 16 et 2 Hz : 1H) ; 5,88 (dt, J = 9,5 et 5 Hz : 1H) ; 5,95 (mt : 1H) ; 6,20 (d, J = 16 Hz : 1H) ; 6,51 (dd, J = 16 et 4 Hz : 1H) ; 8,12 (s : 1H).

L'acide 4-méthylpipérazin-1-yl carbonyl acétique peut être préparé de la manière suivante :

A 4,5 g de 4-méthylpipérazin-1-yl carbonyl acétate d'éthyle en solution dans 100 cm³ d'éthanol on ajoute, à 20°C, 23 cm³ de solution aqueuse d'hydroxyde de sodium 0,1 N. Après 24 heures d'agitation, le mélange réactionnel est concentré à sec sous pression réduite (2,7 kPa). Le résidu obtenu est repris par 50 cm³ d'eau distillée et la solution est lavée par quatre fois 50 cm³ de dichlorométhane. La phase aqueuse est amenée à pH 5-6 par ajout d'une solution aqueuse d'acide chlorhydrique 1 N, extraite par deux fois 50 cm³ de dichlorométhane, puis concentrée à sec sous pression réduite (2,7 kPa). Par trois fois le résidu est recouvert de 100 cm³ de toluène et ramené à sec dans les mêmes conditions. On obtient 4,6 g d'un résidu pâteux jaune qui est agité dans 50 cm³ d'éthanol vers 60°C. L'insoluble est éliminé par filtration et le filtrat est concentré à sec sous pression réduite (2,7 kPa). On obtient ainsi 3,5 g d'acide 4-méthyl-pipérazin-1-yl carbonyl acétique sous forme d'une masse collante, hygroscopique utilisée telle quelle.

Spectre de R.M.N. ¹H (300 MHz, (CD₃)₂SO d6, δ en ppm) : 2,21 (s : 3H) ; 2,29 (t, J = 5 Hz : 2H) ; 2,34 (t, J = 5 Hz : 2H) ; de 3,30 à 3,50 (mt : 4H) ; 3,39 (s : 2H).

Le 4-méthyl-pipérazin-1-yl carbonyl acétate d'éthyle peut être préparé selon Morren H., Trolin S., Denayer R., Grivsky E., Bull. Soc. Chim. Belg. 1950, 59, 228-232.

### Exemple 39

### (16R)-14-O-[4-Carboxybutyryl)-16-désoxo-16-fluoro pristinamycine II_{B}

A 400 mg de (16*R*)-16-désoxo-16-fluoro pristinamycine II_{B} (préparée comme décrit dans l'exemple 1) en solution dans 2 cm³ de pyridine, on ajoute à 20°C, 258 mg d'anhydride glutarique et 84,5 mg de 4-diméthylaminopyridine. Après 20 heures d'agitation, la pyridine est évaporée sous pression réduite (2,7 kPa) et l'huile obtenue est reprise par 20 cm³ d'acétate d'éthyle. La solution résultante est lavée par 2 fois 10 cm³ d'eau distillée puis par une solution aqueuse d'acide chlorhydrique 0,1 N. La phase aqueuse est extraite à l'acétate d'éthyle puis décantée. La phase organique est lavée par de l'eau distillée puis par 2 fois 2 cm³ d'une solution saturée en chlorure de sodium. La phase organique est décantée, séchée sur sulfate de magnésium, filtrée, puis concentrée à sec sous pression réduite (2,7 kPa), pour donner un solide jaune clair qui est agité dans 20 cm³ d'éther diéthylique, filtré puis séché sous pression réduite (2,7 kPa). On obtient ainsi 363 mg d'un solide qui est purifié par chromatographie-flash [éluant : dichlorométhane-méthanol (97-3 en volumes)] pour donner 100 mg de (16*R*)-14-O-[4-carboxybutyryl)-16-désoxo-16-fluoro pristinamycine II_{B} sous forme d'un solide blanc fondant à 98°C.

Spectre de R.M.N. ¹H (400 MHz, CDCl₃, δ en ppm) : 0,96 (d, J = 6,5 Hz : 3H) ; 1,01 (d, J = 6,5 Hz : 3H) ; 1,10 (d, J = 6,5 Hz : 3H) ; de 1,55 à 2,05 (mt : 7H) ; 1,89 (s : 3H) ; 2,17 (mt : 1H) ; 2,25 (mt : 1H) ; 2,37 (t, J = 7 Hz : 2H) ; 2,42 (t, J = 7 Hz : 2H) ; 2,77 (mt : 1H) ; 3,00 (td, J = 17 et 6 Hz : 1H) ; 3,25 (mt : 1H) ; 3,50 (mt : 1H) ; 3,86 (mt : 1H) ; 4,08 (mt : 1H) ; 4,53 (mt : 1H) ; 4,79 (dd, J = 10 et 1,5 Hz : 1H) ; 4,82 (dd, J = 9 et 3 Hz : 1H) ; 5,13 (doublet démultiplié, J _{HF} = 48 Hz : 1H) ; 5,31 (d, J = 9,5 Hz : 1H) ; de 5,75 à 5,90 (mt : 1H) ; 5,77 (mt : 1H) ; 5,83 (dd, J = 17 et 2 Hz : 1H) ; 5,97 (mt : 1H) ; 6,19 (d, J = 16 Hz : 1H) ; 6,53 (dd, J = 17 et 5 Hz : 1H) ; 8,14 (s : 1H).

### Exemple 40

### Chlorhydrate de la (16R)-16-Désoxo-16-fluoro-14-O-[3-(4-méthylpipérazin-1-yl carbonyl)propionyl] pristinamycine II_{B}

A 615 mg de (16*R*)-16-Désoxo-16-fluoro-14-O-[3-(4-méthylpipérazin-1-yl carbonyl)propionyl] pristinamycine II_{B} dissous dans 6 cm³ de méthylisobutyl cétone, on ajoute une solution d'éther chlorhydrique 3 N jusqu'à obtention d'un précipité abondant. Celui-ci est filtré, rincé par 2 fois 2 cm³ de méthylisobutyl cétone puis par 3 fois 10 cm³ d'éther diéthylique. Après séchage à 50°C (90 Pa), on obtient 600 mg de chlorhydrate de (16*R*)-16-désoxo-16-fluoro-14-O-[3-(4-méthylpipérazin-1-yl carbonyl)propionyl] pristinamycine II_{B}, sous forme d'une poudre blanche fondant à 160°C.

La (16*R*)-16-Désoxo-16-fluoro-14-O-[3-(4-méthylpipérazin-1-yl carbonyl)propionyl] pristinamycine II_{B} peut être préparée de la façon suivante :

Dans un tricol placé sous azote on ajoute, 450 mg d'acide 4-(4-méthylpipérazin-1-yl)-4-oxo butyrique, 1g de (16*R*)-16-désoxo-16-fluoro pristinamycine II_{B} (préparée comme décrit dans l'exemple 1), 470 mg de N,N'-dicyclohexylcarbodiimide, 140 mg de 4-diméthylaminopyridine puis 20 cm³ de dichlorométhane. La réaction est agitée à 20°C pendant 48 heures. Le mélange réactionnel est alors dilué par 10 cm³ de dichlorométhane puis versé sur 50 cm³ d'eau distillée. Le mélange obtenu est filtré sur coton. La phase organique est décantée et la phase aqueuse extraite par 2 fois 5 cm³ de dichlorométhane. Les phases organiques sont rasemblées, séchées sur sulfate de sodium, filtrée puis concentrées à sec sous pression réduite (2,7 kPa), pour donner 1,66 g d'un mélange qui est purifié par chromatographie-flash [éluant : dichlorométhane-méthanol (95-5 en volumes)]. On obtient ainsi 975 mg d'un produit qui est agité dans 10 cm³ d'éther diéthylique, filtré et séché, puis repurifié par chromatographie-flash [éluant : acétate d'éthyle-méthanol (93-7 en volumes)]. Les fractions sont concentrées pour donner un solide qui est repris par 10 cm³ d'éther diéthylique et agité 30 minutes. Le solide blanc obtenu est filtré, rincé par de l'éther diéthylique puis séché à 50°C (90 Pa) pour donner 500 mg de (16*R*)-16-désoxo-16-fluoro-14-O-[3-(4-méthylpipérazin-1-yl carbonyl)propionyl] pristinamycine II_{B}, sous forme d'une poudre blanche fondant à 130°C.

Spectre de R.M.N. ¹H (400 MHz, CDCl₃, δ en ppm) : 0,96 (d, J = 6,5 Hz : 3H) ; 1,00 (d, J = 6,5 Hz : 3H) ; 1,10 (d, J = 6,5 Hz : 3H) ; de 1,65 à 2,05 (mt : 5H) ; 1,88 (s : 3H) ; 2,15 (mt : 1H) ; de 2,20 à 2,45 (mt : 5H) ; 2,31 (s : 3H) ; de 2,50 à 2,75 (mt : 4H) ; 2,76 (mt : 1H) ; 3,00 (mt : 1H) ; 3,23 (mt : 1H) ; de 3,40 à 3,55 (mt : 1H) ; 3,50 (mt : 2H) ; 3,63 (mt : 2H) ; 3,87 (mt : 1H) ; 4,06 (mt : 1H) ; 4,53 (mt : 1H) ; 4,78 (d large, J = 10 Hz : 1H) ; 4,82 (dd, J = 9 et 3 Hz : 1H) ; 5,14 (doublet démultiplié, J _{HF} = 48 Hz : 1H) ; 5,33 (d, J = 9,5 Hz : 1H) ; de 5,75 à 5,90 (mt : 1H) ; 5,75 (mt : 1H) ; 5,81 (d large, J = 16 Hz : 1H) ; 5,96 (mt : 1H) ; 6,20 (d, J = 16 Hz : 1H) ; 6,51 (dd, J = 16 et 5 Hz : 1 H) ; 8,12 (s : 1H).

L'acide 4-(4-méthylpipérazin-1-yl)-4-oxo butyrique peut être préparé de la façon suivante :

On ajoute à 20 cm³ de dioxane, 1,17 g d'anhydride succinique puis 1,19 cm³ de N-méthylpipérazine. Après 18 heures d'agitation à température ambiante, le précipité obtenu est filtré, puis rincé successivement par un minimum de dioxane, par 2 fois 10 cm³ d'acétone et par 10 cm³ d'éther diéthylique. Après séchage sous pression réduite (2,7 kPa) à 20°C, on obtient 1,09g d'acide 4-(4-méthylpipérazin-1-yl)-4-oxo butyrique, sous forme d'un solide blanc fondant à 114°C.

### Exemple 41

### (16R)-16-Désoxo-16-fluoro-14-O-{3-[4-(pyridin-2-yl) pipérazin-1-yl carbonyl] propionyl} pristinamycine II_{B}

On opère comme à l'exemple 40, mais à partir de 240 mg d'acide 4-oxo-4-[4-(pyridin-2-yl) pipérazin-1-yl)] butyrique, 400 mg de (16*R*)-16-désoxo-16-fluoro pristinamycine II_{B} (préparée comme décrit dans l'exemple 1), 190 mg de N,N'-dicyclohexylcarbodiimide, 46 mg de 4-diméthylaminopyridine puis 6 cm³ de dichlorométhane. Après 66 heures d'agitation et traitement analogue à celui de l'exemple 40, on obtient 655 mg d'un solide qui est purifié par chromatographie-flash [éluant : dichlorométhane-méthanol (95-5 en volumes)]. On isole un solide qui est agité 20 minutes dans 4 cm³ d'éther diéthylique. Le solide blanc obtenu est filtré, rincé par de l'éther diéthylique puis séché à 50°C (90 Pa) pour donner 308 mg de (16R)-16-désoxo-16-fluoro-14-O-{3-[4-(pyridin-2-yl) pipérazin-1-yl carbonyl]propionyl} pristinamycine II_{B}, sous forme d'une poudre blanche fondant vers 140°C (déc.).

Spectre de R.M.N. ¹H (400 MHz, CDCl₃, δ en ppm) : 0,96 (d, J = 6,5 Hz : 3H) ; 1,01 (d, J = 6,5 Hz : 3H) ; 1,10 (d, J = 6,5 Hz : 3H) ; de 1,65 à 2,05 (mt : 5H) ; 1,88 (s : 3H) ; 2,15 (mt : 1H) ; 2,30 (mt : 1H) ; de 2,55 à 2,80 (mt : 4H) ; 2,76 (mt : 1H) ; 3,00 (mt : 1H) ; 3,23 (mt : 1H) ; 3,46 (mt : 1H) ; 3,51 (mt : 2H) ; 3,63 (mt : 4H) ; 3,75 (mt : 2H) ; 3,87 (mt : 1H) ; 4,06 (mt : 1H) ; 4,53 (mt : 1H) ; 4,78 (dd, J = 10 et 1,5 Hz : 1H) ; 4,82 (dd, J = 9 et 3 Hz : 1H) ; 5,14 (doublet démultiplié, J _{HF} = 48 Hz : 1H) ; 5,34 (d, J = 9,5 Hz : 1H) ; de 5,75 à 5,90 (mt : 1H) ; 5,75 (mt : 1H) ; 5,81 (dd, J = 16 et 1,5 Hz : 1H) ; 5,96 (mt : 1H) ; 6,20 (d, J = 16 Hz : 1H) ; 6,51 (dd, J = 16 et 5 Hz : 1H) ; de 6,60 à 6,70 (mt : 2H) ; 7,52 (mt : 1H) ; 8,12 (s : 1H) ; 8,21 (dd, J = 5 et 1,5 Hz : 1H).

L'acide 4-oxo-4-[4-(pyridin-2-yl) pipérazin-1-yl)] butyrique peut être préparé de la façon suivante :

On ajoute à 15 cm³ de dioxane, 0,97 g d'anhydride succinique puis 1,5 cm³ de 1-(2-pyridyl) pipérazine. Après 4,5 heures d'agitation à 20°C, le solvant est évaporé sous pression réduite (2,7 kPa) à 20°C pour donner un solide qui est recristallisé à chaud dans 33 cm³ d'acétone. Après filtration, le solide est rincé successivement par un minimum d'acétone et par de l'éther diéthylique, puis séché sous pression réduite (2,7 kPa) à 20°C, pour donner 986 mg d'acide 4-oxo-4-[4-(pyridin-2-yl) pipérazin-1-yl)] butyrique sous forme d'un solide blanc fondant à 134°C.

### Exemple 42

### (16R)-16-Désoxo-16-fluoro-14-O-{3-[4-(pyrrolidin-1-yl-carbonylméthyl)pipérazin-1-yl carbonyl]propionyl} pristinamycine II_{B}

En opérant comme à l'exemple 40 mais à partir de 750 mg d'acide 4-oxo-4-[4-(2-oxo-2-pyrrolidin-1-yl éthyl) pipérazin-1-yl)] butyrique, 1 g de (16*R*)-16-désoxo-16-fluoro pristinamycine II_{B} (préparée comme décrit dans l'exemple 1), 190 mg de N,N'-dicyclohexylcarbodiimide, 60 mg de 4-diméthylaminopyridine puis 20 cm³ de dichlorométhane. Après 25 heures d'agitation, on ajoute 95 mg de N,N'-dicyclohexylcarbodiimide et 95 mg de 4-diméthylaminopyridine supplémentaires puis l'agitation est poursuivie 19 heures. Après traitement, on obtient, 1,54 g d'un solide qui est purifié par chromatographie-flash [éluant : dichlorométhane-méthanol (95-5 en volumes)]. Les fractions sont concentrées pour donner 530 mg d'un solide qui est repris par 5 cm³ d'éther diéthylique. Le solide blanc obtenu est filtré, rincé par de l'éther diéthylique puis séché à 50°C (90 Pa) pour donner 480 mg de (16R)-16-désoxo-16-fluoro-14-O-{3-[4-(pyrrolidin-1-yl-carbonylméthyl)pipérazin-1-yl carbonyl] propionyl} pristinamycine II_{B}, sous forme d'une poudre blanche fondant à 132°C (déc.).

Spectre de R.M.N. ¹H (400 MHz, CDCl₃, δ en ppm) : 0,95 (d, J = 6,5 Hz : 3H) ; 1,00 (d, J = 6,5 Hz : 3H) ; 1,09 (d, J = 6,5 Hz : 3H) ; de 1,65 à 2,05 (mt : 9H) ; 1,88 (s : 3H) ; 2,14 (mt : 1H) ; 2,28 (mt : 1H) ; de 2,45 à 2,75 (mt : 8H) ; 2,76 (mt : 1H) ; 3,00 (td, J = 17 et 6 Hz : 1H) ; 3,14 (s : 2H) ; 3,25 (mt : 1H) ; de 3,40 à 3,55 (mt : 7H) ; 3,66 (mt : 2H) ; 3,87 (mt : 1H) ; 4,05 (mt : 1H) ; 4,52 (mt : 1H) ; 4,78 (dd, J = 10 et 1,5 Hz : 1H) ; 4,81 (dd, J = 9 et 3 Hz : 1H) ; 5,12 (doublet démultiplié, J _{HF} = 48 Hz : 1H) ; 5,33 (d, J = 9,5 Hz : 1H) ; 5,74 (mt : 1H) ; de 5,75 à 5,85 (mt : 1H) ; 5,81 (dd, J = 17 et 1,5 Hz : 1H) ; 6,00 (mt : 1H) ; 6,19 (d, J = 16 Hz : 1H) ; 6,51 (dd, J = 17 et 5 Hz : 1H); 8,12 (s : 1H).

L'acide 4-oxo-4-[4-(2-oxo-2-pyrrolidin-1-yl éthyl)- pipérazin-1-yl] butyrique peut être préparé de la façon suivante :

Dans un ballon maintenu sous atmosphère d'azote, on ajoute à 20 cm³ de dioxane, 0,72 g d'anhydride succinique puis 1,5 cm³ de 1-(pyrrolidinocarbonylméthyl) pipérazine à 95%. Après 4,75 heures d'agitation à température ambiante, le solvant est évaporé sous pression réduite (2,7 kPa) à 20°C, pour donner 2,4 g d'acide 4-oxo-4-[4-(2-oxo-2-pyrrolidin-1-yl éthyl) pipérazin-1-yl] butyrique sous forme d'une pâte jaune pâle utilisée telle quelle.

### Exemple 43

### (16R)-16-Désoxo-14-O-[3-(2-diméthylaminoéthylcarbamoyl) propionyl]-16-fluoro pristinamycine II_{B}

En opérant comme à l'exemple 40 mais à partir de 170 mg d'acide N-(2-diméthylaminoéthyl)succinamique, 400 mg de ( 16*R*)-16-désoxo-16-fluoro pristinamycine II_{B} (préparée comme décrit dans l'exemple 1), 190 mg de N,N'-dicyclohexylcarbodiimide, 46 mg de 4-diméthylaminopyridine puis 6 cm³ de dichlorométhane, on obtient, après 18 heures d'agitation et traitement analogue à celui de l'exemple 40, 580 mg d'un solide qui est repris par 15 cm³ de dichlorométhane et 100 cm³ d'eau distillée. On ajoute de l'acide chlorhydrique 0,1 M de façon a ajuster le pH à 4. La phase aqueuse est extraite par 2 fois 5 cm³ de dichlorométhane puis ajustée à pH 7 par addition d'une solution de bicarbonate de sodium. Après décantation, la phase aqueuse est extraite par 3 fois 10 cm³ de dichlorométhane. Les phases organiques sont rassemblées, séchées sur sulfate de sodium, filtrées puis concentrées à sec sous pression réduite (2,7 kPa) à 20°C, pour donner un solide qui est agité dans 5 cm³ d'éther diéthylique, filtré puis séché à 50°C (90 Pa). On obtient 112 mg de (16R)-16-désoxo-14-O-[3-(2-diméthylaminoéthylcarbamoyl)]propionyl]-16-fluoro pristinamycine II_{B}, sous forme d'une poudre blanche fondant à 114°C (déc.).

Spectre de R.M.N. ¹H (400 MHz, CDCl₃, δ en ppm) : 0,97 (d, J = 6,5 Hz : 3H) ; 1,00 (d, J = 6,5 Hz : 3H) ; 1,10 (d, J = 6,5 Hz : 3H) ; de 1,65 à 2,05 (mt : 5H) ; 1,88 (s : 3H) ; de 2,10 à 2,35 (mt : 2H) ; 2,24 (s : 6H) ; de 2,35 à 2,75 (mt : 4H) ; 2,41 (t, J = 6 Hz : 2H) ; 2,77 (mt : 1H) ; 3,00 (td, J = 17 et 6 Hz : 1H) ; 3,23 (mt : 1H) ; 3,33 (q, J = 6 Hz : 2H) ; 3,47 (mt : 1 H) ; 3,87 (mt : 1H) ; 4,07 (mt : 1H) ; 4,54 (mt : 1H) ; 4,78 (d large, J = 10 Hz : 1H) ; 4,82 (dd, J = 9 et 3 Hz : 1 H) ; 5,13 (doublet démultiplié, J _{HF} = 48 Hz : 1H) ; 5,32 (d, J = 9,5 Hz : 1H) ; de 5,75 à 5,90 (mt : 1H) ; 5,75 (mt : 1H) ; 5,82 (d large, J = 17 Hz : 1H) ; 5,98 (mt : 1H) ; de 6,10 à 6,25 (mt : 1H) ; 6,20 (d, J = 16 Hz : 1H) ; 6,51 (dd, J = 17 et 5 Hz : 1 H) ; 8,13 (s : 1H).

L'acide N-(2-diméthylaminoéthyl)succinamique peut être préparé de la façon suivante :

On ajoute à 15 cm³ de dioxane, 1,4 g d'anhydride succinique puis 1,5 cm³ de 2-diméthylamino éthylamine. Après 18 heures d'agitation à température ambiante, le solvant est évaporé sous pression réduite (2,7 kPa) à 20°C, pour donner un résidu qui est repris à chaud dans 40 cm³ de méthyl éthylcétone. Après refroidissement, la fraction solubilisée est éliminée. La gomme résiduelle est cristallisé à chaud dans 15 cm³ d'acétone. Après filtration, le solide est rincé par un minimum d'acétone puis à l'éther diéthylique, séché sous pression réduite (2,7 kPa) à 20°C pour donner 2,03 g d'acide N-(2-diméthylaminoéthyl)succinamique sous forme d'un solide blanc fondant à 155°C.

### Exemple 44

### (16R)-14-O-(3-Carboxypropionyl)-16-désoxo-16-fluoro pristinamycine II_{B}

En opérant comme à l'exemple 39 mais à partir de 5 g de (16R)-16-désoxo-16-fluoro pristinamycine II_{B} (préparée comme décrit dans l'exemple 1), de 2,82 g d'anhydride succinique, de 1,15 g de 4-diméthylaminopyridine et de 25 cm³ de pyridine et après 4 heures 50 minutes d'agitation, on obtient une huile jaune orangée qui est agitée 18 heures dans de l'éther diéthylique pour donner une poudre blanc cassé. Ce solide est filtré puis agité dans de l'éther diéthylique, filtrée et séchée à 30 °C (90 Pa), pour donner 4,96 g de solide. Un gramme de ce solide est agité 1 heure dans 10 cm³ de dichlorométhane, filtré puis rincé à l'éther diéthylique. Le solide obtenu est lavé 3 fois à l'eau distillée puis à l'éther diéthylique pour donner 820 mg de (16*R*)-14-O-[3-carboxypropionyl)-16-désoxo-16-fluoro pristinamycine II_{B}, sous forme d'un solide blanc fondant vers 192°C.

Spectre de R.M.N. ¹H (400 MHz, CDCl₃, δ en ppm) : 0,97 (d, J = 6,5 Hz : 3H) ; 1,01 (d, J = 6,5 Hz : 3H) ; 1,10 (d, J = 6,5 Hz : 3H) ; de 1,55 à 2,05 (mt : 5H) ; 1,89 (s : 3H) ; 2,16 (mt : 1H) ; 2,27 (mt : 1H) ; de 2,55 à 2,70 (mt : 4H) ; 2,77 (mt : 1H) ; 3,00 (td, J = 17 et 6 Hz : 1H) ; 3,25 (mt : 1H) ; 3,50 (mt : 1H) ; 3,87 (mt : 1H) ; 4,07 (mt : 1H) ; 4,52 (mt : 1H) ; 4,79 (dd, J = 10 et 1,5 Hz : 1H) ; 4,83 (dd, J = 9 et 3 Hz : 1H) ; 5,12 (doublet démultiplié, J _{HF} = 48 Hz : 1H) ; 5,33 (d, J = 9,5 Hz : 1H) ; de 5,75 à 5,90 (mt : 1H) ; 5,77 (mt : 1H) ; 5,83 (d large, J = 17 Hz : 1H) ; 5,96 (mt : 1H) ; 6,20 (d, J = 16 Hz: 1H) ; 6,53 (dd, J = 17 et 5 Hz : 1H) ; 8,13 (s : 1H).

### Exemple 45

### Chlorhydrate de la (16R)-16-Désoxo-16-fluoro-14-O-[4-(2-morpholinoéthyl carbamoyl)butyryl)] pristinamycine II_{B}

A 280 mg de (16*R*)-16-désoxo-16-fluoro-14-O-[4-(2-morpholinoéthylcarbamoyl) butyryl] pristinamycine II_{B} en solution dans 3 cm³ de méthyl isobutyl cétone, on ajoute à 4°C, 0,2 cm³ d'éther chlorhydrique 3M. Le précipité formé est filtré sur verre fritté N° 4, rincé plusieurs fois par de la méthyl isobutyl cétone puis par de l'éther diéthylique. La poudre blanche obtenue est séchée à 20°C (90 Pa) pour donner 268 mg de chlorhydrate de (16*R*)-16-désoxo-16-fluoro-14-O-[4-(2-morpholinoéthyl carbamoyl)butyryl)] pristinamycine II_{B}, sous forme d'un solide blanc fondant à 134°C.

La (16*R*)-16-désoxo-16-fluoro-14-O-[4-(2-morpholinoéthylcarbamoyl)butyryl] pristinamycine IIB peut être préparée de la façon suivante :

A 1 g d'acide 5-(2-morpholinoéthylamino)-5-oxo pentanoïque en solution dans 40 cm³ de dichlorométhane, on ajoute 459 mg de (16*R*)-16-désoxo-16-fluoro pristinamycine II_{B} (préparée comme décrit dans l'exemple 1), 388 mg de N,N'-dicyclohexylcarbodiimide, puis 23 mg de 4-diméthylaminopyridine. La réaction est agitée à 20°C pendant 48 heures. La dicyclohexylurée formée est alors filtrée sur verre fritté N° 4 puis rinçée par de l'acétate d'éthyle. Le filtrat est concentrée à sec sous pression réduite (2,7 kPa), pour donner 1,35 g d'un solide qui est purifié par chromatographie-flash [éluant : dichlorométhane-méthanol (95-5 en volumes)]. On obtient ainsi 490 mg d'un produit qui est purifié à nouveau par chromatographie-flash sur alumine [éluant : acétate d'éthyle-méthanol (98-2 en volumes)]. Les fractions sont concentrées sous pression réduite (2,7 kPa) pour donner un solide qui est repris par de l'éther diéthylique et agité 18 heures. Le solide blanc obtenu est filtré, rincé par deux fois de l'éther diéthylique puis séché à 20°C (90 Pa) pour donner 294 mg de (16*R*)-14-O-[4-(2-morpholino éthylcarbamoyl)butyryl]-16-désoxo-16-fluoro pristinamycine IIB sous forme d'une poudre blanche.

Spectre de R.M.N. ¹H (400 MHz, CDCl₃, δ en ppm) : 0,96 (d, J = 6,5 Hz : 3H) ; 1,00 (d, J = 6,5 Hz : 3H) ; 1,10 (d, J = 6,5 Hz : 3H) ; de 1,65 à 2,05 (mt : 5H) ; 1,90 (s : 3H) ; 1,95 (mt : 2H) ; de 2,10 à 2,30 (mt : 2H) ; 2,25 (t, J = 7 Hz : 2H) ; 2,37 (mt : 2H) ; 2,47 (mt : 6H) ; 2,76 (mt : 1H) ; 2,99 (dt, J = 17 et 6 Hz : 1H) ; 3,24 (mt : 1H) ; 3,36 (dt, J = 7 et 6 Hz : 1H) ; 3,48 (mt : 1H) ; 3,71 (t, J = 5 Hz : 4H) ; 3,85 (mt : 1H) ; 4,07 (mt : 1H) ; 4,54 (mt : 1H) ; 4,79 (d large, J = 10 Hz : 1H) ; 4,82 (dd, J = 9 et 3 Hz : 1H) ; 5,12 (doublet démultiplié, J _{HF} = 48 Hz : 1 H) ; 5,30 (d, J = 9 Hz : 1H) ; de 5,70 à 5,85 (mt : 2H) ; 5,82 (d large, J = 17 Hz : 1H) ; 6,00 (mt : 2H) ; 6,19 (d, J = 16 Hz : 1H) ; 6,52 (dd, J = 17 et 5 Hz : 1H) ; 8,12 (s : 1H).

L'acide 5-(2-morpholinoéthylamino)-5-oxo pentanoïque peut être préparé de la façon suivante :

On ajoute à 10 cm³ de dioxane, 1,517 g d'anhydride glutarique puis, goutte à goutte, 1,75 cm³ de N-(2-aminoéthyl)morpholine. L'agitation est poursuivie à 20°C pendant 0,75 heure. Le solide blanc formé est filtré, lavé successivement par du dioxane et de l'éther diéthylique, puis séché à 20°C (90 Pa) pour donner 1,94 g d'acide 5-(2-morpholinoéthylamino)-5-oxo pentanoïque sous forme d'un solide blanc fondant à 96°C.

### Exemple 46

### Chlorhydrate de la (16R)-16-Désoxo-16-fluoro-14-O-{(3RS)-3-méthyl-4-[4-(pyrrolidin-1-yl-carbonylméthyl)pipérazin-1-yl carbonyl]butyryl} pristinamycine II_{B}

Dans un ballon placé sous azote, on introduit 1g de (16*R*)-16-désoxo-16-fluoro pristinamycine II_{B} (préparée comme décrit dans l'exemple 1) et 40 cm³ de dichlorométhane. Le mélange est chauffé jusqu'à dissolution puis on ajoute à 20°C, 620 mg d'acide 3-méthyl-5-oxo-5-[4-(2-oxo -2-pyrrolidin-1-yl éthyl)-pipérazin-1-yl]-pentanoïque, 389 mg de N,N'-dicyclohexylcarbodiimide puis 45 mg de 4-diméthylaminopyridine. Après 24 heures d'agitation à 20°C, on ajoute 124 mg d'acide 3-méthyl-5-oxo-5-[4-(2-oxo-2-pyrrolidin-1-yl-éthyl)pipérazin-1-yl] pentanoïque, 78 mg de N,N'-dicyclohexylcarbodiimide et 9 mg de 4-diméthylaminopyridine supplémentaires. La réaction est agitée à 20°C pendant 24 heures, puis le mélange réactionnel est filtré, rincé à l'acétate d'éthyle et concentré à sec sous pression réduite (2,7 kPa). Le résidu est agité 1 heure dans un mélange de 30 cm³ d'acétate d'éthyle et de 6 cm³ de dichlorométhane puis filtré. L'insoluble est filtré et rincé à l'acétate d'éthyle. Les filtrats sont rassemblés puis successivement lavés par 2 fois 5 cm³ d'une solution aqueuse de bicarbonate de sodium à 5% et par 10 cm³ d'eau saturée en chlorure de sodium. La phase organique résultante est séchée sur sulfate de magnésium, filtrée puis concentrée à sec sous pression réduite (2,7 kPa). Le solide jaune obtenu est agité dans 40 cm³ d'éther diéthylique pendant 18 heures, filtré puis purifié par chromatographie-flash [éluant : dichlorométhane-méthanol (95-5 en volumes)]. On obtient ainsi un produit qui est agité dans l'éther diéthylique, filtré puis séché à 30°C (90 Pa), pour donner 610 mg de (16*R*)-16-désoxo-16-fluoro-14-O-{(3*RS*)-3-méthyl-4-[4-(pyrrolidin-1-yl-carbonylméthyl)pipérazin-1-yl carbonyl]butyryl} pristinamycine II_{B}, sous forme d'une poudre blanche.

A ce solide, dissous dans 3 cm³ d'éthanol absolu, on ajoute 0,25 cm³ d'éther chlorhydrique 3 M puis 10 cm³ d'éther diéthylique. Les solvants sont évaporés sous pression réduite et le solide résultant est concrété dans 20 cm³ d'éther diéthylique. Après filtration et séchage à 25°C (90 Pa), on obtient 610 mg de chlorhydrate de (16*R*)-16-désoxo-16-fluoro-14-O-{(3*RS*)-3-méthyl-4-[4-(pyrrolidin-1-yl-carbonylméthyl)pipérazin-1-yl carbonyl]butyryl} pristinamycine II_{B}, sous forme d'un solide blanc fondant à 180°C.

Spectre de R.M.N. ¹H (400 MHz, (CD₃)₂SO d6, δ en ppm) : 0,88 (d, J = 6,5 Hz : 3H) ; de 0,90 à 1,00 (mt : 6H) ; 1,06 (d, J = 6,5 Hz : 3H) ; 1,58 (mt : 1H) ; de 1,70 à 2,50 (mt : 15H) ; 1,80 (s : 3H) ; 2,78 (mt : 1H) ; de 3,00 à 3,50 (mt : 10H) ; 3,40 (mt : 4H) ; 3,65 (mt : 1H) ; 3,72 (mt : 1H) ; 3,81 (mt : 1H) ; 3,95 (mt : 1H) ; 4,15 (mf : 2H) ; de 4,70 à 4,80 (mt : 2H) ; 5,10 (doublet démultiplié, J _{HF} = 48 Hz : 1H) ; 5,43 (d, J = 9 Hz : 1H) ; 5,66 (mt : 1H) ; 5,76 (mt : 1H) ; 5,83 (dd, J = 16 et 1,5 Hz : 1H) ; 6,20 (d, J = 16 Hz : 1H) ; 6,63 (dd, J = 16 et 4 Hz : 1H) ; 8,04 (t, J = 6 Hz : 1H) ; 8,48 (s : 1H) ; de 9,85 à 10,50 (mf très étalé : 1H).

L'acide (3*RS*)-3-méthyl-5-oxo-5-[4-(2-oxo-2-pyrrolidin-1-yl éthyl) pipérazin-1-yl] pentanoïque peut être obtenu de la façon suivante :

On ajoute à 10 cm³ de dioxane, 1,6 g d'anhydride 3-méthyl glutarique puis 2,62 g de pyrrolidinocarbonylméthyl pipérazine. Après 8 heures d'agitation on ajoute 320 mg d'anhydride 3-méthyl glutarique supplémentaires puis l'agitation est poursuivie à 20°C pendant 18 heures. Le solvant est alors évaporé sous pression réduite puis l'huile résultante est séchée à 45°C (90 Pa) pour donner 4,5 g d'acide (3*RS*)-3-méthyl-5-oxo-5-[4-(2-oxo-2-pyrrolidin-1-yl éthyl)pipérazin-1-yl] pentanoïque, sous forme d'une huile orange utilisée telle quelle.

### Exemple 47

Chlorhydrate de la (16*R*)-16-Désoxo-16-fluoro-14-O-[4-(4-méthylpipérazin-1-yl carbonyl)butyryl] pristinamycine II_{B}

En opérant comme à l'exemple 45 mais à partir de 580 mg d'acide 5-(4-méthylpipérazin-1-yl)-5-oxo pentanoïque, 1,21 g de (16*R*)-16-désoxo-16-fluoro pristinamycine II_{B} (préparée comme décrit dans l'exemple 1), 700 mg de N,N'-dicyclohexylcarbodiimide, 170 mg de 4-diméthylaminopyridine dans 25 cm³ de dichlorométhane, on obtient un solide qui est purifié par chromatographie flash [éluant : dichlorométhane / méthanol 95 / 5 en volumes)]. On obtient ainsi, après concentration à sec des fractions sous pression réduite (2,7 kPa), à 20°C, 1,45 g de ( 16*R*)-16-désoxo-16-fluoro-14-O-[4-(4-méthylpipérazin-1-yl carbonyl)butyryl] pristinamycine II_{B}. A ce solide dissous dans 8 cm³ de méthylisobutylcétone, on ajoute de l'éther chlorhydrique 3N jusqu'à précipitation compléte. Le solide obtenu est filtré, rinçé successivement par 3 fois 2 cm³ de méthylisobutylcétone et par 3 fois 10 cm³ d'éther diéthylique, puis séché (90 Pa) à 50°C, pour donner 1,24g de chlorhydrate de la (16*R*)-16-désoxo-16-fluoro-14-O-[4-(4-méthylpipérazin-1-yl carbonyl)butyryl] pristinamycine II_{B} sous forme d'un solide blanc fondant vers 165°C (déc.).

Spectre de R.M.N. ¹H (400 MHz, (CD₃)₂SO d6, δ en ppm) : 0,86 (mt : 3H) ; 0,94 (d, J = 6,5 Hz : 3H) ; 1,04 (d, J = 6,5 Hz : 3H) ; 1,52 (mt : 1H) ; de 1,65 à 2,15 (mt : 7H) ; 1,77 (s : 3H) ; 2,20 (mt : 1H) ; de 2,25 à 2,45 (mt : 4H) ; de 2,70 à 2,80 (mt : 1H) ; 2,76 (s : 3H) ; de 2,80 à 3,10 (mt : 3H) ; de 3,10 à 3,50 (mt : 5H) ; 3,60 (d large, J = 14 Hz : 1H) ; 3,68 (mt : 1H) ; 3,81 (mt : 1H) ; de 3,90 à 4,10 (mt : 2H) ; 4,43 (mf : 1H) ; de 4,70 à 4,80 (mt : 2H) ; 5,10 (doublet démultiplié, J _{HF} = 48 Hz : 1H) ; 5,43 (d, J = 9 Hz : 1H) ; 5,61 (mt : 1H) ; 5,74 (mt : 1H) ; 5,80 (d large, J = 16 Hz : 1H) ; 6,19 (d, J = 16 Hz : 1H) ; 6,62 (dd, J = 16 et 4 Hz : 1H) ; 8,17 (mt : 1H) ; 8,52 (s : 1H); 10,91 (mf: 1H).

L'acide 5-(4-méthylpipérazin-1-yl)-5-oxo pentanoïque peut être préparé de la façon suivante :

On ajoute à 20 cm³ de dioxane, 1,6 g d'anhydride 3-méthyl glutarique puis 2,62 g de pipérazin-1-yl carboxyméthyl pyrrolidine. Après 8 heures d'agitation on ajoute 320 mg d'anhydride 3-méthyl glutarique supplémentaires puis l'agitation est poursuivie à 20°C pendant 18 heures. Le solvant est évaporé sous pression réduite puis l'huile résultante est séchée à 45°C (90 Pa) pour donner 4,5 g d'acide 5-(4-méthylpipérazin-1-yl)-5-oxo pentanoïque, sous forme d'une huile orange utilisée telle quelle.

### Exemple 48

### Chlorhydrate de la (16R)-16-Désoxo-16-fluoro-14-O-[4-(4-éthylpipérazin-1-yl carbonyl)butyryl] pristinamycine II_{B}

A 390 mg de (16*R*)-16-désoxo-16-fluoro-14-O-[4-(4-éthylpipérazin-1-yl carbonyl)butyryl] pristinamycine II_{B} en solution dans 5 cm³ de méthyl isobutyl cétone, on ajoute à 20°C, 0,35 cm³ d'éther chlorhydrique 3 M. Le précipité formé est filtré, rincé successivement avec un minimum de méthyl isobutyl cétone et avec de l'éther diéthylique, puis séché à 20°C (90 Pa) pour donner 340 mg de chlorhydrate de (16*R*)-16-désoxo-16-fluoro-14-O-[4-(4-éthylpipérazin-1-yl carbonyl)butyryl] pristinamycine II_{B}, sous forme d'un solide blanc fondant vers 150°C (déc.).

Spectre de R.M.N. ¹H (400 MHz, (CD₃)₂SO d6, δ en ppm) : 0,87 (mt : 3H) ; 0,95 (d, J = 6,5 Hz : 3H) ; 1,05 (d, J = 6,5 Hz : 3H) ; 1,25 (t large, J = 7 Hz : 3H) ; 1,53 (mt : 1H) ; de 1,65 à 2,15 (mt : 7H) ; 1,79 (s : 3H) ; 2,20 (mt : 1H) ; de 2,30 à 2,45 (mt : 4H) ; 2,77 (mt : 1H) ; de 2,80 à 3,55 (mt : 10H) ; 3,61 (d large, J = 15 Hz : 1H) ; 3,69 (mt : 1H) ; 3,82 (mt : 1H) ; 3,96 (mt : 1H) ; 4,04 (d large, J = 13,5 Hz : 1H) ; 4,48 (d large, J = 13,5 Hz : 1H) ; de 4,70 à 4,80 (mt : 2H) ; 5,10 (doublet démultiplié, J _{HF} = 48 Hz : 1H) ; 5,43 (d, J = 9 Hz : 1H) ; 5,62 (mt : 1H) ; 5,75 (mt : 1H) ; 5,81 (dd, J = 16 et 1,5 Hz : 1H) ; 6,19 (d, J = 16 Hz : 1H) ; 6,63 (dd, J = 16 et 4 Hz : 1H) ; 8,15 (mt : 1H) ; 8,51 (s : 1H) ; 10,16 (mf large : 1H).

La ( 16*R*)-16-Désoxo-16-fluoro-14-O-[4-(4-éthylpipérazin-1-yl carbonyl)butyryl] pristinamycine II_{B} peut être préparée de la façon suivante :

En opérant comme à l'exemple 45 mais à partir de 430 mg d'acide 5-(4-éthylpipérazin-1-yl)-5-oxo pentanoïque, 1 g de (16*R*)-16-désoxo-16-fluoro pristinamycine II_{B} (préparée comme décrit dans l'exemple 1), 390 mg de N,N'-dicyclohexylcarbodiimide, 23 mg de 4-diméthylaminopyridine dans 40 cm³ de dichlorométhane, et après ajout de 50 mg de sulfate de magnésium, on ajoute après 27 heures d'agitation, 43 mg d'acide 5-(4-éthylpipérazin-1-yl)-5-oxo pentanoïque, 39 mg de N,N'-dicyclohexylcarbodiimide et 2,3 mg de 4-diméthylaminopyridine supplémentaires. Après 4 heures de réaction supplémentaires et traitement, on obtient 1,16 g d'un solide qui est purifié par chromatographie flash [éluant : dichlorométhane - méthanol 95/5 en volumes)]. On obtient ainsi, après concentration à sec des fractions sous pression réduite (2,7 kPa), à 20°C, 260 mg de produit pur et 420 mg d'un solide qui est repurifié par 2 chromatographies CLHP semi-préparatives [silice Hypersil 5 uM, éluant : dichlorométhane / méthanol (95 / 5 en volumes) puis en gradiant dichlorométhane / méthanol (97 / 3 puis 95 / 5 en volumes)]. Les deux lots ainsi obtenus sont réunis pour donner 390 mg de (16*R*)-16-désoxo-16-fluoro-14-O-[4-(4-éthylpipérazin-1-yl carbonyl)butyryl] pristinamycine II_{B}, sous forme d'un solide blanc.

L'acide 5-(4-éthylpipérazin-1-yl)-5-oxo pentanoïque peut être préparé de la façon suivante :

On ajoute sous argon à 10 cm³ de dioxane, 1,5 g de 1-éthylpipérazine puis 1,5 g d'anhydride glutarique en solution dans 5 cm³de dioxane. L'agitation est poursuivie à 25°C pendant 19 heures. Les solvants sont évaporés sous pression réduite (2,7 kPa) pour donner une huile brune qui est agitée dans de l'éther diéthylique. Le solide obtenu est filtré, rincé par de l'éther diéthylique puis séché à 20°C (90 Pa) pour donner 1,95 g d'acide 5-(4-éthylpipérazin-1-yl)-5-oxo pentanoïque sous forme d'un solide rosé hygroscopique utilisé tel quel.

### Exemple 49

### (16R)-16-Désoxo-14-O-[4-(4-éthoxycarbonylméthylpipérazin-1-yl carbonyl)butyryl]-16-fluoro pristinamycine II_{B}

Dans un ballon placé sous azote, on introduit 1g de (16*R*)-16-désoxo-16-fluoro pristinamycine II_{B} (préparée comme décrit dans l'exemple 1) et 30 cm³ de dichlorométhane. Le mélange est chauffé jusqu'à dissolution puis refroidi. On ajoute à 20°C, 540 mg d'acide 5-[4-(éthoxycarbonylméthylpiperazin-1-yl)]- 5-oxo pentanoïque en solution dans 10 cm³ de dichlorométhane, 23 mg de 4-diméthylaminopyridine puis 390 mg de N,N'-dicyclohexylcarbodiimide. Après 18 heures d'agitation à 20°C, on ajoute 54 mg d'acide 5-[4-(éthoxycarbonylméthylpiperazin-1-yl)]-5-oxo pentanoïque, 39 mg de N,N'-dicyclohexylcarbodiimide et 2,3 mg de 4-diméthylaminopyridine supplémentaires. La réaction est agitée à 20°C pendant 2 jours, puis le mélange réactionnel est filtré, rincé au dichlorométhane et concentré à sec sous pression réduite (2,7 kPa). Le solide résultant est agité 20 heures dans 40 cm³ d'éther diéthylique, filtré, séché à 20°C (90 Pa) puis purifié par chromatographie-flash [éluant : dichlorométhane-méthanol (98-2 en volumes)]. On obtient ainsi un produit qui est agité dans l'éther diéthylique, filtré puis séché à 20°C (90 Pa) pour donner 640 mg de (16*R*)-16-désoxo-14-O-[4-(4-éthoxycarbonylméthylpipérazin-1-yl carbonyl)butyryl]-16-fluoro pristinamycine II_{B} sous forme d'une poudre blanche.

A ce solide dissous dans 7 cm³de méthyl éthyl cétone, on ajoute 0,35 cm³ d'éther chlorhydrique 3 M. Le précipité obtenu est filtré sur verre fritté N° 3, rincé par 3 fois 10 cm³ de méthyl éthyl cétone puis par 3 fois 15 cm³ d'éther diéthylique. Après séchage à 20°C (90 Pa), on obtient 450 mg de chlorhydrate de ( 16*R*)-16-désoxo-14-O-[4-(4-éthoxycarbonylméthylpipérazin-1-yl carbonyl)butyryl]-16-fluoro pristinamycine II_{B}, sous forme d'un solide blanc fondant à 156°C.

Spectre de R.M.N. ¹H (400 MHz, (CD₃)₂SO d6, δ en ppm) : 0,88 (d, J = 6,5 Hz : 3H) ; 0,96 (d, J = 6,5 Hz : 3H) ; 1,06 (d, J = 6,5 Hz : 3H) ; 1,27 (t, J = 7 Hz : 3H) ; 1,57 (mt : 1H) ; de 1,70 à 2,35 (mt : 8H) ; 1,79 (s : 3H) ; 2,37 (mt : 4H) ; 2,78 (mt : 1H) ; de 2,90 à 3,50 (mt : 12H) ; 3,65 (mt : 1H) ; 3,72 (mt : 1H) ; 3,81 (mt : 1H) ; 3,95 (mt : 1H) ; 4,23 (q, J = 7 Hz : 2H) ; de 4,70 à 4,80 (mt : 2H) ; 5,10 (doublet démultiplié, J _{HF} = 48 Hz : 1H) ; 5,42 (d, J = 9 Hz : 1H) ; 5,66 (mt : 1H) ; 5,76 (mt : 1H) ; 5,82 (dd, J = 16 et 1,5 Hz 1H) ; 6,19 (d, J = 16 Hz : 1H) ; 6,63 (dd, J = 16 et 4 Hz : 1H) ; 8,03 (t, J = 6 Hz : 1H) ; 8,48 (s : 1H).

L'acide 5-[4-(éthoxycarbonylméthylpiperazin-1-yl)]-5-oxo pentanoïque peut être obtenu de la façon suivante :

Dans un ballon maintenu sous atmosphère d'argon, on ajoute à 10 cm³ de dioxane, 1,5 g d'anhydride glutarique puis 2,26 g de N-éthoxycarbonylméthylpipérazine en solution dans 5 cm³ de dioxane. Après 2 heures d'agitation à température ambiante, le solvant est évaporé sous pression réduite pour donner 3,66 g d'acide 5-[4-(éthoxycarbonylméthylpiperazin-1-yl)]-5-oxo pentanoïque, sous forme d'une huile orange utilisée telle quelle.

### Exemple 50

### (16R)-16-Désoxo-16-fluoro-14-O-{4-[4-(pyrrolidin-1-yl-carbonylméthyl)pipérazin-1-yl carbonyl]butyryl} pristinamycine II_{B}

En opérant comme à l'exemple 46 mais à partir de 1,75 g d'acide 5-oxo-5-[4-(2-oxo-2-pyrrolidin-1-yl éthyl)pipérazin-1-yl] pentanoïque, de 2,5g de (16*R*)-16-désoxo-16-fluoro pristinamycine II_{B} (préparée comme décrit dans l'exemple 1), de 1,16 g de N,N'-dicyclohexylcarbodiimide, et de 113 mg de 4-diméthylaminopyridine dans 100 cm³ de dichlorométhane, on obtient après 4 heures d'agitation à 20°C et après traitement analogue à celui de l'exemple 46, un solide qui est purifié par chromatographie flash [éluant : dichlorométhane - méthanol (95/5 en volumes)]. On obtient, après concentration à sec des fractions sous pression réduite (2,7 kPa), à 20°C, puis agitation du solide obtenu dans l'éther diéthylique pendant 18 heures, filtration et séchage (90 Pa) à 20°C, 2,46g de (16*R*)-16-désoxo-16-fluoro-14-O-{4-[4-(pyrrolidin-1-yl-carbonylméthyl)pipérazin-1-yl carbonyl]butyryl} pristinamycine II_{B}, sous forme d'une poudre jaune pâle fondant à 135°C.

Spectre de R.M.N. ¹H (500 MHz, CDCl₃, δ en ppm) : 0,96 (d, J = 6,5 Hz : 3H) ; 1,00 (d, J = 6,5 Hz : 3H) ; 1,10 (d, J = 6,5 Hz : 3H) ; de 1,65 à 2,05 (mt : 11H) ; 1,89 (s : 3H) ; de 2,10 à 2,30 (mt : 2H) ; 2,37 (mt : 4H) ; 2,54 (t, J = 5 Hz : 2H) ; 2,57 (t, J = 5 Hz : 2H) ; 2,76 (mt : 1H) ; 2,99 (dt, J = 17 et 6 Hz : 1H) ; 3,14 (s : 2H) ; 3,23 (mt : 1H) ; 3,48 (mt : 7H) ; 3,66 (t, J = 5 Hz : 2H) ; 3,86 (mt : 1H) ; 4,06 (mt : 1H) ; 4,53 (mt : 1H) ; 4,79 (dd, J = 10 et 2 Hz : 1H) ; 4,82 (dd, J = 9 et 3 Hz : 1H) ; 5,10 (doublet démultiplié, J _{HF} = 48 Hz : 1H) ; 5,30 (d, J = 9 Hz : 1H) ; de 5,70 à 5,85 (mt : 3H) ; 6,06 (mt : 1H) ; 6,18 (d, J = 16 Hz : 1H) ; 6,52 (dd, J = 16,5 et 5 Hz : 1H) ; 8,12 (s : 1H).

L'acide 5-oxo-5-[4-(2-oxo-2-pyrrolidin-1-yl éthyl)pipérazin-1-yl] pentanoïque peut être préparé de la façon suivante.

On ajoute à 10 cm³ de dioxane sec, 1,52 g d'anhydride glutarique puis 2,62 g de pyrrolidino carbonylméthyl pipérazine. Après 4 heures d'agitation, le précipité formé est dilué par 10 cm³ de dioxane puis agité 1 heure. L'insoluble est filtré sur verre fritté N°4, rincé plusieurs fois à l'éther diéthylique, puis séché (90 Pa) à 20°C pour donner 3,36 g d'acide 5-oxo-5-[4-(2-oxo-2-pyrrolidin-1-yl éthyl)pipérazin-1-yl] pentanoïque sous forme d'un solide blanc.

### Exemple 51

Chlorhydrate de la (16*R*)-16-Désoxo-16-fluoro-14-O-[4-(cis-3,5-diméthylpipérazin-1-yl carbonyl)butyryl)] pristinamycine II_{B}

A 680 mg de (16*R*)-16-désoxo-16-fluoro-14-O-[4-(cis-3,5-diméthylpipérazin-1-yl carbonyl)butyryl)] pristinamycine II_{B} en solution dans 12 cm³ de méthyl isobutyl cétone et la quantité suffisante d'éthanol pour dissoudre le produit à chaud, on ajoute à 20°C, 0,58 cm³ d'éther chlorhydrique 3 M. Le mélange est concentré à sec sous pression réduite (2,7 kPa), à 20°C et le solide obtenu est agité 1,5 heure dans 10 cm³ d'éther diéthylique. Le solide est filtré sur verre fritté N°4, puis séché à 20°C (90 Pa) pour donner 650 mg de chlorhydrate de (16*R*)-16-désoxo-16-fluoro-14-O-[4-(cis-3,5-diméthylpipérazin-1-yl carbonyl)butyryl)] pristinamycine II_{B}, sous forme d'un solide blanc fondant à 205°C.

Spectre de R.M.N. ¹H (600 MHz, (CD₃)₂SO d6, δ en ppm) : 0,87 (mt : 3H) ; 0,95 (d, J = 6,5 Hz : 3H) ; 1,05 (d, J = 6,5 Hz : 3H) ; 1,27 (d, J = 6,5 Hz : 6H) ; 1,53 (mt : 1H) ; de 1,65 à 1,85 (mt : 3H) ; 1,78 (s : 3H) ; de 1,85 à 2,00 (mt : 3H) ; 2,11 (mt : 1H) ; 2,21 (mt : 1H) ; de 2,25 à 2,50 (mt : 5H) ; 2,61 (mt : 1H) ; 2,78 (mt : 1H) ; de 3,05 à 3,30 (mt : 4H) ; 3,61 (d large, J = 15 Hz : 1H) ; 3,69 (mt : 1H) ; 3,82 (mt : 1H) ; de 3,90 à 4,05 (mt : 2H) ; 4,50 (d large, J = 13,5 Hz : 1H) ; de 4,70 à 4,80 (mt : 2H) ; 5,11 (doublet démultiplié, J _{HF} = 48 Hz : 1H) ; 5,44 (d, J = 9 Hz : 1H) ; 5,62 (mt : 1H) ; 5,75 (mt : 1H) ; 5,81 (d large, J = 15,5 Hz : 1H) ; 6,20 (d, J = 15 Hz : 1H) ; 6,63 (dd, J = 15,5 et 4 Hz : 1H) ; 8,17 (t, J = 6 Hz : 1H) ; 8,52 (s : 1H) ; 9,14 (mt : 1H) ; 9,56 (mt : 1H).

La (16*R*)-16-Désoxo-16-fluoro-14-O-[4-(3,5-diméthylpipérazin-1-yl carbamoyl) butyryl)] pristinamycine II_{B} peut être préparée de la façon suivante :

En opérant comme à l'exemple 45 mais à partir de 470 mg d'acide 5-(3,5 diméthylpipérazin-1-yl)-5-oxo pentanoïque, 1 g de (16*R*)-16-désoxo-16-fluoro pristinamycine II_{B} (préparée comme décrit dans l'exemple 1), 430 mg de N,N'-dicyclohexylcarbodiimide, 23 mg de 4-diméthylaminopyridine mais dans un mélange de 40 cm³ de dichlorométhane et de 5 cm³ diméthylformamide, et après 60 heures d'agitation, on isole 2,5 g d'une huile brune qui est purifiée par chromatographie flash [éluant : dichlorométhane - méthanol (97/3 en volumes)]. On obtient ainsi, après concentration à sec des fractions sous pression réduite (2,7 kPa), à 20°C, puis agitation du solide obtenu dans 10 cm³ d'éther diéthylique, filtration et séchage (90 Pa), à 50°C, 680 mg de (16*R*)-16-désoxo-16-fluoro-14-O-[4-(3,5-diméthylpipérazin-1-yl carbonyl)butyryl)] pristinamycine II_{B}, sous forme d'un solide blanc.

L'acide 5-(3,5 diméthylpipérazin-1-yl)-5-oxo pentanoïque peut être préparé de la façon suivante :

On ajoute sous argon à un mélange de 30 cm³ de dioxane et 5 cm³ de dichlorométhane, 1,5 g de 2,6-diméthylpipérazine. Après agitation, l'insoluble résiduel est filtré et le filtrat placé dans un tricol. On ajoute goutte à goutte, 1,5 g d'anhydride glutarique en solution dans 10 cm³de dioxane puis l'agitation est poursuivie à 25°C pendant 2 heures. Les solvants sont évaporés sous pression réduite (2,7 kPa) pour donner un solide qui est repris par 100 cm³ d'éther diéthylique, filtré, rincé par de l'éther diéthylique puis séché à 20°C (90 Pa) pour donner 2,19 g d'acide 5-(3,5 diméthylpipérazin-1-yl)-5-oxo pentanoïque sous forme d'un solide blanc utilisé tel quel.

### Exemple 52

### Chlorhydrate de la (16R)-16-Désoxo-16-fluoro-14-O-{4-[4-(méthyl phényl carbamoylméthyl)pipérazin-1-yl carbonyl)]butyryl} pristinamycine II_{B}

A 470 mg de (16*R*)-16-désoxo-16-fluoro-14-O-{4-[4-(méthylphényl carbamoylméthyl)pipérazin-1-yl carbonyl)]butyryl} pristinamycine II_{B}, en solution dans 5 cm³ de méthyl isobutyl cétone, on ajoute à 4°C, 0,3 cm³ d'éther chlorhydrique 3 M. Après une heure d'agitation, le précipité formé est filtré sur verre fritté N°4 puis rincé successivement par un minimum de méthylisobutyl cétone et par de l'éther diéthylique. Le solide obtenu est séché à 20°C (90 Pa) pour donner 463 mg de chlorhydrate de (16*R*)-16-désoxo-16-fluoro-14-O-{4-[4-(méthylphényl carbamoylméthyl)pipérazin-1-yl carbonyl)]butyryl} pristinamycine II_{B}, sous forme d'un solide blanc fondant vers 155°C.

Spectre de R.M.N. ¹H (500 MHz, (CD₃)₂SO d6, δ en ppm) : 0,87 (mt : 3H) ; 0,95 (d, J = 6,5 Hz : 3H) ; 1,05 (d, J = 6,5 Hz : 3H) ; 1,53 (mt : 1H) ; de 1,65 à 1,85 (mt : 3H) ; 1,77 (s : 3H) ; de 1,85 à 2,05 (mt : 3H) ; 2,11 (mt : 1H) ; 2,19 (mt : 1H) ; 2,34 (mt : 4H) ; 2,78 (mt : 1H) ; de 2,85 à 3,55 et de 3,80 à 4,05 (mt : 1 1H en totalité) ; 3,26 (s : 3H) ; 3,60 (d large, J = 15 Hz : 1H) ; 3,69 (mt : 1H) ; 3,81 (mt : 1H) ; 3,95 (mt : 1H) ; de 4,25 à 4,60 (mf : 1 H) ; de 4,70 à 4,80 (mt : 2H) ; 5,09 (doublet démultiplié, J _{HF} = 48 Hz : 1H) ; 5,42 (d, J = 9,5 Hz : 1H) ; 5,62 (mt : 1H) ; 5,74 (mt : 1H) ; 5,81 (dd, J = 16 et 2 Hz : 1H) ; 6,19 (d, J = 15,5 Hz : 1H) ; 6,63 (dd, J = 16 et 4 Hz : 1H) ; de 7,25 à 7,60 (mt : 5H) ; 8,14 (mt : 1H) ; 8,52 (s : 1H) ; 10,13 (mf étalé).

La (16*R*)-16-Désoxo-16-fluoro-14-O-{4-[4-(méthylphényl carbamoylméthyl) pipérazin-1-yl carbonyl)]butyryl} pristinamycine II_{B} peut être préparée de la façon suivante :

En opérant comme à l'exemple 45 mais à partir de 653 mg d'acide 5-[4-(méthylphényl carbamoylméthyl) pipérazin-1-yl]-5-oxo pentanoïque, 1g de (16*R*)-16-désoxo-16-fluoro pristinamycine II_{B} (préparée comme décrit dans l'exemple 1), 388 mg de N,N'-dicyclohexylcarbodiimide en solution dans 10 cm³ de dichlorométhane ajoutés goutte à goutte en 3 heures, 23 mg de 4-diméthylaminopyridine dans 50 cm³ de dichlorométhane, et après 22 heures d'agitation, on obtient 1,49 g d'un solide qui est purifié par chromatographie flash [éluant : dichlorométhane - méthanol (97/3 en volumes)]. On obtient ainsi, après concentration à sec des fractions sous pression réduite (2,7 kPa), à 20°C, puis agitation du solide obtenu dans l'éther diéthylique, filtration et séchage (90 Pa) à 50°C, 470 mg de (16*R*)-16-désoxo-16-fluoro-14-O-{4-[4-(méthyl phényl carbamoylméthyl)pipérazin-1-yl carbonyl)]butyryl} pristinamycine II_{B}, sous forme d'un solide blanc.

L'acide 5-[4-(méthylphényl carbamoylméthyl) pipérazin-1-yl]-5-oxo pentanoïque peut être obtenu de la manière suivante :

On ajoute à 15 cm³ de dioxane, 1,517 g d'anhydride glutarique puis 1,715 g de N-[2-(pipérazin-1-yl)acétyl]-N-méthylaniline. Après 19 heures d'agitation, à 25°C, le précipité blanc formé est filtré sur verre fritté N°4, rincé au dioxane puis à l'éther diéthylique puis séché à 20°C (90 Pa) pour donner 2 g d'acide 5-[4-(méthylphényl carbamoylméthyl)pipérazin-1-yl]-5-oxo pentanoïque sous forme d'un solide blanc utilisé tel quel.

### Exemple 53

### Chlorhydrate de la (16R)-16-Désoxo-16-fluoro-14-O-{4-[4-(2-méthoxyéthyl) pipérazin-1-yl carbonyl]butyryl} pristinamycine II_{B}

Dans un ballon placé sous azote on ajoute, 1 g de (16*R*)-16-désoxo-16-fluoro pristinamycine II_{B} (préparée comme décrit dans l'exemple 1) et 40 cm³ de dichlorométhane. Le mélange est chauffé jusqu'à dissolution puis refroidi. On ajoute à 20°C, 485 mg d'acide 5-[4-(2-méthoxyéthyl)piperazin-1-yl]-5-oxo pentanoïque puis 45 mg de 4-diméthylaminopyridine. On ajoute ensuite, goutte à goutte, pendant 3 heures, 388 mg de N,N'-dicyclohexylcarbodiimide en solution dans 10 cm³ de dichlorométhane. Après 18 heures d'agitation à 20°C, on ajoute 48 mg acide 5-[4-(2-méthoxyéthyl)piperazin-1-yl)-5-oxo pentanoïque et 38 mg de N,N'-dicyclohexylcarbodiimide supplémentaires en solution dans 5 cm³ de dichlorométhane. La réaction est agitée à 20°C pendant 3 heures, puis le mélange réactionnel est filtré, rincé à l'acétate d'éthyle et le filtrat concentré à sec sous pression réduite (2,7 kPa). Le résidu obtenu est agité 0,5 heure dans 40 cm³ d'acétate d'éthyle puis filtré. L'insoluble est rincé à l'acétate d'éthyle. Les filtrats sont rassemblés puis lavés par 10 cm³ d'une solution aqueuse d'eau saturée en chlorure de sodium, séchés sur sulfate de magnésium, filtrés et concentrés à sec sous pression réduite (2,7 kPa). Le solide obtenu est agitéune première fois dans 40 cm³ d'éther diisopropylique pendant 18 heures puis filtré et à nouveau agitédans un mélange de 40 cm³ d'éther diisopropylique et de 10 cm³ d'éther éthylique. Le solide résultant est filtré puis purifié par chromatographie-flash [éluant : dichlorométhane-méthanol (97-3 en volumes)]. On obtient ainsi un produit qui est agitédans 40 cm³ d'éther diisopropylique, filtré puis séché à 30°C (90 Pa) pour donner 815 mg de (16*R*)-16-désoxo-16-fluoro-14-O-{4-[4-(2-méthoxyéthyl)pipérazin-1-yl carbonyl]butyryl} pristinamycine II_{B}, sous forme d'une poudre blanche.

A ce solide dissous dans 4 cm³ d'éthanol absolu, on ajoute 0,35 cm³ d'éther chlorhydrique 3 M puis lentement 20 cm³ d'éther diéthylique. Les solvants sont évaporés sous pression réduite et le solide résultant agitédans 20 cm³ d'éther diéthylique. Après filtration et séchage à 20°C (90 Pa), on obtient 800 mg de chlorhydrate de (16*R*)-16-désoxo-16-fluoro-14-O-{4-[4-(2-méthoxyéthyl)pipérazin-1-yl carbonyl]butyryl} pristinamycine II_{B}, sous forme d'un solide blanc fondant à 170°C.

Spectre de R.M.N. ¹H (400 MHz, (CD₃)₂SO d6, δ en ppm) : 0,86 (d, J = 6,5 Hz : 3H) ; 0,95 (d, J = 6,5 Hz : 3H) ; 1,04 (d, J = 6,5 Hz : 3H) ; 1,52 (mt : 1H) ; de 1,65 à 2,05 (mt : 6H) ; 1,77 (s : 3H) ; de 2,05 à 2,30 (mt : 2H) ; 2,37 (mt : 4H) ; 2,78 (mt : 1H) ; de 2,85 à 3,55 (mt : 13H) ; 3,59 (mt : 1H) ; de 3,65 à 3,75 (mt : 3H) ; 3,81 (mt : 1H) ; de 3,90 à 4,10 (mt : 2H) ; 4,42 (d large, J = 13 Hz : 1H) ; de 4,70 à 4,80 (mt : 2H) ; 5,10 (doublet démultiplié, J _{HF} = 48 Hz : 1H) ; 5,43 (d, J = 9 Hz : 1H) ; 5,61 (mt : 1H) ; 5,74 (mt : 1H) ; 5,81 (dd, J = 16 et 1,5 Hz : 1H) ; 6,20 (d, J = 16 Hz : 1H) ; 6,64 (dd, J = 16 et 4 Hz : 1H) ; 8,18 (t, J = 6 Hz : 1H) ; 8,52 (s : 1H) ; 10,46 (mf large : 1H).

L'acide 5-[4-(2-méthoxyéthyl)piperazin-1-yl]-5-oxo pentanoïque peut être obtenu de la façon suivante :

On ajoute à 13 cm³ de dioxane, 1,52 g d'anhydride glutarique puis 1,92 g de 1-(2-méthoxyéthyl)pipérazine en solution dans 3 cm³ de dioxane. Après 5 heures d'agitation à température ambiante, on ajoute 300 mg d'anhydride glutarique supplémentaires puis l'agitation est poursuivie à 20°C, pendant 18 heures. Le solvant est évaporé sous pression réduite et l'huile résultante séchée à 20°C (90 Pa) pour donner 3,4 g d'acide 5-[4-(2-méthoxyéthyl)piperazin-1-yl]-5-oxo pentanoïque, sous forme d'une laque orange utilisée telle quelle.

### Exemple 54

### Chlorhydrate de la (16R)-16-Désoxo-16-fluoro-14-O-[4-(4-propylpipérazin-1-yl carbonyl)butyryl] pristinamycine II_{B}

En opérant comme à l'exemple 46 mais à partir de 0,5 g d'acide 5-oxo-5-(4-propylpiperazin-1-yl) pentanoïque, de 1 g de (16*R*)-16-désoxo-16-fluoro pristinamycine II_{B} (préparée comme décrit dans l'exemple 1), de 430 mg de N,N'-dicyclohexylcarbodiimide, et de 23 mg de 4-diméthylaminopyridine dans 50 cm³ de dichlorométhane, on obtient après 4 heures d'agitation à 20°C et après filtration et évaporation à sec sous pression réduite du solvant (2,7 kPa) à 20°C, un solide qui est purifié par chromatographie flash [éluant : dichlorométhane - méthanol (97/3 en volumes)]. On obtient, après concentration à sec des fractions sous pression réduite (2,7 kPa), à 20°C, puis agitation du solide obtenu dans l'éther diéthylique, filtration et séchage (90 Pa) à 20°C, 960 mg de (16*R*)-16-désoxo-16-fluoro-14-O-[4-(4-propylpipérazin-1-yl carbonyl)butyryl] pristinamycine II_{B}, sous forme d'une poudre blanche.

A ce solide dissous dans 10 cm³ de méthyl éthyl cétone, on ajoute 0,75 cm³ d'éther chlorhydrique 3 M. Le précipité obtenu est filtré sur verre fritté N° 3, rincé par 2 fois 10 cm³de méthyl éthyl cétone puis par 2 fois 15 m³ d'éther diéthylique. Après filtration et séchage à 20°C (90 Pa), on obtient 700 mg de chlorhydrate de (16*R*)-16-désoxo-16-fluoro-14-O[4-(4-propylpipérazin-1-yl carbonyl)butyryl] pristinamycine II_{B}, sous forme d'un solide blanc fondant à 145°C.

Spectre de R.M.N. ¹H (400 MHz, (CD₃)₂SO d6, δ en ppm) : 0,86 (d, J = 6,5 Hz : 3H) ; de 0,90 à 1,00 (mt : 6H) ; 1,04 (d, J = 6,5 Hz : 3H) ; 1,54 (mt : 1H) ; de 1,60 à 2,25 (mt : 10H) ; 1,78 (s : 3H) ; de 2,30 à 2,45 (mt : 4H) ; 2,76 (mt : 1H) ; de 2,80 à 3,10 (mt : 6H) ; 3,21 (mt : 1H) ; de 3,40 à 3,55 (mt : 3H) ; 3,60 (mt : 1H) ; 3,68 (mt : 1H) ; 3,82 (mt : 1H) ; de 3,90 à 4,10 (mt : 2H) ; 4,45 (d large, J = 14 Hz : 1H) ; de 4,70 à 4,80 (mt : 2H) ; 5,09 (doublet démultiplié, J _{HF} = 48 Hz : 1H) ; 5,41 (d, J = 9 Hz : 1H) ; 5,61 (mt : 1H) ; 5,74 (mt : 1H) ; 5,80 (d large, J = 16 Hz : 1H) ; 6,19 (d, J = 15,5 Hz : 1H) ; 6,62 (dd, J = 16 et 4 Hz : 1H) ; 8,14 (t large, J = 5,5 Hz : 1H) ; 8,48 (s : 1H) ; 10,41 (mf large : 1H).

L'acide 5-oxo-5-(4-propylpiperazin-1-yl) pentanoïque peut être obtenu de la façon suivante :

Dans un ballon maintenu sous atmosphère d'argon, on ajoute à 10 cm³ de dioxane, 1,5 g d'anhydride glutarique puis 1,66 g de N-propylpipérazine en solution dans 5 cm³de dioxane. Après 2,5 heures d'agitation à température ambiante, le solvant est évaporé sous pression réduite puis l'huile résultante est additionnée de 100 cm³d'éther diéthylique. Après une heure d'agitation, le solide obtenu est filtré, séché à 20°C (90 Pa) pour donner 2,47 g d'acide 5-oxo- 5-(4-propylpiperazin-1-yl) pentanoïque sous forme d'un solide blanc utilisé tel quel.

### Exemple 55

### (16R)-16-Désoxo-16-fluoro-14-O-{4-[4-(3-imidazol-1-yl propyl)pipérazin-1-yl carbonyl]butyryl} pristinamycine II_{B}

Dans un ballon placé sous azote on ajoute, 1,42 g de (16*R*)-14-O-{4-[4-(3-chloropropyl)pipérazin-1-yl carbonyl]butyryl}-16-désoxo-16-fluoro pristinamycine II_{B}, 6 cm³ de diméthylformamide, 0,24 g d'imidazole et 10 mg d'iodure de sodium. Le mélange est chauffé à 75°C pendant 5 heures puis agité 60 heures à 20°C. On rajoute alors à 20°C, 0,12 g d'imidazole supplémentaire en solution dans 2 cm³ de DMF et l'agitation est poursuivie 4 heures à 75°C puis 18 heures à 20°C. Le mélange obtenu est alors concentré à sec sous pression réduite (2,7 kPa). Le solide résultant est repris par 50 cm³ de dichlorométhane et la solution obtenue est lavée par 2 fois 40 cm³ d'eau distillée. La phase organique est décantée, séchée sur sulfate de magnésium, filtrée puis concentrée à sec sous pression réduite pour donner 1,14 g d'un solide qui est purifié par chromatographie-flash [éluant : dichlorométhane-méthanol (gradient 95 / 5 puis 90 / 10 en volumes)]. On obtient ainsi un solide qui est agité dans l'éther diéthylique pendant 40 heures, filtré puis séché à 20°C (90 Pa) pour donner 290 mg de (16*R*)-16-désoxo-16-fluoro-14-O-{4-[4-(3-imidazol-1-yl propyl)pipérazin-1-yl carbonyl]butyryl} pristinamycine II_{B} sous forme d'un solide blanc cassé fondant à 122°C.

Spectre de R.M.N. ¹H (400 MHz, CDCl₃, δ en ppm) : 0,96 (d, J = 6,5 Hz : 3H) ; 1,00 (d, J = 6,5 Hz : 3H) ; 1,09 (d, J = 6,5 Hz : 3H) ; de 1,45 à 2,05 (mt : 9H) ; 1,88 (s : 3H) ; de 2,10 à 2,30 (mt : 2H) ; 2,29 (t, J = 7 Hz : 2H) ; 2,37 (mt : 8H) ; 2,76 (mt : 1H) ; 2,99 (mt : 1H) ; 3,23 (mt : 1 H) ; de 3,40 à 3,55 (mt : 3H) ; 3,62 (mt : 2H) ; 3,86 (mt : 1H) ; de 4,00 à 4,10 (mt : 1H) ; 4,05 (t, J = 7 Hz : 2H) ; 4,53 (mt : 1H) ; 4,78 (dd, J = 10 et 2 Hz : 1H) ; 4,82 (dd, J = 9 et 3 Hz : 1H) ; 5,12 (doublet démultiplié, J _{HF} = 48 Hz : 1H) ; 5,31 (d, J = 9 Hz : 1H) ; 5,75 (mt : 1H) ; de 5,75 à 5,85 (mt : 1H) ; 5,82 (dd, J = 16 et 2 Hz : 1H) ; 6,00 (mt : 1H) ; 6,19 (d, J = 16 Hz : 1H) ; 6,52 (dd, J = 16 et 5 Hz : 1H) ; 6,93 (mf : 1H) ; 7,08 (s large : 1H) ; 7,50 (s large : 1H) ; 8,12 (s : 1H).

La (16*R*)-14-O-{4-[4-(3-chloropropyl)pipérazin-1-yl carbonyl]butyryl}-16-désoxo-16-fluoro pristinamycine II_{B} peut être obtenue en opérant comme à l'exemple 46 mais à partir de 0,94 g d'acide 5-[4-(3-chloropropyl)piperazin-1-yl)]-5-oxo pentanoïque, de 1,5 g de (16*R*)-16-désoxo-16-fluoro pristinamycine II_{B} (préparée comme décrit dans l'exemple 1), de 460 mg de N,N'-dicyclohexylcarbodiimide, et de 23 mg de 4-diméthylaminopyridine dans 40 cm³ de dichlorométhane. On obtient ainsi, après 23 heures d'agitation à 20°C, une suspension qui est filtrée. Le filtrat est concentré à sec sous pression réduite (2,7 kPa) à 20°C, pour donner un solide qui est agité dans de l'éther diisopropylique, filtré puis dissous dans 50 cm³ de dichlorométhane. La solution obtenue est lavée par 30 cm³ d'eau distillée, décantée, séchée sur sulfate de magnésium, filtrée et concentrée à sec sous pression réduite (2,7 kPa) à 20°C, pour donner 1,42 g de (16*R*)-14-O-{4-[4-(3-chloropropyl)pipérazin-1-yl carbonyl]butyryl}-16-désoxo-16-fluoro pristinamycine II_{B}, sous forme d'un solide jaune utilisé tel quel.

L'acide 5-[4-(3-chloropropyl)piperazin-1-yl)]-5-oxo pentanoïque peut être obtenu de la façon suivante :

Dans un ballon maintenu sous atmosphère d'argon, on ajoute à 15 cm³ de dioxane, 1,5 g d'anhydride glutarique puis 2,13 g de N-(chloropropyl) pipérazine en solution dans 5 cm³ de dioxane et 1 cm³ de dichlorométhane. Après 2 heures d'agitation à 20°C, le solvant est évaporé sous pression réduite (2,7 kPa), à 20°C, pour donner 3,75 g d'acide 5-[4-(3-chloropropyl)piperazin-1-yl)]-5-oxo pentanoïque sous forme d'une huile jaune utilisée telle quelle

### Exemple 56

### Chlorhydrate de la (16R)-16-Désoxo-16-fluoro-14-O-[4-(2-morpholinoéthoxy carbonyl)]butyryl} pristinamycine II_{B}

A 550 mg de (16*R*)-16-désoxo-16-fluoro-14-O-[4-(2-morpholinoéthoxycarbonyl)] butyryl} pristinamycine II_{B} en solution dans 5,5 cm³ de méthylisobutyl cétone, on ajoute à 4°C, 0,42 cm³ d'éther chlorhydrique 3 M. Après une heure d'agitation, le précipité formé est filtré sur verre fritté N°4, rincé successivement avec de la méthylisobutyl cétone et de l'éther diéthylique puis séché à 20°C (90 Pa) pour donner 458 mg de chlorhydrate de la (16*R*)-16-désoxo-16-fluoro-14-O-[4-(2-morpholinoéthoxycarbonyl)]butyryl} pristinamycine II_{B}, sous forme d'un solide blanc fondant à 168°C.

Spectre de R.M.N. ¹H (500 MHz, (CD₃)₂SO d6, δ en ppm) : 0,85 (mt : 3H) ; 0,94 (d, J = 6,5 Hz : 3H) ; 1,03 (d, J = 6,5 Hz : 3H) ; 1,50 (mt : 1H) ; de 1,70 à 1,85 (mt : 3H) ; 1,77 (s : 3H) ; de 1,85 à 2,05 (mt : 3H) ; 2,09 (mt : 1H) ; 2,19 (mt : 1H) ; 2,38 (mt : 2H) ; 2,42 (t, J = 7 Hz : 2H) ; 2,77 (mt : 1H) ; de 3,10 à 3,50 (mt : 8H) ; 3,58 (d large, J = 15 Hz : 1H) ; 3,68 (mt : 1H) ; de 3,70 à 3,85 (mt : 3H) ; de 3,90 à 4,00 (mt : 3H) ; 4,39 (mt : 2H) ; de 4,70 à 4,80 (mt : 2H) ; 5,09 (doublet démultiplié, J _{HF} = 48 Hz : 1H) ; 5,42 (d, J = 9 Hz : 1H) ; 5,60 (mt : 1H) ; 5,74 (mt : 1H) ; 5,80 (d large, J = 16 Hz : 1H) ; 6,19 (d, J = 16 Hz : 1H) ; 6,62 (dd, J = 16 et 4 Hz : 1H) ; 8,17 (mt : 1H) ; 8,52 (s : 1H) ; 10,50 (mf étalé : 1H).

La (16*R*)-16-désoxo-16-fluoro-14-O-[4-(2-morpholinoéthoxycarbonyl)]butyryl} pristinamycine II_{B} peut être obtenu de la façon suivante :

En opérant comme à l'exemple 45 mais à partir de 461 mg du mono ester (2-morpholinoéthyl) de l'acide glutarique, 1 g de (16*R*)-16-désoxo-16-fluoro pristinamycine II_{B} (préparée comme décrit dans l'exemple 1), 388 mg de N,N'-dicyclohexylcarbodiimide, 23 mg de 4-diméthylaminopyridine dans 40 cm³ de dichlorométhane, et après 22 heures d'agitation, on obtient 1,33 g d'un solide qui est purifié par deux chromatographies flash successives [éluant : respectivement dichlorométhane / méthanol (97/3 en volumes) et dichlorométhane / méthanol (98 / 2 en volumes)]. Après concentration à sec des fractions sous pression réduite (2,7 kPa), agitation du solide obtenu dans l'éther diéthylique, filtration et séchage (90 Pa), à 20°C, on obtient 550 mg de (16*R*)-16-désoxo-16-fluoro-14-O-[4-(2-morpholinoéthoxycarbonyl)]butyryl} pristinamycine II_{B}, sous forme d'un solide blanc.

Le mono ester (2-morpholinoéthyl) de l'acide glutarique peut être obtenu de la façon suivante :

On ajoute à 10 cm³ de dioxane, 1,517 g d'anhydride glutarique puis 1,61 cm³ de N-2-hydroxyéthylmorpholine. Après 19 heures d'agitation à 25°C, le solvant est évaporé sous pression réduite (2,7 kPa) et le produit obtenu est séché à 55°C (90 Pa) pour donner 3,18 g du mono ester (2-morpholin-4-yl-éthyl) de l'acide glutarique sous forme d'une huile brune utilisée telle quelle.

### Exemple 57

### (16R)-16-Désoxo-16-fluoro-14-O-{4-[4-(2-morpholinoéthyl)pipérazin-1-yl carbonyl]butyryl} pristinamycine II_{B}

En opérant comme à l'exemple 46 mais à partir de 0,7 g d'acide 5-[4-(2-morpholinoéthyl piperazin-1-yl)]-5-oxo pentanoïque, de 1 g de (16R)-16-désoxo-16-fluoro pristinamycine II_{B} (préparée comme décrit dans l'exemple 1), de 460 mg de N,N'-dicyclohexylcarbodiimide, et de 23 mg de 4-diméthylaminopyridine dans 40 cm³ de dichlorométhane, on obtient, après 4 heures d'agitation à 20°C, une suspension qui est filtrée. Le filtrat est concenté à sec sous pression réduite (2,7 kPa) à 20°C, pour donner une poudre beige qui est purifiée par chromatographie flash [éluant : dichlorométhane - méthanol (96/4 en volumes)]. On obtient, après concentration à sec des fractions sous pression réduite (2,7 kPa) à 20°C, puis agitation du solide obtenu dans l'éther diisopropylique pendant 36 heures, filtration et séchage (90 Pa) à 20°C, 750 mg d'un solide. Ce solide est à nouveau agité dans un mélange de 20 cm³ d'éther diisopropylique et de 10 cm³ d'éther diéthylique pendant 60 heures. Après filtration et séchage (90 Pa) à 20°C, le solide obtenu est agité à 60°C, dans 60 cm³ d'éther diisopropylique pendant 1,5 heure puis filtré et séché (90 Pa) à 20°C pour donner 0,55 g de (16*R*)-16-désoxo-16-fluoro-14-O-{4-[4-(2-morpholinoéthyl)pipérazin-1-yl carbonyl]butyryl} pristinamycine II_{B}, sous forme d'une poudre jaune clair fondant à 116°C.

Spectre de R.M.N. ¹H (400 MHz, CDCl₃, δ en ppm) : 0,96 (d, J = 6,5 Hz : 3H) ; 1,00 (d, J = 6,5 Hz : 3H) ; 1,10 (d, J = 6,5 Hz : 3H) ; de 1,55 à 2,05 (mt : 7H) ; 1,89 (s : 3H) ; de 2,10 à 2,30 (mt : 2H) ; 2,38 (mt : 4H) ; de 2,40 à 2,60 (mt : 12H) ; 2,76 (mt : 1H) ; 2,98 (mt : 1H) ; 3,23 (mt : 1H) ; de 3,40 à 3,55 (mt : 3H) ; 3,61 (mt : 2H) ; 3,72 (t, J = 5 Hz : 4H) ; 3,86 (mt : 1H) ; 4,06 (mt : 1H) ; 4,54 (mt : 1H) ; 4,79 (dd, J = 10 et 1,5 Hz : 1H) ; 4,82 (dd, J = 9 et 3 Hz : 1H) ; 5,12 (doublet démultiplié, J _{HF} = 48 Hz : 1H) ; 5,30 (d, J = 9 Hz : 1H) ; 5,75 (mt : 1H) ; de 5,75 à 5,85 (mt : 1H) ; 5,82 (dd, J = 17 et 1,5 Hz : 1H) ; 5,96 (mt : 1H) ; 6,19 (d, J = 16 Hz : 1H) ; 6,51 (dd, J = 17 et 5 Hz : 1H) ; 8,12 (s : 1H).

L'acide 5-[4-(2-morpholinoéthyl pipérazin-1-yl)]-5-oxo pentanoïque peut être obtenu de la façon suivante :

Dans un ballon maintenu sous atmosphère d'argon, on ajoute à 10 cm³ de dioxane, 1,5 g d'anhydride glutarique puis 2,62 g de 1-(2-morpholinoéthyl) pipérazine en solution dans 10 cm³ de dioxane. Après 60 heures d'agitation à température ambiante, le solvant est évaporé sous pression réduite. L'huile résultante est additionnée de 100 cm³ d'éther diéthylique. Après refroidissement à -40°C, le produit se concrète. L'agitation est alors poursuivie 1 heure à 20°C. Le solide obtenu est filtré, rincé à l'éther diéthylique puis séché à 20°C (90 Pa) pour donner 3,57 g d'acide 5-[4-(2-morpholinoéthyl piperazin-1-yl)]-5-oxo pentanoïque sous forme d'un solide blanc utilisé tel quel.

### Exemple 58

### Chlorhydrate de la (16R)-16-Désoxo-16-fluoro-14-O-[(3RS)-3-méthyl-4-(4-méthylpipérazin-1-yl carbonyl butyryl] pristinamycine II_{B}

En opérant comme à l'exemple 46 mais à partir de 516 mg d'acide 3-méthyl-5-(4-méthylpiperazin-1-yl)-5-oxo pentanoïque, de 1 g de (16*R*)-16-désoxo-16-fluoro pristinamycine II_{B} (préparée comme décrit dans l'exemple 1), de 467 mg de N,N'-dicyclohexylcarbodiimide, et de 54 mg de 4-diméthylaminopyridine dans 40 cm³ de dichlorométhane, on obtient 1,25 g d'un solide qui est purifié par chromatographie flash [éluant : dichlorométhane / méthanol (95 / 5 en volumes)]. On obtient, après concentration à sec des fractions sous pression réduite (2,7 kPa) à 20°C, puis agitation du solide obtenu dans l'éther diisopropylique pendant 18 heures, filtration et séchage (90 Pa) à 20°C, 730 mg de (16*R*)-16-désoxo-16-fluoro-14-O-[(3*RS*)-3-méthyl-4-(4-méthylpipérazin-1-yl carbonyl butyryl] pristinamycine II_{B}, sous forme d'un solide blanc. Celui-ci est dissous dans 3,5 cm³ d'éthanol absolu auquel on ajoute 0,34 cm³ d'éther chlorhydrique 3 M. On ajoute ensuite 35 cm³ d'éther diéthylique et la suspension obtenue est agité 2 heures à 20°C. Le solide est filtré, rincé à l'éther diéthylique, puis séché à 45°C (90 Pa) pour donner 645 mg de chlorhydrate de (16*R*)-16-désoxo-16-fluoro-14-O-[(3*RS*)-3-méthyl-4-(4-méthylpipérazin-1-yl carbonyl)butyryl] pristinamycine II_{B}, sous forme d'un solide blanc fondant à 185°C.

Spectre de R.M.N. ¹H (400 MHz, (CD₃)₂SO d6, δ en ppm) : 0,85 (d, J = 6,5 Hz : 3H) ; de 0,90 à 1,00 (mt : 6H) ; 1,03 (d, J = 6,5 Hz : 3H) ; 1,51 (mt : 1H) ; de 1,70 à 2,45 (mt : 11H) ; 1,77 (s : 3H) ; de 2,70 à 3,50 (mt : 9H) ; 2,77 (s large : 3H) ; 3,59 (mt : 1H) ; 3,68 (mt : 1H) ; 3,80 (mt : 1H) ; de 3,90 à 4,20 (mf étalé : 1H) ; 3,97 (mt : 1H) ; 4,44 (mf étalé : 1H) ; de 4,70 à 4,80 (mt : 2H) ; 5,09 (doublet démultiplié, J _{HF} = 48 Hz : 1H) ; 5,43 (d, J = 9 Hz : 1H) ; 5,61 (mt : 1H) ; 5,75 (mt : 1H) ; 5,80 (d large, J = 16 Hz : 1H) ; 6,19 (d, J = 16 Hz : 1H) ; 6,62 (dd, J = 16 et 4 Hz : 1H) ; 8,17 (t, J = 5,5 Hz : 1H) ; 8,52 (s : 1H) ; 10,49 (mf large : 1H).

L'acide (3*RS*)-3-méthyl-5-(4-méthylpiperazin-1-yl)-5-oxo pentanoïque peut être obtenu de la façon suivante :

On ajoute à 20 cm³ de dioxane, 1,6 g d'anhydride 3-méthyl glutarique puis 1,48 cm³ de N-méthylpipérazine. Après 4 heures d'agitation, on ajoute 320 mg d'anhydride 3-méthyl. glutarique supplémentaires. L'agitation est poursuivie à 20°C pendant 18 heures. Le solvant est évaporé sous pression réduite (2,7 kPa), à 50°C. L'huile résultante est séchée à 50°C (90 Pa) pour donner 3,1 g d'acide (3*RS*)-3-méthyl-5-(4-méthylpiperazin-1-yl)-5-oxo pentanoïque, sous forme d'une laque jaune utilisée telle quelle.

### Exemple 59

### (16R)-16-désoxo-16-fluoro-14-O-[(RR,SS)-trans-2-(4-méthylpipérazin-1-yl carbonyl)-1-cyclobutane carbonyl] pristinamycine II_{B} (mélange 50/50 des deux diastéréoisomères)

A 2,1 g de (16*R*)-16-désoxo-16-fluoro pristinamycine II_{B} (préparée comme décrit dans l'exemple 1) en solution dans 100 cm³ de dichlorométhane, on ajoute à 20°C, sous atmosphère d'argon, 2,0 g d'acide (RR, *SS*)-*trans*-2-(4-méthylpipérazin-1-yl-carbonyl)-1-cyclobutane carboxylique, 1,24 g de N,N'-dicyclohexylcarbodiimide et 0,49 g de 4-diméthylaminopyridine. Après 24 heures d'agitation, le mélange réactionnel est filtré pour éliminer l'insoluble. Le filtrat est concentré à sec sous pression réduite (2,7 kPa) pour donner 5,2 g d'un résidu qui est purifié par chromatographie-flash [éluant : gradient dichlorométhane / méthanol (97 / 3 puis 95 / 5 en volumes)]. On obtient, après agitationdans l'éther diisopropylique, 0,26 g de (16*R*)-16-désoxo-16-fluoro-14-O-[(*RR,SS*)-*trans*-2-(4-méthylpipérazin-1-yl carbonyl)-1-cyclobutane carbonyl] pristinamycine II_{B} (mélange 50/50 des deux diastéréoisomères), sous forme d'une poudre blanchâtre.

Spectre de R.M.N. ¹H (400 MHz, CDCl₃, δ en ppm); (2 diastéréoisomères dans les proportions 50-50) : 0,96 (d, J = 6,5 Hz : 3H) ; 1,00 (d, J = 6,5 Hz : 3H) ; 1,11 (d, J = 6,5 Hz : 3H) ; de 1,55 à 2,45 (mt : 15H) ; 1,90 (s : 3H) ; 2,29 et 2,30 (2 s : 3H en totalité) ; 2,77 (mt : 1H) ; 2,98 (dt, J = 17 et 6 Hz : 1H) ; 3,23 (mt : 1H) ; de 3,30 à 3,75 (mt : 7H) ; 3,86 (mt : 1H) ; 4,06 (mt : 1H) ; 4,55 (mt : 1H) ; de 4,75 à 4,85 (mt : 2H) ; 5,11 (doublet démultiplié, J _{HF} = 48 Hz : 1H) ; 5,30 (d, J = 9 Hz : 1H) ; de 5,70 à 5,85 (mt : 3H) ; 5,96 et 6,02 (2 mts : 1H en totalité) ; 6,20 (d, J = 16 Hz : 1H) ; 6,52 (mt : 1H) ; 8,12 (s : 1H).

L'acide (*RR,SS*)-*trans*-2-(4-méthylpipérazin-1-yl-carbonyl)-1-cyclobutane carboxylique, peut être préparé de la manière suivante :

A une solution de 1,8 g de dichlorure de l'acide (RR, *SS*)-*trans*-1,2-cyclobutanedicarboxylique, dans 50 cm³ de dichlorométhane, on ajoute, goutte à goutte, à 25°C, sous atmosphère d'argon, 1,1 cm³ de 1-méthylpipérazine. Après 4 heures d'agitation, on ajoute 10 cm³ d'eau distillée. Après 1 heure d'agitation, le mélange réactionnel est concentré à sec sous pression réduite (2,7 kPa) pour donner un résidu qui est repris par 20 cm' d'eau distillée. Cette solution est amenée à pH 10 par addition d'une solution aqueuse d'hydroxyde de sodium 1 N. La solution obtenue est extraite par 50 cm³ d'acétate d'éthyle, amenée à pH 5-6 par addition d'une solution aqueuse d'acide chlorhydrique 1 N puis concentrée à sec, sous pression réduite (2,7 kPa). Par deux fois le résidu est recouvert par 50 cm' de toluène et ramené à sec sous pression réduite (2,7 kPa). Le résidu est repris par 50 cm³ d'éthanol à 50°C, l'insoluble est éliminé par filtration et le filtrat est concentré à sec sous pression réduite (2,7 kPa). On obtient ainsi 2,4 g d'acide (*RR, SS*)-*trans*-2-(4-méthylpipérazin-1-yl carbonyl)-1-cyclobutane carboxylique, sous forme d'une huile jaune.

Le dichlorure de l'acide (*RR,SS*)-*trans*-1,2-cyclobutanedicarboxylique peut être préparé de la manière suivante :

A 1,5 g d'acide (*RR,SS*)-*trans*-1,2-cyclobutanedicarboxylique, on ajoute, à 20°C, 2,2 cm³ de chlorure de sulfonyle, puis on chauffe à reflux pendant 2 heures. Le mélange réactionnel est ensuite concentré sous pression réduite (2,7 kPa). Par deux fois le résidu est recouvert par 50 cm³ de dichlorométhane et ramené à sec sous pression réduite (2,7 kPa). On obtient ainsi 1,8 g de dichlorure de l'acide (*RR,SS*)-*trans*-1,2-cyclobutanedicarboxylique, sous forme d'une huile jaune.

### Exemple 60

### Méthanesulfonate de la (16R)-16-Désoxo-16-fluoro-14-O-(1-méthylpipéridin-4-yl-carbonyl) pristinamycine II_{A}

A 1,06 g de (16*R*)-16-désoxo-16-fluoro pristinamycine II_{A} (préparée comme décrit dans l'exemple 8), 0,23 g d'acide 1-méthyl-4-pipéridinecarboxylique, 0,123 g de 4-diméthylaminopyridine en solution dans 40 cm³ de dichlorométhane, on ajoute à 20°C, 0,516 g de N,N'-dicyclohexylcarbodiimide. Après 24 heures d'agitation, le mélange réactionnel est lavé par 50 cm³ d'eau, séché sur sulfate de magnésium, filtré, puis concentré à sec sous pression réduite (2,7 kPa) pour donner un résidu qui est purifié par chromatographie flash [éluant : dichlorométhane / méthanol (95 / 5 en volumes)]. On obtient un produit qui est repris par 35 cm³ d'éthanol et 13,4 cm³ d'une solution aqueuse d'acide méthanesulfonique 0,1 N puis concentré à sec sous pression réduite (2,7 kPa), agitédans de l'éther diéthylique, filtré, et concentré pour donner un résidu qui est agité dans 25 cm³ de dichlorométhane et 10 cm³ d'une solution aqueuse à 5% de bicarbonate de sodium. La phase organique est alors séparée puis concentrée à sec sous pression réduite (2,7 kPa) pour donner un résidu qui est repris par 20 cm³ d'éthanol et 10 cm³ d'une solution aqueuse d'acide méthanesulfonique 0,1 N. La solution est concentrée à sec sous pression réduite (2,7 kPa) à 20°C, pour donner 0,65 g du méthanesulfonate de la (16*R*)-16-désoxo-16-fluoro-14-O-(1-méthylpipéridin-4-yl-carbonyl) pristinamycine II_{A}, sous forme d'un solide crème.

Spectre de R.M.N. ¹H (400 MHz, (CD₃)₂SO d6 à une température de 383 K, δ en ppm) : 0,93 (d, J = 6,5 Hz : 3H) ; 1,00 (d, J = 6,5 Hz : 3H) ; 1,13 (d, J = 6,5 Hz : 3H) ; de 1,75 à 2,30 (mt : 7H) ; 1,84 (s : 3H) ; 2,40 (s : 3H) ; de 2,55 à 2,85 (mt : 4H) ; 2,81 (s : 3H) ; de 2,90 à 3,55 (mt : 6H) ; 3,79 (d large, J = 16,5 Hz : 1H) ; 3,94 (dt, J = 16,5 et 6,5 Hz : 1H) ; de 4,05 à 4,25 (mt : 2H) ; 4,81 (dd, J = 9 et 3 Hz : 1H) ; 4,99 (doublet démultiplié, J _{HF} = 48 Hz : 1H) ; 5,33 (d, J = 9,5 Hz : 1H) ; 5,75 (dt, J = 9,5 et 5 Hz : 1H) ; 5,81 (mt : 1H) ; 5,92 (dd, J = 16 et 1,5 Hz : 1H) ; 6,10 (d, J = 16 Hz : 1H) ; 6,28 (t, J = 3 Hz : 1H) ; 6,63 (dd, J = 16 et 6,5 Hz : 1H) ; 7,43 (mf : 1H) ; 8,45 (s : 1H) ; de 8,85 à 9,45 (mf très étalé : 1H).

### L'acide 1-méthyl-4-pipéridinecarboxylique peut être préparé de la manière suivante :

A 7,60 g de 1-méthyl-4-pipéridinecarboxylate d'éthyle en solution dans 35 cm³ d'éthanol, on ajoute à 20°C, 12,5 cm³ d'une solution aqueuse d'hydroxyde de sodium 4 N. Après 20 heures d'agitation, le mélange réactionnel est concentré à volume réduit puis neutralisé par 12,5 cm³ d'une solution aqueuse d'acide chlorhydrique 4 N et enfin concentré à sec sous pression réduite (2,7 kPa). Le résidu est agitédans 60 cm³ d'éthanol anhydre, puis filtré. Le filtrat est concentré à sec sous pression réduite (2,7 Kpa) à 20°C, pour donner 6,3 g d'acide 1-méthyl-4-pipéridinecarboxylique, sous forme d'un solide blanc.

Spectre de R.M.N. ¹H (300 MHz, (CD₃)₂SO d6, δ en ppm) : 1,56 (mt : 2H) ; 1,78 (mt : 2H) ; 1,98 (dt, J = 11,5 et 2,5 Hz : 2H) ; 2,13 (mt : 1H) ; 2,18 (s : 3H) ; 2,74 (d large, J = 11,5 Hz : 2H).

Le 1-méthyl-4-pipéridinecarboxylate d'éthyle peut être préparé de la manière suivante :

A 9,2 g de chlorhydrate de 4-pipéridinecarboxylate d'éthyle en solution dans 80 cm³ de dichlorométhane, on ajoute à 20°C, 12,5 cm³ d'une solution aqueuse d'hydroxyde de sodium 4 N. Après 5 minutes d'agitation, la phase organique est séchée sur sulfate de magnésium, filtrée puis refroidie à 5°C. On ajoute alors sous argon 7,0 cm³ de formaldéhyde et 14,2 g (introduits en deux fois) de triacétoxyborohydrure de sodium. Après 1 heure d'agitation vigoureuse à 20°C, le mélange réactionnel est dilué par 50 cm³ d'eau, alcalinisé avec une solution aqueuse d'hydroxyde de sodium 4 N puis décanté. La phase organique est séchée sur sulfate de magnésium, filtrée et concentrée à sec sous pression réduite (2,7 kPa) à 20°C, pour donner 7,6 g de 1-méthyl-4-pipéridinecarboxylate d'éthyle, sous forme d'une huile incolore.

Spectre de R.M.N. ¹H (250 MHz, CDCl₃, δ en ppm) : 1,24 (t, J = 7 Hz : 3H) ; de 1,65 à 2,05 (mt : 6H) ; de 2,15 à 2,30 (mt : 1H) ; 2,25 (s : 3H) ; 2,81 (d large, J = 11,5 Hz : 2H) ; 4,13 (q, J = 7 Hz : 2H).

### Exemple 61

### (16R)-16-Désoxo-16-fluoro-14-O-[1-(diméthylaminoacétyl)pipéridine-4-carbonyl] pristinamycine II_{B}

A 1,1 g de (16*R*)-16-désoxo-16-fluoro pristinamycine II_{B} (préparée comme décrit dans l'exemple 1) en solution dans 50 cm³ de dichlorométhane, on ajoute, à 20°C, sous atmosphère d'argon, 0,51 g d'acide 1-(diméthylaminoacétyl)-pipéridine-4-carboxylique, 0,5 g de N,N'-dicyclohexylcarbodiimide et 0,15 g de 4-diméthylaminopyridine. Après 48 heures d'agitation, on ajoute 0,5 g de N,N'-dicyclohexylcarbodiimide et 0,1 g de 4-diméthylaminopyridine supplémentaires. Après 48 heures d'agitation, le mélange réactionnel est filtré pour éliminer l'insoluble. Le filtrat est concentré à sec, sous pression réduite (2,7 kPa), pour donner 1,8 g d'un résidu qui est purifié par chromatographie-flash [éluant : gradient dichlorométhane / méthanol (97 / 3 puis 95 / 5 en volumes)]. On obtient, après agitation dans l'éther diéthylique, 0,38 g de (16*R*)-16-désoxo-16-fluoro-14-O-[1-(diméthylaminoacétyl)pipéridine-4-carbonyl] pristinamycine II_{B} sous forme d'un solide blanc.

Spectre de R.M.N, ¹H (400 MHz, CDCl₃, δ en ppm) : 0,96 (d, J = 6,5 Hz : 3H) ; 1,00 (d, J = 6,5 Hz : 3H) ; 1,10 (d, J = 6,5 Hz : 3H) ; de 1,50 à 2,05 (mt : 9H) ; 1,89 (s : 3H) ; de 2,10 à 2,35 (mt : 2H) ; 2,28 (s : 6H) ; 2,52 (mt : 1H) ; 2,77 (mt : 1H) ; 2,80 (mt : 1H) ; 3,00 (dt, J = 17 et 6 Hz : 1H) ; de 3,05 à 3,20 (mt : 1H) ; 3,10 (AB, J = 14 Hz : 2H) ; 3,25 (mt : 1H) ; 3,50 (mt : 1H) ; 3,85 (mt : 1H) ; de 4,00 à 4,10 (mt : 2H) ; 4,38 (mt : 1H) ; 4,53 (mt : 1H) ; 4,78 (d large, J = 10 Hz : 1H) ; 4,82 (dd, J = 9 et 3 Hz : 1H) ; 5,10 (doublet démultiplié, J _{HF} = 48 Hz : 1H) ; 5,29 (d, J = 9 Hz : 1H) ; de 5,70 à 5,90 (mt : 3H) ; 5,93 (mt : 1H) ; 6,19 (d, J = 16 Hz : 1H) ; 6,52 (dd, J = 16 et 4 Hz : 1 H) ; 8,12 (s : 1H).

L'acide 1-(diméthylaminoacétyl)pipéridine-4-carboxylique peut être préparé de la manière suivante :

A 4,5 g de 1-(diméthylaminoacétyl)pipéridine-4-carboxylate d'éthyle en solution dans 50 cm³ d'éthanol, on ajoute à 20°C, 25 cm³ de solution aqueuse d'hydroxyde de sodium 1 N. Après 16 heures d'agitation à 50°C, l'éthanol est éliminé sous pression réduite (2,7 kPa) et la phase aqueuse résiduelle est extraite par 50 cm³ d'acétate d'éthyle. La phase aqueuse est alors amenée à pH 6 par addition d'acide chlorhydrique 1 N, puis est concentrée à sec sous pression réduite (2,7 kPa). Par deux fois le résidu est recouvert par 50 cm³ de toluène et ramené à sec sous pression réduite (2,7 kPa). Le résidu est repris par 50 cm³ d'éthanol à 50°C et l'insoluble est éliminé par filtration. Le filtrat est concentré sous pression réduite (2,7 kPa) pour donner 4,2 g d'acide 1-(diméthylaminoacétyl)pipéridine-4-carboxylique sous forme d'une huile jaune.

Spectre de R.M.N. ¹H (300 MHz, CDCl₃, δ en ppm) : de 1,50 à 1,80 (mt : 2H) ; 1,93 (mt : 2H) ; de 2,40 à 2,60 (mt : 1H) ; 2,51 (s : 6H) ; 2,78 (mt : 1H) ; 3,07 (mt : 1H) ; 3,25 (d, J = 14,5 Hz : 1H) ; 3,55 (d, J = 14,5 Hz : 1H) ; 3,84 (d large, J = 14 Hz : 1H) ; 4,39 (d large, J = 14 Hz : 1H).

Le 1-(diméthylaminoacétyl)pipéridine-4-carboxylate d'éthyle peut être préparé de la façon suivante :

A une solution de 2,3 g de N,N-diméthylglycine, 3,2 cm³ de pipéridine-4-carboxylate d'éthyle, 3,4 cm³ de triéthylamine et de 0,27 g d'hydrate d'hydroxybenzotriazole, dans 100 cm³ de dichlorométhane, on ajoute, à 20°C, sous atmosphère d'argon, 4,6 g de chlorhydrate de N-(3-diméthylaminopropyl)-N'-éthylcarbodiimide. Après 18 heures d'agitation, le mélange réactionnel est lavé avec deux fois 100 cm³ d'eau distillée. La phase organique est séchée sur sulfate de magnésium, filtrée puis concentrée à sec sous pression réduite (2,7 kPa). On obtient 4,6 g de 1-(diméthylaminoacétyl)pipéridine-4-carboxylate d'éthyle sous forme d'une huile jaune.

Spectre de R.M.N. ¹H (250 MHz, (CD₃)₂SO d6 avec ajout de quelques gouttes de CD₃COOD d4, δ en ppm) : 1,22 (t, J = 7 Hz : 3H) ; 1,55 (mt : 2H) ; 1,88 (mt : 2H) ; 2,37 (s : 6H) ; 2,60 (mt : 1H) ; de 2,90 à 3,15 (mt : 2H) ; 3,34 (s : 2H) ; de 3,90 à 4,15 (mt : 2H) ; 4,11 (q, J = 7 Hz: 2H).

### Exemple 62

### (16R)-16-Désoxo-16-fluoro-14-O-[1-(imidazol-1-yl acétyl)pipéridine-4-carbonyl] pristinamycine II_{B}

A 0,8 g de (16*R*)-16-désoxo-16-fluoro-14-O-(1-chloroacétyl pipéridine-4-carbonyl) pristinamycine II_{B} en solution dans 20 cm³ de diméthylformamide, on ajoute à 20°C, sous atmosphère d'argon, 0,15 g d'imidazole et 0,18 g d'iodure de potassium. Après 16 heures d'agitation, on ajoute 100 cm³ de dichlorométhane. Le mélange résultant est lavé par deux fois 50 cm³ de solution aqueuse d'hydroxyde de sodium 0,1 N. La phase organique est décantée, séchée sur du sulfate de magnésium, filtrée puis concentrée à sec sous pression réduite (2,7 kPa) pour donner 0,9 g d'un résidu qui est purifié par chromatographie-flash [éluant : dichlorométhane / méthanol (95 / 5 en volumes)]. On obtient, après agitation dans l'éther d'isopropylique, filtration et séchage (2,7 kPa) à 20°C, 0,38 g de (16*R*)-16-désoxo-16-fluoro-14-O-[1-(imidazol-1-yl acétyl)pipéridine-4-carbonyl] pristinamycine II_{B}, sous forme d'une poudre blanche.

Spectre de R.M.N. ¹H (400 MHz, (CD₃)₂SO d6, δ en ppm) : 0,86 (d, J = 6,5 Hz : 3H) ; 0,95 (d, J = 6,5 Hz : 3H) ; 1,04 (d, J = 6,5 Hz : 3H) ; de 1,35 à 2,30 (mt : 11H) ; 1,78 (s : 3H) ; 2,67 (mt : 1H) ; de 2,75 à 2,85 (mt : 2H) ; de 3,10 à 3,35 (mt : 3H) ; 3,61 (d large, J = 15 Hz : 1H) ; 3,70 (mt : 1H) ; 3,81 (mt : 2H) ; 3,97 (mt : 1H) ; 4,18 (mt : 1H) ; de 4,70 à 4,80 (mt : 2H) ; de 4,95 à 5,20 (mt : 1H) ; 5,00 (AB limite : 2H) ; 5,43 (d, J = 9 Hz : 1H) ; 5,62 (mt : 1H) ; de 5,70 à 5,80 (mt : 1H) ; 5,80 (dd, J = 16 et 2 Hz : 1H) ; 6,19 (d, J = 16 Hz : 1H) ; 6,63 (dd, J = 16 et 4 Hz : 1H) ; 6,86 (s : 1H) ; 7,05 (s : 1H) ; 7,52 (s :1H) ; 8,13 (mt : 1H) ; 8,52 (s : 1H).

La (16*R*)-16-désoxo-16-fluoro-14-O-(1-chloroacétyl pipéridine-4-carbonyl) pristinamycine II_{B} peut être préparée de la manière suivante :

A 1,1 g de (16*R*)-16-désoxo-16-fluoro pristinamycine II_{B} (préparée comme décrit dans l'exemple 1) en solution dans 50 cm³ de dichlorométhane, on ajoute à 20°C, sous atmosphère d'argon, 0,62 g d'acide 1-chloroacétyl pipéridine-4-carboxylique, 0,62 g de N,N'-dicyclohexylcarbodiimide et 0,24 g de 4-diméthylaminopyridine. Après 16 heures d'agitation à 20°C, le mélange réactionnel est filtré pour éliminer l'insoluble. Le filtrat est concentré à sec sous pression réduite (2,7 kPa) pour donner 1,8 g d'un résidu qui est purifié par chromatographie-flash [éluant : dichlorométhane / méthanol (97 / 3 en volumes)]. On obtient ainsi 0,25 g de (16*R*)-16-désoxo-16-fluoro-14-O-(1-chloroacétyl pipéridine-4-carbonyl) pristinamycine II_{B} sous forme d'un solide blanc.

Spectre de R.M.N. ¹H (400 MHz, CDCl₃, δ en ppm) : 0,96 (d, J = 6,5 Hz : 3H) ; 1,01 (d, J = 6,5 Hz : 3H) ; 1,10 (d, J = 6,5 Hz : 3H) ; de 1,70 à 2,05 (mt : 9H) ; 1,89 (s : 3H) ; de 2,10 à 2,30 (mt : 2H) ; 2,55 (mt : 1H) ; 2,77 (mt : 1H) ; de 2,90 à 3,05 (mt : 2H) ; de 3,15 à 3,35 (mt : 2H) ; 3,51 (mt : 1H) ; de 3,75 à 3,90 (mt : 2H) ; de 4,00 à 4,15 (mt : 1H) ; 4,07 (s : 2H) ; 4,31 (mt : 1H) ; 4,52 (mt : 1H) ; 4,78 (d large, J = 10 Hz : 1H) ; 4,82 (dd, J = 9 et 3 Hz : 1H) ; 5,10 (doublet démultiplié, J _{HF} = 48 Hz : 1H) ; 5,29 (d, J = 9 Hz : 1H) ; de 5,75 à 5,90 (mt : 3H) ; 5,92 (mt : 1H) ; 6,19 (d, J = 16 Hz : 1H) ; 6,52 (dd, J = 17 et 5 Hz : 1H) ; 8,12 (s : 1H).

L'acide 1-chloroacétyl pipéridine-4-carboxylique peut être préparé de la manière suivante :

A 2,3 g de 1-chloroacétyl pipéridine-4-carboxylate d'éthyle en solution dans 25 cm³ d'éthanol, on ajoute à 20°C sous atmosphère d'argon, 10 cm³ de solution aqueuse d'hydroxyde de sodium 1 N. Après 16 heures d'agitation, l'éthanol est éliminé sous pression réduite (2,7 kPa). On ajoute alors au résidu 30 cm³ d'eau distillée et de l'acide chlorhydrique 1 N jusqu'à pH 4. La solution obtenue est concentrée à sec, sous pression réduite (2,7 kPa). Par deux fois le résidu est recouvert de toluène et ramené à sec sous pression réduite (2,7 kPa). On obtient ainsi 3 g d'acide 1-chloroacétyl pipéridine-4-carboxylique sous forme d'une pâte blanche.

Spectre de R.M.N. ¹H (250 MHz, (CD₃)₂SO d6, δ en ppm) : de 1,25 à 1,65 (mt : 2H) ; de 1,70 à 1,95 (mt : 2H) ; 2,40 (mt : 1H) ; 2,79 (mt : 1H) ; 3,11 (mt : 1H) ; 3,75 (d large, J = 14 Hz : 1H) ; de 4,05 à 4,25 (mt : 1H) ; 4,36 (AB limite, J = 13 Hz : 2H).

Le 1-chloroacétyl pipéridine-4-carboxylate d'éthyle peut être préparé de la manière suivante :

A 15,7 g de pipéridine-4-carboxylate d'éthyle en solution dans 200 cm³ de dichlorométhane et 14 cm³ de triéthylamine, on ajoute, goutte à goutte en 10 minutes, à 0°C et sous atmosphère d'argon, 8 cm³ de chlorure de chloroacétyle. Après 18 heures d'agitation à 20°C, le mélange réactionnel est successivement lavé par 100 cm³ d'eau distillée, 100 cm³ d'une solution aqueuse saturée d'hydrogénocarbonate de sodium et 100 cm³ d'une solution aqueuse saturée de chlorure de sodium. La phase organique est séchée sur du sulfate de magnésium, filtrée puis concentrée sous pression réduite (2,7 kPa) pour donner 22 g de 1-chloroacétyl pipéridine-4-carboxylate d'éthyle sous forme d'une huile jaune.

Spectre de R.M.N. ¹H (400 MHz, CDCl₃, δ en ppm) : 1,29 (t, J = 7 Hz : 3 H) ; de 1,60 à 1,85 (mt : 2H) ; 1,95 (mt : 2H) ; 2,54 (mt : 1H) ; 2,89 (mt : 1H) ; 3,19 (mt : 1H) ; 3,81 (d large, J = 14 Hz : I H) ; 4,05 (AB limite, J = 12 Hz : 2H) ; 4,13 (q, J = 7 Hz : 2H) ; 4,31 (d large, J = 14 Hz : 1H).

### Exemple 63

### (16R)-16-Désoxo-16-fluoro-14-O-[1-(4-morpholin-4-yl-butyryl)pipéridine-4-carbonyl] pristinamycine II_{B} :

On opère d'une manière analogue à celle décrite dans l'exemple 60 mais à partir de 1,80 g de (16*R*)-16-désoxo-16-fluoro pristinamycine II_{B} (préparée comme décrit dans l'exemple 1), 1,20 g d'acide 1-(4-morpholin-4-yl-butyryl)-pipéridine-4-carboxylique, 0,24 g de 4-diméthylaminopyridine, 30 cm³ de diméthylformamide, et 0,82 g de N,N'-dicyclohexylcarbodiimide. Après traitement, le produit brut est purifié par chromatographie flash [éluant : dichlorométhane / méthanol (97 / 3 en volumes)] puis par deux chromatographies sur colonne d'alumine CBT1 [éluant : respectivement acétate d'éthyle / méthanol (98 / 2 en volumes) et acétonitrile / éther diisopropylique (50 / 50 en volumes)] pour donner 0,12 g de (16*R*)-16-désoxo-16-fluoro-14-O-[1-(4-morpholin-4-yl-butyryl)pipéridine-4-carbonyl]pristinamycine II_{B}, sous la forme d'un solide blanc.

Spectre de R.M.N. ¹H (500 MHz, (CD₃)₂SO d6, δ en ppm). Nous observons la présence de rotamères dans les proportions 60-40 : 0,87 (mt : 3H) ; 0,95 (d, J = 6,5 Hz : 3H) ; 1,04 (mt : 3H) ; de 1,20 à 2,30 (mt : 13H) ; 1,70 et 1,78 (2 s : 3H en totalité) ; 2,27 (t, J = 7,5 Hz : 2H) ; 2,33 (mt : 6H) ; 2,62 (mt : 1H) ; de 2,70 à 2,80 (mt : 2H) ; 3,10 (mt : 1H) ; 3,24 (mt : 1H) ; de 3,50 à 4,00 (mt : 6H) ; 3,57 (mt : 4H) ; 4,22 (mt : 1H) ; de 4,70 à 4,90 (mt : 2H) ; 5,09 et de 5,40 à 5,55 (respectivement doublet démultiplié, J _{HF} = 48 Hz et mt : 1 H en totalité) ; de 5,40 à 5,70 (mt : 2H) ; de 5,70 à 5,85 (mt : 2H) ; 6,19 et 6,24 (2 d, J = 16 Hz : 1 H en totalité) ; 6,63 (dd, J = 16 et 4,5 Hz : 1 H) ; 8,12 et 8,22 (respectivement mt et t, J = 6 Hz : 1H en totalité) ; 8,47 et 8,51 (2 s : 1 H en totalité).

L'acide 1-(4-morpholin-4-yl-butyryl)pipéridine-4-carboxylique peut être préparé de la manière suivante :

A 1,30 g de 1-(4-morpholin-4-yl-butyryl)pipéridine-4-carboxylate d'éthyle en solution dans 20 cm³ d'éthanol, on ajoute 6,0 cm³ d'une solution aqueuse d'hydroxyde de sodium 1 N. Après 1,5 heure d'agitation à 50°C, le mélange réactionnel est concentré presque à sec, dilué dans 20 cm³ d'eau, et le pH ajusté à 5 par addition d'une solution aqueuse d'acide chlorhydrique 1 N. Après concentration à sec sous pression réduite (2,7 kPa), on obtient 1,6 g d'acide 1-(4-morpholin-4-yl-butyryl)-4-pipéridinecarboxylique, sous la forme d'une pâte blanchâtre.

Spectre de R.M.N. ¹H (400 MHz, (CD₃)₂SO d6, δ en ppm) : 1,33 (mt : 1H) ; 1,46 (mt : 1H) ; 1,64 (mt : 2H) ; 1,80 (mt : 2H) ; de 2,20 à 2,40 (mt : 8H) ; 2,43 (mt : 1H) ; 2,69 (mt : 1H) ; 3,05 (mt : 1H) ; 3,55 (t, J = 4,5 Hz : 4H) ; 3,79 (d large, J = 13,5 Hz : 1H) ; 4,20 (d large, J = 13,5 Hz : 1H).

Le 1-(4-morpholin-4-yl-butyryl)pipéridine-4-carboxylate d'éthyle peut être préparé de la manière suivante :

A 1,0 g d'acide 4-morpholinobutyrique, 0,78 g d'isonipécotate d'éthyle, 2,1 cm³ de triéthylamine, et de 20 mg d'hydrate d'hydroxybenzotriazole, en solution dans 80 cm³ de dichlorométhane, on ajoute à 20°C, 0,95 g de chlorhydrate de N-(3-diméthylaminopropyl)-N'-éthylcarbodiimide en solution dans 20 cm³ de dichlorométhane. Après 20 heures d'agitation à 20°C, le mélange réactionnel est lavé avec 20 cm³ d'eau. La phase organique est séchée sur sulfate de magnésium, filtrée, et concentrée à sec sous pression réduite (2,7 kPa) pour donner une huile jaune pâle qui est purifiée par chromatographie sur une colonne d'alumine CBT1 [éluant : dichlorométhane / méthanol (97 / 3 en volumes)]. On obtient ainsi 1,30 g de 1-(4-morpholin-4-yl-butyryl)-4-pipéridinecarboxylate d'éthyle, sous la forme d'une huile jaune pâle.

Spectre de R.M.N. ¹H (300 MHz, (CD₃)₂SO d6, δ en ppm) : 1,20 (t, J = 7 Hz : 3H) ; de 1,25 à 1,60 (mt : 2H) ; 1,65 (mt : 2H) ; 1,84 (mt : 2H) ; de 2,20 à 2,40 (mt : 8H) ; 2,59 (mt : 1H) ; 2,70 (mt : 1H) ; 3,08 (mt : 1H) ; 3,57 (t, J = 5 Hz : 4H) ; 3,82 (d large, J = 14 Hz : 1H) ; 4,08 (q, J = 7 Hz : 2H) ; 4,24 (d large, J = 14 Hz : 1H).

L'acide 4-morpholinobutyrique peut être préparé selon P. A. Cruickshank, et coll., J. Amer. Chem. Soc., 83, 2891 (1961).

### Exemple 64

### (16R)-16-Désoxo-16-fluoro-14-O-[cis-4-(4-morpholino-butyrylamino)-1-cyclohexanecarbonyl] pristinamycine II_{B}:

On opère d'une manière analogue à celle décrite dans l'exemple 60 mais à partir de 3,2 g de (16R)-16-désoxo-16-fluoro pristinamycine II_{B} (préparée comme décrit dans l'exemple 1), 2,0 g d'acide *cis*-4-(4-morpholino-butyrylamino)-1-cyclohexanecarboxylique, 0,40 g de 4-diméthylaminopyridine, 75 cm³ de diméthylformamide, et 1,50 g de N,N'-dicyclohexylcarbodiimide. Après traitement, le produit brut est purifié par chromatographie sur colonne d'alumine CBT1 [éluant : gradient dichlorométhane / méthanol (100 / 0 puis 98 / 2 en volumes)], puis par chromatographie flash [éluant : gradient dichlorométhane / méthanol (100/0 ; 98 / 2 ; 95 / 5 puis 90 / 10 en volumes)] et à nouveau par chromatographie sur alumine CBT1 [éluant : acétate d'éthyle / méthanol (99 / 1 en volumes)]. On obtient ainsi 0,40 g de (16*R*)-16-désoxo-16-fluoro-14-O-[*cis*-4-(4-morpholino-butyrylamino)-1-cyclohexanecarbonyl] pristinamycine II_{B}, sous la forme d'un solide blanc.

Spectre de R.M.N. ¹H (500 MHz, (CD₃)₂SO d6, δ en ppm) : 0,86 (d, J = 6,5 Hz : 3H) ; 0,94 (d, J = 6,5 Hz : 3H) ; 1,04 (d, J = 6,5 Hz : 3H) ; de 1,35 à 1,70 (mt : 8H) ; de 1,75 à 2,25 (mt : 9H) ; 1,78 (s : 3H) ; 2,08 (t, J = 7,5 Hz : 2H) ; 2,22 (t, J = 7,5 Hz : 2H) ; 2,31 (mf : 4H) ; 2,48 (mt : 1H) ; 2,77 (mt : 1H) ; de 3,15 à 3,30 (mt : 2H) ; 3,56 (t, J = 5 Hz : 4H) ; 3,62 (d large, J = 15 Hz : 1H) ; de 3,65 à 3,75 (mt : 2H) ; 3,82 (mt : 1H) ; 3,95 (mt : 1H) ; de 4,70 à 4,80 (mt : 2H) ; 5,09 (doublet démultiplié, J _{HF} = 48 Hz : 1H) ; 5,42 (d, J = 9,5 Hz : 1H) ; 5,62 (mt : 1H) ; 5,75 (mt : 1H) ; 5,80 (d large, J = 16 Hz : 1H) ; 6,19 (d, J = 16 Hz : 1H) ; 6,62 (dd, J = 16 et 5 Hz : 1H) ; 7,65 (d, J = 7,5 Hz : 1H) ; 8,12 (t, J = 6 Hz : 1H) ; 8,51 (s : 1H).

L'acide *cis*-4-(4-morpholinobutyrylamino)-1-cyclohexanecarboxylique peut être préparé de la façon suivante :

A 1,30 g de *cis*-4-(4-morpholino-butyrylamino)-1-cyclohexanecarboxylate de méthyle en solution dans 20 cm³ d'éthanol, on ajoute 6,0 cm³ d'une solution aqueuse d'hydroxyde de sodium 1 N. Après 1,5 heure d'agitation à 50°C, le mélange réactionnel est concentré presque à sec, dilué ensuite dans 20 cm³ d'eau et le pH ajusté à 5 par addition d'une solution aqueuse d'acide chlorhydrique 1 N. Après concentration à sec sous pression réduite (2,7 kPa), on obtient 1,5 g d'acide *cis*-4-(4-morpholino-butyrylamino)-1-cyclohexanecarboxylique, sous la forme d'une pâte blanchâtre, utilisée telle quelle à l'étape suivante.

Spectre de R.M.N. ¹H (400 MHz, CDCl₃, δ en ppm) : de 1,50 à 1,75 (mt : 6H) ; de 1,85 à 2,00 (mt : 4H) ; 2,25 (t, J = 7,5 Hz : 2H) ; 2,44 (mt : 1H) ; 2,61 (t, J = 7,5 Hz : 2H) ; 2,72 (mf : 4H) ; 3,80 (mt : 4H) ; 3,96 (mt : 1H) ; 6,72 (d, J = 8 Hz : 1H).

Le *cis*-4-(4-morpholino-butyrylamino)-1-cyclohexanecarboxylate de méthyle peut être préparé de la façon suivante :

On opère d'une manière analogue à celle décrite dans l'exemple 63, mais à partir de 4,20 g de chlorhydrate de *cis*-4-aminocyclohexane-1-carboxylate de méthyle, 4,0 g d'acide 4-morpholinobutyrique, 50 mg d'hydrate d'hydroxybenzotriazole, 11,2 cm³ de triéthylamine, 200 cm³ de dichlorométhane, et de 4,2 g de chlorhydrate de N-(3-diméthylaminopropyl)-N'-éthylcarbodiimide. Après traitement analogue à celui de l'exemple 60, le produit brut est purifié par chromatographie flash [éluant : dichlorométhane / méthanol (95 / 5 en volumes)] pour donner 5,0 g de *cis*-4-(4-morpholinobutyrylamino)-1-cyclohexanecarboxylate de méthyle, sous la forme d'une huile jaune.

Spectre de R.M.N. ¹H (400 MHz, (CD₃)₂SO d6, δ en ppm) : de 1,40 à 2,00 (mt : 10H) ; 2,10 (t, J = 7,5 Hz : 2H) ; 2,25 (t, J = 7,5 Hz : 2H) ; 2,34 (mf : 4H) ; de 2,45 à 2,60 (mt : 1H) ; 3,59 (t, J = 5 Hz : 4H) ; 3,65 (s : 3H) ; 3,72 (mt : 1H) ; 7,68 (d, J = 7 Hz : 1H).

Le chlorhydrate de *cis*-4-aminocyclohexane-1-carboxylate de méthyle peut être préparé de la manière suivante :

A 50 cm³ de méthanol refroidi à -10°C, on ajoute goutte à goutte 5 cm³ de chlorure de sulfonyle. Après 10 minutes d'agitation à 20°C, on ajoute lentement par fractions 5,0 g d'acide cis-4-aminocyclohexane-1-carboxylique. Après 2 heures d'agitation à 20°C, le mélange réactionnel est concentré à sec sous pression réduite (2,7 kPa) à 20°C, pour donner 7,5 g de chlorhydrate de cis-4-aminocyclohexane-1-carboxylate de méthyle, sous la forme d'une poudre blanche.

Spectre de R.M.N. ¹H (500 MHz, (CD₃)₂SO d6 à une température de 373 K, δ en ppm). Nous observons un mélange de diastéréoisomères dans les proportions 80-20 : 1,46 et de 1,60 à 1,70 (2 mts : 4H en totalité) ; 1,85 et de 1,95 à 2,15 (2 mts : 4H en totalité) ; 2,30 et 2,60 (2 mts : 1H en totalité) ; 3,00 et 3,13 (2 mts : 1H en totalité) ; 3,64 et 3,67 (2 s : 3H en totalité) ; 8,12 (mf : 3H).

### Exemple 65

### (16R)-16-Désoxo-16-fluoro-14-O-[N-(4-méthylpipérazin-1-yl carbonyl) glycinyl] pristinamycine II_{B}

A 3 g de (16*R*)-16-désoxo-16-fluoro pristinamycine II_{B} (préparée comme décrit dans l'exemple 1) en solution dans 100 cm³ de dichlorométhane, on ajoute à 20°C, 1,21 g de N,N'-dicyclohexylcarbodiimide, 1,07 g de N-(4-méthylpipérazin-1-yl carbonyl) glycine, et 0,13 g de 4-diméthylaminopyridine. Après 24 heures d'agitation à 20°C, on ajoute 0,6 g de N,N'-dicyclohexylcarbodiimide et 0,065 g de 4-diméthylaminopyridine. Après 24 heures d'agitation supplémentaires à 20°C, le mélange réactionnel est filtré et l'insoluble est lavé par 70 cm³ de dichlorométhane. Après deux autres cycles de lavage/filtration, le filtrat est concentré à sec sous pression réduite (2,7 kPa). Le résidu est dissout dans 50 cm³ d'éthanol et le pH est ajusté à 2 par addition d'acide méthanesulfonique. Après concentration sous pression réduite (2,7 kPa), le résidu est repris dans 50 cm³ d'eau et extrait avec 2 fois 50 cm³ d'acétate d'éthyle. La phase aqueuse est décantée, ajusté à pH 8 / 9 par addition d'une solution aqueuse d'hydroxyde de sodium 1 N puis extraite par 3 fois 60 cm³ de dichlorométhane. Les phases organiques sont réunies, lavées par 50 cm³ d'eau, séchées sur sulfate de magnésium, filtrées puis concentrées à sec sous pression réduite (2,7 kPa) pour donner 2 g d'une huile rouge qui est purifiée par chromatographie-flash [éluant : gradient dichlorométhane / méthanol / acétonitrile (90 / 5 / 5 à 80 / 10 / 10 en volumes)]. On obtient ainsi, 0,222 g d'un solide jaune qui est agitépendant 2 heures dans 20 cm³ d'éther diéthylique pour donner, après filtration, lavage par 2 fois 10 cm³ d'éther diéthylique et séchage sous pression réduite (2,7 kPa), 0,181 g de (16*R*)-16-désoxo-16-fluoro-14-O-[N-(4-méthylpipérazin-1-yl carbonyl) glycinyl] pristinamycine II_{B}, sous forme d'un solide blanc fondant vers 140°C (déc.).

Spectre de R.M.N. ¹H (400 MHz, CDCl₃, δ en ppm) : 0,96 (d, J = 6,5 Hz : 3H) ; 1,00 (d, J = 6,5 Hz : 3H) ; 1,09 (d, J = 6,5 Hz : 3H) ; de 1,60 à 2,05 (mt : 5H) ; 1,89 (s : 3H) ; 2,15 (mt : 1H) ; de 2,20 à 2,35 (mt : 1H) ; 2,31 (s : 3H) ; 2,40 (mt : 4H) ; 2,76 (mt : 1H) ; 2,99 (dt, J = 17 et 6 Hz : 1H) ; 3,23 (mt : 1H) ; 3,43 (mt : 4H) ; 3,49 (mt : 1H) ; 3,85 (mt : 1H) ; 3,94 (dd, J = 18 et 5 Hz : 1H) ; de 4,00 à 4,10 (mt : 2H) ; 4,54 (mt : 1H) ; 4,78 (d large, J = 10 Hz : 1H) ; 4,82 (dd, J = 9 et 3 Hz : 1H) ; 4,91 (mt : 1H) ; 5,13 (doublet démultiplié, J _{HF} = 48 Hz : 1H) ; 5,32 (d, J = 9 Hz : 1H) ; 5,77 (mt : 1H) ; 5,82 (d large, J = 17 Hz : 1H) ; 5,86 (mt : 1H) ; 5,95 (mt : 1H) ; 6,19 (d, J = 16 Hz : 1H) ; 6,51 (dd, J = 17 et 4 Hz : 1H) ; 8,12 (s : 1H).

La N-(4-méthylpipérazin-1-yl carbonyl) glycine peut être préparée de la manière suivante :

A 0,15 g de palladium sur charbon 5% en suspension dans 100 cm³ de méthanol, on ajoute à 20°C sous atmosphère d'argon, 2,36 g de N-(4-méthylpipérazin-1-yl carbonyl) glycinate de benzyle. Après 2,5 heures d'agitation à 22°C, sous 1,6 bar d'hydrogène, on ajoute 50 cm³ d'eau et on filtre sur Clarcel. Le gateau est lavé par 3 fois 70 cm³ d'eau à 60°C. Le filtrat est ensuite concentré à sec sous pression réduite (2,7 kPa), à 50°C. Le résidu est séché sous pression réduite (2,7 kPa) à 50°C, pour donner 1,5 g de N-(4-méthylpipérazin-1-yl carbonyl) glycine

Spectre de R.M.N. ¹H (400 MHz, (CD₃)₂SO d6 avec ajout de quelques gouttes de CD₃COOD d4, δ en ppm) : 2,65 (s : 3H) ; 2,98 (mt : 4H) ; 3,51 (mt : 4H) ; 3,68 (s : 2H).

Le N-(4-méthylpipérazin-1-yl carbonyl) glycinate de benzyle peut être préparé de la manière suivante :

A 3 g de chlorhydrate du chlorure de 4-méthylpipérazin-1-yl carbonyle en solution dans 150 cm³ de tétrahydrofurane, on ajoute à 20°C, 4,2 cm³ de triéthylamine et 3,02 g de chlorhydrate de glycinate de benzyle. Après 16 heures d'agitation à 60°C, le mélange réactionnel est concentré à sec sous pression réduite (2,7 kPa) et le résidu est repris dans 70 cm³ de dichlorométhane. La phase organique est successivement lavée par 2 fois 100 cm³ d'une solution aqueuse saturée de bicarbonate de sodium et 70 cm³ d'eau, puis séchée sur sulfate de magnésium, filtrée et concentrée à sec sous pression réduite (2,7 kPa) pour donner 1,9 g de N-(4-méthylpipérazin-1-yl carbonyl) glycinate de benzyle, sous forme d'un solide blanc.

Spectre de R.M.N. ¹H (400 MHz, (CD₃)₂SO d6, δ en ppm) : 2,18 (s : 3H) ; 2,25 (t, J = 5 Hz : 4H) ; 3,30 (t, J = 5 Hz : 4H) ; 3,80 (d, J = 6 Hz : 2H) ; 5,13 (s : 2H) ; 7,02 (t, J = 6 Hz : 1H) ; de 7,30 à 7,45 (mt : 5H).

### Exemple 66

### (16R)-16-Désoxo-16-fluoro-14-O-{4-[(3-carboxy)propyldisulfanyl]butyryl} pristinamycine II_{B}

A 1,75 g de (16*R*)-16-désoxo-16-fluoro pristinamycine II_{B} (préparée comme décrit dans l'exemple 1) en solution dans 50 cm³ de dichlorométhane, on ajoute à 20°C, sous atmosphère d'argon, 1,25 g d'acide 4,4'-dithiodibutanoïque, 1,03 g de N,N'-dicyclohexylcarbodiimide et 0,06 g de 4-diméthylaminopyridine. Après 18 heures d'agitation, le mélange réactionnel est additionné de 70 cm³ de dichlorométhane puis filtré pour éliminer l'insoluble. Le filtrat est alors concentré à sec, sous pression réduite (2,7 kPa), pour donner 3,3 g d'un résidu qui est purifié par deux chromatographies-flash successives [éluant : dichlorométhane / méthanol (96 / 4 et 97,5 / 2,5 en volumes)]. On obtient un résidu qui est repris par 25 cm³ d'eau distillée puis dissous par addition d'une solution aqueuse saturée d'hydrogénocarbonate de sodium. La phase aqueuse (pH 7-8) est lavée par quatre fois 50 cm³ d'acétate d'éthyle puis acidifiée par une solution aqueuse d'acide chlorhydrique 1 N jusqu'à pH 2-3. Il apparaît un précipité pâteux qui est dissous par addition de 250 cm³ de dichlorométhane. La phase organique est séparée, séchée sur sulfate de magnésium, filtrée, puis concentrée à sec sous pression réduite (2,7 kPa), pour donner 0,7 g de ( 16*R*)-16-désoxo-16-fluoro-14-O-{4-[(3-carboxy)propyldisulfanyl)butyryl} pristinamycine II_{B} sous la forme d'un solide blanc.

Spectre de R.M.N. ¹H (400 MHz, CDCl₃, δ en ppm) : 0,96 (d, J = 6,5 Hz : 3H) ; 0,99 (d, J = 6,5 Hz : 3H) ; 1, 11 (d, J = 6,5 Hz : 3H) ; de 1,50 à 2,30 (mt : 11H) ; 1,90 (s : 3H) ; de 2,35 à 2,55 (mt : 4H) ; de 2,65 à 2,85 (mt : 5H) ; 3,04 (mt : 1H) ; 3,26 (dt, J = 17 et 5 Hz : 1H) ; 3,56 (mt : 1H); 3,85 (mt : 1H) ; 4,07 (mt : 1H) ; 4,53 (mt : 1H) ; de 4,75 à 4,85 (mt : 2H) ; 5,08 (doublet démultiplié, J _{HF} = 48 Hz : 1H) ; 5,29 (d, J = 9 Hz : 1H) ; de 5,75 à 5,90 (mt : 1H) ; 5,79 (mt : 1H) ; 5,88 (dd, J = 16 et 1,5 Hz : 1H) ; de 6,10 à 6,25 (mt : 1H) ; 6,17 (d, J = 15,5 Hz : 1H) ; 6,58 (dd, J = 16 et 5 Hz : 1H) ; 8,13 (s : 1H).

### Exemple 67

### (16R)-16-Désoxo-16-fluoro-14-O-{4-[3-(4-méthylpipérazin-1-yl carbonyl)propyldisulfanyl] butyryl} pristinamycine II_{B}

A 1 g de (16*R*)-16-désoxo-16-fluoro-14-O-{4-[(3-carboxy)propyldisulfanyl]butyryl} pristinamycine II_{B} (préparé comme décrit dans l'éxemple 70) en solution dans 93 cm³ de dichlorométhane, on ajoute à 20°C sous atmosphère d'argon, 0,97 g de N,N'-dicyclohexylcarbodiimide, 0,5 cm³ de 1-méthyl-pipérazine et 0,08 g de 4-diméthylaminopyridine. Après 96 heures d'agitation, le mélange réactionnel est filtré pour éliminer l'insoluble. Le filtrat est concentré à sec, sous pression réduite (2,7 kPa), pour donner 2 g d'un résidu qui est purifié par chromatographie-flash [éluant : dichlorométhane / méthanol (96 / 4 en volumes)]. On obtient 0,5 g de (16*R*)-16-désoxo-16-fluoro-14-O-{4-[3-(4-méthylpipérazin-1-yl carbonyl)propyldisulfanyl]butyryl} pristinamycine II_{B} sous forme d'un solide blanc.

Spectre de R.M.N. ¹H (400 MHz, CDCl₃, δ en ppm) : 0,96 (d, J = 6,5 Hz : 3H) ; 1,00 (d, J = 6,5 Hz : 3H) ; 1,10 (d, J = 6,5 Hz : 3H) ; de 1,65 à 2,10 (mt : 9H) ; 1,88 (s : 3H) ; 2,15 (mt : 1H) ; 2,24 (mt : 1H) ; 2,32 (s : 3H) ; de 2,35 à 2,50 (mt : 8H) ; de 2,70 à 2,80 (mt : 1H) ; 2,71 (t, J = 7,5 Hz : 2H) ; 2,75 (t, J = 7 Hz : 2H) ; 3,00 (mt : 1H) ; 3,24 (mt : 1H) ; de 3,40 à 3,55 (mt : 3H) ; 3,64 (t, J = 5 Hz : 2H) ; 3,86 (mt : 1H) ; 4,06 (mt : 1H) ; 4,55 (mt : 1H) ; 4,78 (dd, J = 10 et 2 Hz : 1H) ; 4,82 (dd, J = 9 et 3 Hz : 1 H) ; 5,11 (doublet démultiplié, J _{HF} = 48 Hz : 1 H) ; 5,30 (d, J = 9,5 Hz : 1H) ; de 5,75 à 5,85 (mt : 1H) ; 5,76 (mt : 1H) ; 5,81 (dd, J = 16 et 2 Hz : 1H) ; 6,01 (mt : 1H) ; 6,19 (d, J = 16 Hz : 1H) ; 6,52 (dd, J = 16 et 5 Hz : 1H) ; 8,12 (s : 1H).

### Exemple 68

### (16R)-14-O-(3-Carboxypropionyl)-16-désoxo-16-fluoro pristinamycine II_{A}

On place dans un tricol, 560 mg de (16*R*)-16-désoxo-16-fluoro pristinamycine II_{A} (préparée comme décrit dans l'exemple 8) en solution dans 3 cm³ de pyridine puis on ajoute à 20°C, 286 mg d'anhydride succinique puis 120 mg de 4-diméthylaminopyridine. Après 18 heures d'agitation, le mélange réactionnel est versé sur 50 cm³ d'eau distillée et 10 cm³ de dichlorométhane. On ajoute ensuite la quantité suffisante d'acide chlorhydrique 0,1 N pour ajuster le pH à 4. Le mélange est filtré sur coton pour enlever l'insoluble puis décanté. La phase aqueuse est lavée par 2 fois 10 cm³ de dichlorométhane. Les phases organiques sont rassemblées, additionnées de méthanol pour compléter la solubilisation, séchées sur sulfate de magnésium et concentrées à sec sous pression réduite (2,7 kPa) pour donner 670 mg d'un solide qui est purifié par chromatographie-flash [éluant : dichlorométhane-méthanol (97-3 en volumes)]. On obtient ainsi 530 mg d'un produit qui est à nouveau purifié par chromatographie- flash [éluant : dichlorométhane-méthanol (95-5 en volumes)] pour donner un solide blanc qui est agité 15 minutes dans 5 cm³ d'éther diéthylique, filtré puis séché à 50 °C (90 Pa). On obtient 147 mg de (16*R*)-14-O-(3-carboxypropionyl)-16-désoxo-16-fluoro pristinamycine II_{A}, sous forme d'un solide blanc fondant vers 156°C (déc.).

Spectre de R.M.N. ¹H (400 MHz, CDCl₃, δ en ppm) : 0,99 (mt : 6H) ; 1,13 (d, J = 6,5 Hz : 3H) ; 1,83 (s : 3H) ; de 1,90 à 2,10 (mt : 2H) ; 2,27 (mt : 1H) ; de 2,50 à 2,90 (mt : 7H) ; 3,08 (mt : 1H) ; 3,28 (mt : 1H) ; 4,00 (d large, J = 18 Hz : 1H) ; 4,12 (mt : 1H) ; 4,20 (mt : 1H) ; 4,30 (mt : 1H) ; 4,72 (doublet démultiplié, J _{HF} = 48 Hz : 1H) ; de 4,85 à 5,00 (mt : 2H) ; 5,67 (mt : 1H) ; 5,71 (mt : 1H) ; 5,96 (d, J = 16 Hz : 1H) ; 5,98 (d, J = 17 Hz : 1H) ; 6,18 (t large, J = 2 Hz : 1H) ; 6,61 (dd, J = 17 et 7 Hz : 1H) ; 7,02 (mt : 1H) ; 7,96 (s : 1H).

### Exemple 69

### Chlorhydrate de la (16R)-16-Désoxo-16-fluoro-14-O-[3-(4-méthylpipérazin-1-yl carbonyl)propionyl] pristinamycine II_{A}

A 355 mg de (16*R*)-16-désoxo-16-fluoro-14-O-[3-(4-méthylpipérazin-1-yl carbonyl)propionyl] pristinamycine II_{A} dissous dans 3,5 cm³d'éthanol absolu, on ajoute 0,167 cm³ d'éther chlorhydrique 3 M puis, lentement, 7 cm³ d'éther diéthylique jusqu'à précipitation du produit. Après 15 minutes d'agitation, le produit est filtré, rincé avec le minimum d'un mélange éthanol-éther (1/2) puis séché à 20°C (90 Pa) pour donner 305 mg de chlorhydrate de la (16*R*)-16-désoxo-16-fluoro-14-O-[3-(4-méthylpipérazin-1-yl carbonyl)propionyl] pristinamycine II_{A}, sous forme d'un solide blanc fondant à 192°C.

Spectre de R.M.N. ¹H (400 MHz, (CD₃)₂SO d6, δ en ppm) : 0,87 (d, J = 6,5 Hz : 3H) ; 0,96 (d, J = 6,5 Hz : 3H) ; 1,10 (d, J = 6,5 Hz : 3H) ; 1,78 (s : 3H) ; 1,93 (mt : 1H) ; 2,02 (mt : 1H) ; 2,18 (mt : 1H) ; de 2,50 à 2,80 (mt : 7H) ; 2,78 (s : 3H) ; de 2,80 à 3,55 (mt : 8H) ; 3,66 (d large, J = 16 Hz : 1H) ; de 3,90 à 4,25 (mt : 3H) ; 4,14 (mt : 1H) ; de 4,25 à 4,55 (mf : 1H) ; 4,77 (dd, J = 9 et 1,5 Hz : 1H) ; 5,05 (doublet démultiplié, J _{HF} = 48 Hz : 1H) ; 5,38 (d, J = 9,5 Hz : 1H) ; 5,71 (mt : 2H) ; 5,88 (d, J = 16 Hz : 1H) ; 6,12 (d, J = 16 Hz : 1H) ; 6,36 (t large, J = 2 Hz : 1H) ; 6,62 (dd, J = 16 et 5,5 Hz : 1H) ; 8,03 (mt : 1H), 8,61 (s : 1H); 10,58 (mf : 1H).

La (16*R*)-16-Désoxo-16-fluoro-14-O-[3-(4-méthylpipérazin-1-yl carbonyl)propionyl] pristinamycine II_{A} peut être préparée de la façon suivante :

Dans un ballon placé sous azote, on introduit 848 mg de (16R)-16-désoxo-16-fluoro pristinamycine II_{A} (préparée comme décrit dans l'exemple 8), 24 cm³ de dichlorométhane et 352 mg d'acide 4-(4-méthyl pipérazin-1-yl)-4-oxo butyrique. Le mélange obtenu est chauffé jusqu'à dissolution puis on ajoute, à température ambiante, 360 mg de N,N'-dicyclohexylcarbodiimide puis 20 mg de 4-diméthylaminopyridine. Après 18 heures d'agitation à 20°C, on ajoute 36 mg de N,N'-dicyclohexylcarbodiimide et 20 mg de 4-diméthylaminopyridine supplémentaires. Le milieu réactionnel est alors agité à 20°C pendant 1 semaine puis filtré, rincé à l'acétate d'éthyle et concentré à sec sous pression réduite (2,7 kPa). L'huile épaisse obtenue est reprise par 15 cm³ d'acétate d'éthyle et 30 cm³ de dichlorométhane. La phase organique est lavée par 3 fois 30 cm³ d'eau distillée, décantée, séchée sur sulfate de magnésium, filtrée puis concentrée à sec sous pression réduite (2,7 kPa) à 20°C, pour donner 1,05 g d'une huile jaune qui est purifiée par chromatographie-flash [éluant : dichlorométhane-méthanol (95-5 en volumes)]. On obtient ainsi 840 mg d'un produit qui est agité dans 16 cm³ d'éther diéthylique pendant 18 heures, filtré puis séché à 20°C (90 Pa) pour donner 640 mg de (16*R*)-16-désoxo-16-fluoro-14-O-[3-(4-méthylpipérazin-1-yl carbonyl)propionyl] pristinamycine II_{A} sous forme d'un solide blanc fondant à 130°C.

L'acide 4-(4-méthylpipérazin-1-yl)-4-oxo butyrique peut être préparé de la façon suivante :

On ajoute à 20 cm³ de dioxane, 1,17 g d'anhydride succinique puis 1,19 cm³ de N-méthylpipérazine. Après 18 heures d'agitation à température ambiante, le précipité obtenu est filtré, rincé par un minimum de dioxane puis, successivement, par 2 fois 10 cm³ d'acétone et 10 cm³ d'éther diéthylique. Après séchage sous pression réduite (2,7 kPa) à 20°C, on obtient 1,09 g d'acide 4-(4-méthylpipérazin-1-yl)-4-oxo butyrique sous forme d'un solide blanc fondant à 114°C.

### Exemple 70

### (16R)-14-O-(4-Carboxybutyryl)-16-désoxo-16-fluoro pristinamycine II_{A}

A 1,05 g de (16*R*)-16-désoxo-16-fluoro pristinamycine II_{A} (préparée comme décrit dans l'exemple 8) en solution dans 5,5 cm³ de pyridine, on ajoute à 20°C sous atmosphère d'argon, 0,68 g d'anhydride glutarique et 0,24 g de 4-diméthylaminopyridine. Après 24 heures d'agitation, le mélange réactionnel est concentré à sec sous pression réduite (2,7 kPa) pour donner un résidu qui est repris par 110 cm³ d'acétate d'éthyle et 55 cm³ d'eau distillée. La phase organique est décantée, lavée successivement par deux fois 55 cm³ d'eau distillée, 55 cm³ d'une solution aqueuse d'acide chlorhydrique 0,1 N, deux fois 55 cm³ d'eau distillée et deux fois 55 cm³ d'une solution aqueuse saturée de chlorure de sodium. Après séchage sur sulfate de magnésium et filtration, la phase organique est concentrée à sec sous pression réduite (2,7 kPa). On obtient 0,95 g d'un résidu qui est dissous dans 100 cm³ de dichlorométhane et extrait par 100 cm³ d'une solution aqueuse d'hydrogénocarbonate de sodium à 1%. L'émulsion obtenue est additionnée de 500 cm³ d'acétate d'éthyle pour permettre la décantation. La phase aqueuse est alors séparée, lavée à nouveau par trois fois 100 cm³ d'acétate d'éthyle, acidifiée par une solution aqueuse d'acide chlorhydrique 1 N jusqu'à obtention d'un pH voisin de 2 puis extraite trois fois par du dichlorométhane (200, 100 et 100 cm³). Les phases organiques sont réunies, séchées sur du sulfate de magnésium, filtrées, puis concentrées à sec sous pression réduite (2,7 kPa). On obtient ainsi 0,33 g de (16*R*)-14-O-(4-carboxybutyryl)16-désoxo-16-fluoro pristinamycine II_{A} sous forme d'un solide beige fondant à 116°C (déc.).

Spectre de R.M.N. ¹H (400 MHz, CDCl₃, δ en ppm) : 0,98 (mt : 6H) ; 1,12 (d, J = 6,5 Hz : 3H) ; de 1,80 à 2,10 (mt : 4H) ; 1,82 (s : 3H) ; de 2,15 à 2,45 (mt : 5H) ; de 2,65 à 2,90 (mt : 3H) ; 3,08 (mt : 1H) ; 3,26 (dt, J = 16 et 3,5 Hz : 1H) ; 3,99 (d large, J = 18 Hz : 1H) ; de 4,05 à 4,35 (mt : 3H) ; 4,71 (doublet démultiplié, J _{HF} = 48 Hz : 1H) ; de 4,85 à 5,00 (mt : 2H) ; de 5,60 à 5,75 (mt : 2H) ; 5,96 (d, J = 16 Hz : 1H) ; 5,98 (d large, J = 16 Hz : 1H) ; 6,18 (t, J = 3 Hz : 1H) ; 6,61 (dd, J = 16 et 7 Hz : 1H) ; 7,09 (t, J = 5,5 Hz : 1H) ; 7,95 (s : 1H).

### Exemple 71

### (16R)-16-Désoxo-16-fluoro-14-O-[4-(4-méthylpipérazin-1-yl carbonyl)butyryl pristinamycine II_{A}

A 1 g de (16*R*)-16-désoxo-16-fluoro pristinamycine II_{A} (préparée comme décrit dans l'exemple 8) en solution dans 30 cm³ de dichlorométhane, on ajoute à 20°C sous atmosphère d'argon, 0,48 g d'acide 4-(4-méthylpipérazin-1-yl carbonyl)butyrique, 0,48 g de N,N'-dicyclohexylcarbodiimide et 0,14 g de 4-diméthylaminopyridine. Après 20 heures d'agitation à 20°C, le mélange réactionnel est versé sur 50 cm³ d'eau distillée et 10 cm³ de dichlorométhane. Le mélange résultant est filtré pour éliminer l'insoluble. La phase organique est décantée puis la phase aqueuse est extraite par deux fois 10 cm³ de dichlorométhane. Les phases organiques sont réunies, séchées sur du sulfate de magnésium, filtrées, puis concentrées à sec sous pression réduite (2,7 kPa), pour donner 1,6 g d'un résidu qui est purifié par deux chromatographies-flash successives [éluant : dichlorométhane / méthanol (95 / 5 en volumes)]. On obtient après agitation dans de l'éther diéthylique, filtration et séchage (2,7 kPa), 0,6 g de (16*R*)-16-désoxo-16-fluoro-14-O-[4-(4-méthylpipérazin-1-yl carbonyl)butyryl] pristinamycine II_{A} sous forme d'un solide pâteux, fondant vers 120°C.

Spectre de R.M.N. ¹H (500 MHz, CDCl₃, δ en ppm) : 0,99 (mt : 6H) ; 1,13 (d, J = 6,5 Hz : 3H) ; 1,84 (s : 3H) ; de 1,90 à 2,10 (mt : 2H) ; 1,92 (mt : 2H) ; de 2,15 à 2,45 (mt : 9H) ; 2,30 (s : 3H) ; de 2,65 à 2,90 (mt : 3H) ; 3,07 (mt : 1H) ; 3,27 (dt, J = 15 et 3,5 Hz : 1H) ; 3,46 (mt : 2H) ; 3,63 (mt : 2H) ; 3,99 (d large, J = 17,5 Hz : 1H) ; de 4,10 à 4,25 (mt : 2H) ; 4,30 (mt : 1H) ; 4,71 (doublet démultiplié, J _{HF} = 48 Hz : 1H) ; 4,90 (d, J = 9,5 Hz : 1H) ; 4,95 (dd, J = 10 et 2 Hz : 1H) ; 5,65 (dt, J = 9,5 et 4 Hz : 1H) ; 5,72 (mt : 1H) ; 5,96 (d, J = 16 Hz : 1H) ; 5,98 (d large, J = 16,5 Hz : 1H) ; 6,17 (t, J = 3 Hz : 1H) ; 6,60 (dd, J = 16,5 et 7 Hz : 1H) ; 7,02 (t, J = 5,5 Hz : 1H) ; 7,94 (s : 1H).

L'acide 4-(4-méthylpipérazin-1-yl carbonyl)butyrique peut être préparé selon DE 78-2851953.

### Exemple 72

### (16R)-16-Désoxo-16-fluoro-14-O-{(1R, 2R)-[2-(4-méthylpipérazin-1-yl)carbonyl]-1-cyclohexanecarbonyl} pristinamycine II_{B} :

On opère d'une manière analogue à celle décrite dans l'exemple 60 mais à partir de 0,83 g de (16*R*)-16-désoxo-16-fluoro pristinamycine II_{B} (préparée comme décrit dans l'exemple 1), 0,40 g d'acide (*1R, 2R*)-2-[(4-méthylpipérazin-1-yl)-carbonyl] 1-cyclohexanecarboxylique, 0,10 g de 4-diméthylaminopyridine, 30 cm³ de dichlorométhane, et 0,40 g de N,N'-dicyclohexylcarbodiimide. Après traitement analogue à celui de l'exemple 60, le produit brut est purifié par chromatographie flash [éluant : dichlorométhane / acétonitrile / méthanol (90 / 5 / 5 en volumes)] puis par chromatographie sur colonne d'alumine CBT 1 [éluant : acétate d'éthyle / méthanol (98 / 2 en volumes)] pour donner 0,40 g de (16*R*)-16-désoxo-16-fluoro-14-O-{(*1R*, *2R*)-[2-(4-méthylpipérazin-1-yl)carbonyl]-1-cyclohexanecarbonyl} pristinamycine II_{B}, sous la forme d'un solide blanc.

Spectre de R.M.N. ¹H (400 MHz, CDCl₃, δ en ppm) : 0,95 (d, J = 6,5 Hz : 3H) ; 0,99 (d, J = 6,5 Hz : 3H) ; 1,09 (d, J = 6,5 Hz : 3H) ; de 1,20 à 1,45 (mt : 4H) ; de 1,55 à 2,45 (mt : 17H) ; 1,83 (s : 3H) ; 2,50 (mt : 1H) ; de 2,70 à 2,85 (mt : 3H) ; 3,00 (mt : 1H) ; 3,22 (mt : 1H) ; 3,44 (mt : 1H) ; de 3,45 à 3,65 (mt : 4H) ; 3,88 (mt : 1H) ; 4,04 (mt : 1H) ; 4,53 (mt : 1H) ; de 4,70 à 4,85 (mt : 2H) ; 5,10 (doublet démultiplié, J _{HF} = 48 Hz : 1H) ; 5,29 (d, J = 10 Hz : 1H) ; de 5,65 à 5,80 (mt : 2H) ; 5,81 (d large, J = 16 Hz : 1H) ; 6,02 (mt : 1H) ; 6,18 (d, J = 16 Hz : 1H) ; 6,50 (dd, J = 16 et 4 Hz : 1H) ; 8,12 (s : 1H).

L'acide (*1R*, *2R*)-2-[(4-méthylpipérazin-1-yl)-carbonyl]-1-cyclohexane carboxylique peut être préparé de la manière suivante :

A 0,50 g d'anhydride (-)-*trans*-1,2-cyclohexanedicarboxylique en solution à -15°C dans 20 cm³ de dioxane, on ajoute sous atmosphère d'argon, 0,37 cm³ de N-méthylpipérazine en solution dans 5 cm³ de dioxane. Après 1,25 heure à 20°C, le mélange réactionnel est filtré, le solide est lavé avec 20 cm³ d'éther diéthylique, essoré, puis séché sous pression réduite (2,7 kPa) à 20°C, pour donner 0,65 g d'acide (*1R*, *2R*)-2-(4-méthylpipérazin-1-yl)-carbonyl] 1-cyclohexane carboxylique, sous la forme d'un solide blanc.

Spectre de R.M.N. ¹H (400 MHz, CDCl₃, δ en ppm) : de 1,20 à 1,60 (mt : 4H) ; de 1,65 à 1,90 (mt : 3H) ; de 2,05 à 2,35 (mt : 3H) ; 2,37 (s : 3H) ; de 2,70 à 2,95 (mt : 5H) ; 3,43 (mt : 1H) ; 3,84 (d large, J = 13,5 Hz : 1H) ; 4,30 (d, J = 12,5 Hz : 1H).

### Exemple 73

### (16R)-16-Azido-16-désoxo pristinamycine II_{B}

En opérant de manière analogue à l'exemple 4, mis à partir d'azoture de tétra n-butylammonium, on obtient la (16*R*)-16-azido-16-désoxo pristinamycine II_{B}, sous forme d'un solide blanc fondant vers 135°C (déc.).

Spectre de R.M.N. ¹H (400 MHz, CDCl₃, δ en ppm) : 0,96 (d, J = 6,5 Hz : 3H) ; 1,01 (d, J = 6,5 Hz : 3H) ; 1,10 (d, J = 6,5 Hz : 3H) ; de 1,50 à 1,65 (mt : 1H) ; de 1,75 à 2,05 (mt : 5H) ; 1,88 (s : 3H) ; 2,15 (mt : 1H) ; 2,77 (mt : 1H) ; 2,90 (dd, J = 17 et 6 Hz : 1H) ; 3,15 (dd, J = 17 et 7 Hz : 1H) ; 3,49 (mt : 1H) ; 3,88 (mt : 1H) ; de 3,95 à 4,15 (mt : 2H) ; 4,54 (mt : 1H) ; de 4,70 à 4,80 (mt : 2H) ; 4,83 (dd, J = 9 et 3 Hz : 1H) ; 5,37 (d, J = 9,5 Hz : 1H) ; 5,75 (mt : 1H) ; 5,82 (dd, J = 17 et 1,5 Hz : 1H) ; 5,97 (mt : 1H) ; 6,22 (d, J = 16 Hz : 1H) ; 6,52 (dd, J = 17 et 5 Hz : 1H) ; 8,15 (s : 1H).

La présente invention concerne également les compositions pharmaceutiques contenant au moins un dérivé de streptogramine selon l'invention, à l'état pur, associé à au moins un dérivé de streptogramine du groupe B, le cas échéant sous forme de sel, et/ou sous forme d'une association avec un ou plusieurs diluants ou adjuvants compatibles et pharmaceutiquement acceptables.

Les compositions selon l'invention peuvent être utilisées par voie orale, parentérale, topique, rectale ou en aérosols.

Comme compositions solides pour administration orale peuvent être utilisés des comprimés, des pilules, des gélules, des poudres ou des granulés. Dans ces compositions, le produit actif selon l'invention, généralement sous forme d'association est mélangé à un ou plusieurs diluants ou adjuvants inertes, tels que saccharose, lactose ou amidon. Ces compositions peuvent comprendre des substances autres que les diluants, par exemple un lubrifiant tel que le stéarate de magnésium ou un enrobage destiné à une libération contrôlée.

Comme compositions liquides pour administration orale, on peut utiliser des solutions pharmaceutiquement acceptables, des suspensions, des émulsions, des sirops et des élixirs contenant des diluants inertes tels que l'eau ou l'huile de paraffine. Ces compositions peuvent également comprendre des substances autres que les diluants, par exemple des produits mouillants, édulcorants ou aromatisants.

Les compositions pour administration parentérale, peuvent être des solutions stériles ou des émulsions. Comme solvant ou véhicule, on peut employer le propylèneglycol, un polyéthylèneglycol, des huiles végétales, en particulier l'huile d'olive, des esters organiques injectables, par exemple l'oléate d'éthyle. Ces compositions peuvent également contenir des adjuvants, en particulier des agents mouillants, isotonisants, émulsifiants, dispersants et stabilisants.

La stérilisation peut se faire de plusieurs façons, par exemple à l'aide d'un filtre bactériologique, par irradiation ou par chauffage. Elles peuvent également être préparées sous forme de compositions solides stériles qui peuvent être dissoutes au moment de l'emploi dans de l'eau stérile ou tout autre milieu stérile injectable.

Les compositions pour administration topique peuvent être par exemple des crèmes, des pommades, des lotions ou des aérosols.

Les compositions par administration rectale sont les suppositoires ou les capsules rectales, qui contiennent outre le principe actif, des excipients tels que le beurre de cacao, des glycérides semi-synthétiques ou des polyéthylèneglycols.

Les compositions peuvent également être des aérosols. Pour l'usage sous forme d'aérosols liquides, les compositions peuvent être des solutions stériles stables ou des compositions solides dissoutes au moment de l'emploi dans de l'eau stérile apyrogène, dans du sérum ou tout autre véhicule pharmaceutiquement acceptable. Pour l'usage sous forme d'aérosols secs destinés à être directement inhalés, le principe actif est finement divisé et associé à un diluant ou véhicule solide hydrosoluble d'une granulométrie de 30 à 80 µm, par exemple le dextrane, le mannitol ou le lactose.

En thérapeutique humaine, les nouveaux dérivés de streptogramine selon l'invention sont particulièrement utiles dans le traitement des infections d'origine bactérienne. Les doses dépendent de l'effet recherché et de la durée du traitement. Le médecin déterminera la posologie qu'il estime la plus appropriée en fonction du traitement, en fonction de l'âge, du poids, du degré de l'infection et des autres facteurs propres au sujet à traiter. Généralement, les doses sont comprises entre 0,5 et 3 g de produit actif en 2 ou 3 administrations par jour, par voie orale ou parentérale pour un adulte.

L'exemple suivant illustre une composition selon l'invention.

### EXEMPLE

On prépare selon la technique habituelle des comprimés dosés à 250 mg de produit actif, ayant la composition suivante :
- (16*R*)-16-désoxo-16-fluoro pristinamycine II_{B} 175 mg
- pristinamycine I_{B} 75 mg
- excipient : amidon, silice hydratée, dextrine, gélatine, stéarate de magnésium : qsp 500 mg

## Revendications

1. Un dérivé du groupe A des streptogramines de formule générale : 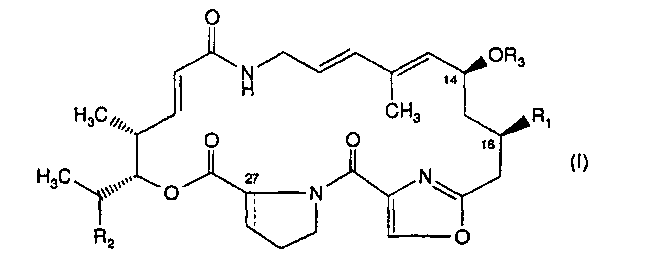 dans laquelle :
― R₁ représente un atome d'halogène ou un radical azido ou thiocyanato,
― R₂ représente un atome d'hydrogène ou un radical méthyle ou éthyle,
― R₃ représente un atome d'hydrogène, ou le reste d'un ester aliphatique, cycloaliphatique, aromatique, araliphatique, hétérocyclique ou hétérocyclylaliphatique pouvant être substitué, et
― la liaison --- représente une liaison simple (stéréochimie 27R) ou une liaison double,
ainsi que ses sels lorsqu'ils existent.

2. Un dérivé du groupe A des streptogramines selon la revendication 1, **caractérisé en ce que** le reste R₃ d'ester aliphatique, cycloaliphatique, aromatique, araliphatique, hétérocyclique ou hétérocyclylaliphatique pouvant être substitué, est un radical R'₃-CO- dans lequel R'₃ représente phényle ou phénylalcoyle non substitués ou substitués sur le radical phényle [par un ou plusieurs radicaux choisis parmi alcoyle, portant éventuellement un radical NR'R" dont les radicaux R' et R" identiques ou différents peuvent être des atomes d'hydrogène ou des radicaux alcoyle pouvant former ensemble avec l'atome d'azote auquel ils sont attachés un radical hétérocyclyle saturé ou insaturé de 3 à 8 chaînons, comprenant éventuellement un autre hétéroatome choisi parmi l'oxygène, le soufre ou l'azote, ledit hétérocycle pouvant être lui même substitué par un ou plusieurs radicaux (alcoyle, hydroxyalcoyle, alcoyloxyalcoyle, alcoyloxycarbonylalcoyle, aryle, hétérocyclyle, hétérocyclylalcoyle saturés ou insaturés de 3 à 8 chaînons ou -CH₂-CO-NR'R"), ou bien R' et/ou R" peuvent être un radical hydroxyalcoyle, phényle, hétérocyclylalcoyle saturé ou insaturé de 3 à 8 chaînons, -CO-NR'R" pour lequel NR'R" est défini comme précédemment, ou alcoyle ou acyle substitués par NR'R" défini tel que ci-dessus], ou bien R'₃ peut être choisi parmi des radicaux phényle ou phénylalcoyle substitués sur le radical phényle par un ou plusieurs radicaux [choisis parmi alcoyle, pouvant être substitués par un radical alcoyloxy ou alcoylthio éventuellement portant eux-même un radical carboxy ou un radical NR'R" tel que défini ci-dessus, ou choisis parmi acyloxy pouvant être substitué par NR'R" défini tel que précédemment], ou bien R'₃ peut être choisi parmi des radicaux alcoyle ou cycloalcoyle éventuellement substitués [par un radical carboxy, carboxyalcoyldisulfanyle ou par un radical NR'R", -CH₂-NR'R", -CO-NR'R", ou par un radical alcoyloxycarbonyle, alcoyloxy, ou alcoyldisulfanyle éventuellement substitués par NR'R" ou -CO-NR'R" pour lesquels NR'R" est défini tel que précédemment], ou bien R'₃ peut être choisi parmi des radicaux hétérocyclyle saturés ou insaturés de 3 à 8 chaînons éventuellement substitués [par alcoyle ou acyle eux-même éventuellement substitués par NR'R"].

3. Un dérivé du groupe A des streptogramines selon l'une des revendications 1 ou 2, **caractérisé en ce que** :
- R₁ représente un atome de fluor, de chlore, de brome ou d'iode ou un radical azido ou thiocyanato,
- R₂ représente un radical méthyle,
- R₃ représente un atome d'hydrogène, ou un radical R'₃-CO- pour lequel R'₃ est phényle ou phénylalcoyle non substitués ou substitués sur le radical phényle [par un ou plusieurs radicaux choisis parmi alcoyle, portant éventuellement un radical NR'R" dont les radicaux R' et R" identiques ou différents peuvent être des atomes d'hydrogène ou des radicaux alcoyle pouvant former ensemble avec l'atome d'azote auquel ils sont attachés un radical hétérocyclyle saturé ou insaturé de 3 à 8 chaînons, comprenant éventuellement un autre hétéroatome choisi parmi l'oxygène, le soufre ou l'azote, ledit hétérocycle pouvant être lui même substitué par un ou plusieurs radicaux (alcoyle, hydroxyalcoyle, alcoyloxyalcoyle, alcoyloxycarbonylalcoyle, aryle, hétérocyclyle, hétérocyclylalcoyle saturés ou insaturés de 3 à 8 chaînons ou -CH₂-CO-NR'R"), ou bien R' et/ou R" peuvent être un radical hydroxyalcoyle, phényle, hétérocyclylalcoyle saturé ou insaturé de 3 à 8 chaînons, -CO-NR'R" pour lequel NR'R" est défini comme précédemment, ou alcoyle ou acyle substitués par NR'R" défini tel que ci-dessus], ou bien R'₃ peut être un radical phényle substitué par un ou plusieurs radicaux [choisis parmi alcoyle, pouvant être substitués par un radical alcoyloxy ou alcoylthio éventuellement portant eux-même un radical carboxy ou un radical NR'R" tel que défini ci-dessus, ou choisis parmi acyloxy pouvant être substitué par NR'R" défini tel que précédemment], ou bien R'₃ peut être choisi parmi des radicaux alcoyle ou cycloalcoyle éventuellement substitués [par un radical carboxy, carboxyalcoyldisulfanyle ou par un radical NR'R", -CH₂-NR'R", - CO-NR'R", ou par un radical alcoyloxycarbonyle, alcoyloxy, ou alcoyldisulfanyle éventuellement substitués par NR'R" ou -CO-NR'R" pour lesquels NR'R" est défini tel que précédemment], ou bien R'₃ peut être choisi parmi des radicaux hétérocyclyle saturés ou insaturés de 3 à 8 chaînons éventuellement substitués [par alcoyle ou acyle eux-même éventuellement substitués par NR'R"], étant entendu que les hétérocycles sont choisis parmi pyrrolidinyle, imidazolyle, pyridyle, pipéridinyle, pipérazinyle, ou morpholynyle, et
- la liaison --- représente une liaison simple (stéréochimie 27R) ou une liaison double,
ainsi que ses sels lorsqu'ils existent.

4. Un dérivé du groupe A des streptogramines selon l'une des revendications 1 à 3, **caractérisé en ce qu'**il s'agit de la (16*R*)-16-désoxo-16-fluoro pristinamycine II_{B}.

5. Un dérivé du groupe A des streptogramines selon l'une des revendications 1 à 3, **caractérisé en ce qu'**il s'agit de la (16*R*)-16-désoxo-16-thiocyanato pristinamycine II_{B}.

6. Un dérivé du groupe A des streptogramines selon l'une des revendications 1 à 3, **caractérisé en ce qu'**il s'agit de la (16*R*)-16-désoxo-16-chloro pristinamycine II_{B}.

7. Un dérivé du groupe A des streptogramines selon l'une des revendications 1 à 3, **caractérisé en ce qu'**il s'agit de la (16*R*)-16-azido-16-désoxo pristinamycine II_{B}.

8. Un dérivé du groupe A des streptogramines selon l'une des revendications 1 à 3, **caractérisé en ce qu'**il s'agit de la (16*R*)-16-désoxo-16-fluoro pristinamycine II_{A}.

9. Un procédé de préparation d'un dérivé du groupe A des streptogramines selon la revendication 1, **caractérisé en ce que** l'on halogène, **en ce que** l'on transforme en azide ou **en ce que** l'on transforme en thiocyanate, un dérivé de streptogramine de formule générale : 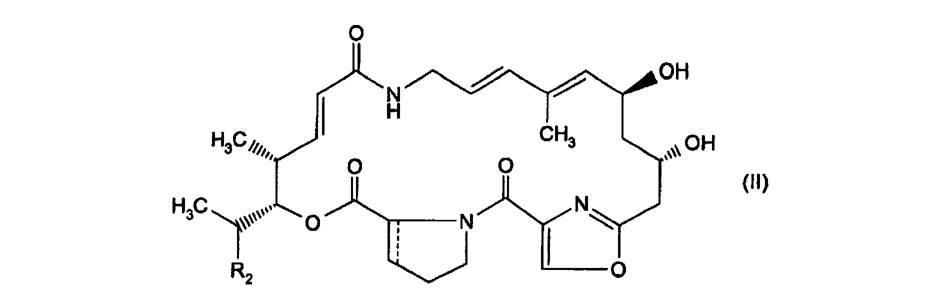 dans laquelle R₂ est défini dans comme précédemment, la liaison --- représente une liaison simple (stéréochimie 27R) ou une liaison double, et dont la fonction hydroxy en position 14 a été préalablement protégée, puis élimine le radical protecteur et le cas échéant, pour obtenir un dérivé de streptogramine du groupe A selon la revendication 1 ou 2, pour lequel R₃ est autre que l'atome d'hydrogène, introduit le reste d'ester aliphatique, cycloaliphatique, aromatique, araliphatique, hétérocyclique ou hétérocyclylaliphatique pouvant être substitué (R₃), et transforme éventuellement en un sel lorsqu'ils existent.

10. Un procédé selon la revendication 9, **caractérisé en ce que** l'on opère en présence d'un trifluorure d'aminosoufre ou en présence de tétrafluorure de soufre, au moyen d'un réactif comme un halogénure, un azoture ou un thiocyanate de tétra alkylammonium, de tri alkyl benzylammonium ou de tri alkyl phénylammonium ou au moyen d'un halogénure, d'un azoture ou d'un thiocyanate de métal alcalin additionné éventuellement d'un éther couronne.

11. Un procédé selon la revendication 9, **caractérisé en ce que** l'on opère, pour la fluoration, par action d'un agent de fluoration choisi parmi un fluorure de soufre, l'hexafluoropropyl diéthylamine ou la N-(chloro-2 trifluoro-1,1,2 éthyl) diéthylamine.

12. Un procédé selon l'une des revendications 9, 10 ou 11, **caractérisé en ce que** le cas échéant, l'estérification est mise en oeuvre par réaction de l'acide ou d'un dérivé réactif de l'acide, en présence ou non d'un agent de couplage comme un carbodiimide et d'une amine tertiaire et éventuellement un catalyseur comme la 4-N-diméthylaminopyridine.

13. Un procédé selon l'une des revendications 9, 10 ou 12, **caractérisé en ce que** le trifluorure d'aminosoufre peut être choisi parmi le trifluorure de diéthylamino soufre, le trifluorure de bis(2-méthoxyéthyl)amino soufre ou le trifluorure de morpholino soufre.

14. Composition pharmaceutique comprenant un dérivé de streptogramine du groupe A selon la revendication 1 ou 2, à l'état pur ou sous forme d'association avec au moins un dérivé de streptogramine du groupe B, le cas échéant sous forme de sel, et/ou sous forme d'association avec un ou plusieurs diluants ou adjuvants compatibles et pharmaceutiquement acceptables.

15. Composition pharmaceutique selon la revendication 14, **caractérisée en ce que** le dérivé de la streptogramine du groupe B est choisi parmi les composantes naturelles : pristinamycine IA, pristinamycine IB, pristinamycine IC, pristinamycine ID, pristinamycine IE, pristinamycine IF, pristinamycine IG, virginiamycine S1, S3 ou S4, vernamycine B ou C, étamycine ou parmi des dérivés d'hémisynthèse de formule générale : 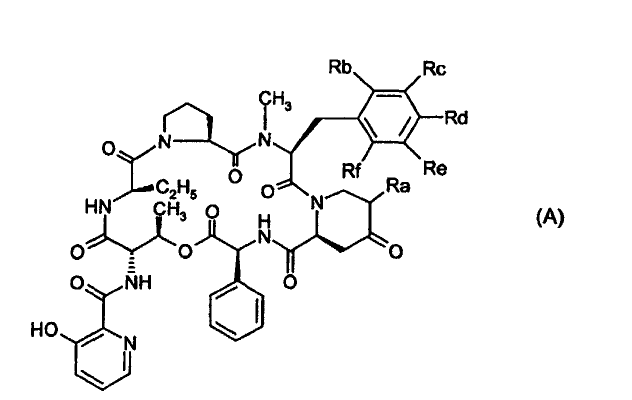 dans laquelle,
1)
Rb, Rc, Re et Rf sont des atomes d'hydrogène, Rd est un atome d'hydrogène ou un radical diméthylamino, et Ra est un radical de structure -CH₂R'a pour lequel R'a est pyrrolidinyl-3thio, pipéridyl-3(ou -4)thio pouvant être substitués par alcoyle, alcoylthio substitué par 1 ou 2 hydroxysulfonyle, alcoylamino, dialcoylamino (lui même éventuellement substitué par mercapto ou dialcoylamino), ou substitué par 1 ou 2 cycles pipérazine éventuellement substitué, morpholino, thiomorpholino, pipéridino, pyrrolidinyle-1, pipéridyle-2,-3 ou -4, ou pyrrolidinyle-2 ou -3 (pouvant être substitués par alcoyle), ou bien Ra est un radical de structure =CHR'a pour lequel R'a est pyrrolidinyl-3amino, pipéridyl-3(ou -4)amino, pyrrolidinyl-3oxy, pipéridyl-3(ou -4)oxy, pyrrolidinyl-3thio, pipéridyl-3(ou -4)thio pouvant être substitués par alcoyle, ou R'a est alcoylamino, alcoyloxy ou alcoylthio substitués par 1 ou 2 hydroxysulfonyle, alcoylamino, dialcoylamino (lui même éventuellement substitué par dialcoylamino), ou par trialcoylammonio, imidazolyl-4 ou -5, ou par 1 ou 2 cycles pipérazine éventuellement substitué, morpholino, thiomorpholino, pipéridino, pyrrolidinyle-1, pipéridyle-2,-3 ou -4, ou pyrrolidinyle-2 ou -3 (pouvant être substitués par alcoyle), ou
Ra est un radical quinuclidinyl-3(ou -4)thiométhyle ou bien
2) Ra est un atome d'hydrogène et
a) soit Rb, Re et Rf sont des atomes d'hydrogène, Rd est un radical -NHCH₃ ou -N(CH₃)₂ et Rc est un atome de chlore ou de brome, ou représente un radical alcényle contenant 3 à 5 atomes de carbone [si Rd est -N(CH₃)₂],
b) soit Rb, Rd, Re et Rf représentent un atome d'hydrogène et Rc est un halogène, ou un radical aminomonoalkyle, aminodialkyle, alcoyloxy, trifluorométhyloxy, thioalcoyle,alcoyle en C₁ à C₃ ou trihalogénométhyle
c) soit Rb, Rc, Re et Rf représentent un atome d'hydrogène et Rd est un halogène, ou un radical éthylamino, diéthylamino ou méthyléthylamino, alcoyloxy ou trifluorométhyloxy, thioalkyle, alcoyle en C₁ à C₆, aryle ou trihalogénométhyle
d) soit Rb, Re et Rf représentent un atome d'hydrogène et Rc est halogène ou un radical aminomonoalkyle ou aminodialkyle, alcoyloxy ou trifluorométhyloxy, thioalkyle, alcoyle en C₁ à C₃, et Rd est halogène ou un radical amino, aminomonoalkyle ou aminodialkyle, alcoyloxy ou trifluorométhyloxy, thioalkyle, alcoyle en C₁ à C₆ ou trihalogénométhyle,
e) soit Rc, Re et Rf représentent un atome d'hydrogène et Rb et Rd représentent un radical méthyle ;
ou encore parmi les dérivés d'hémisynthèse de formule générale : 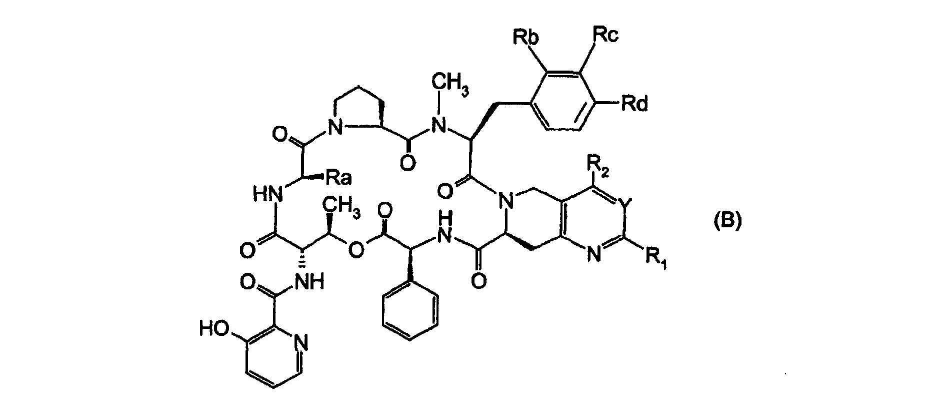 dans laquelle
Y est un atome d'azote ou un radical =CR₃-,
R₁ est un atome d'hydrogène, un radical alcoyle (1 à 8 carbones), alcényle (2 à 8 carbones), cycloalcoyle (3 à 8 carbones), hétérocyclyle saturé ou insaturé (3 à 8 chaînons), phényle, phényle substitué [par un ou plusieurs atomes d'halogène ou radicaux hydroxy, alcoyle, alcoyloxy, alcoylthio, alcoylsulfinyle, alcoylsulfonyle, amino, alcoylamino ou dialcoylamino] ou un radical NR'R", R' et R" identiques ou différents pouvant être des atomes d'hydrogène ou des radicaux alcoyle (1 à 3 carbones), ou pouvant former ensemble avec l'atome d'azote auquel ils sont attachés, un hétérocycle de 3 à 8 chaînons contenant éventuellement un autre hétéroatome choisi parmi l'oxygène le soufre ou l'azote éventuellement substitué [par un radical alcoyle, alcényle (2 à 8 carbones), cycloalcoyle (3 à 6 carbones), hétérocyclyle saturé ou insaturé (4 à 6 chaînons), benzyle, phényle ou phényle substitué tel que défini ci-dessus pour la définition de R₁]
ou bien lorsque Y est un radical =CR₃-, R₁ peut être aussi halogénométhyle, hydroxyméthyle, alcoyloxyméthyle, alcoylthiométhyle dont la partie alcoyle est éventuellement substituée par NR'R", alcoylsulfinylméthyle, alcoylsulfonylméthyle, acyloxyméthyle, benzoyloxyméthyle, cyclopropylaminométhyle ou -(CH₂)ₙNR'R" (n étant un entier de 1 à 4 et R' et R" étant définis comme ci-dessus), ou bien si R₃ est un atome d'hydrogène, R₁ peut être aussi formyle, carboxy, alcoyloxycarbonyle, ou -CONR'R" pour lequel R' et R" sont définis comme ci-dessus,
ou bien lorsque Y est un atome d'azote, R₁ peut être aussi un radical -XR° pour lequel X est un atome d'oxygène ou de soufre, un radical sulfinyle ou sulfonyle,
ou un radical NH et R° est un radical alcoyle (1 à 8 carbones), cycloalcoyle (3 à 6 carbones), hétérocyclyle saturé ou insaturé (3 à 8 chaînons), hétérocyclylméthyle (3 à 8 chaînons) dont la partie hétérocyclyle est attachée au radical méthyle par un atome de carbone, phényle, phényle substitué [par un ou plusieurs atomes d'halogène ou radicaux hydroxy, alcoyle, alcoyloxy, alcoylthio, alcoylsulfinyle, alcoylsulfonyle, amino, alcoylamino ou dialcoylamino] ou un radical - (CH₂)ₙNR'R" pour lequel R' et R" sont définis comme ci-dessus et n est un entier de 2 à 4, ou bien, si X représente NH, R° peut aussi représenter l'atome d'hydrogène,
R₂ est un atome d'hydrogène ou un radical alcoyle (1 à 3 carbones),
R₃ est un atome d'hydrogène ou un radical alcoyle, carboxy, alcoyloxycarbonyle ou carbamoyle de structure -CO-NR'R" dans laquelle R' et R" sont définis comme précédemment,
Ra est un radical méthyle ou éthyle, et
Rb, Rc et Rd ont les définitions ci-après :
1) Rb et Rc sont des atomes d'hydrogène et Rd est un atome d'hydrogène ou un radical méthylamino ou diméthylamino,
2) Rb est un atome d'hydrogène, Rc est un atome d'hydrogène, de chlore ou de brome, ou représente un radical alcényle (3 à 5C), et Rd est un radical -NMe-R''' pour lequel R''' représente un radical alcoyle, hydroxyalcoyle (2 à 4C), ou alcényle (2 à 8C) éventuellement substitué par phényle, cycloalcoyl(3 à 6C)méthyle, benzyle, benzyle substitué [par un ou plusieurs atomes d'halogène ou radicaux hydroxy, alcoyle, alcoyloxy, alcoylthio, alcoylsulfinyle, alcoylsulfonyle, amino, alcoylamino ou dialcoylamino], hétérocyclylméthyle ou hétérocyclyléthyle dont la partie hétérocyclyle est saturée ou insaturée et contient 5 à 6 chaînons et 1 ou 2 hétéroatomes choisis parmi le soufre, l'oxygène ou l'azote éventuellement substitué [par un radical alcoyle, alcényle (2 à 8 carbones), cycloalcoyle (3 à 6 carbones), hétérocyclyle saturé ou insaturé (4 à 6 chaînons), phényle, phényle substitué tel que défini ci-avant pour la définition de R₁ ou benzyle], ou bien R''' représente un radical cyanométhyle, ou -CH₂CORe pour lequel soit Re est -OR'e, R'e étant hydrogène, alcoyle (1 à 6 carbones), alcényle (2 à 6 carbones), benzyle ou hétérocyclylméthyle dont la partie hétérocyclyle contient 5 à 6 chaînons et 1 ou 2 hétéroatomes choisis parmi le soufre, l'oxygène ou l'azote soit Re est un radical alcoylamino, alcoyl méthyl amino, hétérocyclylamino ou hétérocyclyl méthyl amino dont la partie hétérocyclyle est saturée et contient 5 à 6 chaînons et 1 ou 2 hétéroatomes choisis parmi le soufre, l'oxygène ou l'azote éventuellement substitué par un radical alcoyle, benzyle ou alcoyloxycarbonyle,
3) Rb est un atome d'hydrogène, Rd est un radical -NHCH₃ ou -N(CH₃)₂ et Rc est un atome de chlore ou de brome, ou représente un radical alcényle (3 à 5C), [si Rd est -N(CH₃)₂],
4) Rb et Rd sont des atomes d'hydrogène et Rc est un atome d'halogène, ou un radical alcoylamino ou dialcoylamino, alcoyloxy, trifluorométhoxy, thioalcoyle, alcoyle (1 à 6C) ou trihalogénométhyle,
5) Rb et Rc sont des atomes d'hydrogène et Rd est un atome d'halogène, ou un radical éthylamino, diéthylamino ou méthyl éthyl amino, alcoyloxy ou trifluorométhoxy, alcoylthio, alcoylsulfinyle, alcoylsulfonyle, alcoyle (1 à 6C), phényle ou trihalogénométhyle,
6) Rb est un atome d'hydrogène et Rc est un atome d'halogène ou un radical alcoylamino ou dialcoylamino, alcoyloxy ou trifluorométhoxy, thioalcoyle, alcoyle (1 à 3C), et Rd est un atome d'halogène ou un radical amino, alcoylamino ou dialcoylamino, alcoyloxy ou trifluorométhoxy, thioalcoyle, alcoyle (1 à 6C) ou trihalogénométhyle,
7) Rc est un atome d'hydrogène et Rb et Rd représentent un radical méthyle,
ainsi que leurs sels.

## Patentansprüche

1. Derivat der Gruppe A der Streptogramine der allgemeinen Formel (I) 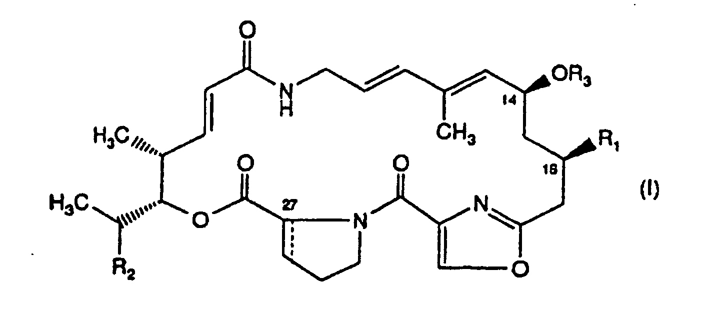 in der
- R₁ ein Halogenatom oder einen Rest Azido oder Thiocyanato darstellt,
- R₂ ein Wasserstoffatom oder einen Rest Methyl oder Ethyl darstellt,
- R₃ ein Wasserstoffatom oder den Rest eines aliphatischen, cycloaliphatischen, aromatischen, araliphatischen, heterocyclischen oder heterocyclylaliphatischen Esters darstellt, der gegebenenfalls substituiert sein kann, und
- die Bindung ----- eine einfache Bindung (Stereochemie 27R) oder eine Doppelbindung bedeutet,
sowie seine Salze, wenn sie existieren.

2. Derivat der Gruppe A der Streptogramine nach Anspruch 1, **dadurch gekennzeichnet, daß** der Rest R₃ vom aliphatischen, cycloaliphatischen, aromatischen, araliphatischen, heterocyclischen oder heterocyclylaliphatischen Ester, der substituiert sein kann, ein Rest R'₃-CO- ist, worin R'₃ Phenyl oder Phenylalkyl, unsubstituiert oder substituiert am Rest Phenyl [durch einen oder mehrere Reste, ausgewählt unter Alkyl, das gegebenenfalls einen Rest NR'R" trägt, dessen Reste R' und R", gleich oder verschieden, Wasserstoffatome oder Reste Alkyl sein können, die zusammen mit dem Stickstoffatom, an das sie gebunden sind, einen gesättigten oder ungesättigten heterocyclischen Rest mit 3 bis 8 Ringgliedern bilden können, der gegebenenfalls ein weiteres Heteroatom umfaßt, ausgewählt unter Sauerstoff, Schwefel oder Stickstoff, wobei der genannte Heterocyclus selbst substituiert sein kann durch einen oder mehrere Reste (Alkyl, Hydroxyalkyl, Alkyloxyalkyl, Alkyloxycarbonylalkyl, Aryl, Heterocyclyl, Heterocyclylalkyl, gesättigt oder ungesättigt und mit 3 bis 8 Ringgliedern, oder -CH₂-CO-NR'R"), oder R' und/oder R" einen Rest Hydroxyalkyl, Phenyl, Heterocyclylalkyl, gesättigt oder ungesättigt und mit 3 bis 8 Ringgliedern, -CO-NR'R", worin NR'R" wie vorstehend definiert sind, oder Alkyl oder Acyl bedeuten können, substituiert durch NR'R" wie vorstehend definiert] darstellt, oder R'₃ auch ausgewählt werden kann unter den Resten Phenyl oder Phenylalkyl, substituiert am Rest Phenyl durch einen oder mehrere Reste [ausgewählt unter Alkyl, das substituiert sein kann durch einen Rest Alkyloxy oder Alkylthio, der gegebenenfalls selbst einen Rest Carboxy oder einen Rest NR'R" trägt, wie vorstehend definiert, oder ausgewählt unter Acyloxy, das substituiert sein kann durch NR'R", wie vorstehend definiert], oder R'₃ auch ausgewählt werden kann unter den Resten Alkyl oder Cycloalkyl, gegebenenfalls substituiert [durch einen Rest Carboxy, Carboxyalkyldisulfanyl oder durch einen Rest NR'R", -CH₂-NR'R", -CO-NR'R", oder durch einen Rest Alkyloxycarbonyl, Alkyloxy oder Alkyldisulfanyl, gegebenenfalls substituiert durch NR'R" oder -CO-NR'R", worin NR'R" wie vorstehend definiert ist], oder R'₃ auch ausgewählt werden kann unter den Resten Heterocyclyl, gesättigt oder ungesättigt und mit 3 bis 8 Ringgliedern, gegebenenfalls substituiert [durch Alkyl oder Acyl, die gegebenenfalls selbst substituiert sind durch NR'R"].

3. Derivat der Gruppe A der Streptogramine nach einem der Ansprüche 1 oder 2, **dadurch gekennzeichnet, daß**
- R₁ ein Atom von Fluor, Chlor, Brom oder Iod oder einen Rest Azido oder Thiocyanato darstellt,
- R₂ einen Rest Methyl darstellt,
- R₃ ein Wasserstoffatom darstellt oder ein Rest R'₃-CO- ist, worin R'₃ Phenyl oder Phenylalkyl, unsubstituiert oder substituiert am Rest Phenyl [durch einen oder mehrere Reste, ausgewählt unter Alkyl, das gegebenenfalls einen Rest NR'R" trägt, dessen Reste R' und R", gleich oder verschieden, Wasserstoffatome oder Reste Alkyl sein können, die zusammen mit dem Stickstoffatom, an das sie gebunden sind, einen gesättigten oder ungesättigten heterocyclischen Rest mit 3 bis 8 Ringgliedern bilden können, der gegebenenfalls ein weiteres Heteroatom umfaßt, ausgewählt unter Sauerstoff, Schwefel oder Stickstoff, wobei der genannte Heterocyclus selbst substituiert sein kann durch einen oder mehrere Reste (Alkyl, Hydroxyalkyl, Alkyloxyalkyl, Alkyloxycarbonylalkyl, Aryl, Heterocyclyl, Heterocyclylalkyl, gesättigt oder ungesättigt und mit 3 bis 8 Ringgliedern, oder -CH₂-CO-NR'R"), oder R' und/oder R" einen Rest Hydroxyalkyl, Phenyl, Heterocyclylalkyl, gesättigt oder ungesättigt und mit 3 bis 8 Ringgliedern, -CO-NR'R", worin NR'R" wie vorstehend definiert sind, oder Alkyl oder Acyl bedeuten können, substituiert durch NR'R" wie vorstehend definiert] darstellt, oder R'₃ auch ein Rest Phenyl sein kann, substituiert durch einen oder mehrere Reste [ausgewählt unter Alkyl, das substituiert sein kann durch einen Rest Alkyloxy oder Alkylthio, der gegebenenfalls selbst einen Rest Carboxy oder einen Rest NR'R" trägt, wie vorstehend definiert, oder ausgewählt unter Acyloxy, das substituiert sein kann durch NR'R", wie vorstehend definiert], oder R'₃ auch ausgewählt werden kann unter den Resten Alkyl oder Cycloalkyl, gegebenenfalls substituiert [durch einen Rest Carboxy, Carboxyalkyldisulfanyl oder durch einen Rest NR'R", -CH₂-NR'R", -CO-NR'R", oder durch einen Rest Alkyloxycarbonyl, Alkyloxy oder Alkyldisulfanyl, gegebenenfalls substituiert durch NR'R" oder -CO-NR'R", worin NR'R" wie vorstehend definiert ist], oder R'₃ auch ausgewählt werden kann unter den Resten Heterocyclyl, gesättigt oder ungesättigt und mit 3 bis 8 Ringgliedern, gegebenenfalls substituiert [durch Alkyl oder Acyl, die gegebenenfalls selbst substituiert sind durch NR'R"], mit der Maßgabe, daß die Heterocyclen ausgewählt werden unter Pyrrolidinyl, Imidazolyl, Pyridyl, Piperidinyl, Piperazinyl oder Morpholinyl, und
- die Bindung ----- eine einfache Bindung (Stereochemie 27R) oder eine Doppelbindung bedeutet,
sowie seine Salze, wenn sie existieren.

4. Derivat der Gruppe A der Streptogramine nach einem der Ansprüche 1 bis 3, **dadurch gekennzeichnet, daß** es sich um (16R)-16-Desoxo-16-fluor-pristinamycin II_{B} handelt.

5. Derivat der Gruppe A der Streptogramine nach einem der Ansprüche 1 bis 3, **dadurch gekennzeichnet, daß** es sich um (16R)-16-Desoxo-16-thiocyanato-pristinamycin II_{B} handelt.

6. Derivat der Gruppe A der Streptogramine nach einem der Ansprüche 1 bis 3, **dadurch gekennzeichnet, daß** es sich um (16R)-16-Desoxo-16-chlor-pristinamycin II_{B} handelt.

7. Derivat der Gruppe A der Streptogramine nach einem der Ansprüche 1 bis 3, **dadurch gekennzeichnet, daß** es sich um (16R)-16-Desoxo-16-azido-pristinamycin II_{B} handelt.

8. Derivat der Gruppe A der Streptogramine nach einem der Ansprüche 1 bis 3, **dadurch gekennzeichnet, daß** es sich um (16R)-16-Desoxo-16-fluor-pristinamycin II_{A} handelt.

9. Verfahren zur Herstellung eines Derivates der Gruppe A der Streptogramine nach Anspruch 1, **dadurch gekennzeichnet, daß** man ein Derivat von Streptogramin der allgemeinen Formel (II) 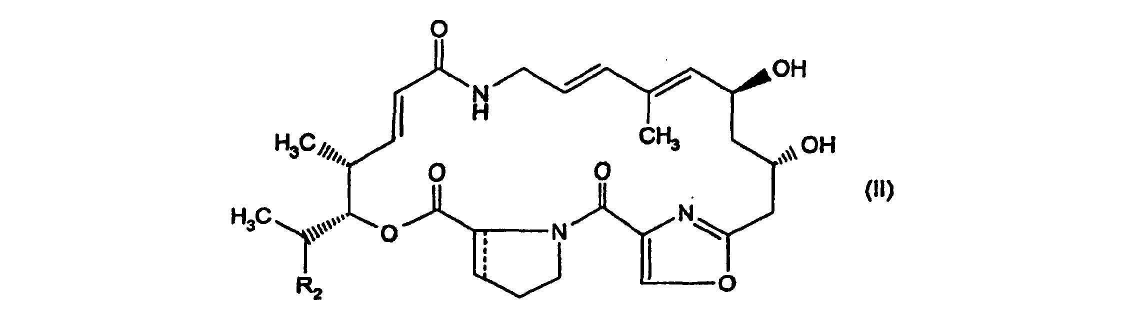 in der R₂ wie vorstehend definiert ist, die Bindung ----- eine einfache Bindung (Stereochemie 27R) oder eine Doppelbindung darstellt, und dessen Hydroxyfunktion in Position 14 zuvor geschützt wurde, halogeniert, in ein Azid umwandelt oder in ein Thiocyanat umwandelt, anschließend die Schutzgruppe wieder entfernt und gegebenenfalls, um ein Derivat von Streptogramin der Gruppe A nach Anspruch 1 oder 2 zu erhalten, worin R₃ anders ist als das Wasserstoffatom, den Rest vom aliphatischen, cycloaliphatischen, aromatischen, araliphatischen, heterocyclischen oder heterocyclylaliphatischen Ester einführt, der substituiert sein kann (R₃), und gegebenenfalls in ein Salz überführt, wenn sie existieren.

10. Verfahren nach Anspruch 9, **dadurch gekennzeichnet, daß** man in Anwesenheit eines Aminoschwefel-trifluorids oder in Anwesenheit von Schwefel-tetrafluorid mit Hilfe eines Reaktanden wie einem Halogenid, einem Azid oder einem Thiocyanat von Tetra-alkylammonium, Tri-alkyl-benzylammonium oder Tri-alkyl-phenylammonium, oder auch einem Halogenid, einem Azid oder einem Thiocyanat von Alkalimetall, gegebenenfalls unter Zusatz eines Kronenethers, arbeitet.

11. Verfahren nach Anspruch 9, **dadurch gekennzeichnet, daß** man bei der Fluorierung mit Hilfe der Einwirkung eines Fluorierungsmittels arbeitet, ausgewählt unter Schwefelfluorid, Hexafluorpropyl-diethylamin oder N-(2-Chlor-1,1,2-trifluorethyl)-diethylamin.

12. Verfahren nach Anspruch 9, 10 oder 11, **dadurch gekennzeichnet, daß** gegebenenfalls die Veresterung mittels Reaktion der Säure oder eines reaktiven Derivates der Säure, in Anwesenheit oder nicht in Anwesenheit eines Kupplungsmittels wie einem Carbodiimid und einem tertiären Amin und gegebenenfalls eines Katalysators wie 4-N-Dimethylaminopyridin durchgeführt wird.

13. Verfahren nach einem der Ansprüche 9, 10 oder 12, **dadurch gekennzeichnet, daß** das Aminoschwefel-trifluorid unter Diethylaminoschwefel-trifluorid, Bis-(2-Methoxyethyl)-aminoschwefel-trifluorid oder Morpholinoschwefel-trifluorid ausgewählt werden kann.

14. Pharmazeutische Zusammensetzung, umfassend ein Derivat der Gruppe A der Streptogramine nach Anspruch 1 oder 2, in reinem Zustand oder in Form einer Assoziation mit mindestens einem Derivat der Gruppe B der Streptogramine, gegebenenfalls in Form von Salz, und/oder in Form einer Assoziation mit einem oder mehreren kompatiblen und pharmazeutisch akzeptablen Verdünnungsmitteln oder Zusatzstoffen.

15. Pharmazeutische Zusammensetzung nach Anspruch 14, **dadurch gekennzeichnet, daß** das Derivat der Gruppe B der Streptogramine unter den folgenden natürlichen Bestandteilen ausgewählt wird: Pristinamycin IA, Pristinamycin IB, Pristinamycin IC, Pristinamycin ID, Pristinamycin IE, Pristinamycin IF, Pristinamycin IG, Virginiamycin S1, S3 oder S4, Vernamycin B oder C, Etamycin, oder unter den Derivaten der Hemisynthese der allgemeinen Formel (A) 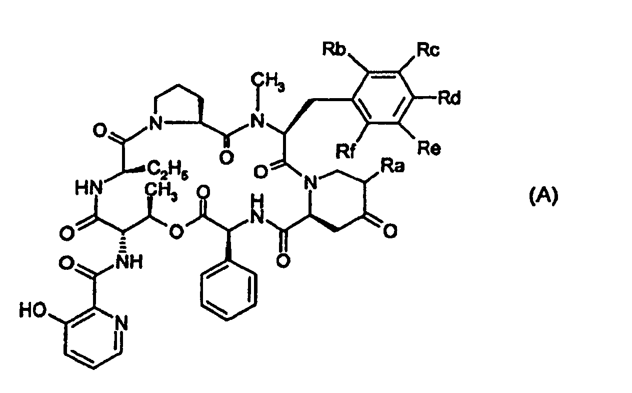 in der
1) Rb, Rc, Re und Rf Wasserstoffatome sind, Rd ein Wasserstoffatom oder ein Rest Dimethylamino ist, und Ra einen Rest der Struktur -CH₂R'a darstellt, worin R'a 3-Pyrrolidinyl-thio, 3-(oder 4-)Piperidyl-thio, die substituiert sein können durch Alkyl, Alkylthio, substituiert durch 1 oder 2 Hydroxysulfonyl, Alkylamino, Dialkylamino (das selbst gegebenenfalls substituiert ist durch Mercapto oder Dialkylamino), oder substituiert durch 1 oder 2 Ringe Piperazin, gegebenenfalls substituiert, Morpholino, Thiomorpholino, Piperidino, 1-Pyrrolidinyl, 2-, 3- oder 4-Piperidyl oder 2- oder 3-Pyrrolidinyl (die durch Alkyl substituiert sein können) bedeutet, oder Ra auch einen Rest der Struktur =CHR'a darstellt, worin R'a 3-Pyrrolidinyl-amino, 3-(oder 4-)-Piperidyl-amino, 3-Pyrrolidinyl-oxy, 3-(oder 4-)Piperidyl-oxy, 3-Pyrrolidinyl-thio, 3-(oder 4-)Piperidyl-thio, die substituiert sein können durch Alkyl, bedeutet, oder R'a Alkylamino, Alkyloxy oder Alkylthio, substituiert durch 1 oder 2 Hydroxysulfonyl, Alkylamino, Dialkylamino (das selbst gegebenenfalls substituiert ist durch Dialkylamino), oder durch Trialkylammonio, 4- oder 5-Imidazolyl, oder durch 1 oder 2 Ringe Piperazin, gegebenenfalls substituiert, Morpholino, Thiomorpholino, Piperidino, 1-Pyrrolidinyl, 2-, 3- oder 4-Piperidyl oder 2- oder 3-Pyrrolidinyl (die durch Alkyl substituiert sein können) bedeutet, oder
Ra einen Rest 3-(oder 4-)Chinuclidinyl-thiomethyl darstellt, oder auch
2) Ra ein Wasserstoffatom ist, und
a) entweder Rb, Re und Rf Wasserstoffatome sind, Rd einen Rest -NHCH₃ oder -N(CH₃)₂ darstellt und Rc ein Atom von Chlor oder Brom ist oder einen Rest Alkenyl mit 3 bis 5 Kohlenstoffatomen bedeutet [wenn Rd -N(CH₃)₂ ist],
b) oder Rb, Rd, Re und Rf ein Wasserstoffatom darstellen und Rc ein Halogen oder einen Rest Aminomonoalkyl, Aminodialkyl, Alkyloxy, Trifluormethyloxy, Thioalkyl, Alkyl mit 1 bis 3 Kohlenstoffatomen oder Trihalogenmethyl bedeutet,
c) oder Rb, Rc, Re und Rf ein Wasserstoffatom darstellen und Rd ein Halogen oder einen Rest Ethylamino, Diethylamino oder Methylethylamino, Alkyloxy oder Trifluormethyloxy, Thioalkyl, Alkyl mit 1 bis 6 Kohlenstoffatomen, Aryl oder Trihalogenmethyl bedeutet,
d) oder Rb, Re und Rf ein Wasserstoffatom darstellen und Rc ein Halogen oder einen Rest Aminomonoalkyl oder Aminodialkyl, Alkyloxy oder Trifluormethyloxy, Thioalkyl, Alkyl mit 1 bis 3 Kohlenstoffatomen bedeutet und Rd ein Halogen oder einen Rest Amino, Aminomonoalkyl oder Aminodialkyl, Alkyloxy oder Trifluormethyloxy, Thioalkyl, Alkyl mit 1 bis 6 Kohlenstoffatomen oder Trihalogenmethyl darstellt,
e) oder Rc, Re und Rf ein Wasserstoffatom darstellen und Rb und Rd einen Rest Methyl bedeuten;
oder auch unter den Derivaten der Hemisynthese der allgemeinen Formel (B) 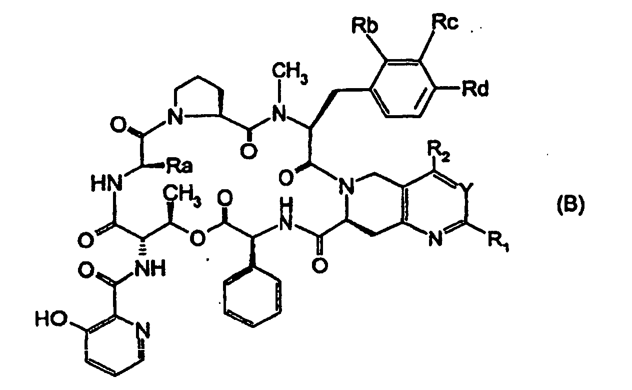 in der
Y ein Stickstoffatom oder einen Rest =CR₃- darstellt,
R₁ ein Wasserstoffatom, einen Rest Alkyl (1 bis 8 Kohlenstoffatome), Alkenyl (2 bis 8 Kohlenstoffatome), Cycloalkyl (3 bis 8 Kohlenstoffatome), Heterocyclyl, gesättigt oder ungesättigt (3 bis 8 Ringglieder), Phenyl, Phenyl substituiert [durch ein oder mehrere Halogenatome oder Reste Hydroxy, Alkyl, Alkyloxy, Alkylthio, Alkylsulfinyl, Alkylsulfonyl, Amino, Alkylamino oder Dialkylamino] oder einen Rest NR'R" darstellt, worin R' und R", gleich oder verschieden, Wasserstoffatome sein können oder Reste Alkyl (1 bis 3 Kohlenstoffatome), oder auch zusammen mit dem Stickstoffatom, an das sie gebunden sind, einen Heterocyclus mit 3 bis 8 Ringgliedern bilden können, der gegebenenfalls ein weiteres Heteroatom enthält, ausgewählt unter Sauerstoff, Schwefel oder Stickstoff, gegebenenfalls substituiert [durch einen Rest Alkyl, Alkenyl (2 bis 8 Kohlenstoffatome), Cycloalkyl (3 bis 6 Kohlenstoffatome), Heterocyclyl, gesättigt oder ungesättigt (4 bis 6 Ringglieder), Benzyl, Phenyl oder Phenyl substituiert wie oben bei der Definition von R₁ angegeben], oder
wenn Y einen Rest =CR₃- darstellt, kann R₁ auch Halogenmethyl, Hydroxymethyl, Alkyloxymethyl, Alkylthiomethyl, dessen Teil Alkyl gegebenenfalls substituiert ist durch NR'R", Alkylsulfinylmethyl, Alkylsulfonylmethyl, Acyloxymethyl, Benzoyloxymethyl, Cyclopropylaminomethyl oder -(CH₂)ₙNR'R" (n ist eine ganze Zahl von 1 bis 4 und R' und R" sind wie oben definiert) sein, oder wenn R₃ ein Wasserstoffatom ist, kann R₁ auch Formyl, Carboxy, Alkyloxycarbonyl oder -CONR'R" sein, worin R' und R" wie oben definiert sind, oder wenn Y ein Stickstoffatom ist, kann R₁ auch ein Rest -XR°, worin X ein Atom von Sauerstoff oder Schwefel ist, ein Rest Sulfinyl oder Sulfonyl oder ein Rest NH sein und R° ist ein Rest Alkyl (1 bis 8 Kohlenstoffatome), Cycloalkyl (3 bis 6 Kohlenstoffatome), Heterocyclyl, gesättigt oder ungesättigt (3 bis 8 Ringglieder), Heterocyclylmethyl (3 bis 8 Ringglieder), dessen Teil Heterocyclyl an den Rest Methyl durch ein Kohlenstoffatom gebunden ist, Phenyl, Phenyl substituiert [durch ein oder mehrere Halogenatome oder Reste Hydroxy, Alkyl, Alkyloxy, Alkylthio, Alkylsulfinyl, Alkylsulfonyl, Amino, Alkylamino oder Dialkylamino] oder ein Rest -(CH₂)ₙNR'R", worin R' und R" wie oben definiert sind und n eine ganze Zahl von 2 bis 4 ist,
oder wenn X NH darstellt, kann R° auch ein Wasserstoffatom sein,
R₂ ist ein Wasserstoffatom oder ein Rest Alkyl (1 bis 3 Kohlenstoffatome),
R₃ ist ein Wasserstoffatom oder ein Rest Alkyl, Carboxy, Alkyloxycarbonyl, oder Carbamoyl der Struktur -CO-NR'R", worin R' und R" wie vorstehend definiert sind,
Ra ist ein Rest Methyl oder Ethyl und
Rb, Rc und Rd besitzen die nachfolgenden Definitionen:
1) Rb und Rc sind Wasserstoffatome und Rd ist ein Wasserstoffatom oder ein Rest Methylamino oder Dimethylamino,
2) Rb ist ein Wasserstoffatom, Rc ist ein Wasserstoffatom, Chloratom oder Bromatom oder stellt einen Rest Alkenyl (3 bis 5 Kohlenstof fatome) dar und Rd ist ein Rest -NMe-R'", worin R'" einen Rest Alkyl, Hydroxyalkyl (2 bis 4 Kohlenstoffatome) oder Alkenyl (2 bis 8 Kohlenstoffatome), gegebenenfalls substituiert durch Phenyl, Cycloalkyl(3 bis 6 Kohlenstoffatome)-methyl, Benzyl, Benzyl substituiert [durch ein oder mehrere Halogenatome oder Reste Hydroxy, Alkyl, Alkyloxy, Alkylthio, Alkylsulfinyl, Alkylsulfonyl, Amino, Alkylamino oder Dialkylamino], Heterocyclylmethyl oder Heterocyclylethyl, deren Teil Heterocyclyl gesättigt oder ungesättigt ist und 5 bis 6 Ringglieder und 1 oder 2 Heteroatome enthält, ausgewählt unter Schwefel, Sauerstoff oder Stickstoff, gegebenenfalls substituiert [durch einen Rest Alkyl, Alkenyl (2 bis 8 Kohlenstoffatome), Cycloalkyl (3 bis 6 Kohlenstoffatome), Heterocyclyl, gesättigt oder ungesättigt (4 bis 6 Ringglieder), Phenyl, Phenyl substituiert wie oben bei der Definition von R₁ oder Benzyl angegeben] bedeutet, oder R'" einen Rest Cyanomethyl oder -CH₂CORe darstellt, worin Re entweder -OR'e ist und R'e Wasserstoff, Alkyl (1 bis 6 Kohlenstoffatome), Alkenyl (2 bis 6 Kohlenstoffatome), Benzyl oder Heterocyclylmethyl darstellt, dessen Teil Heterocyclyl 5 bis 6 Ringglieder und 1 oder 2 Heteroatome enthält, ausgewählt unter Schwefel, Sauerstoff oder Stickstoff, oder Re einen Rest Alkylamino, Alkyl-methylamino, Heterocycylamino oder Heterocycyl-methylamino bedeutet, dessen Teil Heterocyclyl gesättigt ist und 5 bis 6 Ringglieder und 1 oder 2 Heteroatome enthält, ausgewählt unter Schwefel, Sauerstoff oder Stickstoff, gegebenenfalls substituiert durch einen Rest Alkyl, Benzyl oder Alkyloxycarbonyl,
3) Rb ist ein Wasserstoffatom, Rd ist ein Rest -NHCH₃ oder - N(CH₃)₂ und Rc ist ein Atom von Chlor oder Brom oder stellt einen Rest Alkenyl (3 bis 5 Kohlenstoffatome) dar, [wenn Rd -N(CH₃)₂ ist] ,
4) Rb und Rd sind Wasserstoffatome und Rc ist ein Halogenatom oder ein Rest Alkylamino oder Dialkylamino, Alkyloxy, Trifluormethoxy, Thioalkyl, Alkyl (1 bis 6 Kohlenstoffatome) oder Trihalogenmethyl,
5) Rb und Rc sind Wasserstoffatome und Rd ist ein Halogenatom oder ein Rest Ethylamino, Diethylamino oder Methylethylamino, Alkyloxy oder Trifluormethoxy, Alkylthio, Alkylsulfinyl, Alkylsulfonyl, Alkyl (1 bis 6 Kohlenstoffatome), Phenyl oder Trihalogenmethyl,
6) Rb ist ein Wasserstoffatom und Rc ist ein Halogenatom oder ein Rest Alkylamino oder Dialkylamino, Alkyloxy oder Trifluormethoxy, Thioalkyl, Alkyl (1 bis 3 Kohlenstoffatome), und Rd ist ein Halogenatom oder ein Rest Amino, Alkylamino oder Dialkylamino, Alkyloxy oder Trifluormethoxy, Thioalkyl, Alkyl (1 bis 6 Kohlenstoffatome) oder Trihalogenmethyl,
7) Rc ist ein Wasserstoffatom und Rb und Rd bedeuten einen Rest
Methyl,
sowie ihre Salze.

## Claims

1. Group A streptogramin derivative of the formula: 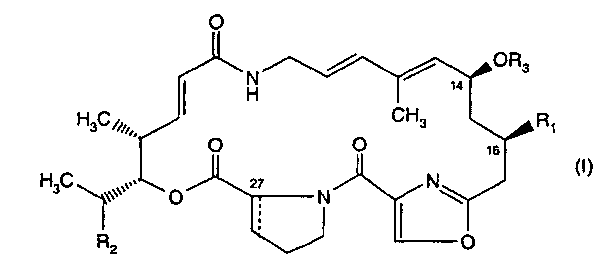 in which
― R₁ represents a halogen atom or an azido or thiocyanato radical,
― R₂ represents a hydrogen atom or a methyl or ethyl radical,
― R₃ represents a hydrogen atom, or the residue of an aliphatic, cycloaliphatic, aromatic, araliphatic, heterocyclic or heterocyclylaliphatic ester which may be substituted, and
― the bond --- represents a single bond (stereochemistry 27R) or a double bond,
as well as its salts when they exist.

2. Group A streptogramin derivative according to Claim 1, **characterized in that** the residue R₃ of an aliphatic, cycloaliphatic, aromatic, araliphatic, heterocyclic or heterocyclylaliphatic ester which may be substituted is a radical R'₃-CO- for which R'₃ is phenyl or phenylalkyl which are unsubstituted or which are substituted on the phenyl radical [with one or more radicals chosen from alkyl, optionally carrying a radical NR'R" in which the radicals R' and R", which are identical or different, may be hydrogen atoms or alkyl radicals which can form together with the nitrogen atom to which they are attached a 3- to 8-membered saturated or unsaturated heterocyclyl radical optionally comprising another heteroatom chosen from oxygen, sulphur or nitrogen, it being possible for the said heterocycle itself to be substituted with one or more radicals (alkyl, hydroxyalkyl, alkyloxyalkyl, alkyloxycarbonylalkyl, aryl, heterocyclyl, heterocyclylalkyl, which are saturated or unsaturated and have 3 to 8 members, or -CH₂-CO-NR'R"), or alternatively R' and/or R" may be a hydroxyalkyl radical, a phenyl radical, a 3- to 8-membered saturated or unsaturated heterocyclylalkyl radical, a radical -CO-NR'R" for which NR'R" is as defined above, or alkyl or acyl radicals which are substituted with NR'R" which is as defined above], or alternatively R'₃ may be chosen from phenyl or phenylalkyl radicals which are substituted on the phenyl radical with one or more radicals [chosen from alkyl, which may be substituted with an alkyloxy or alkylthio radical optionally carrying themselves a carboxyl radical or a radical NR'R" as defined above, or chosen from acyloxy which may be substituted with NR'R" as defined above], or alternatively R'₃ may be chosen from alkyl or cycloalkyl radicals which are optionally substituted [with a carboxyl radical, a carboxyalkyldisulphanyl radical or with a radical NR'R", -CH₂-NR'R", or -CO-NR'R", or with an alkyloxycarbonyl, alkyloxy or alkyldisulphanyl radical which are optionally substituted with NR'R" or -CO-NR'R" for which NR'R" is as defined above], or alternatively R'₃ may be chosen from 3- to 8-membered saturated or unsaturated heterocyclyl radicals which are optionally substituted [with alkyl or acyl which are themselves optionally substituted with NR'R"].

3. Group A streptogramin derivative according to either of Claims 1 and 2, **characterized in that**:
- R₁ represents a fluorine, chlorine, bromine or iodine atom or an azido or thiocyanato radical,
- R₂ represents a methyl radical,
- R₃ represents a hydrogen atom or a radical R'₃-CO- for which R'₃ is phenyl or phenylalkyl which are unsubstituted or substituted on the phenyl radical [with one or more radicals chosen from alkyl, optionally carrying a radical NR'R" in which the radicals R' and R", which are identical or different, may be hydrogen atoms or alkyl radicals which can form together with the nitrogen atom to which they are attached a 3- to 8-membered saturated or unsaturated heterocyclyl radical optionally comprising another heteroatom chosen from oxygen, sulphur or nitrogen, it being possible for the said heterocycle itself to be substituted with one or more radicals (alkyl, hydroxyalkyl, alkyloxyalkyl, alkyloxycarbonylalkyl, aryl, 3- to 8-membered saturated or unsaturated heterocyclyl or heterocyclylalkyl, or -CH₂-CO-NR'R"), or alternatively R' and/or R" may be a radical hydroxyalkyl, phenyl, 3- to 8-membered saturated or unsaturated heterocyclylalkyl, -CO-NR'R" for which NR'R" is as defined above, or alkyl or acyl which are substituted with NR'R" as defined above], or alternatively R'₃ may be a phenyl radical substituted with one or more radicals [chosen from alkyl, which may be substituted with an alkyloxy or alkylthio radical themselves optionally carrying a carboxyl radical or a radical NR'R" as defined above, or chosen from acyloxy which may be substituted with NR'R" as defined above], or alternatively R'₃ may be chosen from alkyl or cycloalkyl radicals which are optionally substituted [with a carboxyl or carboxyalkyldisulphanyl radical or with a radical NR'R", -CH₂-NR'R" or -CO-NR'R", or with an alkyloxycarbonyl, alkyloxy or alkyldisulphanyl radical which are optionally substituted with NR'R" or -CO-NR'R" for which NR'R" is as defined above], or alternatively R'₃ may be chosen from 3- to 8-membered saturated or unsaturated heterocyclyl radicals which are optionally substituted [with alkyl or acyl which are themselves optionally substituted with NR'R"], it being understood that the heterocycles are chosen from pyrrolidinyl, imidazolyl, pyridyl, piperidinyl, piperazinyl or morpholinyl, and
- the bond --- represents a single bond (stereochemistry 27R) or a double bond, as well as their salts when they exist.

4. Group A streptogramin derivative according to one of Claims 1 to 3, **characterized in that** it is (16*R*)-16-deoxo-16-fluoropristinamycin II_{B}.

5. Group A streptogramin derivative according to one of Claims 1 to 3, **characterized in that** it is (16*R*)-16-deoxo-16-thiocyanatopristinamycin II_{B}.

6. Group A streptogramin derivative according to one of Claims 1 to 3, **characterized in that** it is (16*R*)-16-deoxo-16-chloropristinamycin II_{B}.

7. Group A streptogramin derivative according to one of Claims 1 to 3, **characterized in that** it is (16*R*)-16-azido-16-deoxopristinamycin II_{B}.

8. Group A streptogramin derivative according to one of Claims 1 to 3, **characterized in that** it is (16*R*)-16-deoxo-16-fluoropristinamycin II_{A}.

9. Process for preparing a group A streptogramin derivative according to Claim 1, **characterized in that** there is halogenated, **in that** there is converted to an azide or **in that** there is converted to a thiocyanate, a streptogramin derivative of general formula: 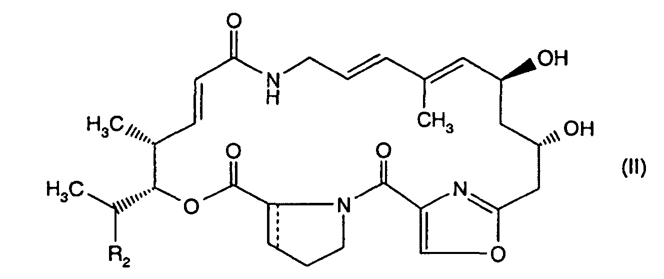 in which R₂ is as defined above, the bond --- represents a single bond (stereochemistry 27R) or a double bond, and in which the hydroxyl function at the 14-position has been previously protected, followed by the removal of the protecting radical and where appropriate, in order to obtain a group A streptogramin derivative according to Claim 1 or 2, for which R₃ is other than a hydrogen atom, introduction of the aliphatic, cycloaliphatic, aromatic, araliphatic, heterocyclic or heterocyclylaliphatic ester residue which may be substituted (R₃), and optionally conversion to a salt when they exist.

10. Process according to Claim 9, **characterized in that** the procedure is carried out in the presence of an aminosulphur trifluoride or in the presence of sulphur tetrafluoride, by means of a reagent such as a tetraalkylammonium, trialkylbenzylammonium or trialkylphenylammonium halide, azide or thiocyanate or by means of an alkali metal halide, azide or thiocyanate optionally supplemented with a crown ether.

11. Process according to Claim 9, **characterized in that** the procedure is carried out, for the fluorination, by the action of a fluorinating agent chosen from a sulphur fluoride, hexafluoropropyldiethylamine or N-(2-chloro-1,1,2-trifluoroethyl)diethylamine.

12. Process according to one of Claims 9, 10 or 11, **characterized in that**, where appropriate, the esterification is carried out by the reaction of the acid or of a reactive derivative of the acid, in the presence or otherwise of a coupling agent such as a carbodiimide, and of a tertiary amine and optionally a catalyst such as 4-N-dimethylaminopyridine.

13. Process according to one of Claims 9, 10 or 12, **characterized in that** the aminosulphur trifluoride may be chosen from diethylaminosulphur trifluoride, bis-(2-methoxyethyl)aminosulphur trifluoride or morpholinosulphur trifluoride.

14. Pharmaceutical composition comprising a group A streptogramin derivative according to Claim 1 or 2, in the pure state or in the form of a combination with at least one group B streptogramin derivative, where appropriate in the form of a salt, and/or in the form of a combination with one or more compatible and pharmaceutically acceptable diluents or adjuvants.

15. Pharmaceutical composition according to Claim 14, **characterized in that** the group B streptogramin derivative is chosen from the natural components: pristinamycin I_{A}, pristinamycin I_{B}, pristinamycin I_{C}, pristinamycin I_{D}, pristinamycin I_{E}, pristinamycin I_{F}, pristinamycin I_{G}, virginiamycin S₁, S₃ or S₄, vernamycin B or C, etamycin or from the semisynthetic derivatives of general formula: 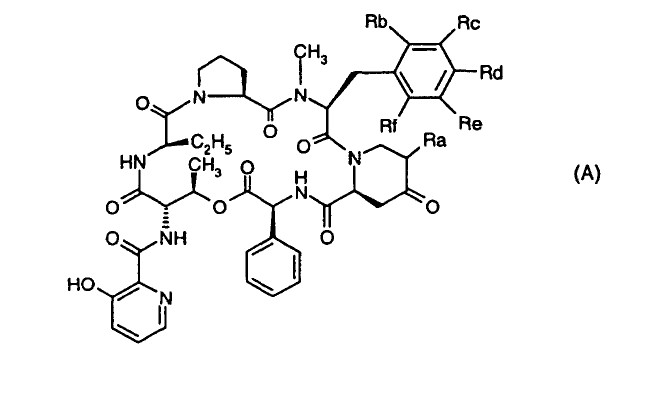 in which,
1) Rb, Rc, Re and Rf are hydrogen atoms, Rd is a hydrogen atom or a dimethylamino radical, and Ra is a radical of structure -CH₂R'a for which R'a is 3-pyrrolidinylthio or 3- or 4-piperidylthio which may be substituted with alkyl, or alkylthio substituted with 1 or 2 hydroxysulphonyl, alkylamino, dialkylamino (itself optionally substituted with mercapto or dialkylamino), or substituted with 1 or 2 optionally substituted piperazine rings, morpholino, thiomorpholino, piperidino, 1-pyrrolidinyl, 2-, 3- or 4-piperidyl or 2- or 3-pyrrolidinyl (which may be substituted with alkyl), or alternatively Ra is a radical of structure =CHR'a for which R'a is 3-pyrrolidinylamino, 3- or 4-piperidylamino, 3-pyrrolidinyloxy, 3- or 4-piperidyloxy, 3-pyrrolidinylthio, 3- or 4-piperidylthio which may be substituted with alkyl, or R'a is alkylamino, alkyloxy or alkylthio substituted with 1 or 2 hydroxysulphonyl, alkylamino, dialkylamino (itself optionally substituted with dialkylamino), or with trialkylammonio, 4- or 5-imidazolyl, or with 1 or 2 optionally substituted piperazine rings, morpholino, thiomorpholino, piperidino, 1-pyrrolidinyl, 2-, 3- or 4-piperidyl or 2- or 3-pyrrolidinyl (which may be substituted with alkyl), or
Ra is a 3- or 4-quinuclidinylthiomethyl radical, or alternatively
2) Ra is a hydrogen atom and
a) either Rb, Re and Rf are hydrogen atoms, Rd is a radical -NHCH₃ or -N(CH₃)₂ and Rc is a chlorine or bromine atom, or represents an alkenyl radical containing 3 to 5 carbon atoms [if Rd is -N(CH₃)₂],
b) or Rb, Rd, Re and Rf represent a hydrogen atom and Rc is a halogen, or an aminomonoalkyl, aminodialkyl, alkyloxy, trifluoromethyloxy, thioalkyl, C₁ to C₃ alkyl or trihalomethyl radical,
c) or Rb, Rc, Re and Rf represent a hydrogen atom and Rd is a halogen, or an ethyl amino, diethylamino or methylethylamino, alkyloxy or trifluoromethyloxy, thioalkyl, C₁ to C₆ alkyl, aryl or trihalomethyl radical,
d) or Rb, Re and Rf represent a hydrogen atom and Rc is halogen or an aminomonoalkyl or aminodialkyl, alkyloxy or trifluoromethyloxy, thioalkyl or C₁ to C₃ alkyl radical, and Rd is halogen or an amino, aminomonoalkyl or aminodialkyl, alkyloxy or trifluoromethyloxy, thioalkyl, C₁ to C₆ alkyl or trihalomethyl radical,
e) or Rc, Re and Rf represent a hydrogen atom and Rb and Rd represent a methyl radical;
or alternatively from the semisynthetic derivatives of general formula: 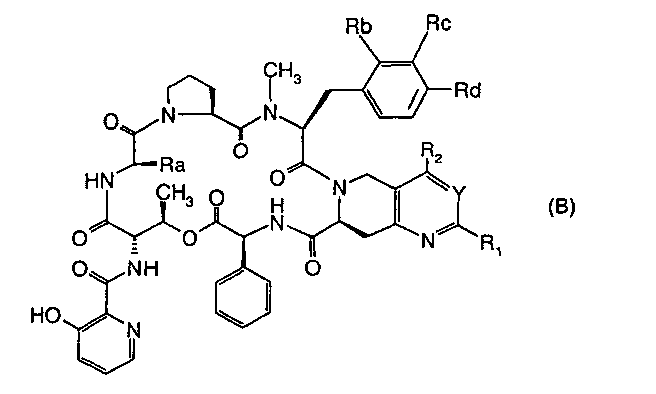 in which
Y is a nitrogen atom or a radical =CR₃-,
R₁ is a hydrogen atom, an alkyl radical (1 to 8 carbons), an alkenyl radical (2 to 8 carbons), a cycloalkyl radical (3 to 8 carbons), a saturated or unsaturated heterocyclyl radical (3 to 8 members), a phenyl radical, a phenyl radical which is substituted [with one or more halogen atoms or hydroxyl, alkyl, alkyloxy, alkylthio, alkylsulphinyl, alkylsulphonyl, amino, alkylamino or dialkylamino radicals] or a radical NR'R", it being possible for R' and R", which are identical or different, to be hydrogen atoms or alkyl radicals (1 to 3 carbons) or to form together with the nitrogen atom to which they are attached a 3- to 8-membered heterocycle optionally containing another heteroatom chosen from oxygen, sulphur or nitrogen, optionally substituted [with an alkyl radical, an alkenyl radical (2 to 8 carbons), a cycloalkyl radical for which R''' represents a radical alkyl, hydroxyalkyl (2 to 4C), or alkenyl (2 to 8C) which is optionally substituted with phenyl, cycloalkyl(3 to 6C)methyl, benzyl, benzyl which is substituted [with one or more halogen atoms or hydroxyl, alkyl, alkyloxy, alkylthio, alkylsulphinyl, alkylsulphonyl, amino, alkylamino or dialkylamino radicals], heterocyclylmethyl or heterocyclylethyl in which the heterocyclyl portion is saturated or unsaturated and contains 5 to 6 members and one or two heteroatoms chosen from sulphur, oxygen or nitrogen optionally substituted [with an alkyl radical, an alkenyl radical (2 to 8 carbons), a cycloalkyl radical (3 to 6 carbons), a saturated or unsaturated heterocyclyl radical (4 to 6 members), a phenyl radical, a phenyl radical which is substituted as defined above for the definition of R₁ or a benzyl radical], or alternatively R''' represents a cyanomethyl radical, or -CH₂CORe for which either Re is -OR'e, R'e being hydrogen, alkyl (1 to 6 carbons), alkenyl (2 to 6 carbons), benzyl or heterocyclylmethyl in which the heterocyclyl portion contains 5 to 6 members and 1 or 2 heteroatoms chosen from sulphur, oxygen or nitrogen or Re is an alkylamino radical, an alkylmethylamino radical, a heterocyclylamino radical or a heterocyclylmethylamino radical in which the heterocyclyl portion is saturated and contains 5 to 6 members and one or two heteroatoms chosen from sulphur, oxygen or nitrogen optionally substituted with an alkyl, benzyl or . alkyloxycarbonyl radical,
3) Rb is a hydrogen atom, Rd is a radical -NHCH₃ or -N(CH₃)₂ and Rc is a chlorine or bromine atom, or represents an alkenyl radical (3 to 5C), [if Rd is -N(CH₃)₂],
4) Rb and Rd are hydrogen atoms and Rc is a halogen atom, or an alkylamino or dialkylamino, alkyloxy, trifluoromethoxy, thioalkyl, alkyl (1 to 6C) or trihalomethyl radical,
5) Rb and Rc are hydrogen atoms and Rd is a halogen atom, or an ethylamino, diethylamino or methylethylamino, alkyloxy or trifluoromethoxy, alkylthio, alkylsulphinyl, alkylsulphonyl, alkyl (1 to 6C), phenyl or trihalomethyl radical,
6) Rb is a hydrogen atom and Rc is a halogen atom or an alkylamino or dialkylamino, alkyloxy or trifluoromethoxy, thioalkyl or alkyl (1 to 3C) radical and Rd is a halogen atom or an amino, alkylamino or dialkylamino, alkyloxy or trifluoromethoxy, thioalkyl, alkyl (1 to 6C) or trihalomethyl radical,
7) Rc is a hydrogen atom and Rb and Rd represent a methyl radical, as well as their salts.
